# EUROPEAN PATENT APPLICATION

(11) **EP 3 026 043 A1**
(43) Date of publication of application: **01.06.2016**
(21) Application number: 14829665.0
(22) Date of filing: 25.07.2014
(51) Int. Cl.: C07D 209/34, A61K 31/404, A61K 31/416, A61K 31/4184, A61K 31/422, A61K 31/423, A61K 31/4245, A61K 31/428, A61K 31/4355, A61K 31/437, A61K 31/4375, A61K 31/4439, A61K 31/4709, A61K 31/4725, A61K 31/497, A61K 31/498, A61K 31/517, A61K 31/519, A61K 31/53, A61P 25/04

(54) **GLYCINE TRANSPORTER INHIBITOR**

(30) Priority: 26.07.2013 JP 2013155268
(71) Applicant: Taisho Pharmaceutical Co., Ltd., Tokyo 170-8633 (JP)
(72) Inventor: WAKASUGI, Daisuke, Tokyo 170-8633 (JP); OHTA, Hiroshi, Tokyo 170-8633 (JP); OKADA, Kumiko, Tokyo 170-8633 (JP); SHIROKAWA, Shin-ichi, Tokyo 170-8633 (JP); MORIYA, Minoru, Tokyo 170-8633 (JP); TAMITA, Tomoko, Tokyo 170-8633 (JP); ABE, Kumi, Tokyo 170-8633 (JP); HATTORI, Nobutaka, Tokyo 170-8633 (JP); ARAKI, Yuko, Tokyo 170-8633 (JP)
(74) Representative: Hoffmann Eitle
(86) International application number: PCT/JP2014/069747
(87) International publication number: WO 2015/012400

(57) **Abstract**

The present invention provides novel compounds of formula [I] or pharmaceutically acceptable salts thereof: 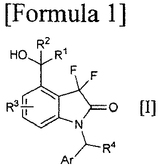 which are useful in the prevention or treatment of diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, or sleep disorders, which is based on the glycine uptake-inhibiting action.

## Description

### TECHNICAL FIELD

The present invention relates to compounds having a glycine transporter-inhibiting action.

### BACKGROUND ART

The NMDA receptor, which is one of glutamate receptors, is located on the nerve cell membranes in the brain and involved in various neurophysiologic events such as neuronal plasticity, cognition, attention, and memory. The NMDA receptor has a plurality of allosteric binding sites, one of which is the glycine binding site (glycine binding site on NMDA receptor complex). It has been reported that the glycine binding site on NMDA receptor complex is involved in the activation of NMDA receptors (Non-Patent Document 1).

Action potential arriving at the presynaptic terminals of glycinergic nerves triggers the release of glycine into synaptic clefts. The released glycine binds to the postsynaptic receptors or the like and is then removed from the synaptic clefts by transporters. Based on this fact, glycine transporters are believed to regulate the functions of NMDA receptors through regulation of the amount of glycine in the extracellular fluid.

Glycine transporters (GlyTs) are proteins involved in the reuptake of extracellular glycine into cells, and two subtypes, GlyT1 and GlyT2, have so far been identified. GlyT1, which is expressed primarily in the cerebral cortex, hippocampus, thalamus and the like, has been reported to be associated with diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, and sleep disorders (Non-Patent Documents 2-4).

Compounds having a GlyT1-inhibiting action have been reported in the documents shown below (Patent Documents 1, 2).

### CITATION LIST

### PATENT DOCUMENTS

Patent Document 1: WO2011012622
Patent Document 2: WO2011023753

### NON-PATENT DOCUMENTS

Non-Patent Document 1: Molecular Psychiatry (2004) 9, 984-997
Non-Patent Document 2: Current Medicinal Chemistry, 2006, 13, 1017-1044
Non-Patent Document 3: Neuropsychopharmacology (2005), 30, 1963-1985
Non-Patent Document 4: Expert Opinion on Therapeutic Patents (2004) 14 (2) 201-214

### SUMMARY OF INVENTION

### TECHNICAL PROBLEM

The present invention aims to provide novel compounds or pharmaceutically acceptable salts thereof which are useful in the prevention or treatment of diseases such as schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, or sleep disorders, which is based on the glycine uptake-inhibiting action.

### SOLUTION TO PROBLEM

As a result of extensive and intensive studies on structurally novel compounds with an inhibitory action against GlyT1, the present inventors found that the compounds having a hydroxy group which are represented by the following formula, are superior GlyT1-inhibiting substances. This finding has led to the completion of the present invention.

The present invention will be described below in detail. Embodiments of the present invention (hereinafter each referred to as "the inventive compound") are as shown below.
(1) A compound of formula [I] or a pharmaceutically acceptable salt thereof: 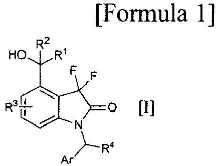 wherein
   Ar represents a phenyl group optionally substituted with one to three substituents selected from substituent group 1, a bicyclic heterocyclyl group optionally substituted with one to three substituents selected from substituent group 1, or a monocyclic heteroaryl group optionally substituted with one to three substituents selected from substituent group 1,
      substituent group 1 is the group consisting of a halogen atom, a C₁₋₆ alkyl group, a C₁₋₆ haloalkyl group, a C₁₋₆ alkoxy group, a cyano group, a triazolyl group, a C₁₋₆ haloalkoxy group, and a C₃₋₆ cycloalkyl group,
   R¹ and R² are the same or different and are each a hydrogen atom, a C₁₋₆ alkyl group, or a C₁₋₆ haloalkyl group, or together with the carbon atom to which they are attached, optionally form a cyclopropane ring, a cyclobutane ring, or an oxetane ring,
   R³ represents a hydrogen atom or a halogen atom, and
   R⁴ represents a hydrogen atom or a C₁₋₆ alkyl group.
(2) A compound represented by formula [I] or a pharmaceutically acceptable salt thereof: 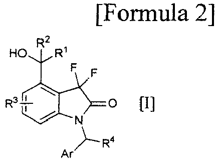 wherein
   Ar represents a phenyl group optionally substituted with one to three substituents selected from substituent group 1, a bicyclic heterocyclyl group optionally substituted with one to three substituents selected from substituent group 1, or a monocyclic heteroaryl group optionally substituted with one to three substituents selected from substituent group 1,
      substituent group 1 is the group consisting of a halogen atom, a C₁₋₆ alkyl group, a C₁₋₆ haloalkyl group, a C₁₋₆ alkoxy group, a cyano group, and a triazolyl group,
   R¹ and R² are the same or different and are each a hydrogen atom, a C₁₋₆ alkyl group, or a C₁₋₆ haloalkyl group, or together with the carbon atom to which they are attached, optionally form a cyclopropane ring, a cyclobutane ring, or an oxetane ring,
   R³ represents a hydrogen atom or a halogen atom, and
   R⁴ represents a hydrogen atom or a C₁₋₆ alkyl group.
(3) The compound according to claim 1 or 2 or a pharmaceutically acceptable salt thereof, wherein Ar is a pyridyl group optionally substituted with one to three substituents selected from substituent group 1.
(4) The compound according to claim 1 or 2 or a pharmaceutically acceptable salt thereof, wherein Ar is a pyridyl group substituted with one to three substituents selected from the group consisting of a halogen atom, a cyano group, a methyl group substituted with one to three halogen atoms, and a methoxy group substituted with one to three halogen atoms.
(5) The compound according to any one of claims 1 to 4 or a pharmaceutically acceptable salt thereof, wherein R⁴ is a hydrogen atom.
(6) The compound according to any one of claims 1 to 5 or a pharmaceutically acceptable salt thereof, wherein R¹ is a C₁₋₆ alkyl group, or a C₁₋₆ haloalkyl group, and R² is a hydrogen atom.
(7) The compound according to claim 1 or a pharmaceutically acceptable salt thereof selected from the group consisting of
   1-[(6-bromo-5-fluoropyridin-2-yl)methyl]-3,3-difluoro-4-[(1S)-1-hydroxyethyl]-1,3-dihydro-2H-indol-2-one,
   1-[(5-bromo-6-fluoropyridin-3-yl)methyl]-3,3-difluoro-4-[(1S)-1-hydroxyethyl]-1,3-dihydro-2H-indol-2-one,
   1-(3-chlorobenzyl)-3,3-difluoro-4-[(1S)-1-hydroxyethyl]-1,3-dihydro-2H-indol-2-one,
   1-[(6-chloro-5-fluoropyridin-2-yl)methyl]-3,3-difluoro-4-[(1S)-1-hydroxyethyl]-1,3-dihydro-2H-indol-2-one,
   6-({3,3-difluoro-4-[(1S)-1-hydroxyethyl]-2-oxo-2,3-dihydro-1H-indol-1-yl}methyl)-3-fluoropyridine-2-carbonitrile,
   1-[(6-bromo-5-fluoropyridin-2-yl)methyl]-4-(2,2-difluoro-1-hydroxyethyl)-3,3-difluoro-1,3-dihydro-2H-indol-2-one,
   3-chloro-6-{[4-(2,2-difluoro-1-hydroxyethyl)-3,3-difluoro-2-oxo-2,3-dihydro-1H-indol-1-yl]methyl}pyridine-2-carbonitrile,
   1-[(6-chloropyridin-2-yl)(2H2)methyl]-3,3-difluoro-4-[(1S)-1-hydroxyethyl]-1,3-dihydro-2H-indol-2-one,
   3,3-difluoro-4-[(1S)-1-hydroxyethyl]-1-{[5-(trifluoromethyl)furan-2-yl]methyl}-1,3-dihydro-2H-indol-2-one,
   1-{[6-chloro-5-(trifluoromethyl)pyridin-2-yl]methyl-3,3-difluoro-4-[(1S)-1-hydroxyethyl]-1,3-dihydro-2H-indol-2-one,
   1-[(6-chloropyridin-2-yl)methyl]-3,3-difluoro-4-(1-hydroxyethyl)-1,3-dihydro-2H-indol-2-one,
   3,3-difluoro-4-[(1S)-1-hydroxyethyl]-1-[3-(trifluoromethyl)benzyl]-1,3-dihydro-2H-indol-2-one,
   1-[(5-chloropyridin-3-yl)methyl]-3,3,5-trifluoro-4-(1-hydroxyethyl)-1,3-dihydro-2H-indol-2-one,
   3-chloro-6-({3,3-difluoro-4-[(1S)-1-hydroxyethyl]-2-oxo-2,3-dihydro-1H-indol-1-yl} methyl)pyridine-2-carbonitrile,
   1-[(6-chloropyridin-2-yl)methyl]-4-(2,2-difluoro-1-hydroxyethyl)-3,3-difluoro-1,3-dihydro-2H-indol-2-one,
   1-{[5-chloro-4-(trifluoromethyl)pyridin-2-yl]methyl}-3,3-difluoro-4-[(1S)-1-hydroxyethyl]-1,3-dihydro-2H-indol-2-one,
   6-{[4-(2,2-difluoro-1-hydroxyethyl)-3,3-difluoro-2-oxo-2,3-dihydro-1H-indol-1-yl]methyl}-3-(trifluoromethyl)pyridine-2-carbonitrile,
   1-[(5-chloro-6-methoxypyridin-3-yl)methyl]-3,3-difluoro-4-[(1S)-1-hydroxyethyl]-1,3-dihydro-2H-indol-2-one,
   1-[(5,6-dichloropyridin-3-yl)methyl]-3,3-difluoro-4-[(1S)-1-hydroxyethyl]-1,3-dihydro-2H-indol-2-one,
   1-(2,1,3-benzoxadiazol-5-ylmethyl)-3,3-difluoro-4-[(1S)-1-hydroxyethyl]-1,3-dihydro-2H-indol-2-one,
   3,3-difluoro-1-(3-fluorobenzyl)-4-[(1S)-1-hydroxyethyl]-1,3-dihydro-2H-indol-2-one,
   6-{[4-(2,2-difluoro-1-hydroxyethyl)-3,3-difluoro-2-oxo-2,3-dihydro-1H-indol-1-yl]methyl}-3-fluoropyridine-2-carbonitrile,
   6-({3,3-difluoro-4-[(1S)-1-hydroxyethyl]-2-oxo-2,3-dihydro-1H-indol-1-yl}methyl)-3-(trifluoromethyl)pyridine-2-carbonitrile,
   4-(2,2-difluoro-1-hydroxyethyl)-3,3-difluoro-1-[(6-methoxypyridin-2-yl)methyl]-1,3-dihydro-2H-indol-2-one,
   1-[(5,6-dichloropyridin-2-yl)methyl]-3,3-difluoro-4-[(1S)-1-hydroxyethyl]-1,3-dihydro-2H-indol-2-one,
   1-[(4-chloro-5-fluoropyridin-2-yl)methyl]-3,3-difluoro-4-[(1S)-1-hydroxyethyl]-1,3-dihydro-2H-indol-2-one,
   4-(2,2-difluoro-1-hydroxyethyl)-1-{[2-(difluoromethoxy)pyridin-4-yl]methyl}-3,3-difluoro-1,3-dihydro-2H-indol-2-one,
   1-[(6-chloro-5-fluoropyridin-2-yl)methyl]-4-(2,2-difluoro-1-hydroxyethyl)-3,3-difluoro-1,3-dihydro-2H-indol-2-one,
   1-[(6-chloropyrazin-2-yl)methyl]-3,3-difluoro-4-[(1S)-1-hydroxyethyl]-1,3-dihydro-2H-indol-2-one,
   3,3-difluoro-4-[(1S)-1-hydroxyethyl]-1-[(2-methoxypyridin-4-yl)methyl]-1,3-dihydro-2H-indol-2-one,
   1-[(2-chloropyridin-4-yl)methyl]-4-(2,2-difluoro-1-hydroxyethyl)-3,3-difluoro-1,3-dihydro-2H-indol-2-one,
   4-({3,3,7-trifluoro-4-[(1S)-1-hydroxyethyl]-2-oxo-2,3-dihydro-1H-indol-1-yl}methyl)pyridine-2-carbonitrile,
   1-[(5-chloropyridin-3-yl)methyl]-3,3-difluoro-4-(1-hydroxyethyl)-1,3-dihydro-2H-indol-2-one,
   1-[(6-chloropyridin-2-yl)methyl]-3,3-difluoro-4-(1-hydroxyethyl)-1,3-dihydro-2H-indol-2-one,
   3-({3,3-difluoro-4-[(1S)-1-hydroxyethyl]-2-oxo-2,3-dihydro-1H-indol-1-yl}methyl)benzonitrile,
   4-(2,2-difluoro-1-hydroxyethyl)-3,3-difluoro-1-(3-fluorobenzyl)-1,3-dihydro-2H-indol-2-one,
   1-{[5-chloro-4-(trifluoromethyl)pyridin-2-yl]methyl}-4-(2,2-difluoro-1-hydroxyethyl)-3,3-difluoro-1,3-dihydro-2H-indol-2-one,
   1-[(6-chloropyridin-2-yl)methyl]-3,3-difluoro-4-(2,2,2-trifluoro-1-hydroxyethyl)-1,3-dihydro-2H-indol-2-one,
   1-[(6-chloropyridin-2-yl)methyl]-3,3-difluoro-4-(2-fluoro-1-hydroxyethyl)-1,3-dihydro-2H-indol-2-one,
   3,3-difluoro-4-[(1S)-1-hydroxyethyl]-1-{[6-(trifluoromethyl)pyridin-2-yl]methyl}-1,3-dihydro-2H-indol-2-one,
   2-({3,3-difluoro-4-[(1S)-1-hydroxyethyl]-2-oxo-2,3-dihydro-1H-indol-1-yl}methyl)-7-fluoro-3-methylquinazolin-4(3H)-one,
   1-[(2-chloropyridin-4-yl)methyl]-3,3-difluoro-4-(1-hydroxyethyl)-1,3-dihydro-2H-indol-2-one,
   3,3-difluoro-4-[(1S)-1-hydroxyethyl]-1-(3-methoxybenzyl)-1,3-dihydro-2H-indol-2-one,
   1-[(4-chloropyridin-2-yl)methyl]-3,3-difluoro-4-[(1S)-1-hydroxyethyl]-1,3-dihydro-2H-indol-2-one,
   4-({3,3-difluoro-4-[(1S)-1-hydroxyethyl]-2-oxo-2,3-dihydro-1H-indol-1-yl}methyl)-3-fluoropyridine-2-carbonitrile,
   1-benzyl-3,3-difluoro-4-[(1S)-1-hydroxyethyl]-1,3-dihydro-2H-indol-2-one,
   2-chloro-5-({3,3-difluoro-4-[(1S)-1-hydroxyethyl]-2-oxo-2,3-dihydro-1H-indol-1-yl}methyl)pyridine-3-carbonitrile,
   1-benzyl-4-(2,2-difluoro-1-hydroxyethyl)-3,3-difluoro-1,3-dihydro-2H-indol-2-one,
   1-[(4-chloro-5-fluoropyridin-2-yl)methyl]-4-(2,2-difluoro-1-hydroxyethyl)-3,3-difluoro-1,3-dihydro-2H-indol-2-one,
   1-[(4-bromopyridin-2-yl)methyl]-3,3-difluoro-4-[(1S)-1-hydroxyethyl]-1,3-dihydro-2H-indol-2-one,
   5-({3,3-difluoro-4-[(1S)-1-hydroxyethyl]-2-oxo-2,3-dihydro-1H-indol-1-yl}methyl)-2-fluoropyridine-3-carbonitrile,
   1-[(2-chloropyridin-4-yl)methyl]-3,3-difluoro-4-(1-hydroxyethyl)-1,3-dihydro-2H-indol-2-one,
   1-[(2-cyclopropylpyridin-4-yl)methyl]-3,3-difluoro-4-[(1S)-1-hydroxyethyl]-1,3-dihydro-2H-indol-2-one,
   6-({3,3-difluoro-4-[(1S)-1-hydroxyethyl]-2-oxo-2,3-dihydro-1H-indol-1-yl}methyl)pyridine-2-carbonitrile,
   1-[(6-chloropyrazin-2-yl)methyl]-4-(2,2-difluoro-1-hydroxyethyl)-3,3-difluoro-1,3-dihydro-2H-indol-2-one,
   3,3-difluoro-4-[(1S)-1-hydroxyethyl]-1-{[2-(trifluoromethyl)pyridin-4-yl]methyl}-1,3-dihydro-2H-indol-2-one,
   3,3-difluoro-1-[(2-methoxypyridin-4-yl)methyl]-4-[(1R)-2,2,2-trifluoro-1-hydroxyethyl]-1,3-dihydro-2H-indol-2-one,
   1-[(6-chloropyridin-2-yl)methyl]-4-(2,2-difluoro-1-hydroxyethyl)-3,3-difluoro-1,3-dihydro-2H-indol-2-one,
   3,3-difluoro-4-[(1S)-1-hydroxyethyl]-1-{[6-(trifluoromethyl)pyridin-3-yl]methyl}-1,3-dihydro-2H-indol-2-one,
   5-{[4-(2,2-difluoro-1-hydroxyethyl)-3,3-difluoro-2-oxo-2,3-dihydro-1H-indol-1-yl]methyl}-2-fluoropyridine-3-carbonitrile,
   3,3-difluoro-4-[(1S)-1-hydroxyethyl]-1-(thiophen-3-ylmethyl)-1,3-dihydro-2H-indol-2-one,
   2-({3,3-difluoro-4-[(1S)-1-hydroxyethyl]-2-oxo-2,3-dihydro-1H-indol-1-yl}methyl)-3-methylquinazolin-4(3H)-one,
   3-({3,3-difluoro-4-[(1S)-1-hydroxyethyl]-2-oxo-2,3-dihydro-1H-indol-1-yl}methyl)-2-methylisoquinolin-1 (2H)-one,
   3,3-difluoro-4-[(1S)-1-hydroxyethyl]-1-{[5-(trifluoromethyl)pyridin-2-yl]methyl}-1,3-dihydro-2H-indol-2-one,
   4-[(2,2-difluoro-1-hydroxyethyl]-3,3-difluoro-1-{[2-(trifluoromethyl)pyridin-4-yl]methyl}-1,3-dihydro-2H-indol-2-one,
   1-(1,3-benzoxazol-6-ylmethyl)-3,3-difluoro-4-[(1S)-1-hydroxyethyl]-1,3-dihydro-2H-indol-2-one,
   1-(1,3-benzoxazol-2-ylmethyl)-3,3-difluoro-4-[(1S)-1-hydroxyethyl]-1,3-dihydro-2H-indol-2-one,
   3,3-difluoro-4-[(S)-1-hydroxy ethyl]-1-(quinoxalin-2-ylmethyl)-1,3-dihydro-2H-indol-2-one, 3,3-difluoro-4-[(1S)-1-hydroxyethyl]-1-[(6-methoxypyridin-3-yl)methyl]-1,3-dihydro-2H-indol-2-one,
   3,3-difluoro-4-[(1S)-1-hydroxyethyl]-1-[(3-methylquinoxalin-2-yl)methyl]-1,3-dihydro-2H-indol-2-one,
   1-{[2-(difluoromethyl)pyridin-4-yl]methyl}-3,3-difluoro-4-[(1S)-1-hydroxyethyl]-1,3-dihydro-2H-indol-2-one,
   1-[(5-chloropyridin-3-yl)methyl]-3,3-difluoro-4-(1-hydroxyethyl)-1,3-dihydro-2H-indol-2-one,
   1-[(5-chloro-4-methoxypyridin-2-yl)methyl]-3,3-difluoro-4-[(1S)-1-hydroxyethyl]-1,3-dihydro-2H-indol-2-one,
   1-{[3-chloro-5-(trifluoromethyl)pyridin-2-yl]methyl}-3,3-difluoro-4-[(1S)-1-hydroxyethyl]-1,3-dihydro-2H-indol-2-one,
   3-({3,3-difluoro-2-oxo-4-[(1R)-2,2,2-trifluoro-1-hydroxyethyl]-2,3-dihydro-1H-indol-1-yl }methyl)quinoxalin-2(1H)-one,
   3,3-difluoro-4-[(1S)-1-hydroxyethyl]-1-[(1-methyl-1H-benzimidazol-2-yl)methyl]-1,3-dihydro-2H-indol-2-one,
   3-({3,3-difluoro-4-[(1S)-1-hydroxyethyl]-2-oxo-2,3-dihydro-1H-indol-1-yl}methyl)quinoxalin-2(1H)-one,
   6-chloro-4-({3,3-difluoro-4-[(1S)-1-hydroxyethyl]-2-oxo-2,3-dihydro-1H-indol-1-yl} methyl)pyridine-2-carbonitrile,
   3,3-difluoro-4-[(1S)-1-hydroxyethyl]-1-{[5-(2H-1,2,3-triazol-2-yl)pyridin-3-yl]methyl}-1,3-dihydro-2H-indol-2-one,
   1-(1,3-benzothiazol-2-ylmethyl)-3,3-difluoro-4-[(1S)-1-hydroxyethyl]-1,3-dihydro-2H-indol-2-one,
   3,3-difluoro-4-[(1S)-1-hydroxyethyl]-1-(quinolin-2-ylmethyl)-1,3-dihydro-2H-indol-2-one,
   1-[(5-chloropyridin-3-yl)methyl]-3,3-difluoro-4-(2,2,2-trifluoro-1-hydroxyethyl)-1,3-dihydro-2H-indol-2-one,
   1-[(2-chloropyridin-4-yl)methyl]-3,3-difluoro-4-[(1R)-2,2,2-trifluoro-1-hydroxyethyl]-1,3-dihydro-2H-indol-2-one,
   4-({3,3-difluoro-4-[(1S)-1-hydroxyethyl]-2-oxo-2,3-dihydro-1H-indol-1-yl }methyl)pyridine-2-carbonitrile,
   4-(2,2-difluoro-1-hydroxyethyl)-3,3-difluoro-1-{[5-(2H-1,2,3-triazol-2-yl)pyridin-3-yl]methyl}-1,3-dihydro-2H-indol-2-one,
   3,3-difluoro-4-[(1S)-1-hydroxyethyl]-1-(quinolin-3-ylmethyl)-1,3-dihydro-2H-indol-2-one,
   3,3-difluoro-4-[(1S)-1-hydroxyethyl]-1-{[5-(trifluoromethyl)pyridin-3-yl]methyl}-1,3-dihydro-2H-indol-2-one,
   1-[(3-bromo-5-fluoropyridin-2-yl)methyl]-3,3-difluoro-4-[(1S)-1-hydroxyethyl]-1,3-dihydro-2H-indol-2-one,
   1-[(6-chloropyrazin-2-yl)methyl]-3,3-difluoro-4-[(1R)-2,2,2-trifluoro-1-hydroxyethyl]-1,3-dihydro-2H-indol-2-one,
   2-({3,3-difluoro-4-[(1S)-1-hydroxyethyl]-2-oxo-2,3-dihydro-1H-indol-1-yl}methyl)pyridine-3-carbonitrile,
   3-({3,3-difluoro-4-[(1S)-1-hydroxyethyl]-2-oxo-2,3-dihydro-1H-indol-1-yl}methyl)-1-methylquinoxalin-2(1H)-one,
   6-{[4-(2,2-difluoro-1-hydroxyethyl)-3,3-difluoro-2-oxo-2,3-dihydro-1H-indol-1-yl]methyl}pyridine-2-carbonitrile,
   5-chloro-4-({3,3-difluoro-4-[(1S)-1-hydroxyethyl]-2-oxo-2,3-dihydro-1H-indol-1-yl}methyl)pyridine-2-carbonitrile,
   1-{[2-(difluoromethoxy)pyridin-4-yl]methyl-3,3-difluoro-4-[(1S)-1-hydroxyethyl]-1,3-dihydro-2H-indol-2-one,
   1-[(4-chloropyridin-2-yl)methyl]-3,3-difluoro-4-[(1R)-2,2,2-trifluoro-hydroxyethyl]-1,3-dihydro-2H-indol-2-one,
   2-({3,3-difluoro-2-oxo-4-[(1R)-2,2,2-trifluoro-1-hydroxyethyl]-2,3-dihydro-1H-indol-1-yl}methyl)-3-methylquinazolin-4(3H)-one,
   1-{[6-(difluoromethyl)pyridin-2-yl]methyl}-3,3-difluoro-4-[(1S)-1-hydroxyethyl]-1,3-dihydro-2H-indol-2-one,
   1-[(5-chloropyridin-2-yl)methyl]-3,3-difluoro-4-[(1S)-1-hydroxyethyl]-1,3-dihydro-2H-indol-2-one,
   2-{[4-(2,2-difluoro-1-hydroxyethyl)-3,3-difluoro-2-oxo-2,3-dihydro-1H-indol-1-yl]methyl}-7-fluoro-3-methylquinazolin-4(3H)-one,
   3,3-difluoro-4-[(1S)-1-hydroxyethyl]-1-{[2-(1H-1,2,4-triazol-1-yl)pyridin-4-yl]methyl}-1,3-dihydro-2H-indol-2-one,
   1-{[2-(difluoromethyl)pyridin-4-yl]methyl}-3,3-difluoro-4-[(1R)-2,2,2-trifluoro-1-hydroxyethyl]-1,3-dihydro-2H-indol-2-one,
   5-chloro-1-[(5-chloropyridin-3-yl)methyl]-3,3-difluoro-4-[(1S)-1-hydroxyethyl]-1,3-dihydro-2H-indol-2-one,
   1-[(5-chloropyridin-3-yl)methyl]-4-(2,2-difluoro-1-hydroxyethyl)-3,3,7-trifluoro-1,3-dihydro-2H-indol-2-one,
   1-[(5-chloropyridin-3-yl)methyl]-4-(2,2-difluoro-1-hydroxyethyl)-3,3,7-trifluoro-1,3-dihydro-2H-indol-2-one,
   4-(2,2-difluoro-1-hydroxyethyl)-3,3-difluoro-1-(quinolin-3-ylmethyl)-1,3-dihydro-2H-indol-2-one,
   4-(2,2-difluoro-1-hydroxyethyl)-3,3-difluoro-1-[(2-methoxypyridin-4-yl)methyl]-1,3-dihydro-2H-indol-2-one,
   3-({3,3-difluoro-4-[(1S)-1-hydroxyethyl]-2-oxo-2,3-dihydro-1H-indol-1-yl}methyl)isoquinolin-1 (2H)-one,
   3,3-difluoro-1-[(2-fluoropyridin-4-yl)methyl]-4-[(1S)-1-hydroxyethyl]-1,3-dihydro-2H-indol-2-one,
   1-[(6-bromopyridin-3-yl)methyl]-3,3-difluoro-4-[(1S)-1-hydroxyethyl]-1,3-dihydro-2H-indol-2-one,
   1-[(6-chloropyridin-2-yl)methyl]-3,3-difluoro-4-(2,2,2-trifluoro-1-hydroxyethyl)-1,3-dihydro-2H-indol-2-one,
   3,3-difluoro-4-[(1S)-1-hydroxyethyl]-1-{[4-(trifluoromethyl)pyridin-2-yl]methyl} -1,3-dihydro-2H-indol-2-one,
   3,3-difluoro-1-[(6-fluoropyridin-3-yl)methyl]-4-[(1S)-1-hydroxyethyl]-1,3-dihydro-2H-indol-2-one,
   4-(2,2-difluoro-1-hydroxyethyl)-3,3-difluoro-1-{[4-(trifluoromethyl)pyridin-2-yl]methyl}-1,3-dihydro-2H-indol-2-one,
   1-[(2-cyclopropylpyridin-4-yl)methyl]-4-(2,2-difluoro-1-hydroxyethyl)-3,3-difluoro-1,3-dihydro-2H-indol-2-one,
   2-{[3,3-difluoro-4-(1-hydroxyethyl)-2-oxo-2,3-dihydro-1H-indol-1-yl]methyl}-3-methylquinazolin-4(3H)-one,
   3,3-difluoro-4-[(1S)-1-hydroxyethyl]-1-[(2-methyl-2H-indazol-3-yl)methyl]-1,3-dihydro-2H-indol-2-one,
   3,3-difluoro-1-[(5-fluoro-6-methoxypyridin-2-yl)methyl]-4-[(1S)-1-hydroxyethyl]-1,3-dihydro-2H-indol-2-one,
   4-(2,2-difluoro-1-hydroxyethyl)-3,3-difluoro-1-{[5-(trifluoromethyl)pyridin-2-yl]methyl}-1,3-dihydro-2H-indol-2-one,
   3,3-difluoro-4-[(1S)-1-hydroxyethyl]-1-{[2-methoxy-6-(trifluoromethyl)pyridin-3-yl]methyl}-1,3-dihydro-2H-indol-2-one,
   1-[(5-chloropyridin-3-yl)methyl]-4-(2,2-difluoro-1-hydroxyethyl)-3,3-difluoro-1,3-dihydro-2H-indol-2-one,
   1-[(5-chloropyridin-3-yl)methyl]-4-(2,2-difluoro-1-hydroxyethyl)-3,3,5-trifluoro-1,3-dihydro-2H-indol-2-one,
   1-[(3,5-dichloropyridin-2-yl)methyl]-3,3-difluoro-4-[(1S)-1-hydroxyethyl]-1,3-dihydro-2H-indol-2-one,
   3-({3,3-difluoro-2-oxo-4-[(1R)-2,2,2-trifluoro-1-hydroxyethyl]-2,3-dihydro-1H-indol-1-yl}methyl)isoquinolin-1(2H)-one,
   1-[(5-chloro-6-methoxypyridin-2-yl)methyl]-3,3-difluoro-4-[(1S)-1-hydroxyethyl]-1,3-dihydro-2H-indol-2-one,
   1-[(3-chloropyridin-2-yl)methyl]-3,3-difluoro-4-[(1S)-1-hydroxyethyl]-1,3-dihydro-2H-indol-2-one,
   6-{[3,3-difluoro-2-oxo-4-(2,2,2-trifluoro-1-hydroxyethyl)-2,3-dihydro-1H-indol-1-yl]methyl}pyridine-2-carbonitrile,
   3,3-difluoro-1-[(6-fluoro-5-methoxypyridin-3-yl)methyl]-4-[(1S)-1-hydroxyethyl]-1,3-dihydro-2H-indol-2-one.
(8) A pharmaceutical composition comprising, as an active ingredient, the compound or pharmaceutically acceptable salt thereof according to any one of (1) to (7).
(9) An agent for preventing or treating diseases of schizophrenia, Alzheimer's disease, cognitive impairment, dementia, anxiety disorders, depression, drug dependence, spasm, tremor, pain, Parkinson's disease, attention deficit hyperactivity disorder, bipolar disorder, eating disorder, or sleep disorders, which comprises, as an active ingredient, the compound or pharmaceutically acceptable salt thereof according to any one of (1) to (7).

### ADVANTAGEOUS EFFECTS OF INVENTION

The inventive compounds have glycine transporter (GlyT1)-inhibiting activity.

### DESCRIPTION OF EMBODIMENTS

The term "C₁₋₆ alkyl group" as used herein refers to a straight-chain or branched-chain alkyl group having 1 to 6 carbon atoms, and includes, for example, a methyl group, an ethyl group, a propyl group, an isopropyl group, a butyl group, an isobutyl group, a tert-butyl group, a pentyl group, an isopentyl group, and a hexyl group.

The term "C₁₋₆ alkoxy group" as used herein refers to a straight-chain or branched-chain alkoxy group having 1 to 6 carbon atoms, and includes, for example, a methoxy group, an ethoxy group, a propoxy group, an isopropoxy group, a butoxy group, an isobutoxy group, a pentyloxy group, an isopentyloxy group, and a hexyloxy group.

The term "halogen atom (halo)" as used herein refers to a fluorine atom, a chlorine atom, a bromine atom, or an iodine atom.

The term "C₁₋₆ haloalkyl group" as used herein refers to a straight-chain or branched-chain alkyl group which has 1 to 6 carbon atoms and which has been substituted by a halogen atom or halogen atoms. The preferred number of the substituting halogen atom(s) is 1 to 3. Examples of the C₁₋₆ haloalkyl group include a fluoromethyl group, a difluoromethyl group, a trifluoromethyl group, and a trichloromethyl group.

The term "monocyclic heteroaryl group" as used herein refers to a monocyclic heteroaryl group having in the ring at least one atom selected from the group consisting of a nitrogen atom, an oxygen atom, and a sulfur atom. When the monocyclic heteroaryl group has a nitrogen atom or nitrogen atoms in the ring, the each nitrogen atom may be N-oxide.

The term "C₁₋₆ haloalkoxy group" as used herein refers to a straight-chain or branched-chain alkoxy group which has 1 to 6 carbon atoms and which has been substituted by a halogen atom or halogen atoms. The preferred number of the substituting halogen atom(s) is 1 to 3. Examples of the C₁₋₆ haloalkoxy group include a fluoromethoxy group, a difluoromethoxy group, a trifluoromethoxy group, and a trichloromethoxy group.

The term "C₃₋₆ cycloalkyl group" as used herein refers to a cycloalkyl group having 3 to 6 carbon atoms, and includes, for example, a cyclopropyl group, a cyclobutyl group, a cyclopentyl group, and a cyclohexyl group.

The monocyclic heteroaryl group is preferably a 5- or 6-membered heteroaryl group, and includes, for example, a pyridyl group, a pyridazinyl group, a pyrimidinyl group, a pyranyl group, a pyrazinyl group, a pyrazolyl group, a thiazolyl group, an imidazolyl group, an oxazolyl group, an isoxazolyl group, a thienyl group, a triazolyl group, an oxadiazolyl group, a thiadiazolyl group, and a furyl group.

The term "bicyclic heterocyclyl group" as used herein refers to a bicyclic heterocyclyl group having in the ring at least one atom selected from the group consisting of a nitrogen atom, an oxygen atom and a sulfur atom, and includes a group having aromaticity (sometimes referred to as a bicyclic heteroaryl group), a partially saturated group, and a completely saturated group. If the bicyclic heterocyclyl group has a nitrogen atom in the ring, the nitrogen atom may be N-oxide, and a partially saturated group as well as a completely saturated group may be substituted by an oxo group.

The bicyclic heterocyclyl group is preferably a 9- or 10-membered heterocyclyl group and may be exemplified by the structures shown below:

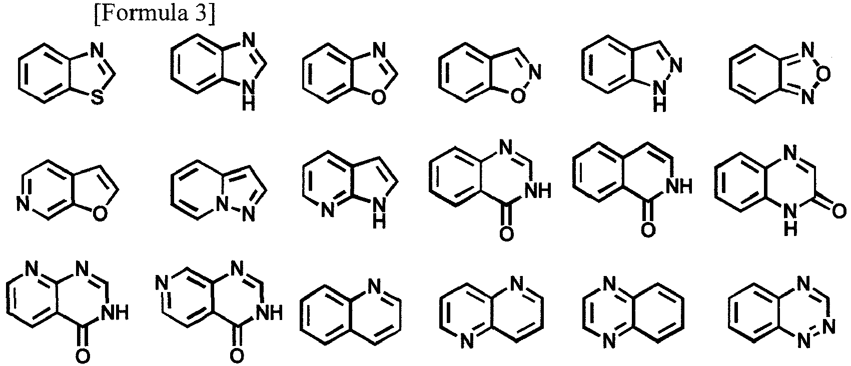

The term "pharmaceutically acceptable salt" as used herein refers to an acid addition salt that may be accepted in pharmaceutical terms. Examples of the acid that may be used include inorganic acids such as sulfuric acid, hydrochloric acid, hydrobromic acid, nitric acid and phosphoric acid, and organic acids such as acetic acid, oxalic acid, lactic acid, citric acid, malic acid, gluconic acid, tartaric acid, fumaric acid, maleic acid, methanesulfonic acid, ethanesulfonic acid, benzenesulfonic acid and p-toluenesulfonic acid. The free forms may be converted to these salts in a conventional manner.

Preferable embodiments of the compounds according to the present invention will be described below. Of the following preferable embodiments, compounds satisfying two or more conditions are more preferable.

Preferable are compounds wherein Ar is a pyridyl group which may be substituted with one to three substituents selected from substituent group 1; more preferable are compounds wherein Ar is a pyridyl group substituted with one to three substituents selected from the group consisting of a halogen atom, a cyano group, a methyl group substituted with one to three halogen atoms, and a methoxy group substituted with one to three halogen atoms.

Preferable are compounds wherein R¹ is a C₁₋₆ alkyl group or a C₁₋₆ haloalkyl group and R² is a hydrogen atom; more preferable are compounds wherein R¹ is a methyl group, a trifluoromethyl group, a difluoromethyl group or a fluoromethyl group, and R² is a hydrogen atom.

In this instsance, the hydroxyl group preferably assumes the following steric configuration:

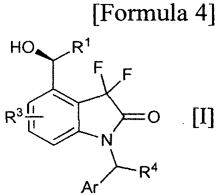

Preferable are compounds wherein R⁴ is a hydrogen atom.

The inventive compounds may contain a plurality of asymmetric centers. Thus, the inventive compounds may exist not only in optically active substances but also as racemic compounds thereof. Further, a plurality of diastereomers may also exist. All of these forms are included in the scope of the present invention. Individual isomers may be obtained by known methods such as, for example, use of optically active starting materials or intermediates, an optically selective reaction or a diastereoselective reaction in the preparation of intermediates or final products, or chromatographic separation in the preparation of intermediates or final products. If the inventive compounds form hydrates or solvates, such hydrates or solvates are also included in the scope of the present invention. Likewise, pharmaceutically acceptable salts of hydrates or solvates of the inventive compounds are also included in the scope of the present invention.

The inventive compounds also encompass compounds in which one or more hydrogen atoms, carbon atoms, nitrogen atoms, oxygen atoms or halogen atoms are replaced by their radioisotopes or stable isotopes. These labeled compounds are useful as in metabolism and/or pharmacokinetics study, or in biological analysis in which they are applied as receptor ligands, etc.

The compound according to the present invention may be administered orally or parenterally. The dosage forms are tablets, capsules, granules, powders, dusts, lozenges, ointments, creams, emulsions, suspensions, suppositories, injections and the like, all of which may be produced by conventional formulation techniques (for example, the methods set forth in the 15th revised Japanese Pharmacopoeia). These dosage forms may be selected as appropriate, according to the symptoms and age of patients and the purpose of treatment.

To produce these preparations, a composition containing the compound of the present invention may be blended with pharmacologically acceptable carriers, namely, excipients (e.g., crystalline cellulose, starch, lactose, mannitol), binders (e.g., hydroxypropylcellulose, polyvinylpyrrolidone), lubricants (e.g., magnesium stearate, talc), disintegrants (e.g., carboxymethylcellulose calcium), and/or various other pharmacologically acceptable additives.

The compounds of the present invention may be used in combination with one or more other therapeutic agents, namely, various antipsychotics, antidepressants, for example, 5HT3 antagonists, 5HT2 antagonists, serotonin agonists, NK-1 antagonists, selective serotonin reuptake inhibitors (SSRIs), serotonin noradrenaline reuptake inhibitors (SNRIs), tricyclic antidepressants, dopaminergic antidepressants, H3 antagonists, 5HT1A antagonists, 5HT1B antagonists, 5HT1D antagonists, D1 agonists, M1 agonists, anticonvulsants, cognitive enhancement drugs, and other psychoactive drugs.

Examples of other therapeutic agents that may be used in combination with the compounds of the present invention include ondansetron, granisetron, metoclopramide, sumatriptan, rauwolscine, yohimbine, fluoxetine, citalopram, escitalopram, femoxetine, fluvoxamine, paroxetine, indalpine, sertraline (registered trademark), zimeldine, venlafaxine, reboxetine, Milnacipran, duloxetine, imipramine, amitriptiline, chlomipramine, nortriptiline, bupropion, amineptine, divalproex, carbamazepine, diazepam, risperidone, olanzapine, ziprasidone, aripiprazole, quetiapine, perospirone, clozapine, haloperidol, pimozide, droperidol, chlorpromazine, thioridazine, mesoridazine, trifluoperazine, perphenazine, fluphenazine, thiflupromazine, prochlorperazine, acetophenazine, thiothixene, chlorprothixene, lamotrigine, loxapine, molindone, and the like. Such combinations may be administered simultaneously (in the same pharmaceutical formulation or in different pharmaceutical formulations), separately, or sequentially.

Particular advantages associated with the use of, and methods for treatment with, combinations of the compounds of the present invention may include comparable or improved effects as achieved by using individual ingredients at lower doses than their usual doses. Such use and treatment methods are also expected to further enhance the therapeutic effects on positive and/or negative symptoms of psychiatric disorders and/or cognitive impairment. The use of and methods for treatment with combinations of the compounds of the present invention may also provide benefits in the treatment of patients who do not sufficiently respond to, or who are resistant to, treatment with certain types of neuroleptics.

The compounds according to the present invention may be administered in doses which, in the case of treating adults, range from 1 to 2000 mg per day, either once daily or in divided portions. The dose may be increased or decreased as appropriate, depending on the age, body weight and symptom of a patient.

The compounds of formula [I] may be produced by various methods of synthesis. The methods described below are only illustrative of the process for producing the inventive compounds and should not be taken as limiting.

In the general production processes, the term "inert solvent" refers to, for example, an alcohol such as methanol, ethanol, isopropyl alcohol, n-butanol, or ethylene glycol; an ether such as diethyl ether, tert-butyl methyl ether, diisopropyl ether, tetrahydrofuran, 1,4-dioxane, or 1,2-dimethoxyethane; a hydrocarbon such as pentane, hexane, heptane, toluene, benzene, or xylene; an ester such as ethyl acetate or ethyl formate; a ketone such as acetone or methyl ethyl ketone; a halogenated carbon-based solvent such as chloroform or dichloromethane; an amide such as N,N-dimethylformamide or N-methylpyrrolidone; acetonitrile; dimethyl sulfoxide; water; or mixed solvents thereof. These solvents are selected as appropriate, according to various reaction conditions known to skilled artisans.

The term "base" refers to, for example, an alkali metal or alkaline earth metal hydride such as lithium hydride, sodium hydride, potassium hydride, or calcium hydride; an alkali metal or alkaline earth metal amide such as lithium amide, sodium amide, lithium diisopropylamide, lithium dicyclohexylamide, lithium hexamethyldisilazide, sodium hexamethyldisilazide, or potassium hexamethyldisilazide; an alkali metal or alkaline earth metal lower alkoxide such as sodium methoxide, sodium ethoxide, or potassium tert-butoxide; an alkyl lithium such as n-butyl lithium, sec-butyl lithium, tert-butyl lithium, or methyl lithium; an alkali metal or alkaline earth metal hydroxide such as sodium hydroxide, potassium hydroxide, lithium hydroxide, or barium hydroxide; an alkali metal or alkaline earth metal carbonate such as sodium carbonate, potassium carbonate, or cesium carbonate; an alkali metal or alkaline earth metal hydrogencarbonate such as sodium hydrogencarbonate or potassium hydrogencarbonate; an amine such as triethylamine, N-methylmorpholine, N,N-diisopropylethylamine, 1,8-diazabicyclo[5.4.0]undec-7-ene, 1,5-diazabicyclo[4.3.0]non-5-ene, or N,N-dimethylaniline; or a basic heterocyclic compound such as pyridine, imidazole, or 2,6-lutidine. These bases are selected as appropriate, according to various reaction conditions known to skilled artisans.

The term "acid" refers to, for example, an inorganic acid such as hydrochloric acid, hydrobromic acid, sulfuric acid, nitric acid, or phosphoric acid; or an organic acid such as p-toluenesulfonic acid, methanesulfonic acid, trifluoroacetic acid, formic acid, acetic acid, citric acid, oxalic acid, or pyridinium p-toluenesulfonate. These acids are selected as appropriate, according to various reaction conditions known to skilled artisans.

The term "Lewis acid" may be exemplified by boron trifluoride, aluminum trichloride, titanium tetrachloride, iron trichloride, zinc chloride, or tin tetrachloride. These Lewis acids are selected as appropriate, according to various reaction conditions known to skilled artisans.

In the general production processes, A¹ represents a chlorine atom, a bromine atom, an iodine atom, or a trifluoromethanesulfonyloxy group; P¹ represents a functional group derived from a chiral optical resolving agent that is used in common optical resolution methods and which has at least one asymmetric point, and may be exemplified by (3aR,6aS)-3a-allylhexahydro-2H-cyclopenta[b]furanyl; X represents a common leaving group such as a chlorine atom, a bromine atom, an iodine atom, a methanesulfonyloxy group, a p-toluenesulfonyloxy group or a trifluoromethanesulfonyloxy group; A² represents a halogen atom, an alkoxy group, an acyloxy group, or an amino group substituted with an alkoxy group or an alkyl group; Mt represents lithium or magnesium halide; the other symbols have the same meanings as defined above.

### General production process 1

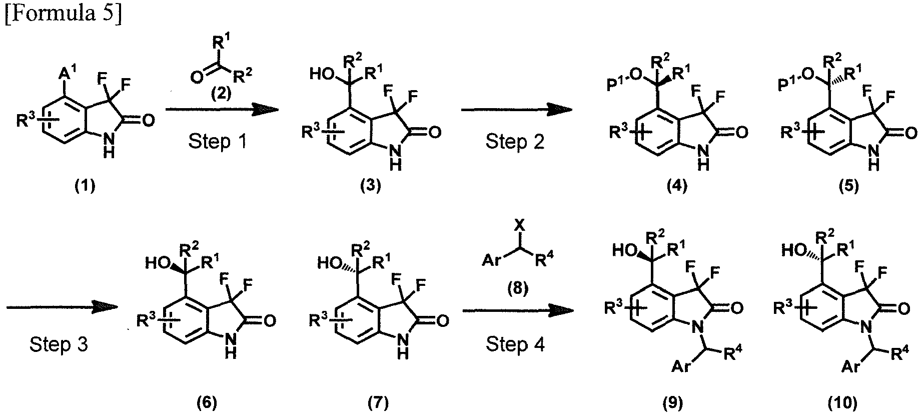

Step 1: In an inert solvent, compound (1) is converted to a metal reagent using, for example, a metal, a Grignard reagent or an alkyl lithium reagent and thereafter the metal reagent may be reacted with compound (2) to give compound (3). The metal as used herein may be exemplified by magnesium or zinc; the Grignard reagent may be exemplified by isopropyl magnesium chloride; and the alkyl lithium reagent may be exemplified by a n-butyl lithium, sec-butyl lithium, t-butyl lithium, or phenyl lithium reagent.
Step 2: Compound (3) may be reacted, in the presence or absence of an acid or a base, with an optical resolving agent used in common optical resolution methods to form diastereomers, thereby yielding compounds (4) and (5). The resulting diastereomeric mixture of compounds (4) and (5) may be separated by fractional crystallization or column chromatography. The optical resolving agent to be used here may be exemplified by (R)-5-allyl-2-oxabicyclo[3.3.0]oct-8-ene.
Step 3: In an inert solvent, compound (4) and compound (5) may be subjected to hydrolysis with an acid or a base or, alternatively, to the hydroxyl group deprotecting reaction that is described in Theodora W. Greene and Peter G. M. Wuts, "Protective Groups in Organic Synthesis Third Edition", thereby yielding compound (6) and compound (7).
Step 4: In an inert solvent, compound (6) or (7) may be subjected to alkylation reaction with compound (8) in the presence or absence of a base and in the presence or absence of an additive, whereupon compound (9) or compound (10) is obtained. Examples of the base include sodium hydride and potassium carbonate, and the additive may be exemplified by potassium iodide. In the case of racemic compounds wherein R¹ and R² are the same, the end product may be obtained from compound (3) through step 4.

### General production process 2

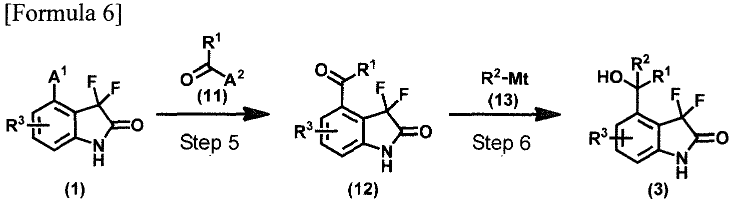

Step 5: The procedure of step 1 may be repeated, except that compound (11) rather than compound (2) is subjected to reaction, thereby yielding compound (12). Compound (11) may be exemplified by ethyl trifluoroacetate, ethyl difluoroacetate, ethyl monofluoroacetate, N,N-dimethylformamide, and N-methoxy-N-methylacetamide.
Step 6: In an inert solvent, compound (12) may be reacted with compound (13) to yield compound (3). Compound (13) may be exemplified by methyl magnesium bromide or ethyl magnesium bromide.

### General production process 3

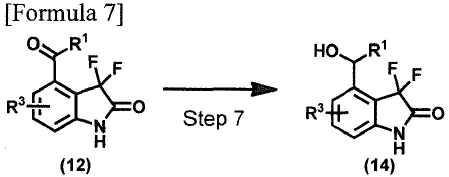

Step 7: In an inert solvent, compound (12) may be subjected to reduction reaction, whereupon compound (14) is obtained. The reducing agent used here may be exemplified by lithium borohydride, sodium borohydride, calcium borohydride, lithium triethyl borohydride, lithium tri-sec-butyl borohydride, potassium tri-sec-butyl borohydride, zinc borohydride, borane, lithium trimethoxyborohydride, lithium triacetoxyborohydride, sodium triacetoxyborohydride, tetramethylammonium borohydride, aluminum lithium hydride, aluminum sodium hydride, sodium bis(2-methoxyethoxy)aluminium hydride, diisobutylaluminum hydride, and trichlorosilane. Alternatively, compound (12) may be reacted with a reducing agent in an inert solvent in the presence of an asymmetric catalyst to yield an optically active compound (14). The asymmetric catalyst referred to above may be exemplified by chloro[(1S,2S)-N-(p-toluenesulfonyl)- ,2-diphenylethanediamine](mesitylene)rutherium(II), and the reducing agent may be exemplified by hydrogen.

### EXAMPLES

Next, the present invention will be further described in more detail with reference to Production Examples, Examples and Test Examples, which are not intended to limit the scope of the present invention.

In the following Production Examples and Examples, the microwave reactor used is Biotage Initiator.

In the following Production Examples and Examples, the "silica gel cartridge" used for purification by column chromatography was a Biotage SNAP Cartridge KP-Sil, HP-Sil, or GRACE REVELERIS Silica.

In the following Production Examples and Examples, purification by preparative high performance liquid chromatography (HPLC) was conducted under the following conditions. It should be noted that when trifluoroacetic acid was used in the main procedure for producing compounds having a basic functional group, neutralization operation or the like was conducted as appropriate for obtaining the compounds in free form.
Apparatus: Gilson Trilution LC
Column: YMC-Actus triart C18 5 µm 20 x 50 mm or Waters SunFire Prep C18 OBD 5 µm 30x50mm
Solvent: A-liquid; 0.1% trifluoroacetic acid containing water, B-liquid; 0.1% trifluoroacetic acid containing acetonitrile
Gradient conditions: 0 min (A-liquid/B-liquid = 90/10), 11 min (A-liquid/B-liquid = 20/80), 12 to 13.5 min (A-liquid/B-liquid = 5/95), flow rate 40 mL/min
Detection method: UV 254 nm

In the following Production Examples and Examples, mass spectra (MS) were measured using the following apparatuses.
MS: Shimadzu LCMS-2010EV, micromass Platform LC, Shimadzu LCMS-IT-TOF, micromass GCT, 1290 Infinity and Agilent 6150

In the following Production Examples and Examples, nuclear magnetic resonance spectra (NMR) were used for structural identification. NMR was measured using the following apparatuses.
NMR spectra: [1H-NMR] 600 MHz: JNM-ECA600 (JOEL Ltd.), 500 MHz: JNM-ECA500 (JOEL Ltd.), 300 MHz: UNITYNOVA300 (Varian Inc.), 200 MHz: GEMINI2000/200 (Varian Inc.)

The RT (Retention Time (min)) indicated in the following Production Examples and Table 1 are values measured using a high performance liquid chromatography mass spectrometer (LCMS) under any of the following conditions.

### Condition A

Measuring instrument: Agilent Agilent 1290 Infinity and Agilent 6150
Column: Waters Acquity CSH C18, 1.7 µm, Φ2.1 x 50 mm
Solvent: A-liquid; 0.1% formic acid containing water, B-liquid; 0.1% formic acid containing acetonitrile,
Gradient: 0 min (A-liquid/B-liquid = 80/20), 1.2 to 1.4 min (A-liquid /B-liquid = 1/99)
Flow rate: 0.8 mL/min, Detection method: 254 nm

### Condition B

Measuring instrument: Agilent Agilent 1290 Infinity and Agilent 6150
Column: Waters Acquity CSH C18, 1.7 µm, Φ2.1 x 50 mm
Solvent: A-liquid; 0.1 % formic acid containing water, B-liquid; 0.1 % formic acid containing acetonitrile
Gradient: 0 min (A-liquid/B-liquid = 95/5), 1.2 min (A-liquid/B-liquid = 50/50), 1.38 min

### (A-liquid/B-liquid = 3/97)

Flow rate: 0.8 mL/min (0 to 1.2 min), 1.0 mL/min (1.2 to 1.38 min)
Detection method: 254 nm

In the following Production Examples and Examples, optical isomer analysis of racemic compounds and optically active compounds was measured using the following instruments, and the RT (min) was shown in the following Production Examples and Table 1.
Measuring instrument: Agilent Agilent 1100 (chiral HPLC)
Measuring Instrument: Waters Waters 2695 and 2998 (chiral HPLC)
Measuring instrument: Shimadzu LC-30AD (chiral HPLC)

In the following Production Examples and Examples, X-ray crystal structure analysis was measured under the following condition.
Measuring instrument: Rigaku R-AXIS RAPID II

In the following Production Examples and Examples, compounds were named in accordance with ACD/Name (ACD/Labs 12.01, Advanced Chemistry Development Inc.).

In the following Production Examples and Examples, chirality confirmation of compounds was conducted by either chiral HPLC analysis or X-ray crystal structure analysis or by a combination thereof.

### Production Example 1: 3,3-Difluoro-4-(trifluoroacetyl)-1,3-dihydro-2H-indol-2-one

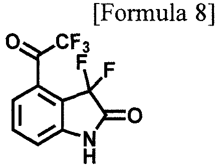

To a diethyl ether suspension (800 ml) of 4-bromo-3,3-difluoro-1,3-dihydro-2H-indol-2-one (40 g, 161.3 mmol), n-butyl lithium (2.69 M hexane solution, 132 ml, 354.8 mmol) was added at -60°C and the resulting mixture was stirred for 30 minutes; to the stirred mixture, ethyl trifluoroacetate (38.5 ml, 322.5 mmol) was added and the resulting mixture was stirred for an hour as it was warmed to room temperature. To the reaction mixture, a saturated aqueous solution of ammonium chloride was added to arrest the reaction and after extraction with ethyl acetate, the extract was washed with water and brine. The organic layer was dried over anhydrous magnesium sulfate; the insoluble matter was separated by filtration and then concentrated under reduced pressure. To the resulting residue, chloroform (200 ml) was added and the resulting mixture was stirred overnight and the precipitate was recovered by filtration. As a result, the titled compound was obtained as a white powder in an amount of 17 g (yield: 40%).
(ESI neg.) m/z : 264(M-H)-
1H NMR (600 MHz, DMSO-d6) d ppm 7.43 - 7.48 (1 H, m), 7.73 - 7.80 (1 H, m), 7.81 - 7.86 (1H, m), 11.55 (1H, br. s.)

### Production Example 2: 3,3-Difluoro-4-(2,2,2-trifluoro-1-hydroxyethyl)-1,3-dihydro-2H-indol-2-one

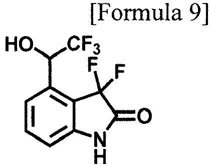

To a chloroform suspension (1200 ml) of the 3,3-difluoro-4-(trifluoroacetyl)-1,3-dihydro-2H-indol-2-one (17 g, 64.1 mmol) obtained in Production Example 1, sodium triacetoxyborohydride (40 g, 192.3 mmol) was added and the resulting mixture was stirred at room temperature for 2 days. After washing the reaction mixture with water, the aqueous layer was extracted with ethyl acetate. The organic layers were combined and dried over anhydrous magnesium sulfate; the insoluble matter was separated by filtration and then concentrated under reduced pressure. To the resulting residue, chloroform (100 ml) was added and the resulting mixture was stirred at room temperature for 30 minutes and the precipitate was recovered by filtration. As a result, the titled compound was obtained as a white powder in an amount of 13.3 g (yield: 31%).
(ESI neg.) m/z : 266(M-H)-
LCMS RT 0.727, Condition A

### Production Example 3: 4-(Difluoroacetyl)-3,3-difluoro-1,3-dihydro-2H-indol-2-one

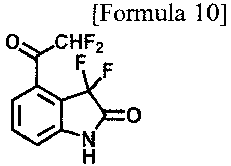

Reaction was carried out in substantially the same manner as in Production Example 1 except that ethyl trifluoroacetate was replaced by ethyl difluoroacetate, and the titled compound was obtained as a pale yellow amorphous in an amount of 123 mg (yield: 25%).
(ESI neg.) m/z : 246(M-H)-
LCMS RT 0.640, Condition A
1H NMR (600 MHz, DMSO-d6) d ppm 7.05 - 7.27 (1 H, m), 7.32 - 7.38 (1 H, m), 7.75 - 7.82 (2 H, m), 11.44 (1 H, br. s.)

### Production Example 4: 3,3-Difluoro-4-(fluoroacetyl)-1,3-dihydro-2H-indol-2-one

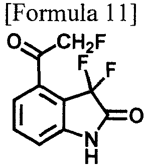

Reaction was carried out in substantially the same manner as in Production Example 1 except that ethyl trifluoroacetate was replaced by ethyl monofluoroacetate, and the titled compound was obtained as a pale yellow amorphous in an amount of 120 mg (yield: 26%).
(ESI neg.) m/z : 228(M-H)-
LCMS RT 0.583, Condition A
1H NMR (600 MHz, DMSO-d6) d ppm 5.70 - 5.85 (2 H, m), 7.27 (1 H, d, J=8.3 Hz), 7.56 - 7.63 (1 H, m), 7.66 - 7.74 (1 H, m), 11.19 - 11.49 (1 H, m)

### Production Example 5: 3,3-Difluoro-2-oxo-2,3-dihydro-1H-indol-4-carbaldehyde

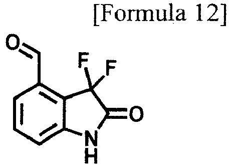

Reaction was carried out in substantially the same manner as in Production Example 1 except that ethyl trifluoroacetate was replaced by N,N-dimethylformamide, and the titled compound was obtained as a pale yellow powder in an amount of 305 mg (yield: 33%).
(ESI neg.) m/z : 196(M-H)-
LCMS RT 0.592, Condition A
1H NMR (600 MHz, DMSO-d6) d ppm 7.26 - 7.34 (1 H, m), 7.63 - 7.73 (1 H, m), 7.74 - 7.82 (1 H, m), 10.06 - 10.13 (1H, m), 11.40 (1H, br. s.)

### Production Example 6: 3,3,5-Trifluoro-4-(trifluoroacetyl)-1,3-dihydro-2H-indol-2-one

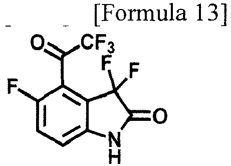

Reaction was carried out in substantially the same manner as in Production Example 1 except that 4-bromo-3,3-difluoro-1,3-dihydro-2H-indol-2-one was replaced by 3,3,5-trifluoro-1,3-dihydro-2H-indol-2-one, and the titled compound was obtained as a pale yellow powder in an amount of 541 mg (yield: 36%).
(ESI neg.) m/z : 282(M-H)-
1H NMR (600 MHz, DMSO-d6) d ppm 7.40 - 7.46 (1 H, m), 7.70 - 7.77 (1 H, m), 11.58 (1 H, br. s.)

### Production Example 7: 4-Acetyl-3,3-difluoro-1,3-dihydro-2H-indol-2-one

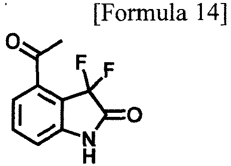

Reaction was carried out in substantially the same manner as in Production Example 1 except that ethyl trifluoroacetate was replaced by N-methoxy-N-methylacetamide, and the titled compound was obtained as a pale yellow powder in an amount of 71 mg (yield: 44%).
(ESI neg.) m/z : 210(M-H)-
1H NMR (600 MHz, CHLOROFORM-d) d ppm 2.66 (3 H, s) 7.06 - 7.11 (1 H, m) 7.44 - 7.51 (1 H, m) 7.53 - 7.60 (2 H, m)

### Production Example 8: 4-(2,2-Difluoro-1-hydroxyethyl)-3,3-difluoro-1,3-dihydro-2H-indol-2-one

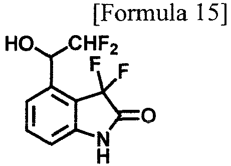

Reaction was carried out in substantially the same manner as in Production Example 2 except that 3,3-difluoro-4-(trifluoroacetyl)-1,3-dihydro-2H-indol-2-one was replaced by the 4-(difluoroacetyl)-3,3-difluoro-1,3-dihydro-2H-indol-2-one obtained in Production Example 3, and the titled compound was obtained as a pale yellow amorphous in an amount of 54 mg (yield: 44%).
(ESI neg.) m/z : 248(M-H)-
LCMS RT 0.979, Condition A

### Production Example 9: 3,3-Difluoro-4-(2-fluoro-1-hydroxyethyl)-1,3-dihydro-2H-indol-2-one

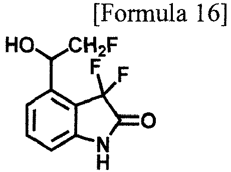

Reaction was carried out in substantially the same manner as in Production Example 2 except that 3,3-difluoro-4-(trifluoroacetyl)-1,3-dihydro-2H-indol-2-one was replaced by the 3,3-difluoro-4-(fluoroacetyl)-1,3-dihydro-2H-indol-2-one obtained in Production Example 4, and the titled compound was obtained as a pale yellow amorphous in an amount of 37 mg (yield: 31 %).
(ESI neg.) m/z : 230(M-H)-
LCMS RT 0.868, Condition A
1H NMR (600 MHz, DMSO-d6) d ppm 4.30 - 4.50 (2 H, m), 5.00 - 5.08 (1 H, m), 6.01 - 6.05 (1 H, m), 6.89 - 6.93 (1 H, m), 7.24 - 7.28 (1 H, m), 7.48 - 7.55 (1 H, m), 11.23 (1 H, br. s.)

### Production Example 10: 3,3-Difluoro-4-(hydroxymethyl)-1,3-dihydro-2H-indol-2-one

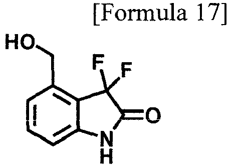

Reaction was carried out in substantially the same manner as in Production Example 2 except that 3,3-difluoro-4-(trifluoroacetyl)-1,3-dihydro-2H-indol-2-one was replaced by the 3,3-difluoro-2-oxo-2,3-dihydro-1H-indol-4-carbaldehyde obtained in Production Example 5, and the titled compound was obtained as a pale yellow amorphous in an amount of 128 mg (yield: 63%).
(ESI neg.) m/z : 198(M-H)-
1H NMR (600 MHz, DMSO-d6) d ppm 4.63 (2 H, s), 5.46 (1 H, br. s.), 6.81 - 6.89 (1 H, m), 7.18 - 7.27 (1 H, m), 7.45 - 7.54 (1 H, m), 11.04 - 11.29 (1 H, m)

### Production Example 11: 3,3,5-Trifluoro-4-(2,2,2-trifluoro-1-hydroxyethyl)-1,3-dihydro-2H-indol-2-one

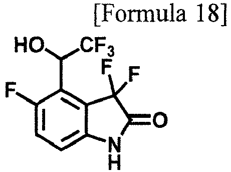

Reaction was carried out in substantially the same manner as in Production Example 2 except that 3,3-difluoro-4-(trifluoroacetyl)-1,3-dihydro-2H-indol-2-one was replaced by the 3,3,5-trifluoro-4-(trifluoroacetyl)-1,3-dihydro-2H-indol-2-one obtained in Production Example 6, and the titled compound was obtained as a pale yellow powder in an amount of 382 mg (yield: 70%).
(ESI neg.) m/z : 284(M-H)-

### Production Example 12: 3,3-Difluoro-4-(1-hydroxyethyl)-1,3-dihydro-2H-indol-2-one

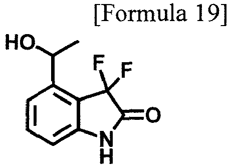

To a diethyl ether solution (400 ml) of 4-bromo-3,3-difluoro-1,3-dihydro-2H-indol-2-one (20 g, 80.6 mmol), n-butyl lithium (2.69 M hexane solution, 89.9 ml, 241.9 mmol) was added at -78°C and the resulting mixture was stirred for 30 minutes; to the stirred mixture, acetaldehyde (5M tetrahydrofuran solution, 35.5 ml, 177.4 mmol) was added and the resulting mixture was stirred for 2 hours as it was warmed to room temperature. To the reaction mixture, a saturated aqueous solution of ammonium chloride was added to arrest the reaction and after extraction with ethyl acetate, the extract was washed with water and brine. The organic layer was dried over anhydrous magnesium sulfate; the insoluble matter was separated by filtration and then concentrated under reduced pressure. The resulting residue was purified with a silica gel cartridge (hexane/ethyl acetate = 80:20 to 20:80). After washing the solids with diisopropyl ether, recovery by filtration gave the titled compound as a pale yellow powder in an amount of 5.2 g (yield: 30%).
(ESI neg.) m/z : 212(M-H)-

### Production Example 13: 3,3-Difluoro-4-(1-hydroxycyclobutyl)-1,3-dihydro-2H-indol-2-one

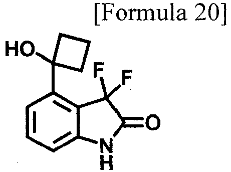

Reaction was carried out in substantially the same manner as in Production Example 12 except that acetaldehyde was replaced by cyclobutanone, and the titled compound was obtained as a pale yellow amorphous in an amount of 560 mg (yield: 83%).
(ESI neg.) m/z : 238(M-H)-
LCMS RT 0.645, Condition A
1H NMR (600 MHz, DMSO-d6) d ppm 1.60 - 1.66 (1 H, m), 1.97 - 2.02 (1 H, m), 2.20 - 2.29 (2 H, m), 2.41 - 2.49 (2 H, m), 5.52 (1 H, br. s.), 6.84 - 6.89 (1 H, m), 7.09 - 7.15 (1 H, m), 7.42 - 7.50 (1 H, m), 11.10 (1 H, br. s.)

### Production Example 14: 3,3-Difluoro-4-(3-hydroxyoxetan-3-yl)-1,3-dihydro-2H-indol-2-one

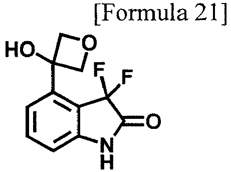

Reaction was carried out in substantially the same manner as in Production Example 12 except that acetaldehyde was replaced by oxetan-3-one, and the titled compound was obtained as a white powder in an amount of 1.1g (yield: 45%).
(ESI neg.) m/z : 240(M-H)-
LCMS RT 0.351, Condition A
1H NMR (600 MHz, DMSO-d6) d ppm 4.70 (2 H, d, J=7.0 Hz), 4.86 (2 H, d, J=7.0 Hz), 6.43 (1 H, br. s.), 6.94 (1 H, d, J=7.8 Hz), 7.21 - 7.28 (1 H, m), 7.48 - 7.57 (1 H, m), 11.19 (1 H, br. s.)

### Production Example 15: 3,3-Difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one

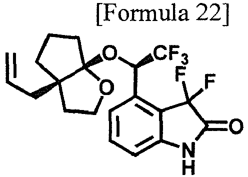

### 3,3-Difluoro-4-[(1S)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one

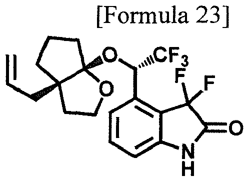

A toluene solution (2 ml) of the 3,3-difluoro-4-(2,2,2-trifluoro-1-hydroxyethyl)-1,3-dihydro-2H-indol-2-one (100 mg, 0.374 mmol) obtained in Production Example 2, (3aR)-3a-(prop-2-en-1-yl)-3,3a,4,5-tetrahydro-2H-cyclopenta[b]furan (0.172 ml, 1.12 mmol) and pyridinium p-toluenesulfonate (9 mg, 0.037 mmol) was heated under reflux for 5 hours. The reaction mixture was diluted with ethyl acetate and washed with water and brine. The organic layer was dried over anhydrous magnesium sulfate and the insoluble matter was separated by filtration and then concentrated under reduced pressure. The resulting residue was purified with a silica gel cartridge (hexane/ethyl acetate = 90:10 to 70:30). As a result, the titled compounds, 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one and 3,3-difluoro-4-[(1S)-2,2,2-trifluoro-l-{[(3aR,6aR)-3a-(prop-2-en-1-yl)hexahydro-6aH-cyclopenta[b]furan-6a-yl]oxy}ethyl]-1,3-dihydro-2H-indol-2-one, were obtained as pale yellow powders in respective amounts of 53mg (yield: 40%) and 67 mg (yield: 43%).

### Data for 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one

(ESI neg.) m/z : 416(M-H)-
1H NMR (600 MHz, DMSO-d6) d ppm 1.44 - 1.53 (2 H, m), 1.60 - 1.70 (4 H, m), 1.78 - 1.84 (1 H, m), 2.09 - 2.22 (2 H, m), 2.27 - 2.33 (1 H, m), 2.99 - 3.06 (1 H, m), 3 .49 - 3.56 (1 H, m), 5.05 - 5.17 (2 H, m), 5.24 - 5.31 (1 H, m), 5.80 - 5.90 (1 H, m), 7.01 - 7.04 (1 H, m), 7.28 - 7.33 (1 H, m), 7.59 - 7.64 (1 H, m), 11.38 (1 H, br. s.)

### Data for 3,3-difluoro-4-[(1S)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one

(ESI neg.) m/z : 416(M-H)-
1H NMR (600 MHz, DMSO-d6) d ppm 1.00 (1 H, br. s.), 1.36 - 1.54 (4 H, m), 1.56 - 1.62 (1 H, m), 1.7 - 1.78 (1 H, m), 1.94 - 2.00 (1 H, m), 2.12 - 2.19 (1 H, m), 2.30 - 2.36 (1 H, m), 3.74 - 3.80 (1 H, m), 3.90 - 3.96 (1 H, m), 5.05 - 5.18 (2 H, m), 5.43 (1 H, br. s.), 5.82 - 5.92 (1 H, m), 7.06 (1 H, d, J=7.8 Hz), 7.28 - 7.33 (1 H, m), 7.60 - 7.67 (1 H, m), 11.42 (1 H, br. s.)

### Production Example 16: 3,3-Difluoro-4-[(1R)-2,2,2-trifluoro-1-hydroxyethyl]-1,3-dihydro-2H-indol-2-one

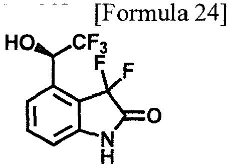

To a methanol solution of the 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one (53 mg, 0.127 mmol) obtained in Production Example 15, p-toluenesulfonic acid (48 mg. 0.254 mmol) was added and the resulting mixture was stirred at 60°C for 4 hours. To the reaction mixture, a saturated aqueous solution of sodium hydrogencarbonate was added and after extraction with ethyl acetate, the extract was washed with brine. The organic layer was dried over anhydrous magnesium sulfate; the insoluble matter was separated by filtration and concentrated under reduced pressure. The resulting residue was purified with a silica gel cartridge (hexane/ethyl acetate = 80:20 to 30:70). As a result, the titled compound was obtained as a white powder in an amount of 28 mg (yield: 83%; enantiomeric excess: 93.8%ee).
(ESI neg.) m/z : 266(M-H)-
1H NMR (600 MHz, DMSO-d6) d ppm 5.12 - 5.19 (1 H, m), 7.00 - 7.04 (1 H, m), 7.22 - 7.28 (1 H, m), 7.28 - 7.33 (1 H, m), 7.56 - 7.62 (1 H, m), 11.38 (1 H, br. s.)

### Chiral analysis conditions

Column: DAICEL CHIRALPAK AD-H, 4.6*(150+150)
Solvent system: Hex : EtOH = 96 : 4
Flow rate: 1 mL/min
RT: 10.31, 11.74, Later

### Production Example 17: 3,3-Difluoro-4-[(1S)-2,2,2-trifluoro-1-hydroxyethyl]-1,3-dihydro-2H-indol-2-one

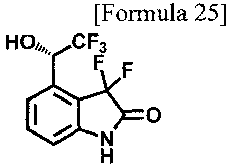

Reaction was carried out in substantially the same manner as in Production Example 16 except that 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one was replaced by the 3,3-difluoro-4-[(1S)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one obtained in Production Example 15, and the titled compound was obtained as a white powder in an amount of 34 mg (yield: 79%; enantiomeric excess: 88.3%ee).
(ESI neg.) m/z : 266(M-H)-

### Chiral analysis conditions

Column: DAICEL CHIRALPAK IC+IC3, 4.6*(150+150)
Solvent system: Hex : EtOH = 96 : 4
Flow rate: 1 mL/min
RT: 10.31, 11.74, Faster

### Production Example 18: 4-(2,2-Difluoro-1-{[(3aR,6aR)-3a-(prop-2-en-1-yl)hexahydro-6aH-cyclopenta[b]furan-6a-yl]oxy}ethyl)-3,3-difluoro-1,3-dihydro-2H-indol-2-one

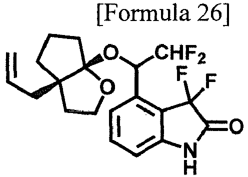

Reaction was carried out in substantially the same manner as in Production Example 15 except that 3,3-difluoro-4-(2,2,2-trifluoro-1-hydroxyethyl)-1,3-dihydro-2H-indol-2-one was replaced by the 3,3-difluoro-4-(2-fluoro-1-hydroxyethyl)-1,3-dihydro-2H-indol-2-one obtained in Production Example 8, and the titled compound was obtained in two isomeric forms, (diastereomer 1) 4-(2,2-difluoro-1-{[(3aR,6aR)-3a-(prop-2-en-1-yl)hexahydro-6aH-cyclopenta[b]furan-6a-yl]oxy}ethyl)-3,3-difluoro-1,3-dihydro-2H-indol-2-one as a white powder in an amount of 63 mg (yield: 40%), and (diastereomer 2) 4-(2,2-difluoro-1-{[(3aR,6aR)-3a-(prop-2-en-1-yl)hexahydro-6aH-cyclopenta[b]furan-6a-yl]oxy}ethyl)-3,3-difluoro-1,3-dihydro-2H-indol-2-one as a pale yellow powder in an amount of 75 mg (yield: 47%).

### Data for diastereomer 1

(ESI neg.) m/z : 398(M-H)-
1H NMR (600 MHz, DMSO-d6) d ppm 1.42 - 1.51 (2 H, m), 1.58 - 1.70 (4 H, m), 1.78 - 1.84 (1 H, m), 2.08 - 2.21 (2 H, m), 2.26 - 2.33 (1 H, m), 3.03 - 3.09 (1 H, m), 3.47 - 3.52 (1 H, m), 4.97 - 5.16 (3 H, m), 5.82 - 5.92 (1 H, m), 6.04 - 6.27 (1 H, m), 6.94 - 6.97 (1 H, m), 7.23 - 7.28 (1 H, m), 7.54 - 7.59 (1 H, m), 11.30 (1 H, br. s.)

### Data for diastereomer 2

(ESI neg.) m/z : 398(M-H)-
1H NMR (600 MHz, DMSO-d6) d ppm 0.98 - 1.06 (1 H, m), 1.35 - 1.53 (4 H, m), 1.61 - 1.67 (1 H, m), 1.68 - 1.75 (1 H, m), 1.92 - 1.98 (1 H, m), 2.09 - 2.15 (1 H, m), 2.29 - 2.36 (1 H, m), 3.75 - 3.82(1 H, m), 3.85 - 3.92 (1 H, m), 5.04 - 5.20 (3 H, m), 5.83 - 5.92 (1 H, m), 6.01 - 6.24 ( H, m), 6.97 - 7. 01 (1 H, m), 7.23 - 7.27 (1 H, m), 7.5 5 - 7.61 (1 H, m), 11.3 5 (1 H, br. s.)

### Production Example 19: 3,3-Difluoro-4-[(1S)-1-{[(3aR,6aR)-3a-(prop-2-en-1-yl)hexahydro-6aH-cyclopenta[b]furan-6a-yl]oxy}methyl]-1,3 -dihydro-2H-indol-2-one

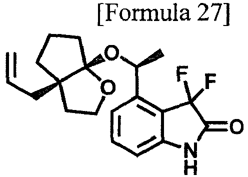

### 3,3-Difluoro-4-[(1R)-1-{[(3aR,6aR)-3a-(prop-2-en-1-yl)hexahydro-6aH-cyclopenta[b]furan-6a-yl]oxy}ethyl]-1,3-dihydro-2H-indol-2-one

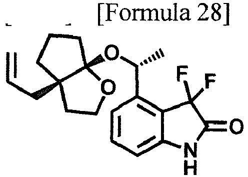

Reaction was carried out in substantially the same manner as in Production Example 15 except that 3,3-difluoro-4-(2,2,2-trifluoro-1-hydroxyethyl)-1,3-dihydro-2H-indol-2-one was replaced by the 3,3-difluoro-4-(1-hydroxyethyl)-1,3-dihydro-2H-indol-2-one obtained in Production Example 12, and the titled compounds, 3,3-difluoro-4-[(1S)-1-{[(3aR,6aR)-3a-(prop-2-en-1-yl)hexahydro-6aH-cyclopenta[b]furan-6a-yl]oxy}ethyl]-1,3-dihydro-2H-indol-2-one and 3,3-difluoro-4-[(1R)-1-{[(3aR,6aR)-3a-(prop-2-en-10-yl)hexahydro-6aH-cyclopenta[b]furan-6a-yl]oxy}ethyl]-1,3-dihydro-2H-indol-2-one, were respectively obtained as a white powder in an amount of 1.0 g (yield: 40%) and as a pale yellow powder in an amount of 1.1 g (yield: 44%).

### Data for 3,3-difluoro-4-[(1 S)-1-{[(3aR,6aR)-3a-(prop-2-en-1-yl)hexahydro-6aH-cyclopenta[b]furan-6a-yl]oxy}ethyl]-1,3-dihydro-2H-indol-2-one

(ESI neg.) m/z : 362(M-H)-
1H NMR (600 MHz, DMSO-d6) d ppm 1.33 (3 H, d, J=6.6 Hz), 1.40 - 1.52 (2 H, m), 1.54 - 1.68 (4 H, m), 1.79 - 1.85 (1 H, m), 2.07 - 2.19 (2 H, m), 2.26 - 2.33 (1 H, m), 3.14 - 3.21 (1 H, m), 3.44 - 3.51 (1 H, m), 5.00 - 5.16 (3 H, m), 5.82 - 5.92 (1 H, m), 6.78 - 6.84 (1 H, m), 7.16 - 7.22 (1 H, m), 7.44 - 7.52 (1 H, m), 11.19 (1 H, br. s.)

### Data for 3,3-difluoro-4-[(1R)-1-{[(3aR,6aR)-3a-(prop-2-en-1-yl)hexahydro-6aH-cyclopenta[b]furan-6a-yl]oxy}ethyl]-1,3-dihydro-2H-indol-2-one

(ESI neg.) m/z : 362(M-H)-
1H NMR (600 MHz, DMSO-d6) d ppm 1.03 - 1.12 (1 H, m), 1.33 (3 H, d, J=6.6 Hz), 1.37 - 1.53 (4 H, m), 1.62 - 1.75 (2 H, m), 1.89 - 1.97 (1 H, m), 2.06 - 2.13 (1 H, m), 2.28 - 2.38 (1 H, m), 3 .70 - 3.79 (1 H, m), 3.80 - 3.90 (1 H, m), 4.95 - 5.20 (3 H, m), 5.78 - 5.93 (1 H, m), 6.80 - 6.88 (1 H, m), 7.15 - 7.23 (1 H, m), 7.46 - 7.56 (1 H, m), 11.11 - 11.35 (1 H, m)

### Production Example 20: 3,3,5-Trifluoro-4-(2,2,2-trifluoro-1-{[(3aR,6aR)-3a-(prop-2-en-1-yl)hexahydro-6aH-cyclopenta[b]furan-6a-yl]oxy}ethyl)-1,3-dihydro-2H-indol-2-one

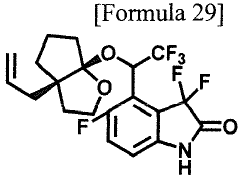

Reaction was carried out in substantially the same manner as in Production Example 15 except that 3,3-difluoro-4-(2,2,2-trifluoro-1-hydroxyethyl)-1,3-dihydro-2H-indol-2-one was replaced by the 3,3,5-trifluoro-4-(2,2,2-trifluoro-1-hydroxyethyl)-1,3-dihydro-2H-indol-2-one obtained in Production Example 11, and the titled compound was obtained in two isomeric forms, (diastereomer 1) 3,3,5-trifluoro-4-(2,2,2-trifluoro-1-{[(3aR,6aR)-3a-(prop-2-en-1-yl)hexahydro-6aH-cyclopenta[b]furan-6a-yl]oxy} ethyl)-1,3-dihydro-2H-indol-2-one and (diastereomer 2) 3,3,5-trifluoro-4-(2,2,2-trifluoro-1-{[(3aR,6aR)-3a-(prop-2-en-1-yl)hexahydro-6aH-cyclopenta[b]furan-6a-yl]oxy}ethyl)-1,3-dihydro-2H-indol-2-one, as pale yellow powders in respective amounts of 108 mg (yield: 23%) and 145 mg (yield: 30%).

### Data for diastereomer 1

(ESI neg.) m/z : 434(M-H)-
1H NMR (600 MHz, DMSO-d6) d ppm 1.43 - 1.54 (2 H, m), 1.56 - 1.70 (4 H, m), 1.78 - 1.85 (1 H, m), 2.08 - 2.30 (3 H, m), 3.12 - 3.20 (1 H, m), 3.56 - 3.63 (1 H, m), 5.02 - 5.14 (2 H, m), 5.24 - 5.35 (1 H, m), 5 .76 - 5.91 (1 H, m), 7.04 - 7.12 (1 H, m), 7.45 - 7.56 (1 H, m), 11.41 (1 H, br. s.)

### Data for diastereomer 2

(ESI neg.) m/z : 434(M-H)-
1H NMR (600 MHz, DMSO-d6) d ppm 1.02 - 1.12 (1 H, m), 1.39 - 1.56 (4 H, m), 1.58 - 1.78 (2 H, m), 1.92 - 2.02 (1 H, m), 2.07 - 2.14 (1 H, m), 2.18 - 2.36 (1 H, m), 3.69 - 3.79 (1 H, m), 3.87 - 3.98 (1 H, m), 5.03 - 5.17 (2 H, m), 5.44 - 5.53 (1 H, m), 5.78 - 5.95 (1 H, m), 7.07 - 7.14 ( H, m), 7.46 - 7.58 (1 H, m), 11.43 (1 H, br. s.)

### Production Example 21: (Enantiomer 1) 4-(2,2-Difluoro-1-hydroxyethyl)-3,3-difluoro-1,3-dihydro-2H-indol-2-one

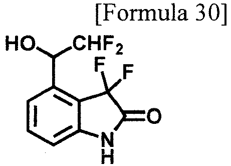

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the (diastereomer 1) 4-(2,2-difluoro-1-{[(3aR,6aR)-3a-(prop-2-en-1-yl)hexahydro-6aH-cyclopenta[b]furan-6a-yl]oxy}ethyl)-3,3-difluoro-1,3-dihydro-2H-indol-2-one obtained in Production Example 18, and the titled compound was obtained as a white powder in an amount of 34 mg (yield: 87%; enantiomeric excess: 90.9%ee).
(ESI neg.) m/z : 248(M-H)-
1H NMR (600 MHz, DMSO-d6) d ppm 4.88 (1 H, br. s.), 5.96 - 6.20 (1 H, m), 6.56 (1 H, br. s.), 6.95 - 6.99 (1 H, m), 7.24 - 7.29 (1 H, m), 7.52 - 7.59 (1 H, m), 11.31 (1 H, br. s.)

### Chiral analysis conditions

Column: DAICEL CHIRALPAK IC+IC3, 4.6*(150+150)
Solvent system: Hex : EtOH = 93 : 7
Flow rate: 1 mL/min
RT: 10.09, 11.92, Later

### Production Example 22: (Enantiomer 2) 4-(2,2-Difluoro-1-hydroxyethyl)-3,3-difluoro-1,3-dihydro-2H-indol-2-one

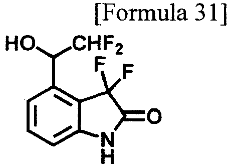

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the (diastereomer 2) 4-(2,2-difluoro-1-{[(3aR,6aR)-3a-(prop-2-en-1-yl)hexahydro-6aH-cyclopenta[b]furan-6a-yl]oxy}ethyl)-3,3 -difluoro-1,3 -dihydro-2H-indol-2-one obtained in Production Example 18, and the titled compound was obtained as a white powder in an amount of 34 mg (yield: 73%; enantiomeric excess: 92.3%ere).
(ESI neg.) m/z : 248(M-H)-

### Chiral analysis conditions

Column: DAICEL CHIRALPAK IC+IC3, 4.6*(150+150)
Solvent system: Hex : EtOH = 93 : 7
Flow rate: 1 mL/min
RT: 10.09, 11.92, Faster

### Production Example 23: 3,3-Difluoro-4-[(1S)-1-hydroxyethyl]-1,3-dihydro-2H-indol-2-one

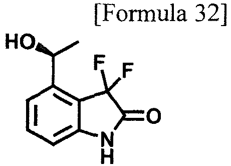

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the 3,3-difluoro-4-[(1S)-1-{[(3aR,6aR)-3a-(prop-2-en-1-yl)hexahydro-6aH-cyclopenta[b]furan-6a-yl]oxy}ethyl]-1,3-dihydro-2H-indol-2-one obtained in Production Example 19, and the titled compound was obtained as a white powder in an amount of
514 mg (yield: 86%; enantiomeric excess: 100%ee).
(ESI neg.) m/z : 212(M-H)-
1H NMR (600 MHz, DMSO-d6) d ppm 1.33 (3 H, d, J=6.6 Hz), 4.92 - 5.00 (1 H, m), 5.43 (1 H, br. s.), 6.81 - 6.86 (1 H, m), 7.24 - 7.28 (1 H, m), 7.45 - 7.52 (1 H, m), 11.19 (1 H, br. s.)

### Chiral analysis conditions

Column : DAICEL CHIRALPAK IC+IC3, 4.6*(150+150)
Solvent system: Hex : EtOH = 95 : 5
Flow rate: 1 mL/min
RT: 21.90,23.55, Later

### Production Example 24: 3,3-Difluoro-4-[(1R)-1-hydroxyethyl]-1,3-dihydro-2H-indol-2-one

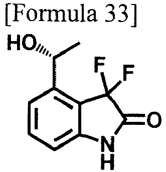

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the 3,3-difluoro-4-[(1R)-1-{[(3aR,6aR)-3a-(prop-2-en-1-yl)hexahydro-6aH-cyclopenta[b]furan-6a-yl]oxy}ethyl]-1,3-dihydro-2H-indol-2-one obtained in Production Example 19, and the titled compound was obtained as a white powder in an amount of
489 mg (yield: 74%; enantiomeric excess: 100%ee).
(ESI neg.) m/z : 212(M-H)-

### Chiral analysis conditions

Column: DAICEL CHIRALPAK IC+IC3, 4.6*(150+150)
Solvent system: Hex : EtOH = 95 : 5
Flow rate: 1 mL/min
RT: 21.90, 23.55, Faster

### Production Example 25: (Enantiomer 1) 3,3,5-Trifluoro-4-(2,2,2-trifluoro-1-hydroxyethyl)-1,3-dihydro-2H-indol-2-one

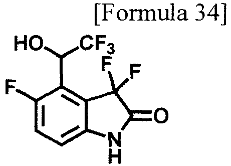

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the (diastereomer 1) 3,3,5-trifluoro-4-(2,2,2-trifluoro-1-{[(3aR,6aR)-3a-(prop-2-en-1-yl)hexahydro-6aH-cyclopenta[b]furan-6a-yl]oxy}ethyl)-1,3-dihydro-2H-indol-2-one obtained in Production Example 20, and the titled compound was obtained as a pale yellow amorphous in an amount of 55 mg (yield: 78%).
(ESI neg.) m/z : 284(M-H)-
1H NMR (600 MHz, DMSO-d6) d ppm 5.30 - 5.39 (1 H, m), 7.03 - 7.09 (1 H, m), 7.28 (1 H, br. s.), 7.43 - 7.50 (1 H, m), 11.35 (1 H, br. s.)

### Production Example 26: (Enantiomer 2) 3,3,5-Trifluoro-4-(2,2,2-trifluoro-1-hydroxyethyl)-1,3-dihydro-2H-indol-2-one

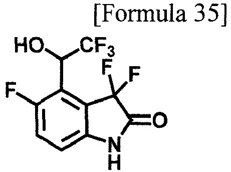

Reaction was carried out in substantially the same manner as in Production Example 16 except that 3,3-difluoro-4-[(1R)-2,2,2-trifluoro-1-{[(3aR,6aR)-3a-(pro-2-en-1-yl)hexahydro-6aH-cyclopenta[b]furan-6a-yl]oxy}ethyl]-1,3-dihydro-2H-indol-2-one was replaced by the (diastereomer 2) 3,3,5-trifluoro-4-(2,2,2-trifluoro-1-{[(3aR,6aR)-3a-(prop-2-en-1-yl)hexahydro-6aH-cyclopenta[b]furan-6a-yl]oxy}ethyl)-1,3-dihydro-2H-indol-2-one obtained in Production Example 20, and the titled compound was obtained as a pale yellow amorphous in an amount of 76 mg (yield: 80%).
(ESI neg.) m/z : 284(M-H)-

### Production Example 27: 3,3-Difluoro-4-(2-hydroxypropan-2-yl)-1,3-dihydro-2H-indol-2-one

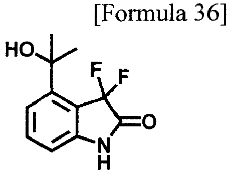

To a tetrahydrofuran solution (1.5 ml) of the 4-acetyl-3,3-difluoro-1,3-dihydro-2H-indol-2-one (70 mg, 0.332 mmol) obtained in Production Example 7, methyl magnesium bromide (1.12 M tetrahydrofuran solution, 1.48 ml, 1.65 mmol) was added under cooling with ice and the resulting mixture was stirred for 2 hours as it was brought back to room temperature. To the reaction mixture, a saturated aqueous solution of ammonium chloride was added to arrest the reaction and after extraction with ethyl acetate, the extract was washed with water and brine. The organic layer was dried over anhydrous magnesium sulfate and after separating the insoluble matter by filtration, the filtrate was concentrated under reduced pressure. The resulting residue was purified with a silica gel cartridge (hexane/ethyl acetate = 80:20 to 30:70; with 1% chloroform added). As a result, the titled compound was obtained as a pale yellow powder in an amount of 50 mg (yield: 66%).
(ESI neg.) m/z : 226(M-H)-
LCMS RT 0.565, Condition A
1H NMR (600 MHz, DMSO-d6) d ppm 1.47 (6 H, s), 5.13 (1 H, s), 6.77 - 6.86 (1 H, m), 7.19 -7.26(1 H, m), 7.37 - 7.46 (1 H, m), 10.97 - 11.29 (1 H, m)

### Production Example 28: 3,3-Difluoro-4-(1-hydroxypropyl)-1,3-dihydro-2H-indol-2-one

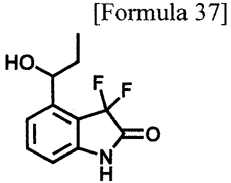

Reaction was carried out in substantially the same manner as in Production Example 27 except that 4-acetyl-3,3-difluoro-1,3-dihydro-2H-indol-2-one was replaced by the 3,3-difluoro-2-oxo-2,3-dihydro-1H-indol-4-carbaldehyde obtained in Production Example 5 and that methyl magnesium bromide (1.12 M tetrahydrofuran solution) was replaced by ethyl magnesium bromide (0.90 M tetrahydrofuran solution), and the titled compound was obtained as a pale yellow amorphous in an amount of 34 mg (yield: 30%).
(ESI neg.) m/z : 226(M-H)-
LCMS RT 0.632, Condition A
1H NMR (600 MHz, DMSO-d6) d ppm 0.82 - 0.89 (3 H, m), 1.55 - 1.66 (2 H, m), 4.69 (1 H, br. s.), 5.36 - 5.42 (1 H, m), 6.83 (1 H, d, J=7.4 Hz), 7.18 - 7.23 (1 H, m), 7.44 - 7.51 (1 H, m), 11.16 ( H, br. s.)

### Production Example 29: Methyl 3,3-difluoro-2-oxo-2,3-dihydro-1H-indol-4-carboxylate

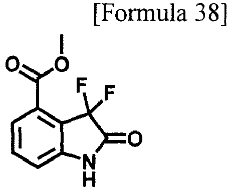

To a chloroform solution (9 ml) of methyl 2,3-dioxo-2,3-dihydro-1H-indole-4-carboxylate (726 mg, 3.54 mmol), N,N-diethylaminosulfur trifluoride (1.16 ml, 8.85 mmol) was added and the resulting mixture was stirred at room temperature for an hour. To the reaction mixture, a saturated aqueous solution of sodium hydrogencarbonate was added under cooling with ice to arrest the reaction and after rendering the mixture acidic with a saturated aqueous solution of ammonium chloride, extraction was conducted. The organic layer was dried and then concentrated under reduced pressure. The resulting residue was purified with a silica gel cartridge (hexane/ethyl acetate = 60:40 to 0:100). As a result, the titled compound was obtained as a pale yellow powder in an amount of 136 mg (yield: 17%). (ESI neg.) m/z : 226(M-H)-
LCMS RT 0.687, Condition A
1H NMR (600 MHz, DMSO-d6) d ppm 3.89 (3 H, s), 7.26 (1 H, dd, J=6.2, 2.1 Hz), 7.63 - 7.70 (2 H, m), 11.40 (1 H, br. s.)

### Production Example 30: 3,3-Difluoro-4-(1-hydroxycyclopropyl)-1,3-dihydro-2H-indol-2-one

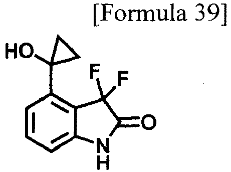

To a tetrahydrofuran solution (4 ml) of the methyl 3,3-difluoro-2-oxo-2,3-dihydro-1H-indole-4-carboxylate (136 mg, 0.599 mmol) obtained in Production Example 29, tetraisopropyl orthotitanate (0.246 ml, 0.838 mmol) and ethyl magnesium bromide (3.0 M diethyl ether solution, 0.758 ml, 3.80 mmol) were added under cooling with ice and the resulting mixture was stirred at room temperature for an hour. To the reaction mixture, an aqueous solution of 1M HC1 was added under cooling with ice to arrest the reaction and after extraction with ethyl acetate, the extract was washed with water and brine. The organic layer was dried and then concentrated under reduced pressure. The resulting residue was purified by preparative HPLC and with a silica gel cartridge (hexane/ethyl acetate = 70:30 to 30:70). As a result, the titled compound was obtained as a pale brown amorphous in an amount of 15 mg (yield: 11%).
(ESI neg.) m/z : 224(M-H)-
LCMS RT 0.556, Condition A
1H NMR (600 MHz, DMSO-d6) d ppm 0.95 - 1.01 (2 H, m), 1.04 - 1.10 (2 H, m), 3.87 (1 H, s), 6.79 - 6.84 (1 H, m), 6.88 - 6.94 (1 H, m), 7.3 5 - 7.42 (1 H, m), 10.99 - 11.23 (1 H, m)

### Production Example 31: 5-Chloro-3,3-difluoro-4-[(1R)-2,2,2-trifluoro-1-hydroxyethyl]-1,3 -dihydro-2H-indol-2-one

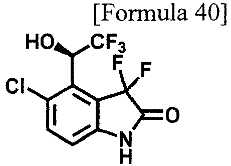

To an acetonitrile solution (1 ml) of the 3,3-difluoro-4-[(1R)-2,2,2-trifluoro-1-hydroxyethyl]-1,3-dihydro-2H-indol-2-one (50 mg, 0.187 mmol) obtained in Production Example 16, N-chlorosuccinimide (125 mg, 0.936 mmol) and trifluoroacetic acid (0.02 ml) were added and the resulting mixture was heated under reflux for 30 hours. To the reaction mixture, a saturated aqueous solution of sodium hydrogencarbonate was added and after extraction with ethyl acetate, the extract was washed with water and brine. After drying the organic layer over anhydrous magnesium sulfate, the insoluble matter was separated by filtration and concentrated under reduced pressure. The resulting residue was purified by preparative HPLC. As a result, the titled compound was obtained as a white solid in an amount of 32 mg (yield: 57%).
(ESI neg.) m/z : 300(M-H)-
1H NMR (600 MHz, DMSO-d6) d ppm 5.50 - 5.65 (1 H, m), 7.03 - 7.10 (1 H, m), 7.18 - 7.27 (1 H, m), 7.64 - 7.70 (1 H, m), 11.41 (1 H, br. s.)

### Production Example 32: 5-Chloro-3,3-difluoro-4-[(1S)-1-hydroxyethyl]-1,3-dihydro-2H-indol-2-one

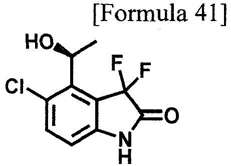

Reaction was carried out in substantially the same manner as in Production Example 31 except that 3,3-difluoro-4-[(1R)-2,2,2-trifluoro-1-hydroxyethyl]-1,3-dihydro-2H-indol-2-one was replaced by the 3,3-difluoro-4-[(1S)-1-hydroxyethyl]-1,3-dihydro-2H-indol-2-one obtained in Production Example 23, and the titled compound was obtained as a white solid in an amount of 50 mg (yield: 86%).
(ESI neg.) m/z : 246(M-H)-
1H NMR (600 MHz, DMSO-d6) d ppm 1.37 (3 H, d, J=6.6 Hz), 5.12 - 5.21 (1 H, m), 5.40 - 5.53 (1 H, m), 6.87 (1 H, d, J=8.3 Hz), 7.52 (1 H, d, J=8.3 Hz), 11.26 (1 H, br. s.)

### Production Example 33: tert-Butyl 3,3-difluoro-4-[(1R)-1-hydroxyethyl]-2-oxo-2,3-dihydro-1H-indole-1-carboxylate

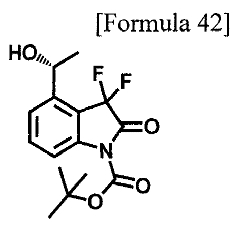

A tetrahydrofuran suspension (5 ml) of the 3,3-difluoro-4-[(1R)-1-hydroxyethyl]-1,3-dihydro-2H-indol-2-one (200 mg, 0.938 mmol) obtained in Production Example 24, di-tert-butyl dicarbonate (307 mg, 1.41 mmol) and sodium carbonate (298 mg, 2.82 mmol) was stirred at room temperature for an hour. The reaction mixture was diluted with ethyl acetate and washed with water and brine. After drying the organic layer over anhydrous magnesium sulfate, the insoluble matter was separated by filtration and concentrated under reduced pressure. The resulting residue was purified with a silica gel cartridge (hexane/ethyl acetate = 80:20 to 50:50). As a result, the titled compound was obtained as a colorless amorphous in an amount of 256 mg (yield: 87%).
(ESI pos.) m/z : 246(M+Na)+
1H NMR (600 MHz, CHLOROFORM-d) d ppm 1.53 (3 H, s), 1.64 (9 H, s), 1.89 - 2.01 (1 H, m), 5.23 - 5.39 (1 H, m), 7.47 - 7.54 (1 H, m), 7.54 - 7.62 (1 H, m), 7.85 - 7.96 (1 H, m)

### Production Example 34: tert-Butyl 4-[(1S)-1-(benzoyloxy)ethyl]-3,3-difluoro-2-oxo-2,3-dihydro-1H-indole-1-carboxylate

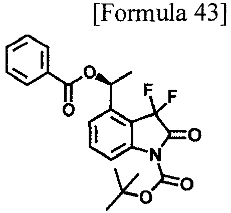

To a tetrahydrofuran solution (4 ml) of the tert-butyl 3,3-difluoro-4-[(1R)-1-hydroxyethyl]-2-oxo-2,3-dihydro-1H-indole-1-carboxylate (256 mg, 0.817 mmol) obtained in Production Example 33, benzoic acid (120 mg, 0.981 mmol) and triphenylphosphine (321 mg, 1.23 mmol), diisopropyl azodicarboxylate (1.9 M toluene solution, 0.645 ml, 1.23 mmol) was added under cooling with ice and the resulting mixture was stirred for an hour under the same conditions. To the reaction mixture, water was added and after extraction with ethyl acetate, the extract was washed with brine. After drying the organic layer over anhydrous magnesium sulfate, the insoluble matter was separated by filtration and concentrated under reduced pressure. The resulting residue was purified with a silica gel cartridge (hexane/ethyl acetate = 90:10 to 50:50). As a result, the titled compound was obtained as a colorless amorphous in an amount of 190 mg (yield: 56%).
(ESI pos.) m/z : 440(M+Na)+
1H NMR (600 MHz, CHLOROFORM-d) d ppm 1.63 (9 H, s), 1.71 (3 H, d, J=6.6 Hz), 6.32 - 6.40 (1 H, m), 7.39 - 7.49 (3 H, m), 7.51 - 7.61 (2 H, m), 7.90 - 7.97 (1 H, m), 8.05 - 8.12 (2 H, m)

### Production Example 35: (1S)-1-(3,3-Difluoro-2-oxo-2,3-dihydro-1H-indol-4-yl)ethyl benzoate

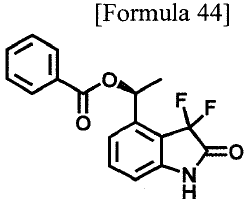

To a chloroform solution (0.5 ml) of the tert-butyl 4-[(1S)-1-(benzoyloxy)ethyl]-3,3-difluoro-2-oxo-2,3-dihydro-1H-indole-1-carboxylate (30 mg, 0.072 mmol) obtained in Production Example 34, trifluoroacetic acid (0.1 ml) was added and the resulting mixture was stirred at room temperature for an hour. The reaction mixture was concentrated under reduced pressure. Without further purification, a crude product mainly consisting of the titled compound was obtained as a pale yellow amorphous in an amount of 23 mg.
(ESI neg.) m/z : 316(M-H)-
1H NMR (600 MHz, DMSO-d6) d ppm 1.63 (3 H, d, J=6.6 Hz), 6.14 - 6.23 (1 H, m), 6.91 - 6.97 (1 H, m), 7.28 - 7.33 (1 H, m), 7.53 - 7.58 (3 H, m), 7.66 - 7.71 (1 H, m), 8.00 - 8.05 (2 H, m), 11.23 - 11.33 (1 H, m)

### Production Example 36: 3,3-Difluoro-4-[(1S)-1-hydroxyethyl]-1,3-dihydro-2H-indol-2-one

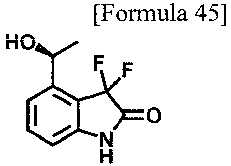

To a methanol solution (1 ml) of the (1S)-1-(3,3-difluoro-2-oxo-2,3-dihydro-1H-indol-4-yl)ethyl benzoate (23 mg, 0.072 mmol) obtained in Production Example 35, an aqueous solution of 1 M sodium hydroxide (0.216 ml, 0.216 mmol) was added under cooling with ice and the resulting mixture was stirred for 3 hours as it was brought back to room temperature. To the reaction mixture, an aqueous solution of 1 M HCl was added for neutralization and after extraction with ethyl acetate, the extract was washed with brine. After drying the organic layer over anhydrous magnesium sulfate, the insoluble matter was separated by filtration and concentrated under reduced pressure. The resulting residue was purified with a silica gel cartridge (hexane/ethyl acetate = 70:30 to 20:80). As a result, the titled compound was obtained as a pale yellow powder in an amount of 9 mg (yield: 59%; enantiomeric excess: 100%ee).
(ESI neg.) m/z : 212(M-H)-

### Chiral analysis conditions

Column: DAICEL CHIRALPAK IC+IC3, 4.6*(150+150)
Solvent system: Hex : EtOH = 95 : 5
Flow rate: 1 mL/min
RT: 21.90, 23.55, Later

### Production Example 37: [6-(2H-1,2,3-Triazol-2-yl)pyridin-2-yl]methanol

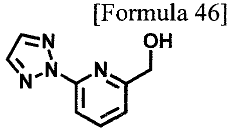

An N,N-dimethylformamide suspension (30 ml) of 2-bromo-6-(hydroxymethyl)pyridine (2.0 g, 10.64 mmol), 2H-1,2,3-triazole (0.741 ml, 12.77 mmol), trans-N,N'-dimethylcyclohexane-1,2-diamine (0.168 ml, 1.07 mmol), copper iodide (202 mg, 1.06 mmol) and cesium carbonate (6.93 g, 21.27 mmol) was subjected to reaction in a microwave reactor at 150°C for one hour and a half. The reaction mixture was filtered through Celite and the filtrate was concentrated under reduced pressure. The resulting residue was purified with a silica gel cartridge (hexane/ethyl acetate = 50:50 to 0:100). As a result, the titled compound was obtained as a pale yellow powder in an amount of 465 mg (yield: 25%).
(ESI pos.) m/z : 177(M+H)+
1H NMR (600 MHz, CHLOROFORM-d) d ppm 3.10 - 3.17 (1 H, m), 4.90 (2 H, d, J=5.4 Hz), 7.42 (1 H, d, J=7.8 Hz), 7.88 - 7.94 (3 H, m), 7.99 (1 H, d, J=7.8 Hz)

### Production Example 38: 2-(Chloromethyl)-6-(2H-1,2,3-triazol-2-yl)pyridine

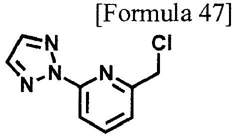

To a chloroform solution (10 ml) of the [6-(2H-1,2,3-triazol-2-yl)pyridin-2-yl]methanol (465 mg, 2.9 mmol) obtained in Production Example 37, thionyl chloride (0.617 ml, 8.71 mmol) was added and the resulting mixture was stirred at room temperature for an hour. The reaction mixture was concentrated under reduced pressure. Without further purification, a crude product mainly consisting of the titled compound was obtained as a pale yellow powder in an amount of 532 mg.
(ESI pos.) m/z : 195(M+H)+
1H NMR (600 MHz, CHLOROFORM-d) d ppm 4.80 (2 H, s), 7.56 - 7.62 (1 H, m), 7.91 (3 H, s), 8.02 - 8.07 (1 H, m)

### Production Example 39: [2-(2H-1,2,3-triazol-2-yl)pyridin-4-yl]methanol

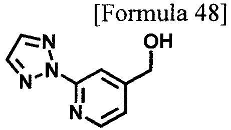

Reaction was carried out in substantially the same manner as in Production Example 37 except that 2-bromo-6-(hydroxymethyl)pyridine was replaced by 2-bromopyridine-4-methanol, and the titled compound was obtained as a pale yellow powder in an amount of 287 mg (yield: 15%).
(ESI pos.) m/z : 177(M+H)+
1H NMR (600 MHz, CHLOROFORM-d) d ppm 2.13 - 2.20 (1 H, m), 4.87 (2 H, d, J=6.2 Hz), 7.31 - 7.37 (1 H, m), 7.90 (2 H, s), 8.10 (1 H, s), 8.53 - 8.58 (1 H, m)

### Production Example 40: [5-(2H-1,2,3-Triazol-2-yl)pyridin-3-yl]methanol

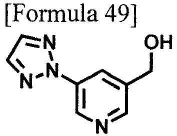

### [5-(1H-1,2,3-Triazol-1-yl)pyridin-3-yl]methanol

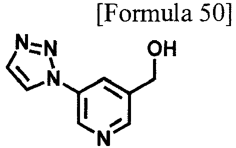

Reaction was carried out in substantially the same manner as in Production Example 37 except that 2-bromo-6-(hydroxymethyl)pyridine was replaced by (5-bromopyridin-3-yl)methanol, and the titled compounds, [5-(2H-1,2,3-triazol-2-yl)pyrdin-3-yl]methanol and [5-(1H-1,2,3-triazol-1-yl)pyridin-3-yl]methanol were obtained in respective amounts of 293 mg (yield: 31 %) and 250 mg (yield: 27%).

### Data for [5-(2H-1,2,3-triazol-2-yl)pyridin-3-yl]methanol

(ESI pos.) m/z : 177(M+H)+
LCMS RT 0.633, Condition B
1H NMR (600 MHz, CHLOROFORM-d) d ppm 4.87 (2 H, s) 7.88 (2 H, s) 8.38 - 8.45 (1 H, m) 8.59-8.65(1 H, m) 9.29 - 9.35 (1 H, m)

### Data for [5-(1H-1,2,3-triazol-1-yl)pyridin-3-yl]methanol

(ESI pos.) m/z : 177(M+H)+
LCMS RT 0.493, Condition B
1H NMR (600 MHz, CHLOROFORM-d) d ppm 2.34 (1 H, s) 4.90 (2 H, s) 7.90 - 7.93 (1 H, m) 8.06-8.10(1 H, m) 8.20 - 8.24 (1 H, m) 8.67 - 8.71 (1 H, m) 8.94 (1 H, d, J=2.48 Hz)

### Production Example 41: [6-(1H-1,2,4-Triazol-1-yl)pyridin-2-yl]methanol

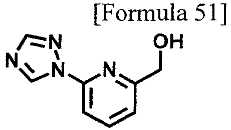

Reaction was carried out in substantially the same manner as in Production Example 37 except that 2H-1,2,3-triazole was replaced by 4H-1,2,4-triazole, and the titled compound was obtained as a pale yellow powder in an amount of 161 mg (yield: 64%).
(ESI pos.) m/z : 177(M+H)+
LCMS RT 0.659, Condition B
1H NMR (600 MHz, CHLOROFORM-d) d ppm 2.80 - 3.06 (1 H, m) 4.83 (2 H, s) 7.31 - 7.37 (1 H, m) 7.79 - 7.86 (1 H, m) 7.88 - 7.95 (1 H, m) 8.12 (1 H, s) 9.20 (1 H, s)

### Production Example 42: [2-(1H-1,2,4-Triazol-1-yl)pyridin-4-yl]methanol

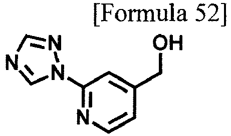

Reaction was carried out in substantially the same manner as in Production Example 37 except that 2-bromo-6-(hydroxymethyl)pyridine was replaced by 2-bromopyridin-4-methanol, and 2H-1,2,3-triazole by 4H-1,2,4-triazole; as a result, the titled compound was obtained as a pale yellow powder in an amount of 842 mg (yield: 90%).
(ESI pos.) m/z : 177(M+H)+
LCMS RT 0.616, Condition B
1H NMR (600 MHz, CHLOROFORM-d) d ppm 2.19 - 2.27 (1 H, m) 4.86 (2 H, s) 7.30 - 7.36 (1 H, m) 7.89 - 7.94 (1 H, m) 8.10 (1 H, s) 8.39 - 8.46 (1 H, m) 9.18 (1 H, s)

### Production Example 43: 4-(Chloromethyl)-2-(2H-1,2,3-triazol-2-yl)pyridine

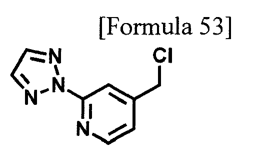

Reaction was carried out in substantially the same manner as in Production Example 38 except that [6-(2H-1,2,3-triazol-2-yl)pyridin-2-yl]methanol was replaced by the [2-(2H-1,2,3-triazol-2-yl)pyridin-4-yl]methanol obtained in Production Example 39, and the titled compound was obtained as a pale yellow powder in an amount of 300 mg.
(ESI pos.) m/z : 195(M+H)+
1H NMR (600 MHz, CHLOROFORM-d) d ppm 4.66 (2 H, s), 7.35 - 7.40 (1 H, m), 7.92 (2 H, s), 8.14 (1 H, s), 8.59 - 8.63 (1 H, m)

### Production Example 44: 3-(Chloromethyl)-5-(2H-1,2,3-triazol-2-yl)pyridine

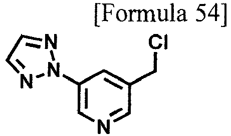

Reaction was carried out in substantially the same manner as in Production Example 38 except that [6-(2H-1,2,3-triazol-2-yl)pyridin-2-yl]methanol was replaced by the [5-(2H-1,2,3-triazol-2-yl)pyridin-3-yl]methanol obtained in Production Example 40, and the titled compound was obtained in an amount of 387 mg.
(ESI pos.) m/z : 195(M+H)+
LCMS RT 0.731, Condition A
1H NMR (600 MHz, CHLOROFORM-d) d ppm 4.82 (2 H, s) 8.01 (2 H, s) 8.77 - 8.82 (1 H, m) 9.01 (1H, s) 9.46(1H,s)

### Production Example 45: 3-(Chloromethyl)-5-(1H-1,2,3-triazol-1-yl)pyridine

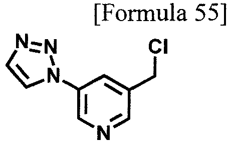

Reaction was carried out in substantially the same manner as in Production Example 38, except that [6-(2H-1,2,3-triazol-2-yl)pyridin-2-yl]methanol was replaced by the [5-(1H-1,2,3-triazol-1-yl)pyridin-3-yl]methanol obtained in Production Example 40, and the titled compound was obtained in an amount of 54 mg.
(ESI pos.) m/z : 195(M+H)+
LCMS RT 0.507, Condition A
1H NMR (600 MHz, CHLOROFORM-d) d ppm 4.70 (2 H, s) 7.93 (1 H, d, J=0.83 Hz) 8.09 (1 H, d, J=0.83 Hz) 8.22 - 8.27 (1 H, m) 8.72 (1 H, d, J=2.06 Hz) 8.98 (1 H, d, J=2.48 Hz)

### Production Example 46: 2-(Chloromethyl)-6-(1H-1,2,4-triazol-1-yl)pyridine

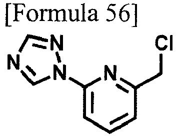

Reaction was carried out in substantially the same manner as in Production Example 38 except that [6-(2H-1,2,3-triazol-2-yl)pyridin-2-yl]methanol was replaced by the [6-(1H-1,2,4-triazol-1-yl)pyridin-2-yl]methanol obtained in Production Example 41, and the titled compound was obtained in an amount of 201 mg.
(ESI pos.) m/z : 195(M+H)+
LCMS RT 0.672, Condition A
1H NMR (600 MHz, CHLOROFORM-d) d ppm 4.69 (2 H, s) 7.56 (1 H, d, J=7.43 Hz) 7.91 (1 H, d, J=7.84 Hz) 7.94 - 8.01 (1 H, m) 8.24 (1 H, br. s.) 9.46 (1H, br. s.)

### Production Example 47: 4-(Chloromethyl)-2-(1H-1,2,4-triazol-1-yl)pyridine

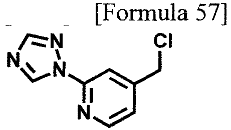

Reaction was carried out in substantially the same manner as in Production Example 38 except that [6-(2H-1,2,3-triazol-2-yl)pyridin-2-yl]methanol was replaced by the [2-(1H-1,2,4-triazol-1-yl)pyridin-4-yl]methanol obtained in Production Example 42, and the titled compound was obtained in an amount of 512 mg.
(ESI pos.) m/z : 195(M+H)+
LCMS RT 1.070, Condition B
1H NMR (600 MHz, CHLOROFORM-d) d ppm 4.63 (2 H, s) 7.31 - 7.38 (1 H, m) 7.96 (1 H, s) 8.11 (1 H, s) 8.46 (1 H, d, J=4.95 Hz) 9.18 (1 H, s)

### Production Example 48: 2-Chloro-6-(1-chloroethyl)pyridine

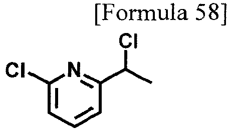

To a chloroform solution (8 ml) of 1-(6-chloropyridin-2-yl)ethanol (340 mg, 2.16 mmol), thionyl chloride (0.313 ml, 4.31 mmol) was added and the resulting mixture was stirred at room temperature for an hour. To the stirred mixture, thionyl chloride (0.313 ml, 4.31 mmol) was added and the resulting mixture was stirred at 40°C for an hour. To the reaction mixture, a saturated aqueous solution of sodium hydrogencarbonate was added to arrest the reaction and extraction was conducted with chloroform. The organic layer was dried and concentrated under reduced pressure. The resulting residue was purified with a silica gel cartridge (chloroform/methanol =0:100 to 95:5). As a result, the titled compound was obtained in an amount of 95 mg (yield: 25%).
(ESI pos.) m/z : 176(M+H)+
1H NMR (600 MHz, CHLOROFORM-d) d ppm 1.86 (3 H, d, J=7.02 Hz) 5.09 (1 H, q, J=6.88 Hz) 7.25 - 7.28 (1 H, m) 7.45 - 7.48 (1 H, m) 7.69 (1 H, t, J=7.84 Hz)

### Production Example 49: (1S)-1-(3,3-Difluoro-2-oxo-1-{[5-(1H-1,2,3-triazol-1-yl)pyridin-3-yl]methyl}-2,3-dihydro-1H-indol-4-yl)ethyl benzoate

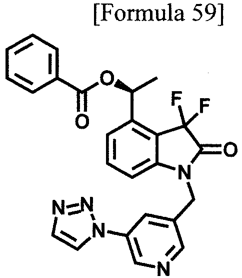

To an N,N-dimethylformamide solution (2 ml) of the (1S)-1-(3,3-difluoro-2-oxo-2,3-dihydro-1H-indol-4-yl)ethyl benzoate (59 mg, 0.185 mmol) obtained in Production Example 35, sodium hydride (60%, 9 mg, 0.222 mmol) was added and the resulting mixture was stirred at room temperature for 15 minutes; thereafter, the 3-(chloromethyl)-5-(1H-1,2,3-triazol-1-yl)pyridine (54 mg, 0.277 mmol) obtained in Production Example 45 was added and the resulting mixture was stirred at 80°C for an hour. To the reaction mixture, water was added to arrest the reaction and purification was conducted by preparative HPLC. As a result, the titled compound was obtained in an amount of 82 mg (yield: 93%).
(ESI pos.) m/z : 476(M+H)+
LCMS RT 1.042, Condition A
1H NMR (600 MHz, CHLOROFORM-d) d ppm 1.72 (3 H, d, J=6.61 Hz) 4.92 - 5.08 (2 H, m) 6.26 - 6.39 (1 H, m) 6.74 - 6.81 (1 H, m) 7.28 - 7.34 (1 H, m) 7.45 (3 H, s) 7.55 - 7.62 (1 H, m) 7.90 (1 H, s) 8.05 (1 H, d, J=0.83 Hz) 8.07 - 8.13 (3 H, m) 8.72 (1 H, d, J=1.65 Hz) 9.00 (1 H, d, J=2.48 Hz)

### Production Example 50: (1S)-1-(1-{[5-(Difluoromethyl)pyridin-3-yl]methyl}-3,3-difluoro-2-oxo-2,3-dihydro-1H-indol-4-yl)ethyl benzoate

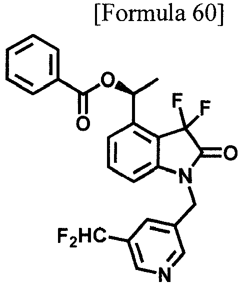

Reaction was carried out in substantially the same manner as in Production Example 49 except that 3-(chloromethyl)-5-(1H-1,2,3-triazol-1-yl)pyridine was replaced by 3-(chloromethyl)-5-(difluoromethyl)pyridine hydrochloride, and the titled compound was obtained in an amount of 27 mg (yield: 46%).
(ESI pos.) m/z : 459(M+H)+
LCMS RT 1.137, Condition A
1H NMR (600 MHz, CHLOROFORM-d) d ppm 1.72 (3 H, d, J=6.61 Hz) 4.87 - 5.03 (2 H, m)6.27-6.39(1 H, m) 6.59 - 6.84 (2 H, m) 7.27 - 7.32 (1 H, m) 7.39 - 7.48 (3 H, m) 7.54 - 7.61 (1 H, m) 7.75 - 7.81 (1 H, m) 8.06 - 8.12 (2 H, m) 8.71 - 8.78 (2 H, m)

### Production Example 51: tert-Butyl3-({3,3-difluoro-4-[(1S)-1-hydroxyethyl]-2-oxo-2,3-dihydro-1H-indol-1-yl}methyl)-1H-indazole-1-carboxylate

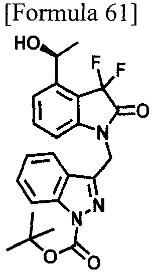

To an N,N-dimethylformamide solution (2 ml) of the 3,3-difluoro-4-[(1S)-1-hydroxyethyl]-1,3-dihydro-2H-indol-2-one (67 mg, 0.312 mmol) obtained in Production Example 23, tert-butyl 3-(chloromethyl)-1H-indazole-1-carboxylate (100 mg, 0.375 mmol) and potassium carbonate (129 mg, 0.936 mmol) were added and the resulting mixture was stirred at 80°C for 30 minutes. The reaction mixture was filtered and the filtrate was purified by preparative HPLC. As a result, the titled compound was obtained in an amount of 95 mg (yield: 19%).
(ESI pos.) m/z : 466(M+H)+
LCMS RT 1.127, Condition A
1H NMR (600 MHz, CHLOROFORM-d) d ppm 1.48 (3 H, d, J=6.61 Hz) 1.54 (1 H, br. s.) 1.71 -1.76 (9 H, m) 5.21 - 5.29 (3 H, m) 7.23 - 7.27 (1 H, m) 7.32 (2 H, s) 7.42 - 7.47 (1 H, m) 7.49 - 7.54 (1 H, m) 7.79 (1 H, d, J=8.26 Hz) 8.04 (1 H, d, J=8.26 Hz)

### Production Example 52: [5-(1H-1,2,4-Triazol-1-yl)pyridin-3-yl]methanol

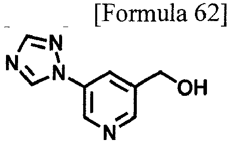

Reaction was carried out in substantially the same manner as in Production Example 37 except that 2-bromo-6-(hydroxymethyl)pyridine was replaced by (5-bromopyridin-3-yl)methanol, and 2H-1,2,3-triazole by 4H-1,2,4-triazole; as a result, the titled compound was obtained as a colorless powder in an amount of 679 mg (yield: 72%).
(ESI pos.) m/z : 177(M+H)+
LCMS RT 0.465, Condition B 1H NMR (600 MHz, CHLOROFORM-d) d ppm 1.64 (1 H, br. s.), 4.88 (2 H, s), 8.11 (1 H, t, J=2.27 Hz), 8.17 (1 H, s), 8.61 - 8.67 (2 H, m), 8.93 (1 H, d, J=2.48 Hz)

### Production Example 53: 3-(Chloromethyl)-5-(1H-1,2,4-triazol-1-yl)pyridine

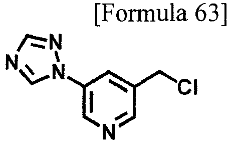

Reaction was carried out in substantially the same manner as in Production Example 38 except that [6-(2H-1,2,3-triazol-2-yl)pyridin-2-yl]methanol was replaced by the [5-(1H-1,2,4-triazol-1-yl)pyridin-3-yl]methanol obtained in Production Example 52, and the titled compound was obtained as a pale yellow powder in an amount of 1.1 g.
(ESI pos.) m/z : 195(M+H)+
LCMS RT 0.493, Condition A

### Production Example 54: 4-(Difluoroacetyl)-3,3,5-trifluoro-1,3-dihydro-2H-indol-2-one

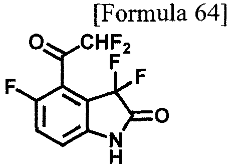

Reaction was carried out in substantially the same manner as in Production Example 1 except that 4-bromo-3,3-difluoro-1,3-dihydro-2H-indol-2-one was replaced by 3,3,5-trifluoro-1,3-dihydro-2H-indol-2-one, and ethyl trifluoroacetate by ethyl difluoroacetate; as a result, the titled compound was obtained as a yellow powder in an amount of 62 mg (yield: 22%).
(ESI neg.) m/z : 264(M-H)-
LCMS RT 0.651, Condition A
1H NMR (600 MHz, CHLOROFORM-d) d ppm 6.22 - 6.48 (1 H, m), 7.12 - 7.19 (1 H, m), 7.37 (1 H, t, J=9.50 Hz), 7.73 (1 H, br. s.)

### Production Example 55: 4-(2,2-Difluoro-1-hydroxyethyl)-3,3,5-trifluoro-1,3-dihydro-2H-indol-2-one

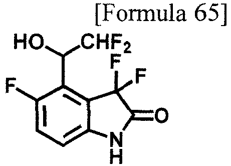

Reaction was carried out in substantially the same manner as in Production Example 2 except that 3,3-difluoro-4-(trifluoroacetyl)-1,3-dihydro-2H-indol-2-one was replaced by the 4-(difluoroacetyl)-3,3,5-trifluoro-1,3-dihydro-2H-indol-2-one obtained in Production Example 54, and the titled compound was obtained as a yellow oil in an amount of 56 mg (yield: 90%).
(ESI pos.) m/z : 268(M+H)+
LCMS RT 0.636, Condition A
1H NMR (600 MHz, CHLOROFORM-d) d ppm 2.75 - 2.80 (1 H, m), 5.18 - 5.27 (1 H, m), 5.94 - 6.18 (1 H, m), 6.88 - 6.94 (1 H, m), 7.21 - 7.25 (1 H, m), 7.35 (1 H, br. s.)

### Production Example 56: 3,3-Difluoro-1-[(3-fluoro-1-oxidopyridin-4-yl)methyl]-4-[(1S)-1-hydroxyethyl]-1,3-dihydro-2H-indol-2-one

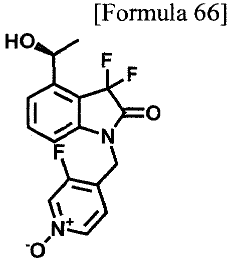

To a chloroform solution (2 ml) of 3,3-difluoro-1-[(3-fluoropyridin-4-yl)methyl]-4-[(1S)-1-hydroxyethyl]-1,3-dihydro-2H-indol-2-one (144 mg, 0.447 mmol), 3-chloroperbenzoic acid (ca. 77%, 300 mg, 1.34 mmol) was added under cooling with ice and the resulting mixture was stirred at room temperature for 4 hours. To the reaction mixture, a saturated aqueous solution of sodium thiosulfate was added to arrest the reaction and extraction was conducted with chloroform. After drying the organic layer over anhydrous magnesium sulfate, the insoluble matter was separated by filtration and concentrated under reduced pressure. The titled compound was obtained as a pale yellow powder in an amount of 177 mg.
(ESI pos.) m/z : 337(M-H)+
1H NMR (600 MHz, DMSO-d6) d ppm 1.35 (3 H, d, J=6.2 Hz), 4.94 - 5.04 (3 H, m), 5.40 - 5.52 (1 H, m), 6.97 - 7.07 (1 H, m), 7.33 - 7.45 (2 H, m), 7.50 - 7.61 (1 H, m), 8.06 - 8.16 (1 H, m), 8.49 - 8.59(1 H, m)

### Production Example 57: (1S)-1-{1-[(5-Cyclopropylpyridin-3-yl)methyl]-3,3-difluoro-2-oxo-2,3-dihydro-1H-indol-4-yl}ethyl benzoate

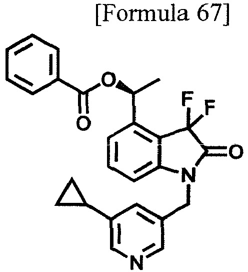

Reaction was carried out in substantially the same manner as in Production Example 49 except that 3-(chloromethyl)-5-(1H-1,2,3-triazol-1-yl)pyridine was replaced by 3-(chloromethyl)-5-(cyclopentyl)pyridine hydrochloride, and the titled compound was obtained in an amount of 39 mg (yield: 67%).
(ESI pos.) m/z : 449(M+H)+
LCMS RT 1.051, Condition A
1H NMR (600 MHz, CHLOROFORM-d) d ppm 0.66 - 0.75 (2 H, m), 1.00 - 1.07 (2 H, m), 1.71 (3 H, d, J=6.61 Hz), 1.81 - 1.92 (1 H, m), 4.78 - 4.90 (2 H, m), 6.33 (1 H, q, J=6.61 Hz), 6.72 (1 H, d, J=7.84 Hz), 7.23 - 7.31 (2 H, m), 7.3 6 - 7.49 (3 H, m), 7.54 - 7.61 (1 H, m), 8.09 (2 H, d, J=7.43 Hz), 8.28 - 8.35 (1 H, m), 8.39 (1 H, d, J=2.06 Hz)

### Production Example 58: 3,3,5-Trifluoro-2-oxo-2,3-dihydro-1H-indole-4-carbaldehyde

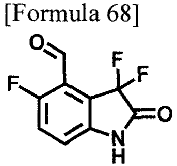

Reaction was carried out in substantially the same manner as in Production Example 1 except that 4-bromo-3,3-difluoro-1,3-dihydro-2H-indol-2-one was replaced by 3,3,5-trifluoro-1,3-dihydro-2H-indol-2-one, and ethyl trifluoroacetate by N,N-dimethylformamide; as a result, the titled compound was obtained as a pale brown solid in an amount of 210 mg (yield: 18%).
(ESI neg.) m/z : 214(M-H)-
1H NMR (600 MHz, DMSO-d6) d ppm 7.28 - 7.35 (1 H, m), 7.63 (1 H, dd, J=10.9, 8.9 Hz), 10.25(1 H, s), 11.19 - 11.54(1 H, m)

### Production Example 59: 3,3,5-Trifluoro-4-(1-hydroxyethyl)-1,3-dihydro-2H-indol-2-one

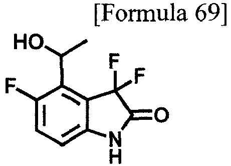

Reaction was carried out in substantially the same manner as in Production Example 27 except that 4-acetyl-3,3-difluoro-1,3-dihydro-2H-indol-2-one was replaced by the 3,3,5-trifluoro-2-oxo-2,3-dihydro-1H-indole-4-carbaldehyde obtained in Production Example 58, and the titled compound was obtained as a pale yellow solid in an amount of 145 mg (yield: 64%).
(ESI neg.) m/z : 230(M-H)-
1H NMR (600 MHz, DMSO-d6) d ppm 1.41 (3 H, d, J=6.6 Hz), 4.97 - 5.09 (1 H, m), 5.40 - 5.55 (1 H, m), 6.80 - 6.90 (1 H, m), 7.27-7.35 (1 H, m), 10.90 - 11.40(1 H, m)

Production Example 60: 3,3,5-Trifluoro-4-(1-{[(3aR,6aR)-3a-(prop-2-en-1-yl)hexahydro-6aH-cyclopenta[b]furan-6a-yl]oxy} ethyl)-1,3-dihydro-2H-indol-2-one

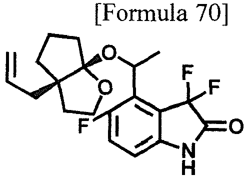

Reaction was carried out in substantially the same manner as in Production Example 15 except that 3,3-difluoro-4-(2,2,2-trifluoro-1-hydroxyethyl)-1,3-dihydro-2H-indol-2-one was replaced by the 3,3,5-trifluoro-4-(1-hydroxyethyl)-1,3-dihydro-2H-indol-2-one obtained in Production Example 59, and the titled compounds, i.e., (diastereomer 1) 3,3,5-trifluoro-4-(1-{[(3aR,6aR)-3a-(prop-2-en-1-yl)hexahydro-6aH-cyclopenta[b]furan-6a-yl]oxy}ethyl)-1,3-dihydro-2H-indol-2-one and (diastereomer 2) 3,3,5-trifluoro-4-(1-{[(3aR,6aR)-3a-(prop-2-en-1-yl)hexahydro-6aH-cyclopenta[b]furan-6a-yl]oxy}ethyl)-1,3-dihydro-2H-indol-2-one, were obtained each as a pale yellow powder and in respective amounts of 78 mg (yield: 33%) and 68 mg(yield: 28%).

### Data for diastereomer 1

(ESI pos.) m/z : 404(M+Na)+
1H NMR (600 MHz, DMSO-d6) d ppm 1.09 - 1.21 (1 H, m), 1.32-1.51 (5 H, m), 1.63 - 1.71 (2 H, m), 1.73 - 1.82 (2 H, m), 1.88 - 1.95 (1 H, m), 2.02 - 2.09 (1 H, m), 2.22 - 2.30 (1 H, m), 3.68 - 3.78(1 H, m), 3.80 - 3.89 (1 H, m), 4.95 - 5.10 (2 H, m), 5.20 - 5.28 (1 H, m), 5.80 - 5.91 (1 H, m), 6.84-6.91 (1 H, m), 7.31 - 7.38 (1 H, m), 11.06 - 11.49 (1 H, m)

### Data for diastereomer 2

(ESI pos.) m/z : 404(M+Na)+
1H NMR (600 MHz, DMSO-d6) d ppm 1.37-1.51 (5 H, m), 1.54-1.67 (4 H, m), 1.75 - 1.84 (1 H, m), 2.05 - 2.17 (2 H, m), 2.19 - 2.30 (1 H, m), 3.17-3.27 (1 H, m), 3.46 - 3.57 (1 H, m), 4.95 - 5.12 (3 H, m), 5.81 - 5.92 (1 H, m), 6.78 - 6.88 (1 H, m), 7.26 - 7.35 (1 H, m), 11.04 - 11.32 (1 H, m)

### Production Example 61: (Enantiomer 1) 3,3,5-Trifluoro-4-(1-hydroxyethyl)-1,3-dihydro-2H-indol-2-one

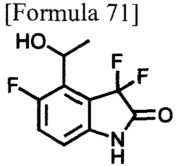

Reaction was carried out in substantially the same manner as in Production Example 16 except that 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one was replaced by the (diastereomer 1) 3,3,5-trifluoro-4-(1-{[(3aR,6aR)-3a-(prop-2-en-1-yl)hexahydro-6aH-cyclopenta[b]furan-6a-yl]oxy}ethyl)-1,3-dihydro-2H-indol-2-one obtained in Production Example 60, and the titled compound was obtained as a pale brown powder in an amount of 30 mg (yield: 64%).
(ESI neg.) m/z : 230(M-H)-
1H NMR (600 MHz, DMSO-d6) d ppm 1.40 (3 H, d, J=6.6 Hz), 4.95 - 5.08 (1 H, m), 5.41 - 5.52 (1 H, m), 6.81 - 6.90 (1 H, m), 7.24 - 7.35 (1 H, m), 10.99 - 11.37 (1 H, m)

### Production Example 62: (Enantiomer 2) 3,3,5-Trifluoro-4-(1-hydroxyethyl)-1,3-dihydro-2H-indol-2-one

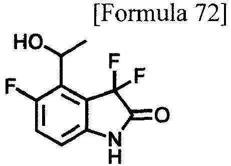

Reaction was carried out in substantially the same manner as in Production Example 16 except that 3,3-difluoro-4-[(1R)-2,2,2-trifluoro-1-{[(3aR,6aR)-3a-(prop-2-en-1-yl)hexahydro-6aH-cyclopenta[b]furan-6a-yl]oxy}ethyl]-1,3-dihydro-2H-indol-2-one was replaced by the (diastereomer 2) 3,3,5-trifluoro-4-(1-{[(3aR,6aR)-3a-(prop-2-en-1-yl)hexahydro-6aH-cyclopenta[b]furan-6a-yl]oxy}ethyl)-1,3-dihydro-2H-indol-2-one obtained in Production Example 60, and the titled compound was obtained as a pale brown powder in an amount of 30 mg (yield: 74%).
(ESI neg.) m/z : 230(M-H)-
1H NMR (600 MHz, DMSO-d6) d ppm 1.40 (3 H, d, J=6.6 Hz), 4.99 - 5.07 (1 H, m), 5.44 - 5.51 (1H,m), 6.81 - 6.89 (1 H, m), 7.27 - 7.35(1 H, m), 11.04-11.31(1 H, m)

### Production Example 63: 1-[(2-Chloro-1-oxidopyridin-4-yl)methyl]-3,3-difluoro-4-[(1S)-1-hydroxyethyl]-1,3-dihydro-2H-indol-2-one

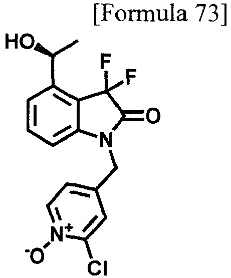

Reaction was carried out in substantially the same manner as in Production Example 56 except that 3,3-difluoro-1-[(3-fluoropyridin-4-yl)methyl]-4-[(1S)-1-hydroxyethyl]-1,3-dihydro-2H-indol-2-one was replaced by 1-[(2-chloropyridin-4-yl)methyl]-3,3-difluoro-4-[(1S)-1-hydroxyethyl]-1,3-dihydro-2H-indol-2-one, and the titled compound was obtained as a colorless amorphous in an amount of 116 mg (yield: 85%).
(ESI pos.) m/z : 355(M+H)+
1H NMR (600 MHz, CHLOROFORM-d) d ppm 1.55 (3 H, d, J=6.2 Hz), 1.99 - 2.09 (1 H, m), 4.84 (2 H, d, J=2.5 Hz), 5.26 - 5.35 (1 H, m), 6.63 (1 H, d, J=7.8 Hz), 7.08 - 7.16 (1 H, m), 7.39 - 7.44 (2 H, m), 7.45 - 7.51 (1 H, m), 8.30 (1 H, d, J=6.6 Hz)

### Production Example 64: 5-Fluoro-4-(hydroxymethyl)pyridine-2-carbonitrile

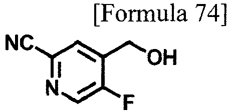

To an N,N-dimethylformamide solution (15 ml) of (2-bromo-5-fluoropyridin-4-yl)methanol (1.0 g, 4.85 mmol) and zinc cyanide (1.71 g, 14.56 mmol), tetrakis(triphenylphosphine)palladium(0) (542 mg, 0.485 mmol) was added and the resulting mixture was stirred at 150°C for an hour. After being left to cool, the reaction mixture was filtered and the filtrate was diluted with ethyl acetate and washed with water and brine. After dying the organic layer over anhydrous magnesium sulfate, the insoluble matter was separated by filtration and concentrated under reduced pressure. The resulting residue was purified by neutral, OH-form silica gel column chromatography (hexane/ethyl acetate = 80:20 to 20:80). The titled compound was obtained as a pale yellow powder in an amount of 527 mg (yield: 71%).
(ESI pos.) m/z : 153(M+H)+
1H NMR (600 MHz, CHLOROFORM-d) d ppm 2.10 - 2.19 (1 H, m), 4.89 (2 H, d, J=5.4 Hz), 7.98 (1 H, d, J=5.4 Hz), 8.49 (1 H, d, J=1.2 Hz)

### Production Example 65: 4-(Chloromethyl)-5-fluoropyridine-2-carbonitrile

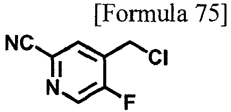

Reaction was carried out in substantially the same manner as in Production Example 38 except that [6-(2H-1,2,3-triazol-2-yl)pyridin-2-yl]methanol was replaced by the 5-fluoro-4-(hydroxymethyl)pyridine-2-carbonitrile obtained in Production Example 64, and the titled compound was obtained as a pale brown oil in an amount of 590 mg.
(ESI pos.) m/z : 171(M+H)+
1H NMR (600 MHz, CHLOROFORM-d) d ppm 4.64 (2 H, s), 7.89 (1 H, d, J=5.8 Hz), 8.57 (1 H, d, J=0.8 Hz)

### Production Example 66: Methyl 5-(difluoromethoxy)pyridine-3-carboxylate

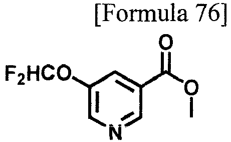

To an acetonitrile solution (20 ml) of methyl 5-hydroxypyridine-3-carboxylate (1.02 g, 6.66 mmol), sodium chlorodifluoroacetate (1.52 g, 9.99 mmol) and potassium carbonate (2.30 g, 16.65 mmol) were added and the resulting mixture was heated under reflux overnight. After leaving the reaction mixture to cool, a saturated aqueous solution of ammonium chloride was added and extraction was conducted with ethyl acetate. After drying the organic layer over anhydrous magnesium sulfate, the insoluble matter was separated by filtration and concentrated under reduced pressure. The resulting residue was purified by neutral, OH-form silica gel column chromatography (hexane/ethyl acetate = 80:20 to 20:80). The titled compound was obtained as a pale yellow oil in an amount of 217 mg (yield: 16%).
(ESI pos.) m/z : 204(M+H)+
LCMS RT 0.718, Condition A
1H NMR (600 MHz, CHLOROFORM-d) d ppm 3.98 (3 H, s), 6.39 - 6.77 (1 H, m), 8.07 (1 H, s), 8.66 (1 H, d, J=2.89 Hz), 9.09 (1 H, d, J=1.65 Hz)

### Production Example 67: [5-(Difluoromethoxy)pyridin-3-yl]methanol

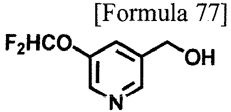

Reaction was carried out in substantially the same manner as in Production Example 2 except that 3,3-difluoro-4-(trifluoroacetyl)-1,3-dihydro-2H-indol-2-one was replaced by the methyl 5-(difluoromethoxy)pyridine-3-carboxylate obtained in Production Example 66, and the titled compound was obtained as a colorless oil in an amount of 91 mg (yield: 72%).
(ESI pos.) m/z : 176(M+H)+
LCMS RT 0.654, Condition B
1H NMR (600 MHz, CHLOROFORM-d) d ppm 1.87 (1 H, br. s.), 4.79 (2 H, s), 6.38 - 6.73 (1 H, m), 7.56 (1 H, s), 8.42 (1 H, d, J=2.48 Hz), 8.47 (1 H, s)

### Production Example 68: 3-(Chloromethyl)-5-(difluoromethoxy)pyridine

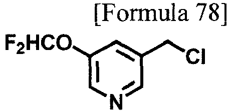

Reaction was carried out in substantially the same manner as in Production Example 38 except that [6-(2H-1,2,3-triazol-2-yl)pyridin-2-yl]methanol was replaced by the [5-(difluoromethoxy)pyridin-3-yl]methanol obtained in Production Example 67, and the titled compound was obtained as a white solid in an amount of 59 mg.
(ESI pos.) m/z : 194(M+H)+
LCMS RT 0.820, Condition A
1H NMR (600 MHz, CHLOROFORM-d) d ppm 4.72 (2 H, s), 6.56 - 6.90 (1 H, m), 8.06 (1 H, s), 8.59 (1 H, br. s.), 8.68 (1 H, br. s.)

### Production Example 69: 1-[(3-Chloro-1-oxidopyridin-4-yl)methyl]-3,3-difluoro-4-[(1S)-1-hydroxyethyl]-1,3-dihydro-2H-indol-2-one

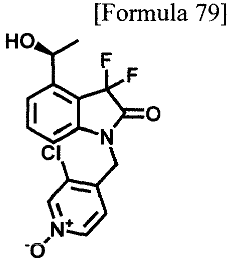

Reaction was carried out in substantially the same manner as in Production Example 56 except that 3,3-difluoro-1-[(3-fluoropyridin-4-yl)methyl]-4-[(1S)-1-hydroxyethyl]-1,3-dihydro-2H-indol-2-one was replaced by 1-[(3-chloropyridin-4-yl)methyl]-3,3-difluoro-4-[(1S)-1-hydroxyethyl]-1,3-dihydro-2H-indol-2-one, and the titled compound was obtained as a pale yellow amorphous in an amount of 147 mg (yield: quant).
(ESI neg.) m/z : 353(M-H)-
1H NMR (600 MHz, CHLOROFORM-d) d ppm 1.55 (3 H, d, J=6.6 Hz), 2.04 (1 H, s), 4.95 (2 H, d, J=1.7 Hz), 5.27 - 5.35 (1 H, m), 6.64 (1 H, d, J=7.4 Hz), 7.05 (1 H, d, J=6.6 Hz), 7.41 -7.45(1 H, m), 7.45 - 7.50 (1 H, m), 8.03 - 8.08 (1 H, m), 8.30 (1 H, d, J=1.7 Hz)

### Production Example 70: 5-Chloro-4-(hydroxymethyl)pyridine-2-carbonitrile

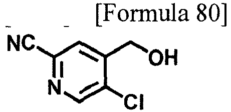

Reaction was carried out in substantially the same manner as in Production Example 64 except that (2-bromo-5-fluoropyridin-4-yl)methanol was replaced by (2-bromo-5-chloropyridin-4-yl)methanol, and the titled compound was obtained as a pale yellow solid in an amount of 577 mg (yield: 61%).
(ESI neg.) m/z : 167(M-H)-
1H NMR (600 MHz, CHLOROFORM-d) d ppm 2.10 - 2.19 (1 H, m), 4.86 (2 H, d, J=5.0 Hz), 7.98 (1 H, s), 8.60 (1 H, s)

### Production Example 71: 5-Chloro-4-(chloromethyl)pyridine-2-carbonitrile

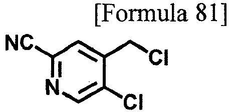

Reaction was carried out in substantially the same manner as in Production Example 38 except that [6-(2H-1,2,3-triazol-2-yl)pyridin-2-yl]methanol was replaced by the 5-chloro-4-(hydroxymethyl)pyridine-2-carbonitrile obtained in Production Example 70, and the titled compound was obtained as a pale brown oil in an amount of 709 mg.
(ESI neg.) m/z : 185(M-H)-
1H NMR (600 MHz, CHLOROFORM-d) d ppm 4.68 (2 H, s), 7.90 (1 H, s), 8.69 (1 H, s)

### Production Example 72: 1-[(5-Chloro-1-oxidopyridin-2-yl)methyl]-3,3-difluoro-4-[(1S)-1-hydroxyethyl]-1,3-dihydro-2H-indol-2-one

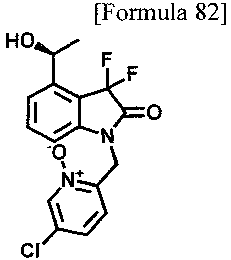

Reaction was carried out in substantially the same manner as in Production Example 56 except that 3,3-difluoro-1-[(3-fluoropyridin-4-yl)methyl]-4-[(1S)-1-hydroxyethyl]-1,3-dihydro-2H-indol-2-one was replaced by 1-[(5-chloropyridin-2-yl)methyl]-3,3-difluoro-4-[(1S)-1-hydroxyethyl]-1,3-dihydro-2H-indol-2-one, and the titled compound was obtained in an amount of 58 mg (yield: quant).
(ESI neg.) m/z : 353(M-H)-
1H NMR (600 MHz, CHLOROFORM-d) d ppm 1.54 (3 H, d, J=6.2 Hz), 1.92 - 2.00 (1 H, m), 5.13 (2 H, s), 5.22 - 5.33 (1 H, m), 6.98 - 7.06 (1 H, m), 7.21 - 7.25 (2 H, m), 7.35 - 7.41 (1H, m), 7.45 - 7.53 (1 H, m), 8.32 (1 H, s)

### production Example 73: 2-({[tert-Butyl(dimethyl)silyl]oxy}methyl)-5-(trifluoromethyl)pyridine

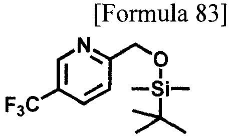

To a chloroform solution (5 ml) of [5-(trifluoromethyl)pyridin-2-yl]methanol (185 mg, 1.04 mmol) and 1H-imidazole (107 mg, 1.57 mmol), tert-butyldimethylchlorosilane (0.271 ml, 1.57 mmol) was added under cooling with ice and the resulting mixture was stirred overnight. The reaction mixture was washed with water. The organic layer was dried and concentrated under reduced pressure. The resulting residue was purified by neutral, OH-form silica gel column chromatography (hexane/ethyl acetate = 99:1 to 95:5). The titled compound was obtained as a pale yellow oil in an amount of 299 mg (yield: 99%).
(ESI pos.) m/z : 292(M+H)+
LCMS RT 1.476, Condition A
1H NMR (600 MHz, CHLOROFORM-d) d ppm 0.14 (6 H, s), 0.97 (9 H, s), 4.88 (2 H, s), 7.66 (1 H, d, J=8.3 Hz), 7.94 (1 H, dd, J=8.3, 2.1 Hz), 8.77 (1 H, s)

### Production Example 74: 2-({[tert-Butyl(dimethyl)silyl]oxy}methyl)-5-(trifluoromethyl)pyridine 1-oxide

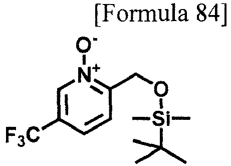

Reaction was carried out in substantially the same manner as in Production Example 56 except that 3,3-difluoro-1-[(3-fluoropyridin-4-yl)methyl]-4-[(1S)-1-hydroxyethyl]-1,3-dihydro-2H-indol-2-one was replaced by the 2-({[tert-butyl(dimethyl)silyl]oxy}methyl)-5-(trifluoromethyl)pyridine obtained in Production Example 73, and the titled compound was obtained as a pale yellow oil in an amount of 307 mg (yield: 97%).
(ESI pos.) m/z : 308(M+H)+
LCMS RT 1.255, Condition A
1H NMR (600 MHz, CHLOROFORM-d) d ppm 0.14 - 0.19 (6 H, m), 0.94 - 0.99 (9 H, m), 4.93 (2 H, s), 7.53 (1 H, d, J=8.3 Hz), 7.72 (1 H, d, J=7.8 Hz), 8.45 - 8.52 (1 H, m)

### Production Example 75: 6-({[tert-Butyl(dimethyl)silyl]oxy}methyl)-3-(trifluoromethyl)pyridine-2-carbonitrile

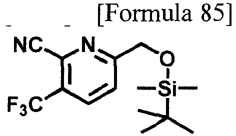

To a chloroform solution (5 ml) of the 2-({[tert-butyl(dimethyl)silyl]oxy}methyl)-5-(trifluoromethyl)pyridine 1-oxide (307 mg, 1.00 mmol) obtained in Production Example 74 and trimethylsilyl anilide (1.12 ml, 9.00 mmol), diethylcarbamoyl chloride (0.624 ml, 9.00 mmol) was added and the resulting mixture was stirred at 60°C for 6 hours. The reaction mixture was washed with a saturated aqueous solution of sodium hydrogencarbonate and the organic layer was dried, then concentrated under reduced pressure. The resulting residue was purified by neutral, OH-form silica gel column chromatography (hexane/ethyl acetate = 100:0 to 94:6). The titled compound was obtained as a pale yellow oil in an amount of 304 mg (yield: 68%).
(ESI pos.) m/z : 317(M+H)+
LCMS RT 1.456, Condition A
1H NMR (600 MHz, CHLOROFORM-d) d ppm 0.15 (6 H, s), 0.97 (9 H, s), 4.90 (2 H, s), 7.90 (1 H, d, J=8.3 Hz), 8.12 (1 H, d, J=8.3 Hz)

### Production Example 76: 6-(Hydroxymethyl)-3-(trifluoromethyl)pyridine-2-carbonitrile

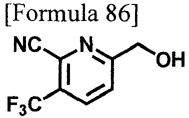

To an ethyl acetate solution (5 ml) of the 6-({[tert-butyl(dimethyl) silyl]oxy}methyl)-3-(trifluoromethyl)pyridine-2-carbonitrile (304 mg, 0.676 mmol) obtained in Production Example 75, HCl (4M ethyl acetate solution, 5 ml) was added and the resulting mixture was stirred at room temperature for 2 hours. The reaction mixture was concentrated under reduced pressure. The resulting residue was purified by neutral, OH-form silica gel column chromatography (hexane/ethyl acetate = 90:10 to 20:80). The titled compound was obtained as a pale yellow oil in an amount of 90 mg (yield: 68%).
(ESI neg.) m/z : 201 (M-H)-
LCMS RT 0.730, Condition A
1H NMR (600 MHz, CHLOROFORM-d) d ppm 2.80 (1 H, t, J=5.6 Hz), 4.92 (2 H, d, J=5.4 Hz), 7.76 (1 H, d, J=9.1 Hz), 8.13 (1 H, d, J=8.3 Hz)

### Production Example 77: 6-(Chloromethyl)-3-(trifluoromethyl)pyridine-2-carbonitrile

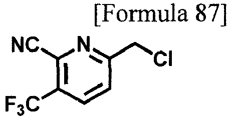

Reaction was carried out in substantially the same manner as in Production Example 38 except that [6-(2H-1,2,3-triazol-2-yl)pyridin-2-yl]methanol was replaced by the 6-(hydroxymethyl)-3-(trifluoromethyl)pyridine-2-carbonitrile obtained in Production Example 76, and the titled compound was obtained as a pale brown oil in an amount of 90 mg. LCMS RT 1.010, Condition A

### Production Example 78: 2-({[tert-Butyl(dimethyl)silyl]oxy}methyl)-5-methoxypyridine

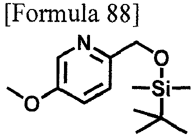

Reaction was carried out in substantially the same manner as in Production Example 73 except that [5-(trifluoromethyl)pyridin-2-yl]methanol was replaced by (5-methoxypyridin-2-yl)methanol, and the titled compound was obtained as a colorless oil in an amount of 463 mg (yield: quant).
(ESI pos.) m/z : 254(M+H)+
LCMS RT 1.149, Condition A
1H NMR (600 MHz, CHLOROFORM-d) d ppm 0.11 (6 H, s), 0.95 (9 H, s), 3.85 (3 H, s), 4.78 (2 H, s), 7.22 (1 H, dd, J=8.7, 2.9 Hz), 7.42 (1 H, d, J=8.7 Hz), 8.21 (1 H, d, J=2.9 Hz)

### Production Example 79: 2-({[tert-Butyl(dimethyl)silyl]oxy}methyl)-5-methoxypyridine 1-oxide

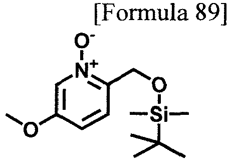

Reaction was carried out in substantially the same manner as in Production Example 74 except that 2-({[tert-butyl(dimethyl)silyl]oxy}methyl)-5-(trifluoromethyl)pyridine was replaced by the 2-({[tert-butyl(dimethyl)silyl]oxy}methyl)-5-methoxypyridine obtained in Production Example 78, and the titled compound was obtained as a colorless oil in an amount of 454 mg.
(ESI pos.) m/z : 270(M+H)+
LCMS RT 1.109, Condition A
1H NMR (600 MHz, CHLOROFORM-d) d ppm 0.12 - 0.16 (6 H, m), 0.93 - 0.98 (9 H, m), 3.83 (3 H, s), 4.89 (2 H, s), 6.96 (1 H, dd, J=8.9, 2.3 Hz), 7.44 (1 H, d, J=9.1 Hz), 7.98 (1 H, d, J=2.1 Hz)

### Production Example 80: 6-({[tert-Butyl(dimethyl)silyl]oxy}methyl)-3-methoxypyridine-2-carbonitrile

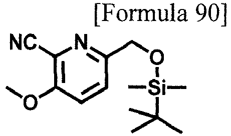

Reaction was carried out in substantially the same manner as in Production Example 74 except that 2-({[tert-butyl(dimethyl)silyl]oxy}methyl)-5-(trifluoromethyl)pyridine 1-oxide was replaced by the 2-({[tert-butyl(dimethyl)silyl]oxy}methyl)-5-methoxypyridine 1-oxide obtained in Production Example 79, and the titled compound was obtained as a colorless solid in an amount of 335 mg (yield: 84%).
(ESI pos.) m/z : 279(M+H)+
LCMS RT 1.353, Condition A
1H NMR (600 MHz, CHLOROFORM-d) d ppm 0.11 (6 H, s), 0.95 (9 H, s), 3.96 (3 H, s), 4.77 (2 H, s), 7.38 (1 H, d, J=8.7 Hz), 7.69 (1 H, d, J=9.1 Hz)

### Production Example 81: 6-(Hydroxymethyl)-3-methoxypyridine-2-carbonitrile

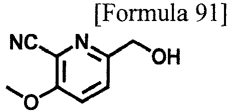

Reaction was carried out in substantially the same manner as in Production Example 74 except that 6-({[tert-butyl(dimethyl)silyl]oxy}methyl)-3-(trifluoromethyl)pyridine-2-carbonitrile was replaced by the 6-({[tert-butyl(dimethyl)silyl]oxy}methyl)-3-methoxypyridine-2-carbonitrile obtained in Production Example 80, and the titled compound was obtained as a colorless solid in an amount of 183 mg (yield: 76%).
(ESI neg.)m/z: 163(M-H)-
LCMS RT 0.459, Condition A
1H NMR (600 MHz, DMSO-d6) d ppm 3.96 (3 H, s), 4.51 (2 H, s), 7.73 - 7.76 (1 H, m), 7.79 -7.82(1 H, m)

### Production Example 82: 6-(Chloromethyl)-3-methoxypyridine-2-carbonitrile

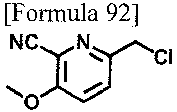

Reaction was carried out in substantially the same manner as in Production Example 38 except that [6-(2H-1,2,3-triazol-2-yl)pyridin-2-yl]methanol was replaced by the 6-(hydroxymethyl)-3-methoxypyridine-2-carbonitrile obtained in Production Example 81, and the titled compound was obtained as a colorless solid in an amount of 57 mg.
(ESI pos.) m/z : 183(M+H)+
LCMS RT 0.828, Condition A

### Production Example 83: 6-Chloro-3-(chloromethyl)-2-methoxypyridine

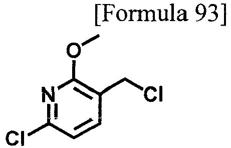

Reaction was carried out in substantially the same manner as in Production Example 38 except that [6-(2H-1,2,3-triazol-2-yl)pyridin-2-yl]methanol was replaced by the (6-chloro-2-methoxypyridin-3-yl)methanol, and the titled compound was obtained as a white solid in an amount of 68 mg.
LCMS RT 1.150, Condition A

### Production Example 84: 4-Bromo-6-fluoro-1H-indoline-2,3-dione

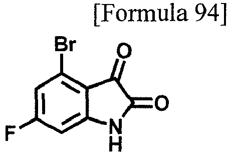

An acetonitrile/water (9:1) suspension (500 ml) of 4-bromo-6-fluoro-1H-indole (5.0 g, 23.4 mmol), cerium chloride (1.1 g, 0.23 mmol) and 2-iodoxybenzoic acid (16.4 g, 58.5 mmol) was stirred at 85°C for 4 hours. To the reaction mixture, ethyl acetate and water were added for extraction and the organic layer was dried, then concentrated under reduced pressure. The resulting residue was purified by neutral, OH-form silica gel column chromatography (hexane/ethyl acetate). The titled compound was obtained as as a brown solid in an amount of 2.0 g (yield: 35%).
(ESI neg.) m/z : 242(M-H)-
LCMS RT 0.752, Condition A
1H NMR (600 MHz, DMSO-d6) d ppm 6.73 - 6.79 (1 H, m), 7.18 - 7.24 (1 H, m), 11.29 - 11.36(1 H, m)

### Production Example 85: 4-Bromo-3,3,6-trifluoro-1,3-dihydro-2H-indol-2-one

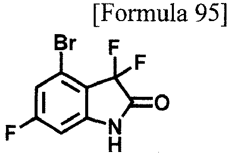

To a chloroform solution (1 ml) of 4-bromo-6-fluoro-1H-indoline-2,3-dione (50 mg, 0.20 mmol), N,N-diethylaminosulfur trifluoride (146 mg, 0.90 mmol) was added and the resulting mixture was stirred at room temperature overnight. Under cooling with ice, a saturated aqueous solution of sodium hydrogencarbonate was added to the reaction mixture to arrest the reaction and extraction was conducted with ethyl acetate. The organic layer was dried and then concentrated under reduced pressure. The resulting residue was purified by neutral, OH-form silica gel column chromatography (hexane/ethyl acetate). The titled compound was obtained as a pale brown solid in an amount of 30 mg (yield: 56%).
(ESI neg.) m/z : 264(M-H)-
LCMS RT 0.976, Condition A
1H NMR (600 MHz, DMSO-d6) d ppm 6.84 - 6.93 (1 H, m), 7.28 - 7.39 (1 H, m), 11.60 (1 H, br. s.)

### Production Example 86: 4-(Difluoroacetyl)-3,3,6-trifluoro-1,3-dihydro-2H-indol-2-one

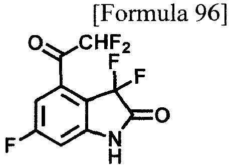

Reaction was carried out in substantially the same manner as in Production Example 1 except that 4-bromo-3,3-difluoro-1,3-dihydro-2H-indol-2-one was replaced by the 4-bromo-3,3,6-trifluoro-1,3-dihydro-2H-indol-2-one obtained in Production Example 85, and ethyl trifluoroacetate by ethyl difluoroacetate; as a result, the titled compound was obtained as a pale brown solid in an amount of 1.18 g (yield: 79%).
(ESI neg.) m/z : 264(M-H)-
LCMS RT 0.761, Condition A
1H NMR (600 MHz, DMSO-d6) d ppm 7.06 - 7.28 (2 H, m), 7.62 - 7.71 (1 H, m), 11.45-11.74(1H, m)

### Production Example 87: 4-(2,2-Difluoro-1-hydroxyethyl)-3,3,6-trifluoro-1,3-dihydro-2H-indol-2-one

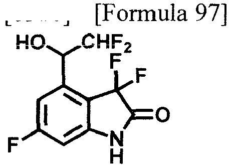

Reaction was carried out in substantially the same manner as in Production Example 2 except that 3,3-difluoro-4-(trifluoroacetyl)-1,3-dihydro-2H-indol-2-one was replaced by the 4-(difluoroacetyl)-3,3,6-trifluoro-1,3-dihydro-2H-indol-2-one obtained in Production Example 86, and the titled compound was obtained as a brown solid in an amount of 1.2 g.
(ESI neg.) m/z : 266(M-H)-
LCMS RT 0.764, Condition A
1H NMR (600 MHz, DMSO-d6) d ppm 4.80 - 4.94 (1 H, m), 5.94 - 6.24 (1 H, m), 6.66 - 6.75 (1 H, m), 6.79 - 6.89 (1 H, m), 7.00-7.10 (1 H, m), 11.35-11.70 (1 H, m)

### Production Example 88: 5-(Bromomethyl)-2-fluoro-3-methoxypyridine

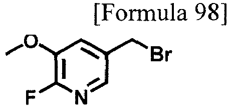

To a carbon tetrachloride solution (10 ml) of 2-fluoro-3-methoxy-5-methylpyridine (84 mg, 0.595 mmol), N-bromosuccinimide (127 mg, 0.714 mmol) and 2,2'-azobis(isobutyronitrile) (10 mg, 0.060 mmol) were added and the resulting mixture was stirred at 90°C for 3 hours. After leaving the mixture to cool, the insoluble matter was separated by filtration and the filtrate was concentrated under reduced pressure. The resulting residue was purified by neutral, OH-form silica gel column chromatography (hexane/ethyl acetate = 100:0 to 50:50). The titled compound was obtained as a white solid in an amount of 79 mg (yield: 60%).
(ESI pos.) m/z : 220(M+H)+
LCMS RT 0.886, Condition A
1H NMR (600 MHz, CHLOROFORM-d) d ppm 3.93 (3 H, s), 4.46 (2 H, s), 7.31 (1 H, dd, J=9.50, 2.06 Hz), 7.72 - 7.76 (1 H, m)

### Production Example 89: 4-(Difluoroacetyl)-3,3,7-trifluoro-1,3-dihydro-2H-indol-2-one

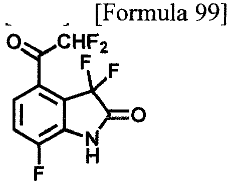

Reaction was carried out in substantially the same manner as in Production Example 1 except that 4-bromo-3,3-difluoro-1,3-dihydro-2H-indol-2-one was replaced by the 4-bromo-3,3,7-trifluoro-1,3-dihydro-2H-indol-2-one, and ethyl trifluoroacetate by ethyl difluoroacetate; as a result, the titled compound was obtained as a brown solid in an amount of 1.07 g.
(ESI neg.) m/z : 264(M-H)-
LCMS RT 0.834, Condition A
1H NMR (200 MHz, DMSO-d6) d ppm 6.85 - 7.47 (1 H, m), 7.66 - 7.92 (2 H, m)

### Production Example 90: 4-(2,2-Difluoro-1-hydroxyethyl)-3,3,7-trifluoro-1,3-dihydro-2H-indol-2-one

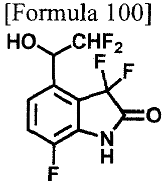

Reaction was carried out in substantially the same manner as in Production Example 2 except that 3,3-difluoro-4-(trifluoroacetyl)-1,3-dihydro-2H-indol-2-one was replaced by the 4-(difluoroacetyl)-3,3,7-trifluoro-1,3-dihydro-2H-indol-2-one obtained in Production Example 89, and the titled compound was obtained as a yellow oil in an amount of 880 mg (yield: 89%).
(ESI pos.) m/z : 268(M+H)+
LCMS RT 0.724, Condition A
1H NMR (200 MHz, DMSO-d6) d ppm 5.75 - 6.43 (1 H, m), 6.54 - 6.70 (1 H, m), 7.29 (1 H, dd, J=8.8, 4.4 Hz), 7.42 - 7.64 (1 H, m), 11.91 (1 H, br. s.)

### Production Example 91: 3-Chloro-4-(hydroxymethyl)pyridine-2-carbonitrile

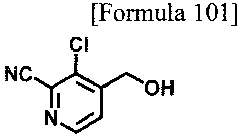

Reaction was carried out in substantially the same manner as in Production Example 64 except that (2-bromo-5-fluoropyridin-4-yl)methanol was replaced by (2,3-dichloropyridin-4-yl)methanol, and the titled compound was obtained as a white solid in an amount of 120 mg (yield: 62%).
(ESI neg.) m/z : 213(M+HCOO)-
LCMS RT 0.590, Condition A
1H NMR (600 MHz, DMSO-d6) d ppm 4.65 (2 H, d, J=5.0 Hz), 5.89 (1 H, t, J=5.6 Hz), 7.86 (1 H, d, J=5.0 Hz), 8.71 (1 H, d, J=4.5 Hz)

### Production Example 92: 3-Chloro-4-(chloromethyl)pyridine-2-carbonitrile

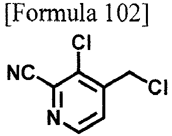

Reaction was carried out in substantially the same manner as in Production Example 38 except that [6-(2H-1,2,3-triazol-2-yl)pyridin-2-yl]methanol was replaced by the 3-chloro-4-(hydroxymethyl)pyridine-2-carbonitrile obtained in Production Example 91, and the titled compound was obtained as a brown amorphous in an amount of 56 mg.
LCMS RT 0.922, Condition A

### Production Example 93: 2-({[tert-Butyl(dimethyl)silyl]oxy}methyl)-5-chloropyridine

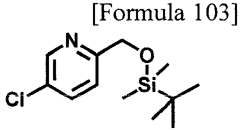

Reaction was carried out in substantially the same manner as in Production Example 73 except that [5-(trifluoromethyl)pyridin-2-yl]methanol was replaced by (5-chloropyridin-2-yl)methanol, and the titled compound was obtained as a colorless oil in an amount of 1.29 g (yield: quant).
(ESI pos.) m/z : 258(M+H)+
LCMS RT 1.494, Condition A
1H NMR (600 MHz, CHLOROFORM-d) d ppm 0.11 - 0.13 (6 H, m), 0.93 - 0.97 (9 H, m), 4.80 (2 H, s), 7.47 (1 H, d, J=8.3 Hz), 7.64 - 7.70 (1 H, m), 8.46 (1 H, d, J=2.5 Hz)

### Production Example 94: 2-({[tert-Butyl(dimethyl)silyl]oxy}methyl)-5-chloropyridine 1-oxide

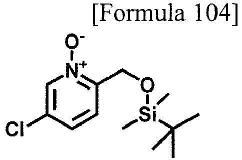

Reaction was carried out in substantially the same manner as in Production Example 74 except that 2-({[tert-butyl(dimethyl)silyl]oxy}methyl)-5-(trifluoromethyl)pyridine was replaced by the 2-({[tert-butyl(dimethyl)silyl]oxy}methyl)-5-chloropyridine obtained in Production Example 93, and the titled compound was obtained as a pale yellow oil in an amount of 1.21 g (yield: 93%).
(ESI pos.) m/z : 274(M+H)+
LCMS RT 1.235, Condition A
1H NMR (600 MHz, CHLOROFORM-d) d ppm 0.12-0.17 (6 H, m), 0.93 - 1.00 (9 H, m), 4.87 (2 H, s), 7.32 (1 H, dd, J=8.7, 1.7 Hz), 7.48 - 7.52 (1 H, m), 8.25 (1 H, d, J=1.7 Hz) [0330] Production Example 95: 6-({[tert-Butyl(dimethyl)silyl]oxy}methyl)-3-chloropyridine-2-carbonitrile

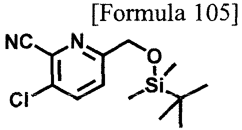

Reaction was carried out in substantially the same manner as in Production Example 75 except that 2-({[tert-butyl(dimethyl)silyl]oxy}methyl)-5-(trifluoromethyl)pyridine 1-oxide was replaced by the 2-({[tert-butyl(dimethyl)silyl]oxy}methyl)-5-chloropyridine 1-oxide obtained in Production Example 94, and the titled compound was obtained as a pale yellow oil in an amount of 2.21 g (yield: quant).
(ESI pos.) m/z : 283(M+H)+
LCMS RT 0.981, Condition A
NMR (600 MHz, CHLOROFORM-d) d ppm 0.10-0.15 (6 H, m), 0.92 - 0.98 (9 H, m), 4.78 - 4.82 (2 H, m), 7.71 (1 H, d, J=8.7 Hz), 7.85 (1 H, d, J=8.7 Hz)

### Production Example 96: 3-Chloro-6-(hydroxymethyl)pyridine-2-carbonitrile

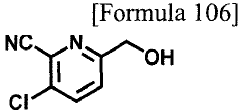

Reaction was carried out in substantially the same manner as in Production Example 76 except that 6-({[tert-butyl(dimethyl)silyl]oxy}methyl)-3-(trifluoromethyl)pyridine-2-carbonitrile was replaced by the 6-({[tert-butyl(dimethyl)silyl]oxy}methyl)-3-chloropyridine-2-carbonitrile obtained in Production Example 95, and the titled compound was obtained as a colorless solid in an amount of 355 mg (yield: 48%).
(ESI pos.) m/z : 169(M+H)+
LCMS RT 0.629, Condition A
1H NMR (600 MHz, CHLOROFORM-d) d ppm 2.82 (1 H, t, J=5.6 Hz), 4.81 (2 H, d, J=5.4 Hz), 7.55 (1 H, d, J=8.7 Hz), 7.87 (1 H, d, J=8.3 Hz)

### Production Example 97: 3-Chloro-6-(chloromethyl)pyridine-2-carbonitrile

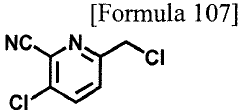

Reaction was carried out in substantially the same manner as in Production Example 38 except that [6-(2H-1,2,3-triazol-2-yl)pyridin-2-yl]methanol was replaced by the 3-chloro-6-(hydroxymethyl)pyridine-2-carbonitrile obtained in Production Example 6, and the titled compound was obtained as a pale yellow solid in an amount of 152 mg.
(ESI pos.) m/z : 187(M+H)+
LCMS RT 0.976, Condition A
1H NMR (600 MHz, CHLOROFORM-d) d ppm 4.67 (2 H, s), 7.71 (1 H, d, J=8.7 Hz), 7.90 (1 H, d, J=8.3 Hz)

### Production Example 98: 4-[(4-Bromo-3,3,7-trifluoro-2-oxo-2,3-dihydro-1H-indol-1-yl)methyl]pyridine-2-carbonitrile

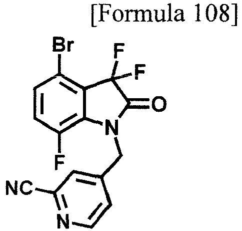

To an N,N-dimethylformamide solution (5 ml) of 4-bromo-3,3,7-trifluoro-1,3-dihydro-2H-indol-2-one (245 mg, 0.921 mmol), 4-(bromomethyl)pyridine-2-carbonitrile (218 mg, 1.11 mmol) and potassium carbonate (382 mg, 2.76 mmol) were added and the resulting mixture was stirred at room temperature for 15 hours. The reaction mixture was filtered and purified by preparative HPLC. The titled compound was obtained as a pale brown solid in an amount of 198 mg (yield: 56%).
(ESI pos.) m/z : 382(M+H)+
LCMS RT 1.100, Condition A
1H NMR (600 MHz, CHLOROFORM-d) d ppm 5.06 (2 H, s), 7.08 - 7.15 (1 H, m), 7.29 (1 H, dd, J=9.1, 3.7 Hz), 7.44 (1 H, d, J=4.5 Hz), 7.61 (1 H, s), 8.71(1 H, d, J=4.5 Hz)

### Production Example 99: 4-[(4-Acetyl-3,3,7-trifluoro-2-oxo-2,3-dihydro-1H-indol-1-yl)methyl]pyridine-2-carbonitrile

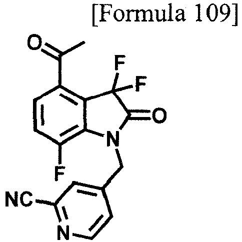

An N,N-dimethylformamide suspension (20 ml) of the 4-[(4-bromo-3,3,7-trifluoro-2-oxo-2,3-dihydro-1H-indol-1-yl)methyl]pyridine-2-carbonitrile (245 mg, 0.641 mmol) obtained in Production Example 98, (1-ethoxyethenyl)tributylstannane (0.286 ml, 0.769 mmol) and tetrakis(triphenylphosphine)palladium(0) (76 mg, 0.064 mmol) was stirred at 120°C for 3 hours. After leaving the suspension to cool, 1M HCl (1.28 ml, 1.28 mmol) was added and the resulting mixture was stirred at room temperature for an hour. To the reaction mixture, water was added and after extraction with ethyl acetate, the extract was washed with brine. The organic layer was dried in a phase separator and the insoluble matter was separated by filtration, then concentrated under reduced pressure. The resulting residue was purified by neutral, OH-form silica gel column chromatography (hexane/ethyl acetate = 88:12 to 0:100). The titled compound was obtained as a pale yellow oil in an amount of 336 mg (yield: quant).
(ESI pos.) m/z : 346(M+H)+
LCMS RT 0.935, Condition A
1H NMR (600 MHz, CHLOROFORM-d) d ppm 2.67 (3 H, s), 5.11 (2 H, s), 7.31 - 7.37 (1 H, m), 7.47 (1 H, dd, J=7.8, 2.9 Hz), 7.53 - 7.58 (1 H, m), 7.62 (1 H, s), 8.71 (1 H, d, J=5.4 Hz)

### Production Example 100: 3,3-Difluoro-4-[(1S)-1-hydroxyethyl]-1,3-dihydro-2H-indol-2-one

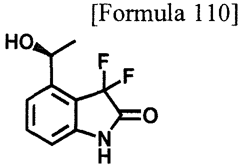

To a DMF solution of the 4-acetyl-3,3-difluoro-1 ,3-dihydro-2H-indol-2-one (50 mg, 0.237 mmol) obtained in Production Example 7, formic acid (0.044 ml, 1.18 mmol) and triethylamine (0.066 ml, 0.474 mmol), chloro[(1S,2S)-N-(p-toluenesulfonyl)-1,2-diphenylethanediamine](mesitylene)ruthenium(II) (4 mg, 0.007 mmol) was added and the resulting mixture was stirred at room temperature for 18 hours. To the reaction mixture, water was added and after extraction with ethyl acetate, the extract was washed with brine. After drying the organic layer over anhydrous magnesium sulfate, the insoluble matter was separated by filtration and concentrated under reduced pressure. The resulting residue was purified by neutral, OH-form silica gel column chromatography (hexane/ethyl acetate = 80:20 to 30:70). The titled compound was obtained as a pale yellow solid in an amount of 39 mg (yield: 83%; enantiomeric excess: 91%ee).

### Production Example 101: (Enantiomer 1)4-(2,2-Difluoro-1-hydroxyethyl)-3,3-difluoro-1,3-dihydro-2H-indol-2-one

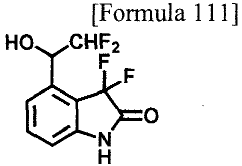

Reaction was carried out in substantially the same manner as in Production Example 100 except that 4-acetyl-3,3-difluoro-1,3-dihydro-2H-indol-2-one was replaced by the 4-(difluoroacetyl)-3,3-difluoro-1,3-dihydro-2H-indol-2-one obtained in Production Example 3, and chloro[(1S,2S)-N-(p-toluenesulfonyl)-1,2-diphenylethanediamine](mesitylene)ruthenium(II) by chloro[(1S,2S)-N-(p-toluenesulfonyl)-1,2-diphenyl-1,2-ethanediamine](p-cymene)ruthenium(II); as a result, the titled compound was obtained as a pale yellow solid in an amount of 50 mg (yield: 99%; enantiomeric excess: 77%ee).

### Production Example 102: 3,3-Difluoro-4-[(1R)-2,2,2-trifluoro-1-hydroxyethyl]-1,3-dihydro-2H-indol-2-one

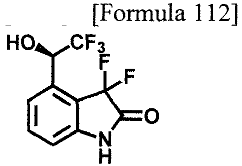

Reaction was carried out in substantially the same manner as in Production Example 100 except that 4-acetyl-3,3-difluoro-1,3-dihydro-2H-indol-2-one was replaced by the 3,3-difluoro-4-(trifluoroacetyl)-1,3-dihydro-2H-indol-2-one obtained in Production Example 1, and chloro[(1S,2S)-N-(p-toluenesulfonyl)-1,2-diphenylethanediamine](mesitylene)ruthenium(II) by chloro[(1R,2R)-N-(p-toluenesulfonyl)-1,2-diphenyl-1,2-ethanediamine](p-cymene)ruthenium(II); as a result, the titled compound was obtained as a pale yellow solid in an amount of 2.94 g (yield: 97%; enantiomeric excess: 53%ee).

### Example 1: 1-[(5-Chloropyridin-3-yl)methyl]-3,3-difluoro-4-(2,2,2-trifluoro-1-hydroxyethyl)-1,3-dihydro-2H-indol-2-one

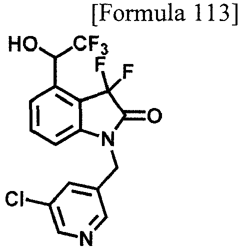

To an N,N-dimethylformamide solution (10 ml) of the 3,3-difluoro-4-(2,2,2-trifluoro-1-hydroxyethyl)-1,3-dihydro-2H-indol-2-one (250 mg, 0.936 mmol) obtained in Production Example 2, 3-chloro-5-(chloromethyl)pyridine (152 mg, 0.945 mmol) and potassium carbonate (265 mg, 1.92 mmol) were added and the resulting mixture was stirred at room temperature overnight. The reaction mixture was filtered and the filtrate was purified by preparative HPLC. As a result, the titled compound was obtained in an amount of 248 mg (yield: 67%). The resulting racemic mixture was resolved through a semi-preparative column under the aforementioned conditions for analysis of racemic compounds (column: DAICEL CHIRALPACK AD3; flow rate: 1.0 ml/min; mobile phase: hexane/ethanol = 70:30; (1) 4.1 min; (2) 4.5 min), and two enantiomers of the titled compound were obtained; enantiomer 1 (Faster (1) 4.1 min) in an amount of 97 mg and enantiomer 2 (Later (2) 4.5 min) in an amount of 98 mg.

### Example 2: 3,3-Difluoro-4-[(1S)-1-hydroxyethyl]-1-{[5-(1H-1,2,3-triazol-1-yl)pyridin-3-yl]methyl}-1,3-dihydro-2H-indol-2-one

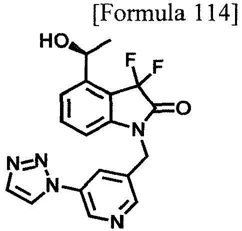

To a methanol solution (2 ml) of the (1S)-1-(3,3-difluoro-2-oxo-1-{[5-(1H-1,2,3-triazol-1-yl)pyridin-3-yl]methyl}-2,3-dihydro-1H-indol-4-yl)ethyl benzoate (82 mg, 0.172 mmol) obtained in Production Example 49, an aqueous solution of 1 M sodium hydroxide (0.259 ml, 0.259 mmol) was added and the resulting mixture was stirred at room temperature for 3 hours; after further adding an aqueous solution of 1M sodium hydroxide (0.300 ml, 0.300 mmol), the mixture was stirred at room temperature overnight. To the reaction mixture, an aqueous solution of 1 M HCl was added for neutralization and the mixture was concentrated under reduced pressure. The resulting residue was purified by preparative HPLC. As a result, the titled compound was obtained in an amount of 36 mg (yield: 56%).

### Example 3: 2-({3,3-Difluoro-4-[(1S)-1-hydroxyethyl]-2-oxo-2,3-dihydro-1H-indol-1-yl}methyl)pyridine-4-carbonitrile

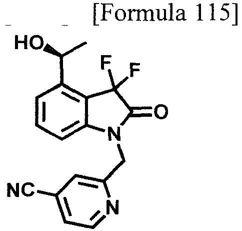

To an N,N-dimethylformamide solution (1 ml) of 1-[(4-bromopyridin-2-yl)methyl]-3,3-difluoro-4-[(1S)-1-hydroxyethyl]-1,3-dihydro-2H-indol-2-one (60 mg, 0.157 mmol) and zinc cyanide (55 mg, 0.470 mmol), tetrakis(triphenylphosphine)palladium(0) (4 mg, 0.031 mmol) was added and the resulting mixture was subjected to reaction in a microwave reactor at 150°C for an hour. The reaction mixture was filtered and purified by preparative HPLC. As a result of freeze-drying, the titled compound was obtained as a pale yellow powder in an amount of 24 mg (yield: 46.5%).

### Example 4: 3,3-Difluoro-4-[(1S)-1-hydroxyethyl]-1-(1H-indazol-3-ylmethyl)-1,3-dihydro-2H-indol-2-one

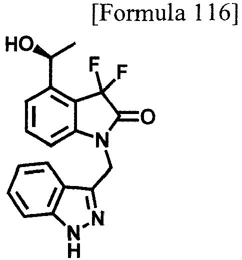

To a chloroform solution (2 ml) of the tert-butyl 3-({3,3-difluoro-4-[(1S)-1-hydroxyethyl]-2-oxo-2,3-dihydro-1H-indol-1-yl}methyl)-1H-indazole-1-carboxylate (95 mg, 0.214 mmol) obtained in Production Example 51, trifluoroacetic acid (1 ml) was added and the resulting mixture was stirred at room temperature for 3 hours. The reaction mixture was concentrated under reduced pressure and the resulting residue was purified by preparative HPLC and with a silica gel cartridge (hexane/ethyl acetate = 80:20 to 0:100). As a result, the titled compound was obtained in an amount of 37 mg (yield: 50%).

### Example 5: 3-({3,3-Difluoro-4-[(1S)-1-hydroxyethyl]-2-oxo-2,3-dihydro-1H-indol-1-yl}methyl)-2-methylisoquinolin-1 (2H)-one

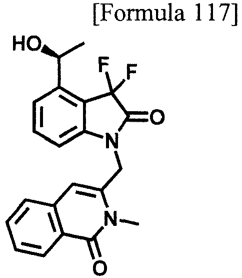

To an N,N-dimethylformamide solution (2 ml) of 3-({3,3-difluoro-4-[(1S)-1-hydroxyethyl]-2-oxo-2,3-dihydro-1H-indol-1-yl}methyl)isoquinolin-1(2H)-one (10 mg, 0.026 mmol), iodomethane (0.003 ml, 0.039 mmol) and potassium carbonate (7 mg, 0.051 mmol) were added and the resulting mixture was stirred at 80°C for 30 minutes. The reaction mixture was filtered and the filtrate was purified by preparative HPLC. As a result, the titled compound was obtained in an amount of 5 mg (yield: 52%).

### Example 6: 4-({3,3-Difluoro-4-[(1S)-1-hydroxyethyl]-2-oxo-2,3-dihydro-1H-indol-1-yl} methyl)-3-fluoropyridine-2-carbonitrile

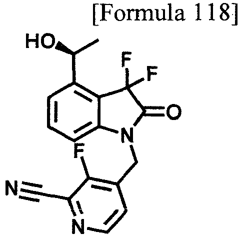

To a chloroform solution (1.5 ml) of the 3,3-difluoro-1-[(3-fluoro-1-oxidopyridin-4-yl)methyl]-4-[(1S)-1-hydroxyethyl]-1,3-dihydro-2H-indol-2-one (100 mg, 0.296 mmol) obtained in Production Example 56 and trimethylsilyl cyanide (0.063 ml, 0.887 mmol), diethylcarbamoyl chloride (0.111 ml, 0.887 mmol) was added and the resulting mixture was stirred at room temperature for 15 hours. After filtering the reaction mixture, the filtrate was purified by preparative HPLC. Treatment with diisopropyl ether gave the titled compound as a white powder in an amount of 61 mg (yield: 59%).

### Example 7: 3,3-Difluoro-4-[(1S)-1-hydroxyethyl]-1-{[6-(1H-1,2,4-triazol-1-yl)pyridin-3-yl]methyl}-1,3-dihydro-2H-indol-2-one

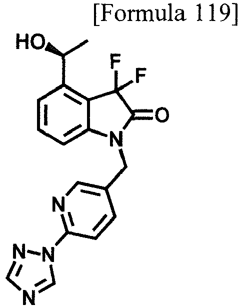

An N,N-dimethylformamide suspension (2 ml) of 1-[(6-bromopyridin-3-yl)methyl]-3,3-difluoro-4-[(1S)-1-hydroxyethyl]-1,3-dihydro-2H-indol-2-one (46 mg, 0.12 mmol), 4H-1,2,4-triazole (11 ml, 0.16 mmol), trans-N,N'-dimethylcyclohexane-1,2-diamine (0.004 ml, 0.024 mmol), copper(I) iodide (6 mg, 0.024 mmol) and cesium carbonate (78 mg, 0.24 mmol) was stirred at 150°C for 6 hours. After leaving the reaction mixture to cool, the insoluble matter was separated by filtration and purified by preparative HPLC and NH-form silica gel column chromatography (hexane/ethyl acetate = 80:20 to 0:100). The titled compound was obtained as a white powder in an amount of 7 mg (yield: 15%).

### Example 8: 1-[(5-Chloro-6-methoxypyridin-2-yl)methyl]-3,3-difluoro-4-[(1S)-1-hydroxyethyl]-1,3-dihydro-2H-indol-2-one

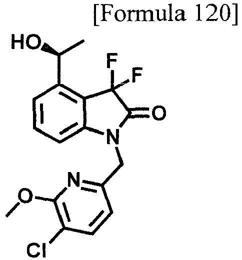

To methanol (2 ml), sodium hydride (60%, 37 mg, 0.938 mmol) was added and after stirring the mixture at room temperature for 30 minutes, 1-[(5,6-dichloropyridin-2-yl)methyl]-3,3-difluoro-4-[(1S)-1-hydroxyethyl]-1,3-dihydro-2H-indol-2-one (50 mg, 0.134 mmol) was added, followed by heating under reflux for 3 hours. After leaving the reaction mixture to cool, a saturated aqueous solution of ammonium chloride was added and extraction was conducted with chloroform; the organic layer was dried in a phase separator and then concentrated under reduced pressure. The resulting residue was purified by neutral, OH-form silica gel column chromatography (hexane/ethyl acetate = 97:3 to 50:50). The titled compound was obtained as a yellow amorphous in an amount of 10 mg (yield: 17%).

### Example 9: 4-({3,3,7-Trifluoro-4-[(1S)-1-hydroxyethyl]-2-oxo-2,3-dihydro-1H-indol-1-yl}methyl)pyridine-2-carbonitrile

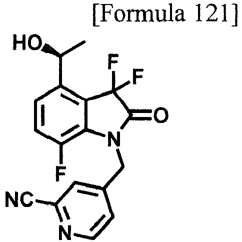

To an N,N-dimethylformamide solution (15 ml) of the 4-[(4-acetyl-3,3,7-trifluoro-2-oxo-2,3-dihydro-1H-indol-1-yl)methyl]pyridine-2-carbonitrile (220 mg, 0.414 mmol) obtained in Production Example 99 and triethylamine (0.314 ml, 2.25 mmol), formic acid (0.212 ml, 5.62 mmol) was added dropwise and after adding chloro[(1S,2S)-N-(p-toluenesulfonyl)-1,2-diphenylethanediamine](mesitylene)ruthenium(II) (34 mg, 0.055 mmol), the resulting mixture was stirred at 35°C for 4 hours. To the reaction mixture, water was added and after extraction with ethyl acetate, the extract was washed with brine. The organic layer was dried in a phase separator and then concentrated under reduced pressure. The resulting residue was purified by neutral, OH-form silica gel column chromatography (hexane/ethyl acetate = 88:12 to 0:100) and by NH-form silica gel column chromatography (hexane/ethyl acetate = 88:12 to 0:100). As a result of freeze-drying, the titled compound was obtained as a colorless amorphous in an amount of 38 mg (yield: 26%).

More compounds were synthesized by substantially the same methods as those used to make the compounds described in Examples 1 to 9, and the structural formulas and names of such compounds and instrumental data for them are shown in Table 1 below. The numbers indicated in the column of "Examples" in the table refer to which of the foregoing Examples 1 to 9 was substantially relied upon to synthesize the respective compounds.

Compounds 36, 120, 126, 143, 144, 146, 147, 148, 150, 151, 152, 153, 154, 156, 157, 158, 159, 160, 161, 162, 164, 165, 167, 168, 169, 170, 171, 172, 176, 178, 179, 180, 186, 191, 218, 219, 220, 255, 256, 257, 258, 259, 260, 263, 264, 265, 266, 267, 268, 269, 270, 271, 272, 273, 274, 275, 276, 278, 280, 282 and 283 were synthesized from the same enantiomeric form as in Production Example 21 whereas compound 35 was synthesized from the same enantiomeric form as in Production Example 22.

In Table 1, the column and solvents used are abbreviated as follows:
Column
   DAICEL CHIRALPAK AD3: AD3
Solvents
   n-hexane: Hex
   ethyl alcohol: EtOH
   isopropyl alcohol: IPA

**[Table 1-1]**

| Compound No | Example | Structural formula | Compound Name | (ESI pos.) m/z (ESI neg.) m/z | LCMS RT (min) condition | 1H-NMR | Chiral HPLC analysis conditions (Column used) (Solvent system) (Flow rate) | Chiral HPLC RT(min) | IC50 (*µ*M) |
|---|---|---|---|---|---|---|---|---|---|
| 1 | 1 | 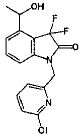 | 1-[(6-chloropyridin-2-yl)methyl]-3,8-difluoro-4-(1-hydroxyethyl)-1,3-dihydro-2H-indol-2-one | 339(M+H) | | | | | 0.015 |
| 2 | 1 | 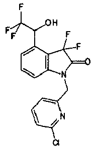 | 1-[(6-chloropyridin-2-yl)methyl]-3,3-difluoro-4-(2,2,2-trifluoro-1-hydroxyethyl)-1,3-dihydro-2H-indol-2-one | 393(M+H) | | | | | 0 051 |
| 3 | 1 | 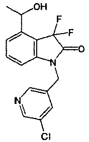 | 1-[(5-chloropyridin-3-yl)methyl]-3,3-difluoro-4-(1-hydroxyethyl)-1,3-dihydro-2H-indol-2-one | 339(M+H) | | | | | 0032 |
| 4 | 1 | 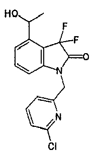 | (enantiomer 1) 1-[(6-chloropyridin-2-yl)methyl]-3,3-difluoro-4-(1-hydroxyethyl)-1,3-dihydro-2H-indol-2-one | 339(M+H) | 0.881, A | 1H NMR (600 MHz. CHLOROFORM-d) d ppm 1.52 - 1.54 (3 H, m),1,95(1H, br. s.), 4.98 (2 H, s), 5.27 - 5.35 (1 H, m), 6.86 (1 H d, J=7.8 Hz) 7.6 (1 H, d, J=7.8 Hz) 7.28 (1 H d, J=7.8 Hz), 7.36 (1 H, d, J=8.3 Hz), 7.45 (1 H, t, J=8.1 Hz) 7.59 - 7.66 (1 H, m) | AD3, 4.6*150mm Hex / IPA=70 / 30 1ml/min, | 4.60, 5.10 Faster | 0.0065 |
| 5 | 1 | 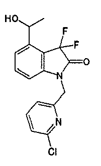 | (enantiomer 2) 1-[(6-chloropyridin-2-yl)methyl]-3,3-difluoro-4-(1-hydroxyethyl)-1,3-dihydro-2H-indol-2-one | 339(M+H) | | | AD3, 4.6*150mm Hex. / IPA=70 / 30 1ml/min | 460, 5.10 Later | 03 |
| 6 | 1 | 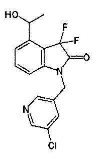 | (enantiomer 1) 1-[(5-chloropyridin-3-yl)methyl]-3,3-difluoro-4-(1-hydroxyethyl)-1,3-dihydro-2H-ndol-2-one | 339(M+H) | | | AD3, 46*150mm Hex. / EtOH=70 / 30 | 4.14, 5.83 Faster | 3.7 |
| 7 | 1 | 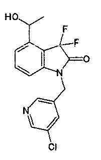 | (enantiomer 2) 1-[(5-chloropyridin-3-yl)methyl]-3,3-difluoro-4-(1-hydroxyethyl)-1,3-dihydro-2H-indol-2-one | 339(M+H) | 0.834, A | 1H NMR (600 MHz DMSO-d6) d ppm 1.34 (3 H, d, J=6.6 Hz), 4.98 - 5.04 (3 H, m) 5.49 (1 H, br. s.), 7.15 (1H, d, J=7.8 Hz), 735 (1 H d J=8.3 Hz) 756 (1 H, t, J=8.1 Hz), 7.91 (1 H t, J=2.1 Hz), 8 54 (1 H, d, J=1.7 Hz) ppm 8.59 (1 H d J=2.1 Hz) | AD3, 46*150mm Hex. / EtOH=70 /30 1 mL / min | 4.14, 5.83 Later | 0014 |
| 8 | 1 | 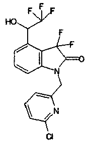 | (enantiomer 1) 1-[(6-chloropyridin-2-yl)methyl]-3,3-difluoro-4-(2,2,2-trifluoro-1-hydroxyethyl)-1,3-dihydro-2H-indol-2-one | 393(M+H) | | | AD3, 4 6*150mm Hex. / EtOH = 90 /10 1 mL/min | 6.16, 6.82 Faster | 0.8 |

**[Table 1-2]**

| Compound No | Example | Structural formula | Compound Name | (ESI pos.) m/z (ESI neg.) m/z | LCMS RT (min) conditions | 1H-NMR | Chiral HPLC analysis conditions (Column used) (Solvent system) (Flow rate) | Chiral HPLC RT(min) | IC50 (*µ*M) |
|---|---|---|---|---|---|---|---|---|---|
| 9 | 1 | 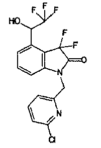 | (enantiomer 2) 1-[(6-chloropyridin-2-yl)methyl]-3,3-difluoro-4-(2,2,2-trifluoro-1-hydroxyethyl)-1,3-dihydro-2H-indol-2-one | 393(M+H) | 0.988, A | 1H NMR (600 MHz, DMSO-d6) d ppm 5.08 (2 H, s), 5.18 - 5.27 (1 H, m), 7.11 - 7 23 (1 H, m), 7.33 (1 H, d. J=4.1Hz), 7.36 - 7.41 (1 H, m), 7.45 (2 H, t, J=7.2 Hz). 7.64 (1 H, t, J=7.8 Hz) 7.89 (1 H, t. J=7.6 Hz) | AD3, 4.6*150mm Hex /EtOH = 90 /10 1 mL / min | 6 16 682 Later | 0.017 |
| 10 | 1 | 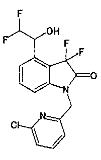 | 1-[(6-chloropyridin-2-yl)methyl]-4-(2,2-difluoro-1-hydroxyethyl)-3,3-difluoro-1,3-dihydro-2H-indol-2-one | 375(M+H) | 0.991, A | 1H NMR (600 MHz, CHLOROFORM-d) d ppm 2.58 - 2.63 (1 H, m), 4.94 - 5.02 (2 H, m), 5.19 - 5.26 (1 H, m) 5.79 - 6.02 (1 H m) 7.01 (1 d, J=7.8Hz), 7.16 - 7.21 (1 H, m) 7.27 - 7.31( 1H, m), 7.36 -7.40 (1 H, m), 7.48 - 7.54 (1 H, m), 7.61 - 7.68 (1 H, m) | | | 0.027 |
| 11 | 1 | 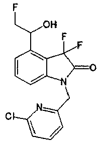 | 1-[(6-chloropyridin-2-yl)methyl]-3,3-difluoro-4-(2-fluoro-1-hydroxyethyl)-1,3-dihydro-2H-ndol-2-one | 357(M+H) | 0.869, A | 1H NMR (600 MHz CHLOROFORM-d) d ppm 2.56 - 2.65 (1 H, m), 4.35 - 4.69 (2 H, m), 4.98 (2 H, s), 5.35 - 5.45 (1 H,(1 m), 6.91 - 6.98(1H, m), 715 - 720 H, m), 7.27 - 7.31 (1 H, m) 7.36 - 7.40 (1 H, m), 7.44 - 7.51 (1 H, m), 7.58 - 7.67 (1 H, m) | | | 0.017 |
| 12 | 1 | 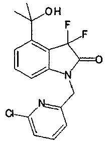 | 1-[(6-chloropyridin-2-yl)methyl]-3,3-difluoro-4-(2-hydroxypropan-2-yl)-1,3-dihydro-2H-indol-2-one | 353(M+H) | 0.906, A | 1H NMR (600 MHz CHLOROFORM-d) d ppm 1.65 (6 H, s), 2.07 - 2.15 (1 H, m), 4.99 (2 H, s), 6.83 - 6.87 (1 H, m), 7.12 - 7.21 (2 H, m), 7.26 - 7.30 (1 H, m), 7.34 - 7.41 (1 H m), 758 - 7.67(1 H, m) | | | 07 |
| 13 | 1 | 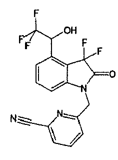 | 6{[3,3-difluoro-2-oxo-4-(2,2,2-trifluoro-1-hydroxyethyl)-2,3-dihydro-1 H-indol-1-yl]methyl}pyridyne-2-carbonitrile | 384(M+H) | | | | | 0.096 |
| 14 | 1 | 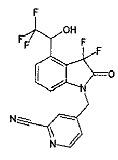 | 4-{[3,3-difluoro-2-oxo-4-(2,2,2-trifluoro-1-hydroxyethyl)-2,3-dihydro-1H-indol-1-yl]methyl}pyridine-2-carbonitrile | 384(M+H) | | | | | 0.12 |
| 15 | 1 | 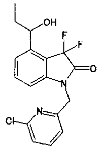 | 1-[(6-chloropyridin-2-yl)methyl]3,3-difluoro-4-(1-hydroxypropyl)-1,3-dihydro-2-indol-2-one | 353(M+H) | 0.946, A | 1H NMR (600 MHz, CHLOROFORM-d) d (2 H, m), 1.96 - 2.03 (1 H, m), 4.91 - 5.05 (3 H, m), 6.83 - 6.88 (1 H, m), 7.14 - 7 20 (1 H, m), 7 27 - 7 33 (2 H m). 7.41 - 7.48 (1 H, m), 7.59 - 7.66 (1 H, m) | | | 0.18 |
| 16 | 1 | 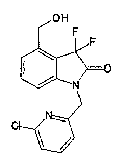 | 1-[(6-chloropyridin-2-yl)methyl]-3,3-difluoro-4-(hydroxymethyl)-1,3-dihydro-2H-indol-2-one | 325(M+H) | 0.820, A | 1H NMR (600 MHz, CHLOROFORM-d) d ppm 1.89 (1H, br, s.), 4.91 (2H, br, s.), 4.98 (2 H, s), 6.88 (1 H, d, J=7.8 Hz), 7.14 - 719 (1H,m), 7.27 - 7.05 (2 H, m), 7.40 - 7.48 (1 H, m), 7.59 - 7.66 (1 H m) | | | 0.14 |

**[Table 1-3]**

| Compound No | Example | Structural formula | Compound Name | (ESI pos.) m/z (ESI neg.) m/z | LCMS RT (min) condition | 1H-NMR | Chiral HPLC analysis conditions (Column used) (Solvent system) (Flow rate) | Chiral HPLC RT (min) | IC50 (*µ*M) |
|---|---|---|---|---|---|---|---|---|---|
| 17 | 1 | 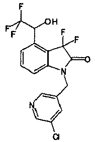 | 1-[(5-chloropyridin-3-yl)methyl]-3,3-difluoro-4-(2,2,2-trifluoro-1-hydroxyethyl)-1,3-dihydro-2H-mdol-2-one | 393(M+H) | | | | | 0.072 |
| 18 | 1 | 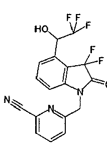 | (enantiomer 1) 6-{[3,3-difluoro-2-oxo-4-(2,2,2-trifluoro-1-hydroxyethyl)-2,3-dihydro-1H-indol-1-yl]methyl}pyridine-2-carbonitrile | 384(M+H) | | | AD3, 4.6*150mm Hex./EtOH=30 1 mL/min | 2.27, 2.75 Faster | 33 |
| 19 | 1 | 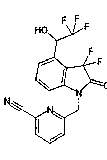 | (enantiomer 2) 6-{[3,3difluoro-2-oxo-4-(2,2,2-trifluoro-1-hydroxyethyl)-2,3-dihydro-1 H-indol-1-yl]methyl}pyridine-2-carbonitrile | 384(M+H) | 0.925, A | 1H NMR (600 MHz CHLOROFORM-d) d ppm 2.91 (1 H, d, J=5.0 Hz) 5.05 (2 H, s), 5.36 - 5.45 (1 H, m), 7.08 (1H, d, J=7 8 Hz), 7 48 (1 H d J=7.9 Hz), 7 52 - 7.59 (2 H, m), 7.67 (1 H, d, J=7.5 Hz), 7.80 - 7.93 (1H, m) | AD3, 4.6*150mm Hex. /EtOH = 30 / 70 1 mL / min | 2.27, 2.75 Later | 0.06 |
| 20 | 1 | 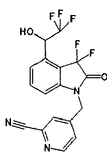 | (enantiomer 1) 4-([3,3-difluoro-2-oxo-4-(2,2,2-trifluoro-1-hydroxyethyl)-2,3-dihydro-1H-indol-1-yl]methyl)pyridine-2-carbonitrile | 384(M+H) | | | AD3, 4.6*150mm Hex. / EtOH=80 1 mL/ min | 4.23, 4.69 Faster | 29 |
| 21 | 1 | 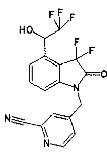 | (enantiomer 2) 4-{[3,3-difluoro-2-oxo-4-(2,2,2-trifluoro-1-hydroxyethyl)-2,3-dihydro-1H-indol-1-yl]methyl}pyridine-2-carbonitrile | 384(M+H) | 0.878, A | 1H NMR (600 MHz, CHLOROFORM-d) d ppm 2.83 (1 H, d, J=5.0 Hz), 4.88 - 5.04 (2 H, m), 5.38 - 5.51 (1 H, m), 6.73 (1 H, d, J=7.0 Hz), 7.34 - 7.47 (1H, m), 7.50 - 7.57 (2 H, m), 7.58 - 7.65 (1 H, m), 8.69 - 8.79 (1 H, m) | AD3. 4.6*150mm Hex /EtOH = 80 / 20 1 mL / min | 4.23, 4.69 Later | 0.071 |
| 22 | 1 | 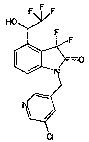 | (enantiomer 1) 1-[(5-chloropyridin-3-yl)methyl]-3,3-difluoro-4-(2,2,2-trifluoro-1-hydroxyethyl)-1,3-dihydro-2H-indol-2-one | 393(M+H) | | 1H NMR (600 MHz DMSO-d6) d ppm 5.03 (2 H, s), 5.16 - 5.26 (1 H, m), 7.31 (1 H, d, J=5.7 Hz), 7.35 (1 H, d, J=8.0 Hz) 7.40 (1 H, d, J=8.3 Hz), 7.64 - 7.70 (1 H, m), 7.92 - 7.95 (1 H, m), 8.55(1 H, d, J=2.1 Hz), 8.59 (1 H, d, J=2.1 Hz) | AD3, 4.6*150mm Hex. / EtOH = 70 /30 1 mL / min | 4.08 447 Faster | 1.9 |
| 23 | 1 | 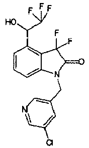 | (enantiomer 2) 1-[(5-chloropyridin-3-yl)methyl]-3,3-difluoro-4-(2,2,2-trifluoro-1-hydroxyethyl)-1,3-dihydro-2H-indol-2-one | 393(M+H) | 0.937, A | 1H NMR (600 MHz, CHLOROFORM-d) d ppm 3.39 (1 H, br, s.), 4.79 - 4.97 (2 H, m), 5.37 - 5.44 (1 H, m), 6.83 (1H, d. J=7.8 Hz), 7.48 - 7.55 (2 H, m), 7.64 (1 H, t J=2.3 Hz), 8 48 (1 H, d, J=1 7 Hz) 854 (1 H, d, J=2.1 Hz) | AD3, 46*150mm Hex. /EtOH = 70 /30 1 mL / min | 4.08. 447 Later | 0.038 |
| 24 | 1 | 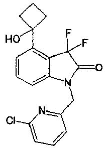 | 1-[(6-chloropyridin-2-yl)methyl]-3,3-difluoro-4-(1-hydroxycyclobutyl)-1,3-dihydro-2H-indol-2-one | 365(M+H) | 0.960, A | 1 H NMR (600 MHz, CHLOROFORM-d) d ppm 1.77 - 1.88 (1 H, m), 2.21 - 2.33 (2 H, m), 2.35 - 2.46 (2 H, m) 2.56 - 2.71 (2 H, m), 4.98 (2 H, s), 6.85 - 6.92 (1 H, m), 7.10 -7.19 (2 H, m), 7 27 - 7.30 (1 H, m), 7.38 - 7.47 (1 H, m), 7.57 - 7.67 (1 H, m) | | | 5.9 |

**[Table 1-4]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 25 | 1 | 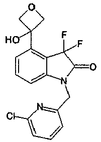 | 1-[(6-chloropyridin-2-yl)methyl]-3,3-difluoro-4-(3-hydroxyoxetan-3-yl)-1,3-dihydro-2H-indol-2-one | 367(M+H) | 0.772 A | 1H NMR (600 MHz, CHLOROFORM-d) d ppm 2.84 (1H, s). 4.90 (2 H, d, ,J=7.8 Hz). 4.99 (2 H, s), 5.07 (2 H d, J= 7.8 Hz) 7.01 (1H H d. J=7 8 Hz). 7 17 -7 20 (1H,m),7.21-7.25(1Hm),727-7.31 (1 H,m),748-754(1 H m) 761 - 7 68 (1 H m) d | 7.6 |
| 26 | 1 | 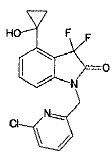 | 1-[(6-chloropyridin-2-yl)methyl]-3,3-difluoro-4-(1-hydroxycyclopropyl)-1,3-dihydro-2H-indol-2-one | 351(M+H) | 0.900 A | 1H NMR (600 MHz. CHLOROFORM-d) d ppm 1.07 - 1.14 (2 H, m), 1.29 - 1.34 (2 H, m), 2.68 (1 H. br. s) 4.99 (2 H. s). 6.87 (1 H, d J=7,4 Hz) 6.96 - 7.02(1 H m).7.14-720(1H, m).727-730(1 H,m),735-741 (1 H m) 759-767 (1 H m) | 1.5 |
| 27 | 1 | 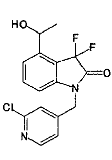 | 1-[(2-chloropyridin-4-yl)methyl]-3,3-difluoro-4-(1-hydroxyethyl)-1,3-dihydro-2H-indol-2-one | 339(M+H) | | | 0.023 |
| 28 | 1 | 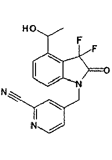 | 4-{[3,3-difluoro-4-(1-hydroxyethyl)-2-oxo-2,3-dihydro-1H-indol-1-yl]methyl}pyridine-2-carbonitrile | 330(M+H) | | | 0.098 |
| 29 | 1 | 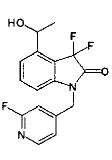 | 3,3-difluoro-1-[(2-fluoropyridin-4-yl)methyl]-4-(1-hydroxyethyl)-1,3-dihydro-2H-indol-2-one | 323(M+H) | | | 0.077 |
| 30 | 1 | 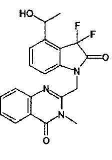 | 2-{[3,3-difluoro-4-(1-hydroxyethyl)-2-oxo-2,3-dihydro-1H-indol-1-yl]methyl}-3-methylquinazolin-4(3H)-one | 386(M+H) | | | 0.054 |
| 31 | 1 | 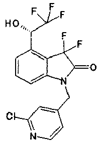 | 1-[(2-chloropyridin-4-yl)methyl]-3,3-difluoro-4-[(1S)-2,2,2-trifluoro-1-hydroxyethyl]-1,3-dihydro-2H-indol-2-one | 393(M+H) | | | 0.35 |
| 32 | 1 | 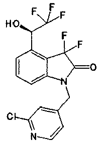 | 1-[(2-chloropyridin-4-yl)methyl]-3,3-difluoro-4-[1R)-2,2,2-trifluoro-1-hydroxyethyl]-1,3-dihydro-2H-indol-2-one | 393(M+H) | 0.932 A | 1H NMR (600 MHz CHLOROFORM-d) d ppm 2.81 - 2.85 (1 H, m). 4.84 - 4.96 (2 H, m), 5.39 - 5.46 (1 H, m), 6.73-6.76 (1 H, m), 7.10 - 7.13 (1 H. m), 7.25 (1H. s). 7.48 - 7.56 (2 H, m), 8.36 - 8.42 (1 H, m). | 0.038 |

**[Table 1-5]**

| Compound No | Example | Structural formula | Compound Name | I (ESI pos.) m/z (ESI neg.) m/z | LCMS RT (min) condition | 1H-NMR | Chiral HPLC analysis conditions. (Column used) (Solvent system) (Flow rate) | Chiral HPLC RT (min) | IC50 (µM) |
|---|---|---|---|---|---|---|---|---|---|
| 33 | 1 | 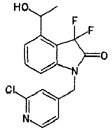 | (enantiomer 1) 1-[(2-chloropyridin-4-yl)methyl]-3,3-difluoro-4-(1-hydroxyethyl)-1,3-dihydro-2H-indol-2-one | 339(M+H) | 0.826 A | 1H NMR (600 MHz CHLOROFORM-d) d ppm 1.50 - 1.60 (3 H, m) 1 90 - 203 (1 H m) 4.88 (2 H, s) 5.28 - 5.36 (1 H m) 655-661 (1 H, m) 7.10-7.14 (1 H, m) 7 24 (1 H, s) 7.38 - 7.38 (2H m) 8.38 (1 H, d, J=5 37 Hz) | AD3 4.6*250 Hex EtOH = 85 15 1mL / min | 6.80, 7.29 Faster | 0.018 |
| 34 | 1 | 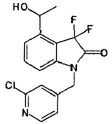 | (enantiomer 2) 1-[(2-chloropyridin-4-yl)methyl]-3,3-difluoro-4-(1-hydroxyethyl)-1,3-dihydro-2H-indol-2-one | 339(M+H) | | | AD3, 4 6*250 Hex EtOH = 85 15 1mL / min | 6.80, 7.29 later | 4.6 |
| 35 | 1 | 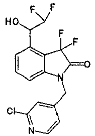 | (enantiomer 2) 1-[(2-chloropyridin-4-yl)methyl]-4-[(1S)-2,2-difluoro-1-hydroxyethyl]-3,3-difluoro-1,3-dihydro-2H-indol-2-one | 375(M+H) | | | | | 0.28 |
| 36 | 1 | 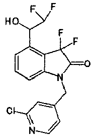 | (enantiomer 1) 1-[(2-chloropyridin-4-yl)methyl]-4-(2,2-difluoro-1-hydroxyethyl)-3,3-difluoro-1,3-dihydro-2H-indol-2-one | 375(M+H) | 0.848 A | 1H NMR(600 MHz CHLOROFORM-d) d ppm 2 63 (1 H. d, J=3 7 Hz), 4.89 (2 H.s) 5.22 - 5.30 (1 H, m) 5.61-6.04 (1H,m), 6.68-673 (1 H, m), 7.10-7 13 (1 H, m), 7 23 - 7,25 (1H m), 7.40-7.45 (1 H, m) 748-754 (1 H, m) 8.36 - 8.43 (1 H,m) | | | 0.013 |
| 37 | 1 | 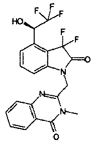 | 2-({3,3-difluoro-2-oxo-4-[(1R)-2,2,2-tnfluoro-1-hydroxyethyl]-2,3-dihydro-1H-indol-1-yl)methyl)-3-methylquinazolin-4(3H)-one | 440(M+H) | 0.925 A | 1H NMR (600 MHz. CHLOROFORM-d) d ppm 2.86 - 2.91 (1 H, m). 3.68 (3 H, s) 4.97 - 5.08 (2 H, m) 5.41 - 5.49 (1 H. m), 7.10-714 (1 H, m), 745-7.55 (4 H, m), 7.67 - 773(1 H, m), 823 - 8.29 (1 H. m) | | | 0.043 |
| 38 | 1 | 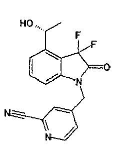 | 4-({3>3-difluoro-4-[(1R)-1-hydroxyethyl]-2-oxo-2,3-dihydro-1H-indol-1-yl}methyl)pyridine-2-carbonitrile | 330(M+H) | | | | | 53 |
| 39 | 1 | 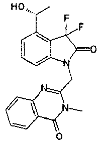 | 2-({3,3-difluoro-4-[(1R)-1-hydroxyethyl]-2-oxo-2,3-dihydro-1H-indol-1-yl}methyl)-3-methylquinazolin-4(3H)-one | 386(M+H) | | | | | 0.62 |
| 40 | 1 | 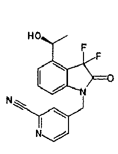 | 4-({3,3-difluoro-4-[(1S)-1-hydroxyethyl]-2-oxo-2,3-dihydro-1H-indol-1-yl}methyl)pyridine-2-carbonitrile | 330(M+H) | 0.752 A | 1H NMR (600 MHz. CHLOROFORM-d) d ppm 1.55 - 1.57 (3 H, m) 1.98 (1 H d, J=330Hz) 4,88-501 (2H m) 5.28 - 5.38 (1 H, m) 6,52 - 6 59 (1 H, m) 7.39 - 7.50 (3 H. m) 759 (1 H d. J=0.83 Hz) 8 71 (1 H d. J=495 Hz) | | | 0.038 |

**[Table 1-6]**

| Compound No | Example ple | Structural formula | Compound Name | (ESI pos.) m/z (ESI neg.) m/z | LCMS RT (min) conditions | 1H-NMR | Chiral HPLC analysis conditions column used) (Solvent system) (Flow rate) | Chiral HPLC RT (min) | IC50 (µM) |
|---|---|---|---|---|---|---|---|---|---|
| 41 | 1 | 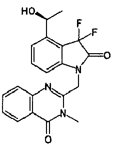 | 2-({3,3-difluoro-4-[(1S)-1-hydroxyethyl]-2-oxo-2,3-dihydro-1H-indol-1-yl}methyl)-3-methylquinazolin-4(3H)-one | 386(M+H) | 0.809 A | 1H NMR (600 MHz CHLOROFORM-d) d ppm 1.55 - 1.57 (3 H m) 1.97 (1 H, d, J=3.30 Hz) 3.67 (3 H, s) 4.97 - 5.07 (2 H. m) 5.30 - 5.38 (1 H m) 6 93 -7 01 (1 H, m) 7.36-7.40 (1 H, m) 7.41-745 (1H, m) 7,46 - 750 (1 H, m) 7.54 - 7,58 (1 H, m) 7.68 - 7.74 (1 H, m) 8.23 - 8.28 (1 H, m) | | | 0.029 |
| 42 | 1 | 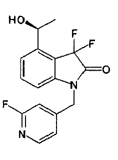 | 3,3-difluoro-1-[(2-fluoropyridin-4-yl)methyl)-4-[(1S)-1-hydroxyethyl]-1,3-dihydro-2H-indol-2-one | 323(M+H) | 0.783 A | 1H NMR (600 MHz, CHLOROFORM-d) d ppm 1.54 - 1.57 (3 H, m) 191 - 1,99 (1 H, m) 4.92 (2 H, m, J=3.70 Hz) 5,28 - 5.36 (1 H. m) 6.56 - 6.56 - (1 H m)6.81 - 6.84 (1 H, m) 7.07 - 7.12 (1 H, m) 7.38 -747(2H,m)819-825(1 H m) | AD3 4.6*250 Hex EtOH = 90 10 1mL / min | 1028 1188 Faster | 0.05 |
| 43 | 1 | 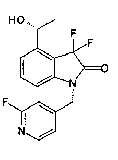 | 3,3-difluoro-1-[(2-fluoropyridin-4-yl)methyl]4-[(1R)-1-hydroxyethyl]-1,3-dihydro-2H-indol-2-one | 323(M+H) | | | AD3, 4.6*250 Hex : EtOH = 90 10 1mL/min mo" | 10.28 1188 Later | 44 |
| 44 | 1 | 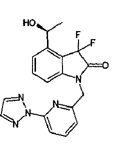 | 3,3-difluoro-4-[(1S)-1-hydroxyethyl]-1-{[6-(2H-1,2,3-triazol-2-yl)pyridin-2-yl]methyl}-1,3-dihydro-2H-indol-2-one | 372(M+H) | 0.797 A | 1H NMR (600 MHz, CHLOROFORM-d) d ppm 1.54 (3 H, d, J=6.6 Hz) 1.94-2.00 (1 H, m), 5.11-5.20 (2H (1H, m) 5.28-5.36 (1 H, m), 6.86 - 6.90 (1 H, m) 7.18 - 7.25 (1 H, m), 7.32-7.46 (2 H m) 7.82 - 7.89 (1 H, m), 7.94 (2 H, s), 8. 01 - 8.06 (1 H, m) | | | 0.068 |
| 45 | 1 | 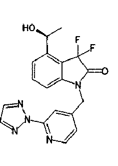 | 3,3-difluoro-4-[(1S)-1-hydroxyethyl]-1-{[2-(2H-1,2,3-triazol-2-yl)pyridin-4-yl]methyl}-1,3-dihydro-2H-indol-2-one | 372(M+H) | 0.720 A | 1 H NMR (600 MHz CHLOROFORM-d) d ppm 1.52 - 1.57 (3 H, m), 1.97 - 2.00 (1 H, m), 5.01 (2 H, s) - 5.36 (1 H. H, m), 7.36 - 7.46 (2 H, m), 7.91 (2 H, s), 8.04 (1 H, s), 8.54 - 8.60 (1 H, m) | | | 0.11 |
| 46 | 1 | 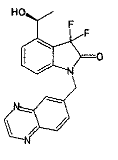 | 3,3-difluoro-4-[(1S)-1-hydroxyethyl]-1-(quinoxalin-6-ylmethyl)-1,3-dihydro-2H-indol-2-one | 356(M+H) | 0.752 A | 1 H NMR (600 MHz, CHLOROFORM-d) d ppm 1.51 - 1.57 (3 H, m) 2.05 - 2.11 (1 H, m). 5.07 - 5.20 (2 H, m) 5.28 - 5.36 (1H, m), 6.67 - 6.73 (1 H, m),7.32 - 7.42 (2 H m), 7.69 - 7.75 (1 H, m) 7,97 - 8.04 (1 H, m), 8.08 - 8.17 (1 H, m) 8.81 ← 890 (2 H, m) | | | 014 |
| 47 | 1 | 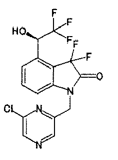 | 1-[(6-chloropyrazin-2-yl)methyl]-3,3-difluoro-4-[(1R)-2,2,2-trifluoro-1-hydroxyethyl]-1,3-dihydro-2H-indol-2-one | 394(M+H) | 0.933 A | 1 H NMR (600 MHz CHLOROFORM-d) d ppm 2.75 - 2.82 (1 H, m) 4.96 - 5.09 (2 H, m) 5.32 - 5.47 (1 H, m) 7.04 - 7.11 (1 H, m) 7.46 - 7.51 (1 H, m) 7,54 -7.60 (1 H, m) 8.54 (1 H, s) 8.58 (1 H, s) | | | 004 |
| 48 | 1 | 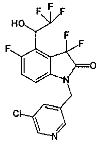 | 1-[(5-chloropyridin-3-yl)methyl]-3,3,5-trifluoro-4-(2,2,2-trifluoro-1-hydroxyethyl)-1,3-dihydro-2H-indol-2-one | 411(M+H) | | | | | 0.085 |

**[Table 1-7]**

| Compound No | Example | Structural formula | Compound Name | (ESI pos.) m/z (ESI neg.) m/z | LCMS RT (min) conditions | 1H-NMR | Chiral HPLC analysis conditions (Column used) (Solvent system) (Flow rate) | Chiral HPLC RT (min) | IC50 (*µ*M) |
|---|---|---|---|---|---|---|---|---|---|
| 49 | 1 | 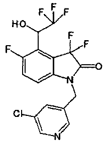 | (enantiomer 1) 1-[(5-chloropyridin-3-yl)methyl]-3,3,5-trifluoro-4-(2,2,2-trifluoro-1-hydroxyethyl)-1,3-dihydro-2H-indol-2-one | 411 (M+H) | | | Shsmadzu AD3. 4.6*250 Hex IPA= 80 20 1mL / min | 4.32, 5.25 Faster | 1.5 |
| 50 | 1 | 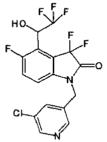 | (enantiomer 2) 1-[(5-chloropyridin-3-yl)methyl]-3,3,5-trifluoro-4-(2,2,2-trifluoro-1-hydroxyethyl)-1,3-dihydro-2H-indol-2-one | 411(M+H) | 0.927 A | 1H NMR (600 MHz, DMSO-d6) d ppm 4.98 - 5.08 (2 H, m), 5.34 - 5.44 (1H m). 7.33 - 7.43 (2 H, m) 7 52 - 760 (1 H.m), 7.91 - 7.96 (1 H, m), 8.51 - 8.64 (2 H m) | Shimadzu AD3 4.6*250 Hex IPA=80 20 ImL / min | 4.31, 5.25 Later | 0.17 |
| 51 | 1 | 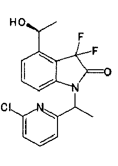 | 1-[1-(6-chloropyridin-2-yl)ethyl]-3,3-difluoro-4-[(1-S)-1-hydroxyethyl]-1,3-dihydro-2H-ndol-2-one | 353(M+H) | | | | | 0.26 |
| 52 | 1 | 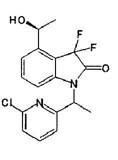 | (diastereomer 1) 1-[1-(6-chloropyridin-2-yl)ethyl]-3,3-difluoro-4-[(1S)-1-hydroxyethyl]-1,3-dihydro-2H-indol-2-one | 353(M+H) | 0.972 A | 1 H NMR (600 MHz, CHLOROFORM-d) d ppm 1.50 - 1.57 (3H, m) 1.90 (3 H, d J=7.02 Hz) 5.24 - 5.33 (1 H, m) 5,68 - 5.74 (1 H, m) 6.74 - 6.78 (1 H m)7.21 - 7.28 (2 H, m) 7,31 - 7.28 (2 H, m) 7,59 - 7 64 (1 H, m). | | | 0.33 |
| 53 | 1 | 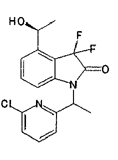 | (diastereomer 2) 1-[1-(6-chloropyridin-2-yl)ethyl)-3,3-difluoro-4-[(1S)-1-hydroxyethyl]-1,3-dihydro-2H-indol-2-one | 353(M+H) | 0.958 A | 1H NMR (600 MHz. CHLOROFORM-d) d ppm 1.47 - 1.53 (3 H, m) 1.87 - 1.94 (3 H.m) 5.23 - 5.34 (1 H m) 5.69 (1H. q. J=7.16 Hz) 6.79 (1 H d J=7 84Hz) 7.21 - 7.30 (2 H, m) 7.31 - 740 (2 H, m) 759-765(1 H, m) | | | 0.14 |
| 54 | 1 | 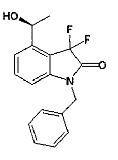 | 1-benzyl-3,3-difiuoro-4-[(1S)-1-hydroxyethyl]-1,3-dihydro-2H-indol-2-one | 304(M+H) | 0.944 A | 1H NMR (600 MHz, CHLOROFORM-d) d ppm 1.53 (3 H, d, J=6.2 Hz) 1.55 - 1.70 (1 H, m), 4.85 - 4.94 (2 H, m), 5.25 - 5.35 (1 H, m), 6.63 - 6.71 (1 H, m), 7.27 - 7.42 (7 H, m) | | | 0.02 |
| 55 | 1 | 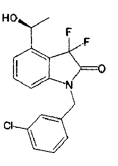 | 1-(3-chlorobenzyl)-3,3-difluoro-4-[(1S)-1-hydroxyethyl]-1,3-dihydro-2H-indol-2-one | 360(M+Na) | 1.022 A | 1H NMR (600 MHz CHLOROFORM-d) d ppm 1.50 - 1.60 (3 H, m), 1.96 (1 H, br s.), 4.86 (2 H. d. J=1.2 Hz) 5.30 (1H.6 s). 6.60 - 6.69 (1 H m) 7.14 - 7.21(1 H m). 7.27 - 7.46 (5 H. m) | | | 0.0022 |
| 56 | 1 | 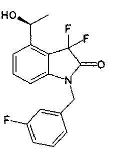 | 3,3-difluoro-1-(3-fluorobenzyl)-4-[(1S)-1-hydroxyethyl]-1,3-dihydro-2H-indol-2-one | 322(M+H) | 0.954 A | 1H NMR (600 MHz. CHLOROFORM-d) d ppm 1.54 (3 H. d. J=6.6 Hz). 1.95 (1H. br. s.), 4.88 (2 H, d, J=3.3 Hz), 5.26-5.37 (1 H, m), 6.59 - 6.69 (1H, m) 6.96 - 7.04 (2 H, m), 7.05 - 7.11 (1 H, m) 7.29 - 7.45 (3 H, m) | | | 0.0091 |

**[Table 1-8]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 57 | 1 | 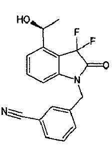 | 3-({3,3-difluoro-4-[(1S)-1-hydroxyethyl]-2-oxo-2,3-dihydro-1H-indol-1-yl}methyl)benzonitrile | 329(M+H) | 0.868 A | 1H NMR (600 MHz. CHLOROFORM-d) d ppm 1.55 (3 H, d, J=6.6 Hz),1.96 (1H, br. s.), 4.92 (2 H. d. J=6 6 Hz), 5.24 - 5.36 (1 H, m), 6.58 - 6.67 (1 H,m), 7.35 - 7.66 (6 H, m) | 0.015 |
| 58 | 1 | 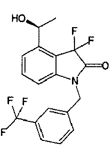 | 3,3-difluoro-4-[(1S)-1-hydroxyethyl]-1-[3-(trifluoromethyl) benzyl)-1,3-dihydro-2H-indol-2-one | 372(M+H) | 1.042 A | 1H NMR (600 MHz CHLOROFORM-d) d ppm 1.54 (3 H, d, J=6.6Hz), 1.95 (1 H br. s.), 4.94 (2 H, s), 5.24 - 5.39 (1H, m), 6.60 - 6.70 (1 H, m). 7.33 - 7.62(6 H m) | 0.0066 |
| 59 | 1 | 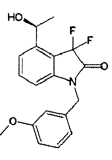 | 3,3-difluoro-4-[(1S)-1-hydroxyethyl]-1-(3-methoxybenzyl)-1,3-dihydro-2H-indol-2-one | 334(M+H) | 0.943 A | 1 H NMR (600 MHz, CHLOROFORM-d) d ppm 1.53 (3 H, d, J=6.2 Hz) 1.88 - 2.00 (1 H, m), 3.78 (3 H, s) 4.88 (2 H, d, J=3.3 Hz) 5.25 - 5.35 (1 H, m). 6.68(1 H, d. J=7.8 Hz), 6.82 (3 H s). 7.22 - 7.28(1 H,m),7.29-734 (1 H, m) 736 - 7.41 (1 H, m) | 0.018 |
| 60 | 1 | 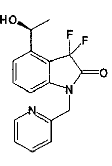 | 3,3-difluoro-4-[(1S)-1-hydroxyethyl]-1-(pyridin-2-ylmethyl)-1,3-dihydro-2H-indol-2-one | 305(M+H) | 0.702 A | 1H NMR (600 MHz CHLOROFORM-d) d ppm 1.52-1.54 (3 H,m) 1.93 (1H, br. s.) 5.02 (2 H, s) 5.25 - 5.35 (1H m) 6.87 (1 H, d, J=7.84 Hz) 7.21 - 7.29 (2H, m) 7.31 - 7.37 (1 H, m) 7.38 - 7.45 (1 H, m) 7.64 - 7.70 (1 H m) 8.57 (1 H. d, J=4 13 Hz) | 0.23 |
| 61 | 1 | 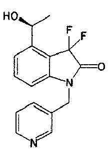 | 3,3-difluoro-4-[(1S)-1-hydroxyethyl)-1-(pyridin-3-ylmethyl)-1,3-dihydro-2H-indol-2-one | 305(M+H) | 0.423 A | 1H NMR (600 MHz CHLOROFORM-d) d ppm 1.51 - 1.54 (3 H, m) 1.90 - 1.98 (1 H, m) 4.86 - 4.99 (2H m) 5.24 - 5.36 (1 H, m) 6.66 - 6.72 (1 H,m) 7.29 - 7.46 (3 H, m) 7.62 - 7.69 (1 H, m) 8.56 - 8.60 (1 H, m) 8.65 (1 H, s) | 0.42 |
| 62 | 1 | 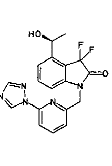 | 3,3-difluoro-4-[(1S)-1-hydroxyethyl]-1-{[6-(1H-1,2,4-triazol-1-yl)pyridin-2-yl]methyl}-1,3-dihydro-2H-indol-2-one | 372(M+H) | 0.751 A | 1H NMR (600 MHz. CHLOROFORM-d) d ppm 1 55 (3 H d, J=6 61 Hz) 5.04 (2 H s) 5.27 - 5.37 (1 H, m) 6.78 - 6.85 (1 H, m) 7.28 - 7.32 (1H, m) 7.36 -7.40 (1 H m)7.42-7.47(1 H, m)7.85(1 H,s) 7.86 - 7.93 (1 H, m) 8.07 (1 H s) 8.98 (1H, s) | 0.23 |
| 63 | 1 | 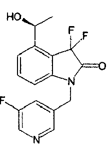 | 3,3-difluoro-1-[(5-fluoropyridin-3-yl)methyl]-4-[(1S)-1-hydroxyethyl]-1,3-dihydro-2H-indol-2-one | 323(M+H) | 0.744 A | 1H NMR (600 MHz. DMSO d6) d ppm 1.34 (3 H. d, J=6.19 Hz) 4.97 - 5.02 (1 H, m) 5.03 (2 H, s) 5.48 (1 H, d, J=4.13 Hz) 7.10 - 7.16 (1 H, m) 7.32 - 7.38 (1H, m) 7.52 - 7.59 (1 H, m) 7,68 - 7.74 (1 H. m) 8.46 (1 H. s) 8.54 (1 H. d. J=2 89 Hz) | 0076 |
| 64 | 1 | 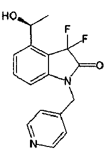 | 3,3-difluoro-4-[(1S)-1-hydroxyethyl]-1-(pyridin-4-ylmethyl)-1,3-dihydro-2H-indol-2-one | 305(M+H) | 0.317 A | 1H NMR(600 MHz CHLOROFORM-d) d ppm 1.55 (3 H, d, J=6.6 Hz), 1.94 - 2 07 (1 H, m), 4.94 (2 H, s), 5.27 - 5.37 (1 H m), 6.53 - 6.62 (1 H, m). 7 28 -7 33 (2 H, m), 7.36 - 7.46 (2H, m),8.59-8.67 (2 H, m). | 039 |

**[Table 1-9]**

| Compound No | Examples | Structural formula | Compound Name | (ESI pos.) m/z (ESI neg.) m/z | LCMS RT (min) condition | 1H-NMR | IC50 (*µ*M) (*µ*M) |
|---|---|---|---|---|---|---|---|
| 65 | 1 | 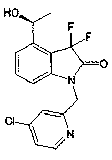 | 1-[(4-chloropyridin-2-yl)methyl]-3,3-difluoro-4-[(1S)-1-hydroxyethyl]-1,3-dihydro-2H-ndol-2-one | 339(M+H) | 0.860 A | 1 H NMR (600 MHz. CHLOROFORM-d) d ppm 1.54 (3 H. d. J=6.6 Hz), 1,95 (1 H, br. s.). 4.99 (2 H, s), 5.25 - 5.35 (1 H, m),6.82-6.89 (1H,m),7.22-7.26(1 H, m), 7.27 - 7.30 (1 H, m) 7.33 - 7.39 (1 H, m),7.39-7.47(1 H,m),8.42-8.50 (1 H, m) | 0.018 |
| 66 | 1 | 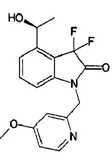 | 3,3-difluoro-4-[(1S)-1-hydroxyethyl]-1-[(4-methoxypyridin-2-yl)methyl]-1,3-dihydro-2H-indol-2-one | 335(M+H) | 0.500 A | 1H NMR (600 MHz CHLOROFORM-d) d ppm 1 53 (3 H, d, J=6 6 Hz), 1 96 - 2 04 (1H, m), 380 (3 H, s), 4.96 (2 H s) 5.26 - 5.34 (1 H, m), 6 70 - 6 80 (2H, m). 6.85 - 6.92 (1 H, m), 7.30 - 7.37 (1 H, m), 7.39 - 746 (1 H,m) 8.34 - 8.43 (1H, m). | 0.13 |
| 67 | 1 | 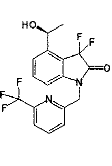 | 3,3-difluoro-4-[(1S)-1-hydroxyethyl]-1-{[6-(trifluoromethyl)pyridi n-2-yl]methyl}-1,3-dihydro-2H-indol-2-one | 373(M+H) | 0.952 A | 1H NMR (600 MHz CHLOROFORM-d) d ppm 1 53 (3 H, d J=6.61 Hz) 1.90 - 1.96 (1 H, m) 5.08 (2 H, s) 5.25 - 5.34 (1 H, m) 6.91 - 6.96 (1 H. m) 7.33 -7.38 (1 H, m) 7.42 - 7.50 (2 H, m)7.61-7.65 (1 H, m) 7.83 - 790 (1H, m) | 0.017 |
| 68 | 1 | 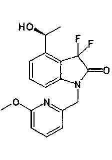 | 3,3-difluoro-4-[(1S)-1-hydroxyethyl]-1-[(6-methoxypyridin-2-yl)methyl]-1,3-dihydro-2H-indol-2-one | 335(M+H) | 0.914 A | 1H NMR (600 MHz CHLOROFORM-d) d ppm 1.53 (3 H, d J=6.61 Hz) 1.94 (1H. br. s.) 3.81 (3 H, s) 4,90 (2 H, s) 5,26 - 5.36(1 H m)6.60-666(1 H, m)6,82-6.85 (1 H, m) 6.85 - 6.88 (1 H, m) 7.32 - 7.35 (1 H, m) 738 - 744 (1 H, m) 7.50 - 7.55 (1 H. m) | 0.076 |
| 69 | 1 | 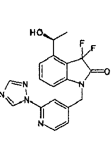 | 3,3-difluoro-4-[(1S)-1-hydroxyethyl]-1-{[2-(1 H-1,2,4-triazol-1-yl)pyridin-4-yl]methyl}-1,3-dihydro-2H-indol-2-one | 372(M+H) | 0.725 A | 1H NMR (600 MHz CHLOROFORM-d) d ppm 1 55 (3 H, d J=6 19 Hz) 4 98 (2 H s) 5.27 - 5.36 (1 H, m) 6.63 (1 H,dd, J=7.64, 1.03 Hz) 7.13 - 7.19 (1 H,m) 7.36 - 7.47 (2 H, m) 7.88 (1 H s) 8.09 (1H,s) 8.43 (1H d, J=5.37 Hz) 9.17 (1 H.s) | 0.045 |
| 70 | 1 | 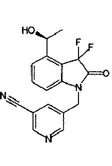 | 5-({3,3-difluoro-4-[(1S)-1-hydroxyethyl]-2-oxo-2,3-dihydro-1H-indol-1-yl}methyl)pyridine-3-carbonitrile | 330(M+H) | 0.715 A | 1H NMR (600 MHz CHLOROFORM-d) d ppm 1.46 - 1.58 (3 H, m) 1.91 - 199 (1 H,m)4.83-5.02 (2H, m)5.21-5.38 (1 H, m) 6.59 - 6.69 (1 H, m) 7.39 -7.45 (1 H, m) 7.45 - 7.51 (1 H, m) 7.85-7.92 (1 H ,m) 8.79 - 8.89 (2 H, m) | 0.091 |
| 71 | 1 | 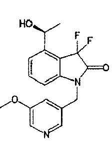 | 3,3-difluoro-4-[(1S)-1-hydroxyethyl]-1-[(5-methoxypyridin-3-yl)methyl]-1,3-dihydro-2H-indol-2-one | 335(M+H) | 0.605 A | 1 H NMR (600 MHz CHLOROFORM-d) d ppm 1.53 (3 H, d, J=6.19 Hz) 1.90 - 2.00 (1 H,m) 3.84 (3 H,s) 4.81 - 496 (2 H, m) 5.24 - 535 (1 H, m) 667 - 674 (1 H, m) 7.11 - 7.16 (1 H, m) 7.34 - 7.38 (1 H, m) 7.40 - 7,46 (1 H, m) 8.18 - 8 29 (2 H m) | 0.16 |
| 72 | 1 | 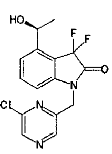 | 1-[(6-chloropyrazin-2-yl)methyl]-3,3-difluoro-4-[(1S)-1-hydroxyethyl]-1,3-dihydro-2H-ndol-2-one | 340(M+H) | 0.813 A | 1H NMR (600 MHz. CHLOROFORM-d) d ppm 1.52 - 1.54 (3 H, m) 1.95 (1 H, d, J=2.89 Hz) 4.94 - 5.07 (2 H, m) 5.25 - 5.35 (1 H, m) 6.82 - 6.92 (1 H, m)7,37 - 7.42 (1 H, m) 7.45 - 7.51 (1 H, m) 8.52 (1 H, s) 8.56 (1 H, s) | 0.012 |

**[Table 1-10]**

| Compound No | Example | Structural formula | Compound Name | (ESI pos) m/z (ESI neg.) m/z | LCMS RT (min) condition | 1H-NMR | IC50 (µM) |
|---|---|---|---|---|---|---|---|
| 73 | 1 | 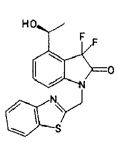 | 1-(1,3-benzothiazol-2-ylmethyl)-3,3-difluoro-4-[(1S)-1-hydroxyethyl]-1,3-dihydro-2H-indol-2-one | 361(M+H) | 0.950 A | 1H NMR (600 MHz CHLOROFORM-d) d ppm 1 53 (3 H, d, J=6.61 Hz) 1 92 - 2.03 (1 H, m) 5.22 - 5.37 (3 H, m) 6.91 - 7.00 (1 H, m) 7.34 - 7.55 (4 H, m)7.84 (1 H d, J=7 43 Hz) 8.03 (1 H, d, J=8.26 Hz) | 0.037 |
| 74 | 1 | 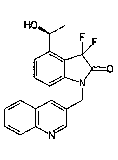 | 3,3-difluoro-4-[(1S)-1-hydroxyethyl]-1-(quinolin-3-ylmethyl)-1,3-dihydro-2H-indol-2-one | 355(M+H) | 0.751 A | 1H NMR (600 MHz CHLOROFORM-d) d ppm 1.52 - 1.54 (3 H, m) 1.97 (1 H, d, J=3.30 Hz) 5.02 - 5.15 (2 H, m) 5.26 - 5.36 (1 H, m) 6.73 (1 H, d, J=7.84Hz) 7.36 (2 H, s) 7.54 - 7.60 (1 H, m) 7.71-7.76 (1 H, m) 7.78 - 7.83 (1 H, m) 8.05 - 8.08 (1 H, m) 8.11 (1 H, d, J=8.67 Hz) 8.92 (1 H, d. J=2.48 Hz) | 0.039 |
| 75 | 1 | 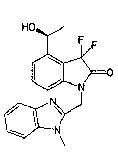 | 3,3-difluoro-4-[(1S)-1-hydroxyethyl]-1-[(1-methyl-1H-benzimidazol-2-yl)methyl]-1,3-dihydro-2H-indol-2-one | 358(M+H) | 0.684 A | 1H NMR (600 MHz CHLOROFORM-d) d ppm 1 50 (3 H, d, J=6.61 Hz) 1.93 (1 H br. s.) 3.83 (3 H, s) 5.18 - 5.32 (3 H, m) 7.28 - 7.38 (4 H, m) 7.42 - 7.52 (2 H, m) 7.78 (1 H, d, J=7.43 Hz) | 0.034 |
| 76 | 1 | 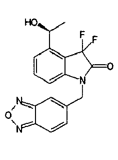 | 1-(2,1,3-benzoxadiazol-5-ylmethyl)-3,3-difluoro-4-[(1S)-1-hydroxyethyl]-1,3-dihydro-2H-indol-2-one | 368(M+Na) | 0.908 A | 1H NMR (600 MHz. CHLOROFORM-d) d ppm 1.55 (3 H, s) 1.94 - 1.99 (1 H, m) 4.91 - 5.04 (2 H, m) 5.27 - 5.37 (1 H, m) 6.67 - 6.74 (1 H, m) 7.32 - 7.48 (3H, m) 7.70 - 7.75 (1 H, m) 7.88 (1 H, d, J=9.08 Hz) | 0.0087 |
| 77 | 1 | 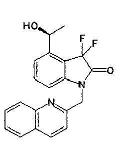 | 3,3-difluoro-4-[(1S)-1-hydroxyethyl]-1-(quinolin-2-ylmethyl)-1,3-dihydro-2H-indol-2-one | 355(M+H) | 0.920 A | 1H NMR (600 MHz, CHLOROFORM-d) d ppm 1.51 - 1.54 (3 H, m) 1.94 (1 H, d, J=3.30 Hz) 5.20 (2 H, s) 5.26 - 5.35 (1 H, m) 6.87 - 6.97 (1 H, m) 7.32 (1 H, s) 7.34 - 7.40 (2 H, m) 7.53 - 7.60 (1 H, m) 7.71 - 7.77 (1 H, m) 7.79 - 7.85 (1 H, m) 8.04 - 8.10 (1 H, m) 8.14 (1 H, d, J=8.67 Hz) | 0037 |
| 78 | 1 | 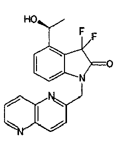 | 3,3-difluoro-4-[(1S)-1-hydroxyethyl]-1-(1,5-naphthyridin-2-ylmethyl)-1,3-dihydro-2H-indol-2-one | 356(M+H) | 0.712 A | 1H NMR (600 MHz, CHLOROFORM-d) d ppm 1.52 - 1.55 (3 H, m) 1.92 - 1.98 (1 H, m) 5.22 (2 H, s) 5.28 - 5.37 (1 H, m) 6.82 - 6.89 (1 H, m) 7.31 - 7.41 (2 H, m) 7.59 - 7.63 (1 H, m) 7.64 - 7.69 (1 H, m) 8.33 - 8.37 (1 H, m) 8.38 - 8.42 (1 H, m) 8.98 (1 H, dd, J=4.54, 1.65 Hz) | 0.32 |
| 79 | 1 | 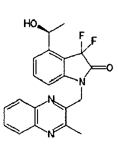 | 3,3-difluoro-4-[(1S)-1-hydroxyethyl]-1-[(3-methylquinoxalin-2-yl)methyl]-1,3-dihydro-2H-indol-2-one | 370(M+H) | 0.870 A | 1H NMR (600 MHz, CHLOROFORM-d) d ppm 1.50 - 1.57 (3 H m) 1.96 (1 H, d, J=3.30 Hz) 2.82 (3 H s) 5.23 (2 H, s) 5.30 - 5.37 (1 H, m) 6.86 (1 H, d, J=7.43 Hz) 7.30 - 7.40 (2 H, m) 7.64 - 7.70 (1 H, m) 7.70 - 7.76 (1 H, m) 7.91 - 7.96 (1 H, m) 7.98 - 8.03 (1 H, m) | 0.031 |
| 80 | 1 | 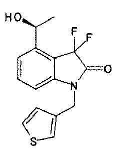 | 3,3-difluoro-4-[(1S)-1-hydroxyethyl]-1-(thiophen-3-ylmethyl)-1,3-dihydro-2H-indol-2-one | 310(M+H) | 0.909 A | 1 H NMR (600 MHz, CHLOROFORM-d) d ppm 1.52 - 1.53 (3 H, m) 1.88 - 1.96 (1 H, m) 4.84 - 4.93 (2 H, m) 5.25 - 5.33 (1 H, m) 6.74 - 6.78 (1 H, m) 7.01 - 7.05 (1 H, m) 7.20 - 7.22 (1 H, m) 7.30 - 7.32 (1 H, m) 7.34 (1 H, d, J=8.26 Hz) 7.41 - 7.46 (1 H, m) | 0.028 |

**[Table 1-11]**

| Compound No | Example | Structural formula | Compound Name | (ESI pos.) m/z (ESI neg.) m/z | LCMS RT (min) conditions | 1H-NMR | IC50 (µM) |
|---|---|---|---|---|---|---|---|
| 81 | 1 | 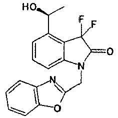 | 1-(1,3-benzoxazol-2-ylmethyl)-3,3-difluoro-4-[(1S)-1-hydroxyethyl]-1,3-dihydro-2H-indol-2-one | 345(M+H) | 0.884 A | 1H NMR (600 MHz CHLOROFORM-d) d ppm 1.52 - 1.54 (3 H, m) 1.94 (1 H, d. J=2.89 Hz) 5.12 - 5.22 (2 H, m) 5.28-5.36 (1 H, m) 6.92 - 6.98 (1 H, m) 7.32 - 7.40 (3 H, m) 7.44 - 7.49 (1 H, m) 7.49 - 7.53 (1 H, m) 7.69 - 7.74 (1 H, m) | 003 |
| 82 | 1 | 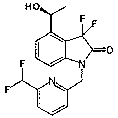 | 1-{[6-(difluoromethyl) pyridin-2-yl]methyl}-3,3-difluoro-4-[(1S)-1-hydroxyethyl]-1,3-dihydro-2H-indol-2-one | 355(M+H) | 0.881 A | 1H NMR (600 MHz, CHLOROFORM-d) d ppm 1.52 - 1.53 (3 H, m) 1.88 - 1.96 (1 H, m) 5.04 (2 H, s) 5.25 - 5.35 (1 H, m) 6.47 - 6.72 (1 H, m) 6.83 - 6.89 (1 H, m) 7.33 - 7.40 (2 H, m) 7.41 - 7.46 (1 H, m) 7.56 - 7.60 (1 H, m) 7.80 - 7.86 (1 H, m) | 0.044 |
| 83 | 1 | 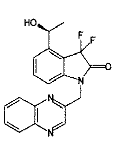 | 3,3-difluoro-4-[(1S)-1-hydroxyethyl]-1-(quinoxalin-2-ylmethyl)1,3-dihydro-2H-indol-2-one | 356(M+H) | 0.850 A | 1H NMR (600 MHz CHLOROFORM-d) d ppm 1.52 - 1.54 (3 H, m) 1.89 - 2.00 (1 H, m) 5.19 - 5.28 (2 H, m) 5.28 - 5.34 (1 H, m) 6.90 - 6.95 (1 H, m) 7.36 (1 H, s) 7.39 - 7.44 (1 H, m) 7.79 (2 H, s) 8.04 - 8.08 (1 H, m) 8.09 - 8.14 (1 H, m) 8.88 (1 H, s) | 003 |
| 84 | 1 | 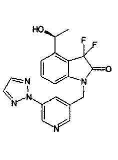 | 3,3-difluoro-4-[(1S)-1-hydroxyethyl]-1-{[5-(2H-1,2,3-triazol-2-yl)pyridin-3-yl]methyl}-1,3-dihydro-2H-indol-2-one | 372(M+H) | 0.782 A | 1H NMR (600 MHz. CHLOROFORM-d) d 1H NMR ppm 1.52 - 1.53 (3 H, m) 1.90 - 1.97 (1 H, m) 4.94 - 5.07 (2 H, m) 5.24 - 5.37 (1 H, m) 6.70 - 6.75 (1 H, m) 7.35 - 7.40 (1 H, m) 7.41 - 7.48 (1 H, m) 7.87 (2 H, s) 8.30 - 8.37 (1 H, m) 8.58 - 8.63 (1 H, m) 9.35 (1 H, d, J=2.06 Hz) | 0035 |
| 85 | 3 | 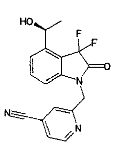 | 2-({3,3-difluoro-4-[(1S)-1-hydroxyethyl]-2-oxo-2,3-dihydro-1H-indol-1-yl}methyl)pyridine-4-carbonitrile | 330(M+H) | 0.770 A | 1H NMR (600 MHz. CHLOROFORM-d) d ppm 1.55 (3 H, d, J=6.6 Hz), 1.57 - 1.68 (1 H, m), 5.02 - 5.10 (2 H, m), 5.27 - 5.37 (1 H, m), 6.75 - 6.84 (1 H, m), 7.35 - 7.54 (4 H, m), 8.73 - 8.80 (1 H, m) | 042 |
| 86 | 1 | 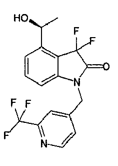 | 3,3-difluoro-4-[(1S)-1-hydroxyethyl]-1-{[2-(trifluoromethyl) pyridin-4-yl]methyl}-1,3-dihydro-2H-indol-2-one | 373(M+H) | 0.864 A | 1H NMR (600 MHz, CHLOROFORM-d) d 1H NMR ppm 1.55 (3 H, d, J=6.6 Hz), 1.87 - 2.05 (1 H, m), 4.97 (2 H, s), 5.28 - 5.37 (1 H, m), 6.53 - 6.62 (1 H, m), 7.34 - 7.50 (3 H, m), 7.58 - 7.63 (1 H, m), 8.66 - 8.78 (1 H, m) | 0 024 |
| 87 | 2 | 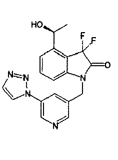 | 3,3-difluoro-4-[(1S)-1-hydroxyethyl]-1-{[5-(1H-1,2,3-triazol-1-yl)pyridin-3-yl]methyl}-1,3-dihydro-2H-indol-2-one | 372(M+H) | 0.645 A | 1H NMR (600 MHz, CHLOROFORM-d) d ppm 1.52 - 1.53 (3 H, m) 1.92 - 1.99 (1 H, m) 4.95 - 5.08 (2 H, m) 5.27 - 5.34 (1 H, m) 6.72 - 6.77 (1 H, m) 7.38 - 7.42 (1 H, m) 7.44 - 7.50 (1 H, m) 7.90 (1 H, d, J=0.83 Hz) 8.05 (1 H, d, J=0.83 Hz) 8.10 - 8.13 (1 H, m) 8.71 (1 H, d, J=2.50 Hz) 8.98 (1 H, d, J=2.48 Hz) | 1.5 |
| 88 | 1 | 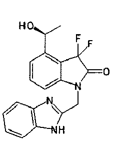 | 1-(1H-benzimidazol-2-ylmethyl)-3,3-difluoro-4-[(1S)-1-hydroxyethyl]-1,3-dihydro-2H-indol-2-one | 344(M+H) | 0.567 A | 1H NMR (600 MHz DMSO-d6) d ppm 1.36 (3 H, d, J=6.61 Hz) 4.98 - 5.07 (1 H, m) 5.16 (2 H, s) 5.47 - 5.52 (1 H, m) 6.93 - 6.98 (1 H, m) 7.11 - 7.18 (2 H,m) 7.33 (1 H, m, J=7.80 Hz) 7.50 (2 H, d, J=7.84 Hz) 1266 (1 H, br. s.) | 0.087 |

**[Table 1-12]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 89 | 4 | 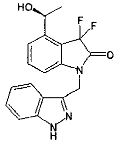 | 3,3-difluoro-4-[(1S)-1-hydroxyethyl]-1-(1H-indazol-3-ylmethyl)-1,3-dihydro-2H-indol-2-one | 344(M+H) | 0.836 A | 1H NMR (600 MHz. CHLOROFORM-d) d ppm 1 48 (3 H, d, J=6 19 Hz) 1.82 - 1.97 (1 H, m) 5.20 - 5.32 (3 H, m) 7.14 - 7.22 (2 H, m) 7.28 - 7.31 (1 H, m) 7.44 (3 H, s) 7.81 - 7.86 (1 H, m) 9.86 - 10.01 (1 H, m) | 19 |
| 90 | 1 | 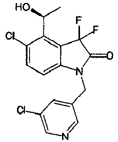 | 5-chloro-1-[(5-chloropyridin-3-yl)methyl]-3,3-difluoro-4-[(1S)-1-hydroxyethyl]-1,3-dihydro-2H-indol-2-one | 373(M+H) | 0.906 A | 1H NMR (600 MHz CHLOROFORM-d) d ppm 1.62 (3 H, d, J=6.6 Hz), 2.30 - 2.51 (1 H, m), 4.75 - 4.99 (2 H, m), 5.32 - 5.45 (1 H, m), 6.58 - 6.68 (1 H, m), 7.36 -7.46 (1 H, m), 7.57 - 7.65 (1 H, m). 8.43 - 8.61 (2 H, m) | 0.046 |
| 91 | 1 | 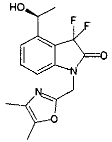 | 1-[(4,5-dimethyl-1,3-oxazol-2-yl)methyl]-3,3-difiuoro-4-[(1S)-1-hydroxyethyl]-1,3-dihydro-2H-indol-2-one | 323(M+H) | 0.812 A | 1H NMR (600 MHz CHLOROFORM-d) d ppm 1.53 (3 H, d, J=6.19 Hz) 2.05 (3 H, s) 2.18 (3 H, s) 4.89 (2 H, s) 5.27 - 5.33 (1 H, m) 6.94 - 6.99 (1 H, m) 7.37(1 H, d, J=8.26 Hz) 7.45 - 7.50 (1 H, m) | 0.13 |
| 92 | 1 | 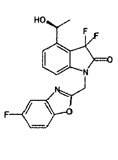 | 3,3-difluoro-1-[(5-fluoro-1,3-benzoxazol-2-yl)methyl]-4-[(1S)-1-hydroxyethyl]-1,3-dihydro-2H-indol-2-one | 363(M+H) | 0.921 A | 1H NMR (600 MHz, CHLOROFORM-d) d ppm 1.52 - 1.54 (3 H, m) 1.96 (1 H, br. s.) 5.11 - 5.20 (2 H, m) 5.28 - 5.34 (1 H, m) 6.93 (1 H, d, J=7.84 Hz) 7.06 - 7.13 (1 H, m) 7.36 - 7.42 (2 H, m) 7.42 - 7.51 (2 H, m) | 0.069 |
| 93 | 1 | 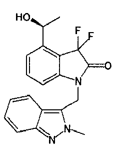 | 3,3-difluoro-4-[(1S)-1-hydroxyethyl]-1-[(2-methyl-2H-indazol-3-yl)methyl]-1,3-dihydro-2H-indol-2-one | 358(M+H) | 0.821 A | 1H NMR (600 MHz CHLOROFORM-d) d ppm 1.51 (3 H, d, J=6.61 Hz) 1.86 - 1.97 (1 H, m) 4.17 (3 H, s) 5.23 - 5.35 (3 H, m) 6.67 - 6.72 (1 H, m) 7.18 - 7.23 (1 H, m) 7.30 - 7.37 (3 H, m) 7.65 - 7 73 (2 H m) | 0.054 |
| 94 | 1 | 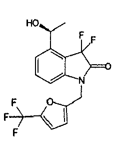 | 3,3-difluoro-4-[(1S)-1-hydroxyethyl]-1-{[5-(trifluoromethyl)furan-2-yl]methyl}-1,3-dihydro-2H-indol-2-one | 362(M+H) | 0.995 A | 1H NMR (600 MHz. CHLOROFORM-d) d ppm 1.52 - 1.54 (3 H, m) 1.94 (1 H, d, J=2.89 Hz) 4.85 - 4.94 (2 H, m) 5.26 - 5.34 (1 H, m) 6.42 (1 H, d, J=2.89Hz) 6.73 - 6.76 (1 H, m) 6.91 - 6.96 (1 H, m) 7.40 (1 H, s) 7.49 - 7.54 (1 H, m) | 0.006 |
| 95 | 1 | 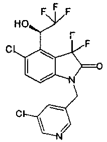 | 5-chloro-1-[(5-chloropyridin-3-yl)methyl]-3,3-difluoro-4-[(1R)-2,2,2-trifluoro-1-hydroxyethyl]-1,3-dihydro-2H-indol-2-one | 427(M+H) | 0.991 A | 1H NMR (600 MHz CHLOROFORM-d) d ppm 3.32 - 3.47 (1 H, m), 4.81 - 4.99 (2 H, m), 5.62 - 5.76 (1 H, m), 6.76 - 6.83 (1 H, m), 7.48 - 7.55 (1 H, m), 7.57-7.63 (1 H, m), 8.46 - 8.52 (1 H, m), 8.54 - 8.60 (1 H, m) | 1 |
| 96 | 1 | 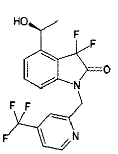 | 3,3-difluoro-4-[(1S)-1-hydroxyethyl]-1-{[4-(trifluoromethyl) pyridin-2-yl]methyl}-1,3-dihydro-2H-indol-2-one | 373(M+H) | 0.944 A | 1H NMR (600 MHz CHLOROFORM-d) d ppm 1.54 (3 H, d, J=6.6 Hz), 1.92 - 1.98 (1 H, m), 5.07 (2H, s), 5.27 - 5.35 (1 H, m) 6.79 - 6.91 (1 H, m) 7.34 - 7.55 (4 H, m), 8.69 - 8.80 (1 H, m) | 0.051 |

**[Table 1-13]**

| Compound No | Example | Structural formula | Compound Name | (ESI pos.) m/z (ESI neg.) m/z | LCMS RT (min) conditions | 1H-NMR | IC50 (*µ*M) |
|---|---|---|---|---|---|---|---|
| 97 | 1 | 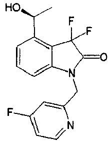 | 3,3-difluoro-1-[(4-fluoropyridin-2-yl)methyl]-4-[(1S)-1-hydroxyethyl]-1,3-dihydro-2H-indol-2-one | 323(M+H) | 0.802 A | 1H NMR (600 MHz CHLOROFORM-d) d ppm 1 52 - 1 58 (3 H, m), 1.92 - 1.97 (1 H m), 5.01 (2 H, s), 5.27 - 5.35 (1 H, m), 6.79 - 6.86 (1 H, m), 6.88 - 7.06 (2 H, m) 7.32 - 7.38 (1 H, m), 7.39 - 7.47 (1 H, m), 8.45 - 8.59 (1 H, m) | 0.091 |
| 98 | 1 | 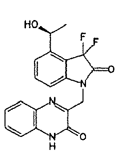 | 3-({3,3-difluoro-4-[(1S)-1-hydroxyethyl]-2-oxo-2,3-dihydro-1H-indol-1-yl}methyl)quinoxalin-2(1H)-one | 372(M+H) | 0.765 A | 1H NMR (600 MHz. CHLOROFORM-d) d ppm 1.55 - 1.58 (3 H, m) 5.09 - 5.20 (2 H, m) 5.31 - 5.39 (1 H, m) 6.69 - 6.75 (1 H, m) 7.15 - 7.20 (1 H m) 7.28 - 7.32 (1 H, m) 7.33 - 7.37 (1 H, m) 7.39 - 7.43 (1 H, m) 7.49 - 7.54 (1 H, m) 7.71 (1 H, d, J=8.26 Hz) 9.86 (1 H, br. s.) | 0034 |
| 99 | 1 | 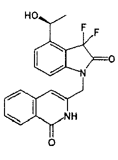 | 3-({3,3difluoro-4-[(1S)-1-hydroxyethyl]-2-oxo-2,3-dihydro-1H-indo-1-yl}methyl)isoquinolin-1(2H)-one | 371(M+H) | 0.778 A | 1H NMR (600 MHz CHLOROFORM-d) d ppm 1.52 - 1.55 (3 H, m) 4.81 - 4.90 (2 H, m) 5.27 - 5.34 (1 H, m) 6.50 (1 H, s) 6.93 (1 H, d, J=7.84 Hz) 7.36 - 7.40 (1 H, m) 7.41 - 7.46 (1 H, m) 7.48 - 7.55 (2 H, m) 7.64 - 7.70 (1 H, m) 8.38 (1 H, d, J=8.26 Hz) 10.42 (1 H, br. s.) | 0.048 |
| 100 | 5 | 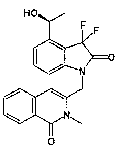 | 3-({3,3-difluoro-4-[(1S)-1-hydroxyethyl]-2-oxo-2,3-dihydro-1H-indol-1-yl}methyl)-2-methylisoquinolin-1(2H)-one | 385(M+H) | 0.842 A | 1H NMR (600 MHz CHLOROFORM-d) d ppm 1.57 (3 H, d, J=6.61 Hz) 3.66 (3 H, s) 4.88 - 4.98 (2 H, m) 5.30 - 5.38 (1 H, m) 6.33 - 6.36 (1 H, m) 6.75 - 6.82 (1 H, m) 7.38 - 7.46 (3 H, m) 7.46 - 7.52 (1 H, m) 7.60 - 7.65 (1 H, m) 8.40 (1 H, d, J=8.26 Hz) | 0029 |
| 101 | 5 | 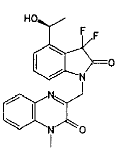 | 3-({3,3-difluoro-4-[(1S)-1-hydroxyethyl]-2-oxo-2,3-dihydro-1H-indol-1-yl}methyl)-1-methylquinoxalin-2(1 H)-one | 386(M+H) | 0.831 A | 1H NMR (600 MHz. CHLOROFORM-d) d ppm 1.56 - 1.59 (3 H, m) 1.98 (1 H, s) 3.74 (3 H, s) 5.09 - 5.19 (2 H, m) 5.33 - 5.40 (1 H, m) 6.69 (1 H, d, J=7.84Hz) 7.28 - 7.36 (3 H, m) 7.37 - 7.42 (1 H, m) 7.53 - 7.59 (1 H, m) 7.70 (1 H, dd, J=8.05, 1.44 Hz) | 0041 |
| 102 | 1 | 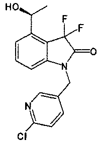 | 1-[(6-chloropyridin-3-yl)methyl]-3,3-difluoro-4-[(1S)-1-hydroxyethyl]-1,3-dihydro-2H-indol-2-one | 339(M+H) | 0.846 A | 1H NMR (600 MHz, CHLOROFORM-d) d ppm 1.53 (3 H, d, J=6.6 Hz), 1.54 - 1.68 (1 H, m), 4.84 - 4.93 (2 H, m), 5.21 - 5.37 (1 H, m), 6.63 - 6.71 (1 H, m), 7.29 - 7.49 (3 H, m), 7.56 - 7.63 (1 H, m), 8.39 - 8.46 (1 H, m) | 0.091 |
| 103 | 1 | 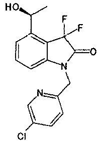 | 1-[(5-chloropyridin-2-yl)methyl]-3,3-difluoro-4-[(1S)-1-hydroxyethyl]-1,3-dihydro-2H-indol-2-one | 339(M+H) | 0.897 A | 1H NMR (600 Mhz, CHLOROFORM-d) d ppm 1.48 - 1.60 (3 H, m), 1.91 - 1.99 (1 H, m), 4.98 (2 H, s), 5.24 - 5.34 (1 H, m), 6.81 - 6.88 (1 H, m), 7.24 - 7.46 (3 H, m), 7.59 - 7.69 (1 H, m), 8.46 - 8.56 (1 H, m) | 0044 |
| 104 | 1 | 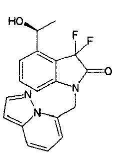 | 3,3-difluoro-4-[(1S)-1-hydroxyethyl]-1-(pyrazolo[1,5-a]pyridin-7-ylmethyl)-1,3-dihydro-2H-indol-2-one | 344(M+H) | 0.904 A | 1H NMR (600 MHz CHLOROFORM-d) d ppm 1.56 (3 H, d, J=6.19 Hz) 1.95 (1 H, br. s.) 5.30 - 5.37 (1 H, m) 5.44 (2 H, s) 6.56 - 6.61 (1 H, m) 6.63 (1 H, d, J=2.48 Hz) 6.83 - 6.87 (1 H, m) 7.03 - 7.10 (1 H, m) 7.35 - 7.40 (1 H, m) 7.42 (1 H, d J=7.84 Hz) 7.53 - 7.59 (1 H, m) 8.05 (1 H, d, J=2.48 Hz) | 0.088 |

**[Table 1-14]**

| Compound No | Example | Structural formula | Compound Name | (ESI pos.) m/z (ESI neg.) m/z | LCMS RT (min) conditions | 1H-NMR | Chiral HPLC analysis conditions (Column used) (Solvent system) (Flow rate) | Chiral HPLC RT (min) | IC50 (*µ*M) |
|---|---|---|---|---|---|---|---|---|---|
| 105 | 1 | 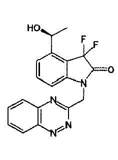 | 1-(1,2,4-benzotriazin-3-ylmethyl)-3,3-difluoro-4-[(1S)-1-hydroxyethyl]-1,3-dihydro-2H-indol-2-one | 357(M+H) | 0.813 A | 1H NMR(600 MHz CHLOROFORM-d) d ppm 1.56 (3 H, d, J=6.61 Hz) 1.96 (1 H, br. s.) 5.28 - 5.39 (1 H, m) 5.59 (2 H, s) 6.74 - 6.79 (1 H, m) 7.32 - 7.42 (2H, m) 7.87 - 7.93 (1 H, m) 7.97 - 8.03 (2 H, m) 8.50 - 8.58 (1 H, m) | | | 1 |
| 106 | 1 | 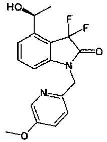 | 3,3-difluoro-4-[(1S)-1-hydroxyethyl]-1-[(5-methoxypyridin-2-yl)methyl]-1,3-dihydro-2H-indol-2-one | 335(M+H) | 0.791 A | 1H NMR(600 MHz CHLOROFORM-d) d ppm 1.52 (3 H, d, J=6.2 Hz), 1.98 (1 H, br. s.), 3.83 (3 H, s), 4.95 (2 H, s), 5.23 - 5.35 (1 H, m), 6.87 - 6.95 (1 H, m), 7.12 - 7.18 (1 H, m) 7.19 - 7.25 (1 H, m), 7.30 - 7.36 (1 H, m), 7.36 - 7.46 (1 H, m), 8.20 - 8.27 (1 H, m) | | | 081 |
| 107 | 1 | 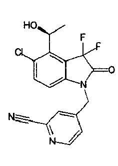 | 4-({5-chloro-3,3-difluoro-4-[(1S)-1-hydroxyethyl]-2-oxo-2,3-dihydro-1H-indol-1-yl}methyl)pyridine-2-carbonitrile | 364(M+H) | 0.846 A | 1H NMR(600 MHz CHLOROFORM-d) d ppm 1.64 (3 H, d, J=6.6 Hz), 2.20 - 2.59 (1 H, m), 4.82 - 5.03 (2 H, m), 5.37 - 5.48 (1 H, m), 6.48 - 6.56 (1 H, m), 7.36 - 7.47 (2 H, m), 7.54 - 7.62 (1 H, m), 8.67 - 8.76 (1 H, m) | | | 015 |
| 108 | 1 | 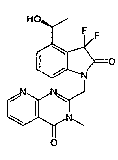 | 2-({3,3-difluoro-4-[(1S)-1-hydroxyethyl]-2-oxo-2,3-dihydro-1H-indol-1-yl}methyl)-3-methylpyrido[2,3-d]pyrimidin-4(3H)-one | 387(M-H) | 0.643 A | 1H NMR(600 MHz CHLOROFORM-d) d ppm 1.52 - 1.55 (3 H, m) 1.55 - 1.57 (1 H, m) 3.69 (3 H, s) 5.07 - 5.18 (2 H, m) 5.27 - 5.34 (1 H, m) 7.15 - 7.20 (1H, m) 7.36 - 7.41 (1 H, m) 7.42 - 7.51 (2 H, m) 8.62 (1 H, dd, J=7.84, 2.06 Hz) 8.99 (1 H, dd, J=4.54, 2.06 Hz) | | | 0.24 |
| 109 | 1 | 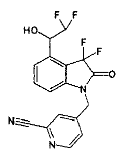 | (enantiomer 1) 4-{[4-(2,2-difluoro-1-hydroxyethyl)-3,3-difluoro-2-oxo-2,3-dihydro-1H-indol-1-yl]methyl}pyridine-2-carbonitrile | 366(M+H) | 0.802 A | 1H NMR (600 MHz. CHLOROFORM-d) d ppm 2.70 - 2.76 (1 H, m), 4.96 (2 H, s), 5.20 - 5.31 (1 H, m), 5.77 - 6.07 (1 H, m) 6.66 - 6.71 (1 H, m), 739-7.47 (2 H, m), 7.49 - 7.56 (1 H, m), 7.57 - 7.62 (1 H, m), 8.67 - 8.75 (1 H, m) | Shimadzu AD3. 4.6*250 Hex IPA= 70 : 30 1mL / min | 6.62, 7.93 Faster | 0.088 |
| 110 | 1 | 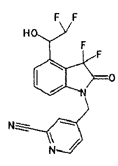 | (enantiomer 2) 4-{[4-(2,2-difluoro-1-hydroxyethyl)-3,3-difluoro-2-oxo-2,3-dihydro-1H-indol-1-yl]methyl}pyridine-2-carbonitrile | 366(M+H) | | | Shimadzu AD3, 4.6*250 Hex : IPA = 70 : 30 1mL / min | 6.62, 7.93 Later | 2.1 |
| 111 | 1 | 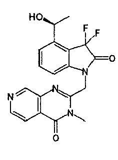 | 2-({3,3-difluoro-4-[(1s)-1-hydroxyethyl]-2-oxo-2,3-dihydro-1H-indol-1-yl}methyl)-3-methylpyrido[3,4-d]pyrimidin-4(3H)-one | 387(M+H) | 0.666 A | 1H NMR (600 MHz, CHLOROFORM-d) d ppm 1.57 - 1.58 (3 H, m) 2.01 - 2.08 (1 H, m) 3.71 (3 H, s) 5.04 (2 H, s) 5.29 - 5.39 (1 H, m) 6.94 (1 H, d, J=7.84 Hz) 7.39 - 7.49 (2 H, m) 8.02 (1 H, d, J=5.37 Hz) 8.69 (1 H, d, J=5.37 Hz) 8.98 (1 H, s) | | | 0 13 |
| 112 | 1 | 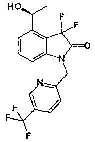 | 3,3-difluoro-4-[(1S)-1-hydroxyethyl]-1-{[5-(trifluoromethyl) pyridin-2-yl]methyl}-1,3-dihydro-2H-indol-2-one | 373(M+H) | 0.952 A | 1H NMR(600 MHz CHLOROFORM-d) d ppm 1.54 (3 H, d, J=6.2 Hz), 1.86 - 2.05 (1 H, m), 5.07 (2 H, s), 5.27-5.34 (1 H, m) 6.79 - 6.86 (1 H, m), 7.34 - 7.47 (3 H, m), 7.88 - 7.95 (1 H, m), 8.80 - 8.86 (1 H, m) | | | 0.029 |

**[Table 1-15]**

| Compound No | Example | Structural formula | Compound Name | (ESI pos.) m/z (ESI neg.) m/z | LCMS RT (min) condition | 1H-NMR | lC50 (*µ*M) |
|---|---|---|---|---|---|---|---|
| 113 | 1 | 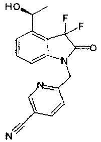 | 6-({3,3,difluoro-4-[(1S)-1-hydroxyethyl]-2-oxo-2,3-dihydro-1H-indol-1-yl}methyl) pyridine-3-carbonitrile | 330(M+H) | 0.775 A | 1H NMR (600 MHz CHLOROFORM-d) d ppm 1.54 (3 H, d, J=6.6 Hz), 1.98 (1 H, br. s.), 5.06 (2 H, s), 5.26 - 5.34 (1 H, m), 6.75 - 6.82 (1 H, m) 7.35 - 7.47 (3 H, m), 7.89 - 8.00 (1 H, m), 8.79 - 8.87 (1 H, m) | 094 |
| 114 | 1 | 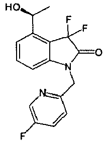 | 3,3-difluoro-1-[(5-fluoropyndin-2-yl)methyl]-4-[(1S)-1-hydroxyethyl)-1,3-dihydro-2H-indol-2-one | 323(M+H) | 0.812 A | 1H NMR (600 MHz, CHLOROFORM-d) d ppm 1.53 (3 H, d, J=6.2 Hz), 1.91 - 1.99 (1 H, m), 4.99 (2 H, s), 5.25 - 5.34 (1 H, m), 6.85 - 6.90 (1 H, m), 7.29 - 7.46 (4 H, m), 8.37 - 8.44 (1 H, m) | 0061 |
| 115 | 1 | 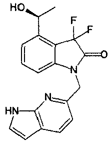 | 3,3-difluoro-4-[(1S)-1-hydroxyethyl-1-(1H-pyrrolo[2,3-b]pyridin-6-ylmethyl)-1,3-dihydro-2H-indol-2-one | 344(M+H) | 0.813 A | 1H NMR (600 MHz, CHLOROFORM-d) d ppm 1.52 (3 H, d, J=6.2 Hz), 1.99 - 2.07 (1 H, m), 5.07 (2 H, s), 5.25 - 5.34 (1 H, m), 6.44 - 6.53 (1 H, m), 6.84 - 6.89 (1 H, m), 7.04 - 7.11 (1 H, m), 7.28 - 7.40 (3 H, m), 7.86 - 7.93 (1 H, m), 8.77 (1 H, br. s.) | 0.098 |
| 116 | 2 | 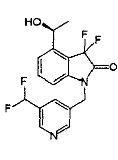 | 1-{[5-(difluoromethyl) pyridin-3-yl]methyl}-3,3-difluoro-4-[(1S)-1-hydroxyethyl]-1,3-dihydro-2H-indol-2-one | 355(M+H) | 0.768 A | 1H NMR (600 MHz, CHLOROFORM-d) d ppm 1.52 - 1.54 (3 H, m) 1.96 (1 H, br. s.) 4.89 - 5.02 (2 H, m) 5.26 - 5.35 (1 H, m) 6.59 - 6.82 (2 H, m) 7.37 - 7.41 (1 H, m) 7.43 - 7.48 (1 H, m) 7.77 (1 H, s) 8.71 - 8.76 (2 H, m) | 0.11 |
| 117 | 1 | 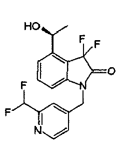 | 1-{[2-(difluoromethyl) pyridin-4-yl]methyl}-3,3-difluoro-4-[(1S)-1-hydroxyethyl]-1,3-dihydro-2H-indol-2-one | 355(M+H) | 0.798 A | 1H NMR (600 MHz, CHLOROFORM-d) d ppm 1.55 - 1.57 (3 H, m) 1.98 (1 H, br. s.) 4.96 (2 H, s) 5.27 - 5.36 (1 H, m) 6.51 - 6.77 (2 H, m) 7.27 - 7.30 (1 H,m) 7.38 - 7.47 (2 H, m) 7.56 (1 H, s) 8.63 (1 H, d, J=4.95 Hz) | 0031 |
| 118 | 1 | 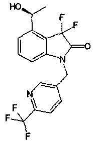 | 3,3-difluoro-4-[(1S)-1-hydroxyethyl]-1-{[6-(trifluoromethyl) pyridin-3-yl]methyl}-1,3-dihydro-2H-indol-2-one | 373(M+H) | 0.917 A | 1H NMR (600 MHz, CHLOROFORM-d) d ppm 1.53 (3 H, d, J=6.2 Hz), 1.97 (1 H, br. s.), 4.92 - 5.03 (2 H, m), 5.25 - 5.34 (1 H, m), 6.61 - 6.71 (1 H, m), 7.36 - 7.49 (2 H, m), 7.64 - 7.72 (1 H, m), 7.76 - 7.85 (1 H, m), 8.69 - 8.77 (1 H, m) | 0027 |
| 119 | 1 | 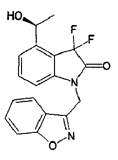 | 1-(1,2-benzoxazol-3-ylmethyl)-3,3-difluoro-4-[(1S)-1-hydroxyethyl]-1,3-dihydro-2H-indol-2-one | 365(M+H) | 0875 A | 1H NMR (600 MHz, CHLOROFORM-d) d ppm 1.50 (3 H, d, J=6.2 Hz), 1.17 - 2.03 (1 H, m), 5.24 - 5.30 (1 H, m), 5.31 - 5.41 (2 H, m), 6.99 - 7.07 (2 H, m), 7.28 - 7.33 (1 H, m), 7.36 - 7.40 (1 H, m), 7.47 - 7.52 (1 H, m), 7.53 - 7.59 (2 H, m) | 0077 |
| 120 | 1 | 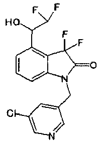 | (enantiomer 1) 1-[(5-chloropyridin-3-yl)methyl]-4-(2,2-difluoro-1-hydroxyethyl)-3,3-difluoro-1,3-dihydro-2H-indol-2-one | 375(M+H) | 0873 A | 1H NMR (600 MHz, CHLOROFORM-d) d ppm 1.56 (1 H, br. s.), 4.91 (2 H, s), 5.17 - 5.30 (1 H, m), 5.74 - 6.03 (1 H, m), 6.77 - 6.83 (1 H, m), 7.37 - 7.44 (1 H, m), 7.48 - 7.55 (1 H, m), 7.59 - 7.66 (1 H, m), 8.47 - 8.59 (2 H, m) | 0.057 |

**[Table 1-16]**

| Compound No | Example | Structural formula | Compound Name | (ESI pos.) m/z (ESI neg.) m/z | LCMS RT (min) condition | 1H-NMR | 1050 (µM) |
|---|---|---|---|---|---|---|---|
| 121 | 1 | 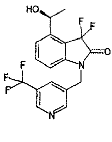 | 3,3-difluoro-4-[(1S)-1-hydroxyethyl]-1-{[5-trifluoromethyl)pyridin-3-yl]methyl}-1,3-dihydro-2H-indol-2-one | 373(M+H) | 0.880 A | 1H NMR (600 MHz, CHLOROFORM-d) d ppm 1.53 - 1.55 (3 H, m)1,98 (1H d J=3.30 Hz) 4.97 (2H m) 5.27 - 5.34 (1 H, m) 6.66 - 6.71 (1 H,m) 7.37-7 43 (1H, m) 7.44 - 7.50 (1H, m) 7.87 (1 H, s) 8.81 (1 H, s) 8.86 (1 H, s) | 0 039 |
| 122 | 1 | 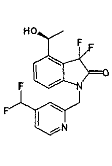 | 1-{[4-(difluoromethyl)pyridin-2-yl]methyl}-3,3-difluoro-4-{(1S)- -1-hydroxyethyl]-1,3-dihydro-2H-indol-2-one | 355(M+H) | 0.836 A | 1H NMR (600 MHz, CHLOROFORM-d) d ppm 1.52 - 1 55 (3 H, m) 1 94 - 1 97 (1 H, m) 5.05 (2 H, s) 5.27 - 5 35 (1 H m) 6.47 - 6.72 (1 H, m) 6.84 - 6.89 (1H, m) 7.34 - 7.40 (3 H, m) 7.41-7.46 (1 H, m) 8.69 (1 H, d J=4.95 Hz) | 0.22 |
| 123 | 1 | 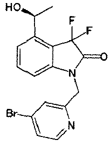 | 1-[(4-bromopyridin-2-yl)methyl]-3,3-difluoro-4-[(1S)-1-hydroxyethyl]-1,3-dihydro-2H-indol-2-one | 384(M+H) | 0890 A | 1H NMR (600 MHz CHLOROFORM-d) d ppm 1.54 (3 H d J=6 2 Hz) 184-2.05 (1 H, m), 4.97 (2 H, s), 5.27-5.35 (1 H, m), 6.80 - 6.89 (1 H, m), 7.33 - 7.48 (4 H, m). 8.33 - 8.40 (1 H, | 0.022 |
| 124 | 1 | 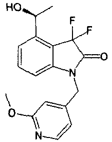 | 3,3-difluoro-4-[(1S)-1-hydroxyethyl]-1-[(2-methoxypyridin-4-yl)methyl]-1,3-dihydro-2H-indol-2-one | 335(M+H) | 0823 A | 1H NMR(600 MHz, CHLOROFORM-d) d ppm 1.54 (3 H, d, J=6.2 Hz), 1.90-2.04 (1 H, m), 3.92 (3 H, s), 4.77-4.89 (2 H, m), 5.28 - 5.36 (1H, m), 6.55 -6.64 (2 H, m), 6.74 - 6.80 (1 H, m), 7.34 - 7.45 (2H, m), 8.10 - 8.16 (1 H, m) | 0012 |
| 125 | 1 | 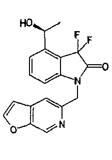 | 3,3-difluoro-1-(furo[2,3-c]pyridin-5-ylmethyl)-4-[(1S)-1-hydroxyethyl]-1,3-dihydro-2H-indol-2-one | 345(M+H) | 0.785 A | 1H NMR (600 MHz, CHLOROFORM-d) d ppm 1.51 - 1.54 (3 H, m) 1.92 - 1.96 (1 H, m) 5.11 (2 H, s) 5.27 - 5.33 (1 H, m) 6.75 - 6.80 (1 H, m) 6.93 - 6.99 (1H, m) 7.31 - 7.35 (1 H, m) 7.38-7 44 (1 H, m) 7.55 (1 H, s) 7.76 (1 H, s) 8.82 (1H, s) | 013 |
| 126 | 1 | 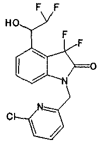 | (enantiomer 1) 1-[(6-chloropyridin-2-yl)methyl]-4-(2,2-difluoro-1-hydroxyethyl)-3,3-difluoro-1,3-dihydro-2H-indol-2-one | 375(M+H) | 0.922, A | 1H NMR (600 MHz CHLOROFORM-d) d ppm 2.51 - 2.69 (1 H, m), 4.99 (2 H, d. J=1.7 Hz) 5.19 - 5.27 (1 H, m), 5.78 -6.02 (1 H, m), 6.98 - 7.03 (1H, m) 7.16 - 7.21 (1 H, m), 7.27 - 7.31 (1 H, m). 7.35 - 7.40 (1H, m), 7.48 - 7.54 (1 H, m), 7.59 - 7.71 (1 H, m) | 00079 |
| 127 | 1 | 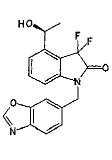 | 1-(1,3-benzoxazol-6-ylmethyl)-3,3-difluoro-4-[(1S)-1-hydroxyethyl]-1,3-dihydro-2H-indol-2-one | 345(M+H) | 0.802, A | 1H NMR (600 MHz, CHLOROFORM-d) d ppm 1 52 (3 H, d, J=10 32 Hz) 1 93 (1 H d, J=3.30 Hz), 4.98 - 5.07 (2 H, m), 5.26 - 5.35 (1H, m), 6.69 (1 H, d, J=7.84 Hz), 7.33 - 7 37 (2 H, m) 7 37 - 7.41 (1 H, m), 7.54 (1 H, s), 7.74-7.80 (1 H, m), 8.09 (1 H, s) | 0.029 |
| 128 | 1 | 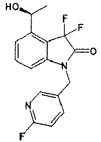 | 3,3-difluoro-1-[(S-fluoropyridin-3-yl)methyl]-4-[(1S)-1-hydroxyethyl]-1,3-dihydro-2H-indol-2-one | 323(M+H) | 0.791, A | 1H NMR(600 MHz CHLOROFORM-d) d ppm 1.53 (3 H, d, J=6.2 Hz), 1.86-201 (1 H, m), 4.89 (2 H d J=6.6 Hz) 5.24 - 5.33 (1 H, m), 6.66 - 6.73 (1 H, m), 6.90 - 6.96 (1 H, m), 7.35 - 7.48 (2 H, m), 7.70 - 7.80 (1 H, m), 8.21 - 8.27 (1 H, m) | 0052 |

**[Table 1-17]**

| Compound No | Example | Structural formula | Compound Name | (ESI pos.) m/z (ESI neg.) m/z | LCMS RT (min) condition | 1H-NMR | Chiral HPLC analysis conditions (Column used) (Solvent system) (Flow rate) | Chiral HPLC RT (min) | IC50 (µM) |
|---|---|---|---|---|---|---|---|---|---|
| 129 | 1 | 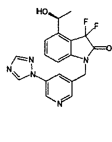 | 3,3-difluoro-4-[(1S)-1-hydroxyethyl]-1-([5-(1H-1,2,4-triazol-1-yl)pyndn-3-yl]methyl-1,3-dihydro-2H-indol-2-one | 372(M+H) | 0.645, A | 1H NMR (600 MHz, CHLOROFORM-d) d ppm 1.52 - 1.54 (3H, m), 1.92 - 2.09 (1 H, m), 4.94 - 5.05(2H, m), 5.26-5.34 (1 H, m), 6.73 (1 H, d, J=7.84 Hz), 7.37 - 7.42 (1H, m) 7.44 - 7.48 (1 H, m), 8.01 (1 H, t, J=2.27 Hz), 8.15 (1 H, s), 8.60 (1H, s), 8.86 (1 H d, J=1.65 Hz), 8.96 (1 H, d, J=2.48 Hz) | | | 69 |
| 130 | 1 | 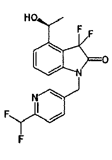 | 1-([6-(difluoromethyl) pyndin-3-yl]methyl}-3,3-difluoro-4-[(1S)-1-hydroxyethyl]-1,3-dihydro-2H-indol-2-one | 355(M+H) | 0.853, A | 1 H NMR (600 MHz, CHLOROFORM-d) d ppm 1.52 - 1.54 (3 H, m), 1.95 (1 H, br, s.), 4.91 - 5.02 (2 H, m), 5.26-5.33 (1 H, m). 6.50 - 6.74 (1 H, m), 6.67 (1 H, d, J=7.84 Hz), 7 36 - 7 41 (1 H, m), 7.42 - 7.46 (1 H, m) 7.63 (1 H, d, J=8.26 Hz). 7 78 (1 H, dd, J=8.05, 1.86 Hz). 866 (1 H, s) | | | 0.40 |
| 131 | 1 | 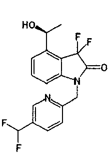 | 1-([5-(difluoromethyl) pyridin-2-yl]methyl)-3,3-difluoro-4-[(1S)-1-hydroxyethyl]-1,3-dihydro-2H-indol-2-one | 355(M+H) | 0.871, A | 1H NMR(600 MHz, CHLOROFORM-d) d ppm 1.52 - 1.54 (3 H, m), 1.92 (1 H, br. s). 5.06 (2 H, s), 5.26 - 5.34 (1 H, m), 6.57 - 6.80 (1 H, m), 6.83 (1 H, d, J=7.84 Hz), 7 33 - 7 46 (3 H, m) 7.83 (1 H, d, J=7.84 Hz), 8.70 (1 H, s) | | | 0.93 |
| 132 | 5 | 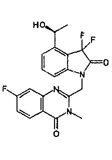 | 2-({3,3-difluoro-4-[(1S)-1-hydroxyethyl]-2-oxo-2,3-dihydro-1H-indol-1-yl}methyl)-7-fluoro-3-methylquinazolin-4(3H)-one | 404(M+H) | 0.857, A | 1H NMR (600 MHz CHLOROFORM-d) d ppm 1 57 (3 H, d J=6.61 Hz), 1.98-2.00 (1 H, m), 3.67 (3 H, s), 4.95-5.04 (2 H, m), 5.31 - 5.38 (1 H, m), 6.92 (1 H, d, J=7.43 Hz), 7.16 - 7.21 (2 H, m) 7.38 -7.42 (1 H, m), 7.62 - 7.47 (1 H, m), 8.21 - 8.30 (1 H, m) | | | 0018 |
| 133 | 1 | 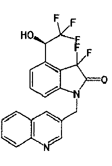 | 3,3-difluoro-1-(quinolin-3-ylmethyl)-4-[(1R)-2,2,2-trifluoro-1-hydroxyethyl]-1,3-dihydro-2H-indol-2-one | 409(M+H) | 0.905, A | 1H NMR (600 MHz, CHLOROFORM-d) d ppm 3.02 (1 H, d, J=4.95 Hz), 5.05-5.17 (2 H, m) 5.36 - 5.46 (1 H, m), 6.88 (1 H, d, J=6.61 Hz), 7.41 - 7.52 (2 H, m), 7.56 - 7.61 (1 H, m), 7.74 (1 H, ddd, J=8.46, 6.81, 1.24 Hz), 7.81 (1 H, d, J=8.26 Hz), 8.07 (1 H, d. J=1.24 Hz) 8.11 (1 H, d, J=8.26 Hz), 8.92 (1 H, d, J=2.06 Hz) | | | 0.062 |
| 134 | 1 | 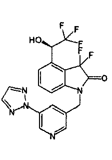 | 3,3-difluoro-1-{[5-(2H-1,2,3-triazol-2-yl)pyridin-3-yl]methyl}-4-[(1R)-2,2,2-trifluoro-1-hydroxyethyl]-1,3-dihydro-2H-indol-2-one | 426(M+H) | 0.911,A | 1H NMR (600 MHz, CHLOROFORM-d) d ppm 2 97 (1 H, d, J=4 95 Hz), 4.95-5.09 (2 H, m), 5.35 - 5 45 (1 H, m), 6.89 (1 H, d, J=7.84 Hz), 7.46 - 7.49 (1 H, m), 7.50 - 75.4 (1 H, m), 7.87 (2 H, s), 8.33 (1 H, t, J=2.27 Hz), 8.60 (1 H, d J=2.06 Hz), 9.36 (1 H d, J=2.06 Hz) | | | 0.066 |
| 135 | 1 | 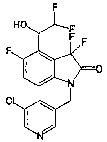 | (enantiomer 1) 1-[(5-chloropyridin-3-yl)methyl]-4-(2,2-difluoro-1-hydroxyethyl)-3,3,5-trifluoro-1,3-dihydro-2H-mdol-2-one | | | | AD3, 4.6*250mm Hex / IPA=80 / 20 1ml/min. | 9.79, 11.68 Faster | 0.057 |
| 136 | 1 | 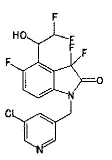 | (enantiomer 2)1-[(5-chloropyridin-3-yl)methyl-4-(2,2-difluoro-1-hydroxyethyl)-3,3,5-trifluoro-1,3-dihydro-2H-indol-2-one | | | | AD3, 4.6*250mm Hex / IPA=80 / 20 1 ml/min | 979 1168 Latter | 1.7 |

**[Table 1-18]**

| Compound No | Example | Structural formula | Compound Name | (ESI pos.) m/z (ESI neg) m/z | LCMS RT (min) condition | 1H-NMR | IC50 (*µ*M) |
|---|---|---|---|---|---|---|---|
| 137 | 1 | 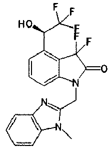 | 3,3-difluoro-1-[(1-methyl-1H-benzimidazol-2-yl)methyl-4-[(1R)-2,2,2-trifluoro-1-hydroxyethyl]-1,3-dihydro-2H-indol-2-one | 412(M+H) | 0.862, A | 1H NMR (600 MHz CHLOROFORM-d) d ppm 3.85 - 3.78 (3 H, m) 3.82 (3 H, s), 5.14 - 5.29 (2H, m), 5.33 - 5.41 (1 H, m) 7.27 - 7.37 (3 H, m), 742-747 (1H, m), 7.48 - 7.54 (1 H, m), 7.55-7 63 (1 H, m), 7.69 - 7.77 (1 H, m) | 015 |
| 138 | 1 | 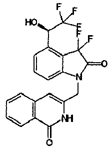 | 3-({3,3-difluoro-2-oxo-4-[(1R)-2,2,2-trifluoro-1-hydroxyethyl]-2,3-dihydro-1H-indol-1-yl}methyl)isoquinolin-1(2H)-one | 425(M+H) | 0.878, A | 1H NMR (600 MHz, CHLOROFORM-d) d ppm 3.67 - 3.90 (1 H, m), 4.81 - 4.98 (2 H, m), 5.33 - 5.44 (1 H, m), 6.49 (1 H, s), 7.02 - 7.12 (1 H, m), 7.35 - 7.53 (4 H, m) 7.57 - 7.75 (1 H, m) 8.27-8.42 (1 H, m), 11 60 - 11 96 (1 H, m) | 0 058 |
| 139 | 1 | 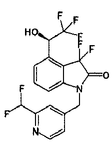 | 1-[[2-(difluoromethyl) pyridin-4-yl]methyl]-3,3-difluoro-4-[(1R)-2,2,2-trifluoro-1-hydroxyethyl]-1,3 dihydro-2H-indol-2-one | 409(M+H) | 0.923, A | 1H NMR (600 MHz, CHLOROFORM-d) d ppm 2.90 (1 H, d, J=4.95 Hz), 4.91-5.04 (2 H, m), 5.37 - 5.46 (1 H, m), 6.52 - 6.73 (1 H, m), 6.72 - 6.76 (1 H, m) 7.27 - 7.30 (1 H, m), 7.47 - 7.54 (2 H, m), 7.57 (1 H, s), 8.65 (1 H, d, J=4.95 Hz) | 0045 |
| 140 | 1 | 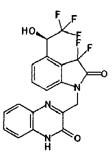 | 3-({3,3-difluoro-2-oxo-4-[(1R)-2,2,2-trifluoro-1-hydroxyethyl]-2,3-dihydro-1 H-indol-1-yl}methyl)quinoxalin-2(1H)-one | 426(M+H) | 0.872, A | 1H NMR (600 MHz, CHLOROFORM-d) d ppm 5.10 - 5.28 (2 H, m), 5.36 - 5.51 (1 H, m), 6.88 - 6.93 (1 H m) 7.21-7.29 (2 H, m), 7.30 - 7.35 (1 H, m), 7.38 - 7.50 (2 H, m), 7.51 - 7.57 (1 H, m),768-774(1 H, m), 11.05 - 11.22 (1 H, m) | 0033 |
| 141 | 1 | 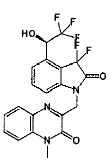 | 3-({3,3-difluoro-2-oxo-4-[(1R)-2,2,2-trifluoro-1-hydroxyethyl]-2,3-dihydro-1H-indol-1-yl}methyl)-1-methylquinoxalin-2(1H)-one | 440(M+H) | 0.952, A | 1H NMR (600 MHz, CHLOROFORM-d) d ppm 3.16 - 3.31 (1 H, m), 3.73 (3 H s), 5.15 (2 H, d, J=2.5 Hz), 5.41 - 5.50 (1 H, m), 6.81 - 6.88 (1 H, m), 7.28-7.36 (2H, m), 7.41 - 7.49 (2H, m), 7.53 - 7.60 (1 H, m), 7.65 - 7.71 (1 H, m) | 0.10 |
| 142 | 1 | 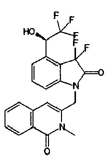 | 3-({3,3-difluoro-2-oxo-4-[(1R)-2,2,2-trifluoro-1-hydroxyethyl]-2,3-dihydro-1H-indol-1-yl}methyl)-2-methylisoquinolin-1(2H)-one | 439(M+H) | 0.933, A | 1H NMR (600 MHz CHLOROFORM-d) d ppm 3.63 (3 H, s), 3.77 - 3.95 (1 H, m), 4.82 - 5.03 (2 H, m), 5.39 - 5.53 (1 H, m), 6.37 (1 H, s), 6.90 - 6.98 (1 H, m), 7.34 - 7.57 (4 H, m), 7.57 - 7.66 (1 H, m) 8.31 - 8.40 (1 H, m) | 013 |
| 143 | 1 | 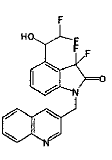 | (enantiomer 1) 4-(2,2-difluoro-1-hydroxyethyl)-3,3-difluoro-1-(quinolin-3-ylmethyl)-1,3-dihydro-2H-indol-2-one | 391(M+H) | 0.806, A | 1H NMR (600 MHz. CHLOROFORM-d) d ppm 2.93 (1 H, d, J=3.72 Hz) 5.10 (2 H, s), 5.25 (1 H, br. s.), 5.78 - 6.03 (1 H, m), 6.85 (1 H, d, J=7.84 Hz), 7.38 (1 H. d, J=7.84 Hz). 7.46 (1 H. t, J=8.05 Hz). 7 58 (1 H, t J=7.63 Hz). 7 74 (1 H. td J=7.64. 1 24 Hz). 7 80 (1 H, d J=8.26 Hz), 8.07 (1 H, d. J=1.65 Hz). 8.11 (1 H, d, J=8.26 Hz), 8.91 (1 H, d J=2.48 Hz) | 0 048 |
| 144 | 1 | 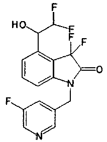 | (enantiomer 1) 4-(2,2-difluoro-1-hydroxyethyl)-3,3-difluoro-1-[(5-fluoropyridin-3-yl)methyl]-1,3-dihydro-2H-indol-2-one | 359(M+H) | 0.790, A | 1 H NMR (600 MHz, CHLOROFORM-d) d ppm 2.65 (1 H, d, J=3.72 Hz), 4.90-4.98 (2 H, m), 5.19 - 5.28 (1 H, m). 5.79 - 6.02 (1 H, m), 6.80 (1 H, d, J=7.84 Hz), 7.33 - 7.38 (1 H, m), 7.41 (1 H, d, J=7.84 Hz), 7.48 - 7.54 (1 H, m) 8.43 - 8.49 (2 H, m) | 0.24 |

**[Table 1-19]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 145 | 1 | 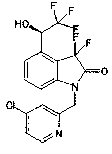 | 1-[(4-chloropyridin-2-yl)methyl]-3,3-difluoro-4-[(1R)-2,2,2-trifluoro-1-hydroxyethyl]-1,3-dihydro-2H-indol-2-one | 393(M+H) | 0.989, A | 1H NMR (600 MHz, CHLOROFORM-d) d ppm 2.80 - 2.92 (1 H, m) 4.99 (2 H, d, J=6.6 Hz), 5.33 - 5.46 (1 H, m), 7.00 - 7.08 (1 H, m), 7.24 - 7.28 (1H, m), 7.28 - 7.34 (1 H, m), 7.41 - 7.55 (2 H, m) 8.38 - 8.49 (1 H, m) | 0043 |
| 146 | 1 | 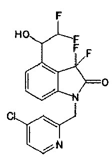 | (enantiomer 1) 1-[(4-chloropyridin-2-yl)methyl]-4-(2,2-difluoro-1-hydroxyethyl)-3,3-difluoro-1,3-dihydro-2H-indol-2-one | 375(M+H) | 0.903, A | 1H NMR (600 MHz, CHLOROFORM-d) d ppm 2.61 - 2.77 (1 H, m), 4.99 (2 H, s), 5.17 - 5.29 (1 H, m), 5.78 - 6.03 (1 H, m), 6.97 - 7.01 (1 H, m), 7.24 - 7.27 (1 H, m), 7.29 - 7.32 (1 H m), 7.35-7.40 (1 H, m), 7.45 - 7.53 (1 H, m), 8.38 - 8.49 (1 H, m) | 0.061 |
| 147 | 1 | 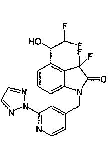 | (enantiomer 1) 4-(2,2-difluoro-1-hydroxyethyl)-3,3-difluoro-1-{[2-(2H-1,2,3-triazol-2-yl)pyridin-4-yl]methyl}-1,3-dihydro-2H-indol-2-one | 408(M+H) | 0.768, A | 1H NMR(600 MHz, CHLOROFORM-d) d ppm 2 74 (1 H, d, J=4.13 Hz), 502 (2 H, s), 5.21 - 5.32 (1 H, m), 5.78 - 6.09 (1 H, m), 6 75 (1 H, d. J=7 84 Hz) 7 20 (1 H, dd J=5.16, 103 Hz), 741 (1 H, d, J=8.26 Hz), 748 (1 H t. J=8.05 Hz), 7.91 (2 H, s), 8.04 (1 H, s), 8.58 (1 H, d. J=4.95 Hz) | 0.36 |
| 148 | 1 | 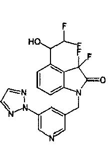 | (enantiomer 1) 4-(2,2-difluoro-1-hydroxyethyl)-3,3-difluoro-1-{[5-(2H-1(2,3-triazol-2-yl)pyridin-3-yl]methyl]-1,3-dihydro-2H-indol-2-one | 408(M+H) | 0.821, A | 1H NMR(600 MHz CHLOROFORM-d) d ppm 2.67 (1 H, d, J=4.13 Hz), 502 (2 H, s), 5.18 - 5.30 (1 H, m), 5.79 - 6.02 (1 H, m), 6.85 (1 H, d, J=7.84 Hz), 7.40 (1 H d, J=7.84 Hz), 7.47 - 7.54 (1 H, m), 7.87 (2 H, s), 8.33 (1 H, t, J=2.06 Hz) 8.61 (1 H, d, J=2.06 Hz), 9.36 (1 H, d, J=2 48 Hz) | 0039 |
| 149 | 1 | 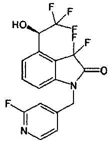 | 3,3-difluoro-1-[(2-fluoropyridin-4-yl)methyl]-4-[(1R)-2,2,2-trifluoro-1-hydroxyethyl]-1,3-dihydro-2H-indol-2-one | 377(M+H) | 0.909, A | 1H NMR (600 MHz, CHLOROFORM-d) d ppm 2.86 - 2.96 (1 H, m), 4.85 - 5.02 (2 H, m), 5.37 - 5.46 (1 H, m), 6.68-6.77 (1 H, m), 6.83 (1 H, s), 7.03-7.13 (1 H, m), 7.44 - 7.57 (2H, m), 8 14 - 8.27 (1 H, m) | 0.20 |
| 150 | 1 | 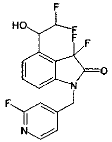 | (enantiomer 1) 4-(2,2-difluoro-1-hydroxyethyl)-3,3-difluoro-1-[(2-fluoropyridin-4-yl)methyl]-1,3-dihydro-2H-indol-2-one | 358(M+H) | 0.817, A | 1H NMR (600 MHz, CHLOROFORM-d) d ppm 2.69 - 2.79 (1 H, m), 4.93 (2 H, s), 5.19 - 5.32 (1 H, m), 5.77 - 6.06 (1 H, m), 6.69 - 6.73 (1 H, m), 6.83 (1 H, s), 7.07 - 7.13 (1 H, m), 7.38 - 7.45 (1 H, m), 7.47 - 7.55 (1 H, m), 8.18 - 8.26 (1 H, m) | 031 |
| 151 | 1 | 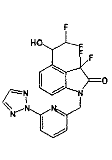 | (enantiomer 1) 4-(2,2-difluoro-1-hydroxyethyl)-3,3-difluoro-1-{[5-(2H-1,2,3-triazol-2-yl)pyridin-2-yl]methyl}-1,3-dihydro-2H-indol-2-one | 408(M+H) | 0.846, A | 1H NMR(600 MHz, CHLOROFORM-d) d ppm 2 65 (1 H. d. J=3 72 Hz). 5.12-5.20 (2 H, m), 5.21 - 5.29 (1H, m), 5.80 - 6.04 (1 H, m), 7.05 (1 H, d, J= 7.84 Hz). 7 23 (1 H, d, J=7.43 Hz). 7.37 (1 H, d, J=8.26 Hz), 7.44 - 7.50 (1 H, m), 7 88 (1 H, t. J=7.84 Hz). 7 94 (2 H s), 8.05 (1 H, d, J=8.26 Hz) | 0.27 |
| 152 | 1 | 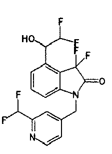 | (enantiomer 1) 4-(2,2-difluoro-1-hydroxyethyl)-1-{[2-(difluoromethyl) pyridin-4-yl]methyl}-3,3-difluoro-1,3-dihydro-2H-indol-2-one | 391(M+H) | 0.842, A | 1H NMR (600 MHz, DMSO-d6) d ppm 4.88 - 5.00 (1 H, m), 5.11 (2 H, s), 6.00 - 6.24 (1 H, m), 6.63 (1 H, br. s.), 6.83 - 7.09 (1 H, m), 7.16 (1 H, d, J=7.84 Hz) 7 37 (1 H, d J=8.26 Hz) 7.45 (1 H, d, J=4.95 Hz), 7.58 - 7.67 (2 H m) 8.66 (1 H, d J=5 37 Hz) | 0.088 |

**[Table 1-20]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 153 | 1 | 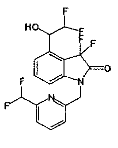 | (enantiomer 1) 4-(2,2-difluoro-1-hydroxyethyl)-1-{[6-(difluoromethyl) pyridin-2-yl]methyl]-3,3-difluoro-1,3-dihydro-2H-indol-2-one | 391(M+H) | 0.921, A | 1H NMR (600 MHz CHLOROFORM-d) d ppm 2 59 (1 H, d, J=4 13 Hz). 5.00-5.09 (2 H, m), 5.18 - 5.30 (1 H, m), 5.77 - 6.03 (1 H, m), 6.45 - 6.69 (1 H, m), 7.01 (1 H, d, J=8.26 Hz), 7.35-7.42 (2 H, m), 7.46 - 7.52 (1 H, m), 7.59 (1 H, d, J=7.84 Hz), 7.84 (1 H, t, J=7.84 Hz) | 0.13 |
| 154 | 1 | 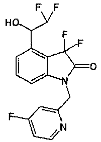 | (enantiomer 1) 4-(2,2-difluoro-1-hydroxyethyl)-3,3-difluoro-1-[(4-fluoropyridin-2-yl)methyl]-1,3-dihydro-2H-indol-2-one | 359(M+H) | 0.830, A | 1H NMR (600 MHz CHLOROFORM-d) d ppm 2.74 (1 H, d, J=4.1 Hz), 5.01 (2 H, s), 5.18 - 5.31 (1 H, m), 5.76 - 6.07 (1 H, m), 6.91 - 7.07 (3 H, m), 7.32-7.41 (1 H, m), 7.42 - 7.54 (1 H, m), 8.40 - 8.57 (1 H, m) | 0.26 |
| 155 | 1 | 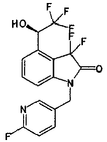 | 3,3-difluoro-1-[(6-fluoropyridin-3-yl)methyl]-4-[(1R)-2,2,2-trifluoro-1-hydroxyethyl]-1,3-dihydro-2H-indol-2-one | 377(M+H) | 0.921, A | 1H NMR (600 MHz, CHLOROFORM-d) d ppm 2.86 - 2.94 (1 H, m), 4.91 (2 H, s), 5.33 - 5.45 (1 H, m), 6.83 - 6.88 (1 H, m), 6.91 - 6.98 (1 H, m), 7.42 - 7.56 (2 H, m), 7.70 - 7.81 (1 H, m), 8.26 (1 H H, s) | 0.18 |
| 156 | 1 | 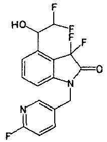 | (enantiomer 1) 4-(2,2-difluoro-1-hydroxyethyl)-3,3-difluoro-1-[(6-fluoropyridin-3-yl)methyl]-1,3-dihydro-2H-indol-2-one | 359(M+H) | 0.828, A | 1 H NMR (600 MHz. CHLOROFORM-d) d ppm 2.61 - 2.69 (1 H, m), 4.90 (2 H, d, J=3.7 Hz), 5.15 - 5.29 (1 H, m), 5.75 - 6.02 (1 H, m), 6.79 - 6.85 (1 H, m), 6.91 - 6.98 (1 H, m), 735 - 743 (1 H, m), 7.46 - 7.55 (1 H, m), 7.71 - 7.79 (1 H, m), 8.26 (1 H, s) | 0.11 |
| 157 | 1 | 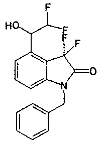 | (enantiomer 1) 1-benzyl-4-(2,2-difluoro-1-hydroxyethyl)-3,3-difluoro-1,3-dihydro-2H-indol-2-one | 340(M+H) | 0.973, A | 1H NMR (600 MHz, CHLOROFORM-d) d ppm 2.57 - 2.64 (1 H, m), 4.90 (2 H, s), 5.19 - 5.29 (1 H, m), 5.79 - 6.04 (1 H, m) 6.76 - 6.81 (1 H, m), 7.27 - 7.38 (6 H, m), 7.44 (1 H, s) | 0.021 |
| 158 | 1 | 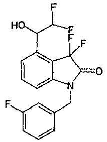 | (enantiomer 1) 4-(2,2-difluoro-1-hydroxyethyl)-3,3-difluoro-1-(3-fluorobenzyl)-1,3-dihydro-2H-indol-2-one | 358(M+H) | 0.979, A | 1H NMR (600 MHz CHLOROFORM-d) d ppm 2.58 - 2.66 (1 H, m), 4.89 (2 H, s), 5.18 - 5.30 (1 H, m), 5.79 - 6.03 (1 H, m), 6.74 - 6.80 (1 H, m), 6.96 - 7.10 (3 H, m), 7.29 - 7.40 (2 H, m), 7.43-7.50 (1 H, m) | 0016 |
| 159 | 1 | 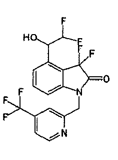 | (enantiomer 1) 4-(2,2-difluoro-1-hydroxyethyl)-3,3-difluoro-1-{[4-(trifluoromethyl) pyridin-2-yl]methyl}-1,3-dihydro-2H-indol-2-one | 409(M+H) | 0.966, A | 1H NMR (600 MHz CHLOROFORM-d) d ppm 2.55 - 2.67 (1 H, m), 5.08 (2 H, s), 5.18 - 5.29 (1 H, m), 5.78 - 6.03 (1 H, m) 6.95 - 7.05 (1 H, m), 7.35 - 7.41 (1 H, m), 7.44 - 7.54 (3 H, m), 8.68-8.77 (1 H, m) | 0.053 |
| 160 | 1 | 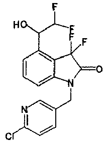 | (enantiomer 1) 1-[(6-chloropyridin-3-yl)methyl]-4-(2,2-difluoro-1-hydroxyethyl)-3,3-difluoro-1,3-dihydro-2H-indol-2-one | 375(M+H) | 0.880, A | 1H NMR (600 MHz, CHLOROFORM-d) d ppm 2.62 - 2.68 (1 H, m), 4.89 (2 H, m), d, J=3.7 Hz), 5.17 - 5.28 (1 H, m), 5.77 - 6.00 (1 H, m), 6.74 - 6.84 (1 H, m) 7.31 - 7.36 (1 H, m), 7.37 - 7.43 (1 H, m), 7.47 - 7.54 (1 H, m) , 7.57 - 7.63 (1 H, m), 8.39 - 8.45 (1 H, m) | 035 |

**[Table 1-21]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 161 | 1 | 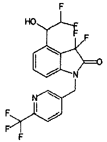 | (enantiomer 1) 4-(2,2-difluoro-1-hydroxyethyl)-3,3-difluoro-1-{[6-(trifluoromethyl) pyridin-3-yl]methyl}-1,3-dihydro-2H-indol-2-one | 409(M+H) | 0.949. A | 1 H NMR (600 MHz, CHLOROFORM-d) d ppm 2.62 - 2.68 (1 H, m), 5.00 (2 H, d, J=5.8 Hz), 5.18 - 5.21 (1 H.m), 5.76 - 6.03 (1H, m), 6.75 - 6.82 (1 H, m), 7.38 - 7.43 (1 H, m), 7.48 - 7.53 (1 H, m), 7.66 - 7.72 (1 H, m). 7.77 - 7.84 (1 H, m), 8.75 (1 H, s) | 0.090 |
| 162 | 1 | 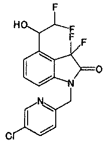 | (enantiomer 1) 1-[(5-chloropyridin-2-yl)methyl]-4-(2,2-difluoro-1-hydroxyethyl)-3,3-difluoro-1,3-dihydro-2H-indol-2-one | 375(M+H) | 0.932, A | 1H NMR (600 MHz, CHLOROFORM-d) d ppm 2.59 - 2.69 (1 H m), 4.99 (2 H, d, J=2.1 Hz), 5 15 - 5.28 (1 H, m), 5.73 - 6.02 (1 H, m), 6.96 - 7.03 (1 H, m), 719 - 729 (1 H. m). 732 - 739 (1 H, m), 7.44 - 7.52 (1 H, m), 7.61 - 7.70 (1 H, m), 8.46 - 8.56 (1 H, m) | 0 22 |
| 163 | 1 | 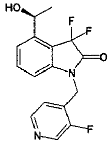 | 3,3-difluoro-1-[(3-fluoropyridin-4-yl)methyl]-4-[(1S)-1-hydroxyethyl]-1,3-dihydro-2H-indol-2-one | 323(M+H) | 0.724, A | 1H NMR (600 MHz, CHLOROFORM-d) d ppm 1.47 - 1.61 (3 H, m) 1.93 - 2.02 (1 H, m), 4.97 (2 H, s), 5.21 - 5.37 (1 H, m), 6.61 - 6.73 (1 H, m), 7.11 - 7.21 (1 H, m), 7.35 - 7.51 (2 H, m). 8.31 - 8.41 (1 H, m), 8.45 - 8.56 (1 H, m) | 041 |
| 164 | 1 | 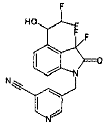 | (enantiomer 1) 5-{[4-(2,2-difluoro-1-hydroxyethyl)-3,3-difluoro-2-oxo-2,3-dihydro-1 H-indol-1-yl]methyl}pyridine-3-carbonitrile | 366(M+H) | 0.766 A | 1H NMR (600 MHz, CHLOROFORM-d) d ppm 2.68 (1 H, d, J=4.13 Hz), 4.91-5.02 (2 H, m), 5.18 - 5.31 (1 H, m) 5.77 - 6.05 (1 H, m), 6.79 (1 H, d, J=8.26 Hz), 7.44 (1 H, d, J=8.26 Hz), 7.50 - 7.58 (1 H, m), 7.90 (1 H, t, J=2.06 Hz), 8.78 - 8.91 (2 H, m) | 046 |
| 165 | 1 | 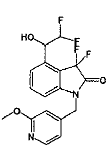 | (enantiomer 1) 4-(2,2-difluoro-1-hydroxyethyl)-3,3-difluoro-1-[(2-methoxypyridin-4-yl)methyl]-1,3-dihydro-2H-indol-2-one | 371(MtH) | 0.865, A | 1H NMR (600 MHz, CHLOROFORM-d) d ppm 2.63 1 H, d, J=4.13 Hz), 3.94 (3 H, s), 4.85 (2 H, s), 5.19 - 5.29 (1 H, m), 5.78 - 6.05 (1 H, m), 6 81 (i H, s), 6.72 (1 H, d, J=7.84 Hz), 6.77 (1 H, dd, J=5.37, 1.65 Hz), 7.38 (1 H, d, J=8.26 Hz), 7.44 - 7.50 (1 H, m), 8.14 ( 1 H, d, J=5.37 Hz) | 0.048 |
| 166 | 1 | 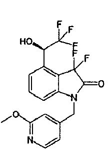 | 3,3-difluoro-1-[(2-methoxypyridin-4-yl)methyl]-4-[(1 R)-2,2,2-trifluoro-1-hydroxyethyl]-1,3-dihydro-2H-indol-2-one | 389(M+H) | 0.953, A | 1 H NMR (600 MHz, CHLOROFORM-d) d ppm 2.82 (1 H, d, J=4.95 Hz), 3.92 (3 H, s), 4.77 - 4.94 (2 H, m), 5.36 - 5.46 (1 H, m), 6.61 (1 H, s), 6.72 - 6.80 (2 H, m), 7.44 - 7.53 (2 H, m), 8.15 (1 H, d, J=4.95 Hz) | 0.026 |
| 167 | 1 | 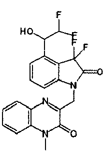 | (enantiomer 1) 3-{[4-(2,2-difluoro-1-hydroxyethyl)-3,3-difluoro-2-oxo-2,3 dihydro-1H-indol-1-yl]methyl}-1-methylquinoxalin-2(1H)-one | 422(M+H) | 0.873, A | 1H NMR (600 MHz, CHLOROFORM-d) d ppm 2.64 (1 H, d, J=3.72 Hz), 3.74 (3 H, s), 5.11 - 5.21 (2 H, m), 5.25 - 5.35 (1 H, m), 5.81 - 6.07 (1 H, m), 6.81 (1 H, d, J=7.84 Hz), 7.28 - 7.39 (3 H, m), 7.41 - 7.49 (1 H, m), 7.57 (1 H, td, J=7.84, 1.65 Hz), 7.69 (1 H, dd, J=8.26. 1 24 Hz) | 0.062 |
| 168 | 1 | 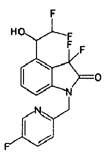 | (enantiomer 1) 4-(2,2-difluoro-1-hydroxyethyl)-3,3-difluoro-1-[(5-fluoropyridin-2-yl)methyl]-1,3-dihydro-2H-indol-2-one | 359(M+H) | 0.857, A | 1H NMR (600 MHz, CHLOROFORM-d) d ppm 2.58 - 2.72 (1 H, m), 5.00 (2 H, d. J=2.5 Hz), 5.16 - 5.28 (1 H, m), 5.76 - 6 03 (1 H, m), 6 99 - 7 06 (7 H, m), 7.30 - 7.43 (3 H, m), 7.46 - 7.52 (1 H, m), 8.38 - 8.44 (1 H, m) | 0 28 |

**[Table 1-22]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 169 | 1 | 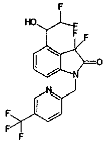 | (enantiomer 1) 4-(2,2-difluoro-1-hydroxyethyl)-3,3-difluoro-1-{[5-(trifluoromethyl)pyridin -2-yl]methyl}1,3-dihydro-2H-indol-2-one | 409(M+H) | 0.973, A | 1H NMR (600 MHz, CHLOROFORM-d) d ppm 2.61 - 2.67 (1 H, m), 5.08 (2 H, d, J=2.5 Hz), 5.19 - 5.28 (1 H, m), 5.79 - 6.03 (1 H, m), 6.93 - 7.00 (1 H, m), 7.36 - 7.40 (1 H, m), 7.41 - 7.46 (1 H, m), 7.47 - 7.54 (1 H, m), 7.98 - 7.96 (1 H, m), 8.81 - 8.86 (1 H, m) | 0056 |
| 170 | 1 | 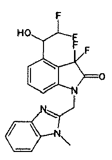 | (enantiomer 1) 4-(2,2-difluoro-1-hydroxyethyl)-3,3-difluoro-1-[(1-methyl-1H-benzimidazol-2-yl)methyl]-1,3-dihydro-2H-indol-2-one | 394(M+H) | 0.754, A | 1H NMR (600 MHz, CHLOROFORM-d) d ppm 2.74 - 2.85 (1 H, m), 3.83 (3 H, s),5.16 - 5.30 (3 H, m) 573-599(1 H, m), 7.27 - 7.39 (4 H, m), 7.48 - 7.55 (1 H, m), 7.57 - 7.63 (1 H, m), 7.76 (1 H. d, J=7.4 Hz) | 0.11 |
| 171 | 1 | 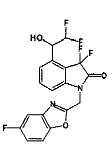 | (enantiomer 1) 4-(2,2-difluoro-1-hydroxyethyl)-3,3-difluoro-1-[(5-fluoro-1,3-benzoxazol-2-yl)methyl]-1,3 dihydro-2H-indol-2-one | 399(M+H) | 0.947, A | 1H NMR (600 MHz, CHLOROFORM-d) d ppm 2.79 - 2.97 (1 H, m), 5.17 (2 H, s), 5.20 - 5.30 (1 H, m), 5.76 - 6.03 (1 H, m), 7.00 - 7.15 (2 H, m), 7.33 - 7.48 (3 H, m), 7.50 - 7.57 (1 H, m) | 017 |
| 172 | 1 | 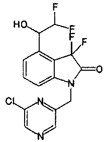 | (enantiomer 1) 1-[(6-chloropyrazin-2-yl)methyl]-4-(2,2-difluoro-1-hydroxyethyl)-3,3-difluoro-1,3-dihydro- , 2H-indol-2-one | 376(M+H) | 0.854, A | 1H NMR (600 MHz, CHLOROFORM-d) d ppm 2.63 (1 H, d, J=4.13 Hz), 4.98-5.06 (2 H, m), 5.19 - 5.29 (1 H, m), 5.79 - 6.02 (1 H, m), 7.03 (1 H, d J=7.84 Hz), 7.41 (1 H, d, J=7.84 Hz), 7.54 (1 H, t, J=8.05 Hz), 8.54 (1 H, s), 8.57 (1 H, s) | 0024 |
| 173 | 6 | 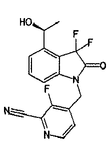 | 4-({3,3-difluoro-4-[(1S)-1-hydroxyethyl]-2-oxo-2,3-dihydro-1 H-indol-1-yl}methyl)-3-fluoropyridine-2-carbonitrile | 348(M+H) | 0.835, A | 1H NMR (600 MHz, CHLOROFORM-d) d ppm 1.48 - 1.59 (3 H, m) 1.95 - 2.02 (1 H, m), 5.01 (2 H, s), 5.26 - 5.35 (1 H, m), 6.65 - 6.72 (1 H, m), 7.39 - 7.54 (3 H, m), 8.47 - 8.53 (1 H, m) | 0019 |
| 174 | 2 | 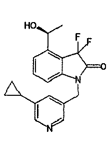 | 1-[(5-cyclopropylpyridin-3 yl)methyl]-3,3-difluoro-4-[(1S)-1-hydroxyethyl]-1,3-dihydro-2H-indol-2-one | 345(M+H) | 0.589, A | 1H NMR (600 MHz, CHLOROFORM-d) d ppm 0.67 - 0.73 (2 H, m), 0.99 - 1.07 (2 H, m), 1.53 (3 H, d. J=6.61 Hz), 1.83 - 1.90 (1 H, m), 1.96 (1 H, d, J=3.30 Hz), 4.80 - 4.89 (2 H, m), 5.26 - 5.34 (1 H, m), 6.70 (1 H, d, J=7.84 Hz), 7.21 - 7.30 (1 H, m), 7.34 - 7.37 (1 H, m), 7.40 - 7.45 (1 H, m), 8.33 (1 H, d, J=1.65 Hz), 8.38 (1 H, d, J=1.65 Hz) | 0095 |
| 175 | 1 | 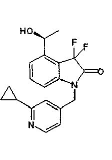 | 1-[(2-cyclopropylpyridin-4-yl)methyl]-3,3-difluoro-4-[(1S)-1-hydroxyethyl]-1,3-dihydro-2H-indol-2-one | 345(M+H) | 0.499, A | 1H NMR (600 MHz, CHLOROFORM-d) d ppm 0.93 - 1.06 (4 H, m), 1.49 - 1.55 (3 H, m), 1.95 - 2.02 (2 H, m), 4.84 (2 H, s), 5.26 - 5.37 (1 H, m), 6.61 (1 H, d, J=7.84 Hz), 6.91 (1 H, d, J=4.13 Hz), 7.03 (1 H, s), 7.36 - 7.39 (1 H, m), 7.39 - 7.46 (1 H, m), 8.39 (1 H, d, J=5.37 Hz) | 0023 |
| 176 | 1 | 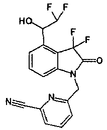 | (enantiomer 1) 6-{[4-(2,2-difluoro-1-hydroxyethyl)-3,3-difluoro-2-oxo-2,3 dihydro-1 H-indol-1-yl]methyl}pyridine-2-carbonitrile | 364(M-H) | 0.838, A | 1H NMR (600 MHz CHLOROFORM-d) d ppm 2.58 - 2.66 (1 H, m), 5.04 (2 H, d, J=3.3 Hz), 5.16 - 5.27 (1 H, m), 5.75 - 6.05 (1 H, m), 6.99 - 7.07 (1 H, m), 7.39 (1 H, d, J=7.8 Hz), 7.48 - 7.58 (2 H, m), 7.64 - 7.71 (1 H, m), 7.80-7.91 (1 H, m) | 0.041 |

**[Table 1-23]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 177 | 1 | 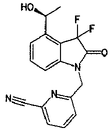 | 6-({3,3-difluoro-4-[(1 S)-1-hydroxyethyl]-2-oxo-2,3-dihydro-1H-indol-1-yl}methyl) pyridine-2-carbonitrile | 328(M-H) | 0.800, A | 1H NMR (600 MHz CHLOROFORM-d) d ppm 1.53 (3 H, d, J=6.2 Hz), 1.94 - 1.98 (1 H, m), 5.03 (2 H, s), 5.26 - 5.33 (1 H, m), 6.82 - 6.92 (1 H, m), 7.35 - 7.40 (1 H, m), 7.44 - 7.49 (1 H, m), 7.50 - 7.56 (1 H, m), 7.63 - 7.68 (1 H, m), 7.80 - 7.87 (1 H, m) | 0.024 |
| 178 | 1 | 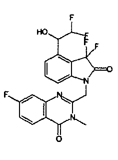 | (enantiomer 1) 2-{[4-(2,2-difluoro-1-hydroxyethyl)-3,3-difluoro-2-oxo-2,3-dihydro-1 H-indol-1-yl]methyl}-7-fluoro-3-methylquinazolin-4(3H)-one | 440(M+H) | 0.951, A | 1H NMR (600 MHz CHLOROFORM-d) d ppm 2.67 (1 H, d, J=3.72 Hz), 3.68 (3 H, s), 5.00 (2 H, s), 5.23 - 5.34 (1 H, m), 5.80 - 6.09(1 H, m), 7.04 (1 H, d, J=7.84 Hz), 7.13 - 7.21 (2 H, m), 7.39 - 7.45 (1 H, m), 7.48 - 7.54 (1 H, m), 8.22 - 8.30 (1 H, m) | 0.044 |
| 179 | 1 | 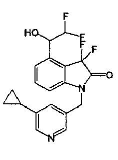 | (enantiomer 1) 1-[(5-cyclopropylpyridin-3-yl)methyl]-4-(2,2-difluoro-1-hydroxyethyl)-3,3-difluoro-1,3-dihydro-2H-indol-2-one | 381(M+H) | 0.704, A | 1H NMR (600 MHz, CHLOROFORM-d) d ppm 0.66 - 0.75 (2 H, m), 1.00 - 1.08 (2 H, m),1.82 - 1.92 (1 H, m), 2.77 (1 H, d, J=3.72 Hz), 4.86 (2 H, s), 5.18 - 5.29 (1 H, m), 5.75 - 6.04 (1 H, m), 6.81 (1H, d, J=7.84 Hz), 7.22 - 7.29 (1 H, m), 7.38 (1 H, d, J=8.26 Hz), 7.48 (1 H, t, J=8.05 Hz). 8.33 (1 H, d, J=2.06 Hz), 8.38 (1 H, d, J₌2. 06 Hz) | 0.48 |
| 180 | 1 | 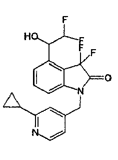 | (enantiomer 1) 1-[(2-cyclopropylpyridin-4-yl)methyl]-4-(2,2-difluoro-1-hydroxyethyl)-3,3-difluoro-1,3-dihydro-2H-indol-2-one | 381(M+H) | 0.616, A | 1H NMR (600 MHz, CHLOROFORM-d) d ppm 0.94 - 1.07 (4 H, m), 1.95 - 2.02 (1 H, m), 2.73 (1 H, d, J=4.13 Hz), 4.85 (2 H, s), 5.19 - 5.31 (1 H, m), 5.79 - 6.06 (1 H, m), 6.72 (1 H, d, J=7.84 Hz), 6.88 - 6.94 (1 H, m), 7.03 (1 H, s), 7.39 (1 H, d, J=8.26 Hz), 7.45 - 7.51 (1 H, m), 8.40 (1 H, d, J=5.37 Hz) | 0.053 |
| 181 | 1 | 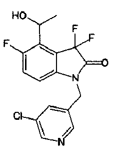 | (enantiomer 1) 1-[(5-chloropyridin-3-yl)methyl]-3,3,5-trifluoro-4-(1-hydroxyethyl)-1,3-dihydro-2H-indol-2-one | 357(M+H) | | | 0.31 |
| 182 | 1 | 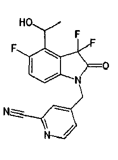 | (enantiomer 1) 4-{[3,3,5-trifluoro-4-(1-hydroxyethyl)-2-oxo-2,3-dihydro-1H-indol-1-yl]methyl}pyridine-2-carbonitrile | 348(M+H) | | | 1.7 |
| 183 | 1 | 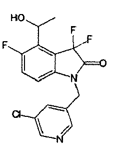 | (enantiomer 2) 1-[(5-chloropyridin-3-yl)methyl]-3,3,5-trifluoro-4-(1-hydroxyethyl)-1,3-dihydro-2H-indol-2-one | 357(M+H) | 0.825, A | 1H NMR (600 MHz. CHLOROFORM-d) d ppm 1.64 (3 H, d, J=6.6 Hz), 2.06 - 2.27 (1 H, m) 4.88 (2 H, d, J= 14.0 Hz), 5.20 - 5.36 (1 H, m), 6.55 - 6.67 (1 H, m), 7.05 - 7.19 (1H, m), 7.54 - 7.68 (1 H, m), 8.42 - 8.60 (2 H, m) | 0.0067 |
| 184 | 1 | 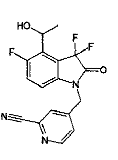 | (enantiomer 2) 4-{[3,3,5-trifluoro-4-(1-hydroxyethyl)-2-oxo-2,3-dihydro-1H-indol-1-yl]methyl}pyridine-2-carbonitrile | 348(M+H) | 0.761, A | 1H NMR (600 MHz. CHLOROFORM-d) d ppm 1.66 (3 H, d, J=6.6 Hz), 2.24 (1 H, br. s.), 4.85 - 5.01 (2 H, m), 5.31 (1 H, br. s.), 6.46 - 6.56 (1 H, m), 7.09 - 7.20 (1 H, m), 7.36 - 7.43 (1 H, m), 7.54 - 7.62 (1 H, m), 8.68 - 8.75 (1 H, m) | 0.095 |

**[Table 1-24]**

| Compound No | Example | Structural formula | Compound Name | (ESt pos.) m/z (ESI neg.) m/z | LCMS RT (min) condition | 1H-NMR | IC50 (*µ*M) |
|---|---|---|---|---|---|---|---|
| 185 | 1 | 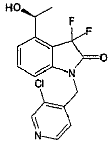 | 1-[(3-chloropyridin-4-yl)methyl]-3,3-difluoro-4-[(1S)-1-hydroxyethyl]-1,3-dihydro-2H-indol-2-one | 339(M+H) | 0.800, A | 1H NMR (600 MHz, CHLOROFORM-d) d ppm 1.55 - 1.58 (3 H,), 2.04 (1 H, s), 4.97 - 5.05 (2 H, m), 5.27 - 5.37 (1 H, m), 6.58 (1 H, d, J=7.4 Hz), 7.02 (1 H, d, J=5.0 Hz), 6.98 - 7.04 (1 H, m), 7.37 - 7.48 (2 H, m), 8.39 - 8.45 (1 H, m),8.64 (1 H, s) | 0.28 |
| 186 | 1 | 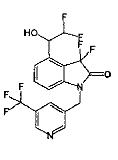 | (enantiomer 1) 4-(2,2-difluoro-1-hydroxyethyl)-3,3-difluoro-1-{[5-(trifluoromethyl) pyridin-3-yl]methyl}-1,3-dihydro-2H-indol-2-one | 409(M+H) | 0.972, A | 1H NMR (600 MHz, CHLOROFORM-d) d ppm 2.60 - 2.71 (1 H, m), 4.99 (2 H, s), 5.17 - 5.29 (1 H, m), 5.76 - 6.05 (1 H, m), 6.81 (1 H, d, J=7.84 Hz), 7.42 (1 H, d, J=8.26 Hz), 7.49 - 7.57 (1 H, m), 7.87 (1 H, s), 8.82 (1 H, s), 8.87 (1 H, s) | 0.064 |
| 187 | 6 | 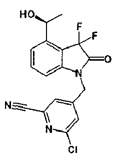 | 6-chloro-4-({3,3-difluoro-4-[(1S)-1-hydroxyethyl]-2-oxo-2,3-dihydro-1H-indol-1-yl}methyl)pyridine-2-carbonitrile | 362(M-H) | 0.902, A | 1H NMR (600 MHz, CHLOROFORM-d) d ppm 1.57 (3 H, d, J=6.6 Hz), 1.95 - 2.02 (1 H, m), 4.92 (2 H, d, J=8.3 3 Hz), 5.28 - 5.36 (1H, m), 6.53 - 8.59 (1 H, m), 7.43 - 7.53 (4 H, m) | 0.034 |
| 188 | 1 | 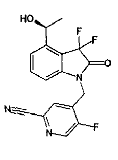 | 4-({3,3-difluoro-4-[(1 S)-1-hydroxyethyl]-2-oxo-2,3-dihydro-1H-indol-1-yl}methyl)-5-fluoropyridine-2-carbonitrile | 346(M-H) | 0.804, A | 1H NMR (600 MHz, CHLOROFORM-d) d ppm 1.51 - 1.60 (3 H, m), 2.01 (1 H, s), 4.99 (2 H, m), 6.62 - 6.67 (1 H, m), 7.41 - 7.53 (2 H, m), 7.55 - 7.61 (1 H, m), 8.61 (1 H, s) | 0.062 |
| 189 | 1 | 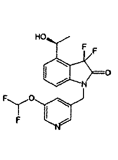 | 1-{[5-(difluoromethoxy) pyridin-3-yl]methyl}-3,3-difluoro-4-[(1S)-1-hydroxyethyl]-1,3-dihydro-2H-indol-2-one | 371(M+H) | 0.875, A | 1H NMR (600 MHz, CHLOROFORM-d) d ppm 1.54 (3 H, d, J=6.61 Hz), 1.95 (1 H, br. s.), 4.86 - 4.99 (2 H, m), 5.25 - 5.35 (1, H m), 6.41 - 6.67 (1 H, m), 6.69 (1 H, d, J=7.84 Hz), 7.33 - 7.49 (3 H, m), 8.46 (1 H, d. J=2.06 Hz), 8.49 (1 H, d, J=1.65 Hz) | 0.079 |
| 190 | 1 | 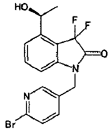 | 1-[(6-bromopyridin-3-yl)methyl]-3,3-difluoro-4-[(1S)-1-hydroxyethyl]-1,3-dihydro-2H-indol-2-one | 383(M+H) | 0.939, A | 1H NMR (600 MHz CHLOROFORM-d) d ppm 1.53 (3 H, d, J=6.61 Hz), 1.94 (1 H, br s.), 4.77 - 4.92 (2 H, m), 5.24 - 5.33 (1 H, m), 6.67 (1 H, d, J=8.26 Hz), 7.36 - 7.40 (1 H, m), 7.42 - 7.54 (3 H, m), 8.40 (1 H, d, J₌2. 06 Hz) | 0.051 |
| 191 | 1 | 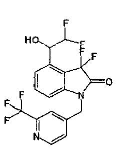 | (enantiomer 1) 4-(2,2-difluoro-1-hydroxyethyl)-3,3-difluoro-1-{[2-(trifluoromethyl) pyridin-4-yl]methyl}-1,3-dihydro-2H-indol-2-one | 409(M+H) | 0991 A | 1H NMR (600 MHz, CHLOROFORM-d) d ppm 2.64 (1 H, d, J=4.13 Hz), 4.99 (2 H, s), 5.20 - 5.33 (1 H, m), 5.78 - 6.05 (1 H, m), 6.70 (1 H, d, J=7.84 Hz), 7.37 (1 H, d, J=4.95 Hz), 7.43 (1 H, d, J=8.26 Hz), 7.49 - 7.55 (1 H, m), 7.61 (1 H, s), 8.73 (1 H, d, J=4.95 Hz) | 0.029 |
| 192 | 6 | 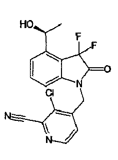 | 3-chloro-4-({3,3-difluoro-4-[(1S)-1-hydroxyethyl]-2-oxo-2,3-dihydroxyethyl]-2-oxo-2,3-dihydro-1H-indol-1-yl}methyl) pyridine-2-carbonitrile | 362(M-H) | 0.880, A | 1H NMR (600 MHz, CHLOROFORM-d) d ppm 1.53 - 1.59 (3 H, m), 1.99 - 2.02 (1 H, m), 5.04 (2 H, s), 5.29 - 5.37 (1 H, m), 6.53 - 6.59 (1 H, m), 7.21 - 7.25 (1 H, m), 7.40 - 7.52 (3 H, m) 8.54 (1 H, s) | 0.067 |

**[Table 1-25]**

| Compound No | Example | Structural formula | Compound Name | (ESI pos.) m/z (ESI neg.) m/z | LCMS RT (min) condition | 1H-NMR | IC50 (*µ*m) |
|---|---|---|---|---|---|---|---|
| 193 | 7 | 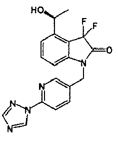 | 3,3-difluoro-4-[(1S)-1-hydroxyethyl]-1-{[6-(1H-1,2,4-triazol-1-yl)pyridin-3-yl]methyl}-1,3-dihydro-2H-indol-2-one | 372(M+H) | 0.799, A | 1H NMR (600 MHz, CHLOROFORM-d) d ppm 1.52 - 1.54 (3 H, m), 1.94 (1 H, s), 4.87 - 5.02 (2 H, m), 5.25 - 5.35 (1 H, m), 6.72 (1 H, d, J=7.84 Hz), 7.36 - 7.41 (1 H, m), 7.42 - 7.48 (1 H, m), 7.81 - 7.87 (1H, m), 7.89 - 7.94 (1 H, m), 8.09 (1 H, s), 8.47 (1 H, d, J=1.65 Hz), 9.14 (1 H, s) | 1.4 |
| 194 | 1 | 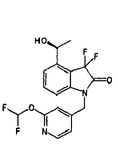 | 1-{[2-(difluoromethoxy) pyridin-4-yl]methyl}-3,3-difluoro-4-[(1S)-1-hydroxyethyl]-1,3-dihydro-2H-indol-2-one | 371(M+H) | 1.013, A | 1H NMR (600 MHz, CHLOROFORM-d) d ppm 1.54 - 1.56 (3 H, m), 1.96 (1 H, br. s), 4.83 - 4.94 (2 H, m), 5.28 - 5.36 (1 H, m), 6.58 (1 H, d, J=7.84 Hz), 6.78 (1 H, s), 7.00 (1 H, d, J=4.95 Hz), 7.31 - 7.60 (3 H, m), 8.17 (1 H, d, J=5.37 Hz) | 0.043 |
| 195 | 1 | 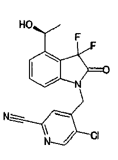 | 5-chloro-4-({3,3-difluoro-4-[(1S)-1-hydroxyethyl]-2-oxo-2,3-dihydro-1H-indol-1-yl}methyl)pyridine-2-carbonitrile | 362(M-H) | 0.873, A | 1H NMR (600 MHz, CHLOROFORM-d) d ppm 1.59 (3 H, d, J=6.2 Hz), 2.02 - 2.06 (1 H, m) 5.01 (2 H, d, J=11.6 Hz), 5.30 - 5.39 (1 H, m), 6.51 - 6.57 (1 H, m), 7.37 (1 H, d, J=0.8 Hz), 7.44 - 7.51 (2 H, m), 8.73 (1 H, s) | 0.042 |
| 196 | 1 | 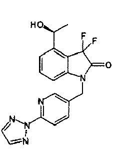 | 3,3-difluoro-4-[(1S)-1-hydroxyethyl]-1-{[6-(2H-1,2,3-triazol-2-yl)pyridin-3-yl]methyl}-1,3-dihydro-2H-indol-2-one | 372(M+H) | 0.816, A | 1H NMR (600 MHz, CHLOROFORM-d) d ppm 1.51 - 1.58 (4 H, m), 4.91 - 5.04 (2 H, m), 5.25 - 5.34 (1 H, m), 6.71 (1 H, d, J=7.43 Hz), 7.34 - 7.39 (1 H, m), 7.41 - 7.46 (1 H, m), 7.84 (1 H, dd, J=8.46, 2.27 Hz), 7.90 (2 H, s) 8.08 (1 H, d, J=8.26 Hz), 8.61 (1 H, d, J=2.06 Hz) | 4.2 |
| 197 | 1 | 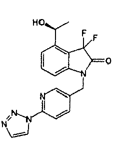 | 3,3-difluoro-4-[(1S)-1-hydroxyethyl]-1-{[6-(1H-1,2,3-triazol-1-yl)pyridin-3-yl]methyl}-1,3-dihydro-2H-indol-2-one | 372(M+H) | 0.830, A | 1H NMR (600 MHz. CHLOROFORM-d) d ppm 1.54 (3 H, d, J=6.61 Hz), 1.96 (1 H, br. s.), 4.91 - 5.03 (2 H, m), 5.27 - 5.34 (1 H, m), 6.72 (1 H, d, J=7.43 Hz), 7.35 - 7.41 (1 H, m) 7.42 - 7.48 (1 H, m), 7.83 (1 H, d, J=1.24 Hz), 7.88 (1 H, dd, J=8.46, 2.27 Hz), 8.22 (1H, d, J=8.67 Hz), 8.51 (1 H, d, J=1.65 Hz), 8.56 (1 H, d, J=1.24 Hz) | 6.7 |
| 198 | 1 | 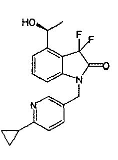 | 1-[(6-cyclopropylpyridin-3-yl)methyl]-3,3-difluoro-4-[(1S)-1-hydroxyethyl]-1,3-dihydro-2H-indol-2-one | 345(M+H) | 0.673, A | 1H NMR (600 MHz, CHLOROFORM-d) d ppm 0.96 - 1.02 (4 H, m), 1.52 (3 H, d, J=6.61 Hz), 1.96 (1 H, d, J=3.30 Hz), 1.98 - 2.06 (1 H, m), 4.77 - 4.90 (2 H, m), 5.24 - 5.33 (1 H, m), 6.70 (1 H, d, J=7.43 Hz), 7.11 (1 H, d, J=8.26 Hz), 7.34 (1 H, d, J=7.84 Hz), 7.41 (1 H, t, J=7.87 Hz), 7.48 (1 H, dd, J=7.84 2.48 Hz), 8.43 (1 H, d, J=2.06 Hz) | 0.16 |
| 199 | 1 | 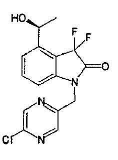 | 1-[(5-chloropyrazin-2-yl)methyl]-3,3-difluoro-4-[(1S)-1-hydroxyethyl]-1,3-dihydro-2H-indol-2-one | 340(M+H) | 0.918, A | 1H NMR (600 MHz, CHLOROFORM-d) d ppm 1.53 (3 H, d, J=6.61 Hz), 1.96 (1 H br. s.), 4.97 - 5.09 (2 H, m), 5.26 - 5.34 (1 H, m), 6.86 (1 H, d, J=8.26 Hz), 7.38 (1 H, d, J=7.84 Hz), 7.43 - 7.50 (1 H, m), 8.44 (1 H, s), 8.53 (1 H, d, J=1.65 Hz) | 0.11 |
| 200 | 1 | 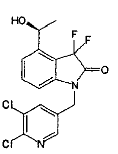 | 1-[(5,6-dichloropyridin-3-yl)methyl]-3,3-difluoro-4-[(1S)-1-hydroxyethyl]-1,3-dihydro-2H-indol-2-one | 373(M+H) | 1.041, A | 1 H NMR (600 MHz, CHLOROFORM-d) d ppm 1.53 - 1.54 (3 H, m), 1.94 (1 H, d, J=3.30 Hz), 4.78 - 4.93 (2 H, m), 5.24 - 5.34 (1 H, m), 6.68 (1 H, d, J=7.84 Hz), 7.40 (1 H, d, J=7.84 Hz), 7.44 - 7.50 (1 H, m), 7.73 (1 H, d, J=2.06 Hz), 8.32 (1 H, d, J=2.06 Hz) | 0.0086 |

**[Table 1-26]**

| Compound No | Example | Structural formula | Compound Name | (ESI pos.) m/z (ESI neg.) m/z | LCMS RT (min) condition | 1H-NMR | IC50 (µM) |
|---|---|---|---|---|---|---|---|
| 201 | 1 | 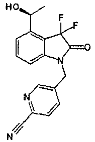 | 5-({3,3-difluoro-4-[(1S)-1-hydroxyethyl)-2-oxo-2,3-dihydro-1-indol-1-yl}methyl) pyridine-2-carbonitrile | 328(M-H) | 0.784, A | 1H NMR (600 MHz CHLOROFORM-d) d ppm 1.54 (3 H d, J=6.2 Hz). 1.95 - 2.02 (1 H, m), 4.97 (2 H, d, J=7.4 Hz), 5.25 - 5.35 (1 H, m). 6 56 - 6.68 (1 H, m), 7.36 - 7.51 (2 H, m), 7.64 - 7.81 (2 H, m), 8 73 (1 H, d, J=1.7 Hz) | 0.83 |
| 202 | 6 | 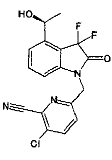 | 3-chloro-6-({3,3-difluoro-4-[(1S)-1-hydroxyethyl]-2-oxo-2,3-dihydro-1H-indol-1-yl}methyl)pyridine-2-carbonitrile | 362(M-H) | 0.895, A | 1H NMR (600 MHz CHLOROFORM-d) d ppm 1.51 - 1.54 (3 H, m), 1.93 - 1.98 (1 H, m), 5.01 (2 H, s), 5.25 - 5.33 (1 H, m), 6.85 - 6.92 (1 H, m), 7.37 - 7.42 (1 H, m), 7.44 - 7.54 (2 H, m), 7.85 (1 H, d d, J=8.3 Hz) | 0.0069 |
| 203 | 1 | 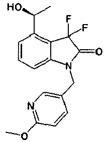 | 3,3-difluoro-4-[(1S)-1-hydroxyethyl]-1-[(6-methoxypyndin-3-yl)methyl]-1,3-dihydro-2H-ndol-2-one | 335(M+H) | 0.837, A | 1H NMR (600 MHz, CHLOROFORM-d) d ppm 1.52 (3 H, d, J=6.6 Hz), 1.54 - 1.79 (1 H, m), 3.94 (3 H, s), 4.82 (2 H, d, J=10.3 Hz), 5.18 - 5.32 (1 H, m), 6.67 - 6.79 (2 H, m) 7.32 - 7.38 (1 H, m), 7.39 - 7.47 (1 H, m), 7.51 - 7.63 (1 H, m), 8.06 - 8.24 (1 H, m) | 0031 |
| 204 | 1 | 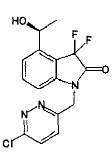 | 1-[(6-chloropyridazin-3-yl)methyl]-3,3-difluoro-4-[(1S)-1-hydroxyethyl]-1,3-dihydro-2H-ndol-2-one | 340(M+H) | 0.809, A | 1H NMR (600 MHz, CHLOROFORM-d) d ppm 1 52 (3 H, d, J=6 61 Hz), 1.89 - 1.96 (1 H, m), 5.16 - 5.25 (2 H, m), 525 - 5.31 (1 H, m), 7.03 (1 H, d, J=7.43 Hz), 7 37 (1 H, d, J=8.26 Hz), 7.44 - 7.49 (1 H, m), 7.51 - 7.55 (2 H, m) | 0.11 |
| 205 | 1 | 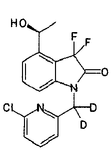 | 1-[(6-chloropyridin-2-yl)(2H2)methyl]-3,3-difluoro-4-[(1S)-1-hydroxyethyl]-1,3-dihydro-2H-ndol-2-one | 341(M+H) | 0.967, A | 1H NMR (600 MHz, CHLOROFORM-d ppm 1.52 - 1.54 (3 H, m), 1.95 (1 H, br. s.), 5.25 - 5.36 (1 H, m), 6.87 (1 H, d, J=7.84 Hz), 7.17 (1 H, dd, J=7.84, 0.83 Hz), 7.28 (1 H, dd, J=7.84, 0.83 Hz), 7.36 (1 H, d, J=7.84 Hz), 7.45 (1 H, t, J=7.84 Hz), 7.63 (1 H, t, J=7.84 Hz) | 0.0056 |
| 206 | 1 | 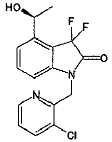 | 1-[(3-chloropyridin-2-yl)methyl]-3,3-difluoro-4-[(1S)-1-hydroxyethyl]-1,3-dihydro-2H-indol-2-one | 339(M+H) | 0.915, A | 1H NMR (600 MHz, CHLOROFORM-d) d ppm 1.54-1.56 (3 H, m), 1.94 (1 H, d, J=3.30 Hz), 5.09 - 5 17 (2 H, m), 5 28 - 5 37 (1 H, m), 6.67 (1 H, d, J=7.43 Hz) 7.20 (1 H, dd, J=8.05, 4.75 Hz), 7.31 - 7.34 (1 H, m), 7.35 - 7.40 (1 H, m), 7.70 (1 H, dd, J=8.05, 1.44 Hz). 8.40 (1 H, dd, J=4.75, 1 45 Hz) | 0.060 |
| 207 | 1 | 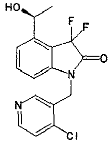 | 1-[(4-chloropyridin-3-yl)methyl]-3,3-difluoro-4-[(1S)-1-hydroxyethyl]-1,3-dihydro-2H-indol-2-one | 339(M+H) | 0793 A | 1H NMR (600 MHz, CHLOROFORM-d) d ppm 1.52 - 1.55 (3 H, m), 1.98 (1 H, d, J=2.89 Hz), 4.98 - 5.11 (2 H, m) 5.27 - 5.36 (1 H m) 6 65 (1 H, d, J=7.84 Hz), 7.35 - 7.40 (2 H, m), 7.40 - 7.45 (1 H, m), 8.44 (1 H, s), 8.48 (1 H, d, J=4.95 Hz) | 0.094 |
| 208 | 1 | 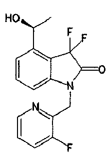 | 3,3-difluoro-1-[(3-fluoropyridin-2-yl)methyl)-4-[(1S)-1-hydroxyethyl]-1,3 dihydro-2H-indol-2-one | 323(M+H) | 0.864, A | 1H NMR (600 MHZ, CHLOROFORM-d) d ppm 1.52 - 1.54 (3 H, m), 1.95 (1 H, br, s.), 5.10 (2 H, d, J=1.65 Hz), 5.27 - 5.35 (1 H, m), 6.81 (1 H, d J=7.84 Hz), 7.24 - 7.30 (1 H, m), 7.33 (1 H, d, J=7.84 Hz), 7.37 - 7.45 (2 H, m), 8.36 (1 H, d, J=4.54 Hz) | 015 |

**[Table 1-27]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 209 | 1 | 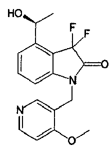 | 3,3-difluoro-4-[(1S)-1-hydroxyethyl]-1-[(4-methoxypyridin-3-yl)methyl]-1,3-dihydro-2H-indol-2-one | 335(M+H) | 0.674, B | 1H NMR (600 MHz, CHLOROFORM-d) d ppm 1 53 (3 H, d, J=6.61 Hz), 1.97 (1 H, br. s.), 3.91 (3 H, s), 4.85 - 4.95 (2 H, m), 5.24 - 5.34 (1 H, m), 6.72 (1 H, d, J=7.84 Hz), 6.81 (1 H, d, J=5.37 Hz), 7.29 - 7.35 (1 H, m), 7.36 - 7.43 (1 H, m), 8.37 (1 H, s.), 8.46 (1 H, d, J=5.37 Hz) | 28 |
| 210 | 1 | 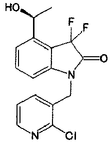 | 1-[(2-chloropyridin-3-yl)methyl]-3,3-difluoro-4-[(1S)-1-hydroxyethyl]-1,3-dihydro-2H-indol-2-one | 339(M+H) | 0.894, A | 1H NMR (600 MHz, CHLOROFORM-d) d ppm 1.51 - 1.54 (3 H, m), 1.97 (1 H, d, J=3.30 Hz), 5.02 (2 H, s), 5.27 - 5.37 (1 H, m), 6.68 (1 H, d, J=7.43 Hz), 7.21 - 7.24 (1 H, m), 7.38 - 7.41 (1 H, m), 7.42 - 7.51 (2 H, m), 8.37 (1 H, dd, J=4.75, 1.44 Hz) | 0 68 |
| 211 | 1 | 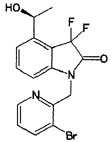 | 1-[(3-bromopyridin-2-yl)methyl]-3,3-difluoro-4-[(1S)-1-hydroxyethyl]-1,3-dihydro-2H-indol-2-one | 383(M+H) | 0.984, A | 1H NMR (600 MHz, CHLOROFORM-d) d ppm 1.55 (3 H, d, J=6.61 Hz), 1.95 (1 H, d, J=3.30 Hz), 5.04 - 5.15 (2 H, m), 5.29 - 5.38 (1 H, m), 6.62 (1 H, d, J=7.43 Hz) 7 11 (1 H, dd, J=8.05, 4.75 Hz), 7.31 - 7.41 (2 H, m), 7.87 (1 H, dd, J=8.05, 1.45 Hz), 8.42 (1 H, dd, J=4.75, 1.44 Hz) | 0085 |
| 212 | 1 | 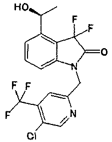 | 1-{[5-chloro-4-(trifluoromethyl) pyridin-2-yl]methyl}-3,3-difluoro-4-[(1S)-1-hydroxyethyl]-1,3-dihydro-2H-indol-2-one | 405(M-H) | 1.042, A | 1H NMR (600 MHz, CHLOROFORM-d) d ppm 1.54 (3 H, d, J=6.6 Hz), 1.92 - 1.99 (1 H, m), 5.03 (2 H, s) 5.26 - 5.35 (1 H, m), 7.41 - 7.48 (1 H, m), 7.57 (1 H, s), 8.68 (1 H, s) | 0.0082 |
| 213 | 1 | 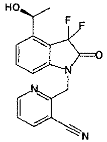 | 2-({3,3-difluoro-4-[(1S)-1-hydroxyethyl]-2-oxo-2,3dihydro-1-indol-1-yl}methyl) pyridine-3-carbonitrile | 330(M+H) | 0.834, A | 1H NMR (600 MHz CHLOROFORM-d) d ppm 1.55 (3 H, d, J=6.61 Hz), 1.96 (1 H, d, J=3.30 Hz), 5.19 - 5.28 (2 H, m), 5.29 - 5.36 (1 H, m), 6.63 (1 H, d, J=7.84 Hz), 7.31 - 7.45 (3 H, m), 8.00 (1 H, dd, J=7.84, 206 Hz), 8.70 (1 H dd, J=4.95, 1.65 Hz) | 0040 |
| 214 | 1 | 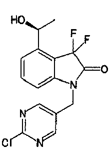 | 1-[(2-chloropyrimidin-5-yl)methyl]-3,3-difluoro-4-[(1S)-1-hydroxyethyl]-1,3-dihydro-2H-indol-2-one | 340(M+H) 0837, 340(M+H) | 0.837, A | 1H NMR (600 MHz, CHLOROFORM-d) d ppm 1.52 - 1.54 (3 H, m), 1.95 (1 H, d, J=3.30 Hz), 4.89 (2 H, q, J=16.10 Hz), 5.25 - 5.34 (1 H, m), 6.70 (1 H, d, J=7.43 Hz), 7.39 - 7.44 (1 H, m), 7.46 - 7.52 (1 H, m), 8.64 (2 H, s) | 0.14 |
| 215 | 1 | 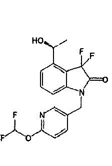 | 1-{[6 (difluoromethoxy) pyridin-3-yl]methyl}-3,3-difluoro-4-[(1S)-1-hydroxyethyl]-1,3-dihydro-2H-indol-2-one | 371(M+H) | 1.019, A | 1H NMR (600 MHz, CHLOROFORM-d) d ppm 1.53 (3 H, d, J=6.61 Hz), 1.93 (1 H, d, J=3.30 Hz), 4.79 - 4.93 (2 H, m), 5.25 - 5.33 (1 H, m), 6.70 (1 H, d, J=7.84 Hz). 6 90 (1 H, d, J=8.67 Hz), 7.29 - 7 57 (3 H, m), 7 70 (1 H, dd. J=8.67 2 48 Hz), 8 20 (1 H, d, J=2.48 Hz) | 0.24 |
| 216 | 1 | 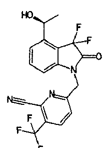 | 6-({3,3-difluoro-4-[(1S)-1-hydroxyethyl]-2-oxo-2,3dihydro-1H-indol-1-yl}methyl)-3-(trifluoromethyl) pyridine-2-carbonitrile | 396(M-H) | 1.013, A | 1H NMR (600 MHz CHLOROFORM-d) d ppm 1.51 - 1.56 (3 H, m), 1.95 (1 H, br. s.), 5.10 (2 H, d, J=1.7 Hz), 5.30 (1 H, d, J = 7.0 Hz), 6.86 (1 H, d, J=7.8 Hz), 7.41 J=7.0 Hz), 6.86 (1 H, d, J=7.8 Hz), 7.41 (1 H, d, J=8.3 Hz), 7.47 - 7.52 (1 H, m), 7.68 (1 H d, J=8.3 Hz), 8.11 (1 H, d, J=8.3 Hz) | 00094 |

**[Table 1-28]**

| Compound No | Examples | Structural formula | Compound Name | (ESI pos.) m/z (ESI neg.) m/z | LCMS RT (min) condition | 1H-NMR | IC50 (*µ*M) |
|---|---|---|---|---|---|---|---|
| 217 | 1 | 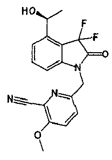 | 6-({3,-difluoro-4-[(1S)-1-hydroxyethyl]-2-oxo-2,3-dihydro-1H-indol-1-yl}methyl)-3-methoxypyridine-2-carbonitrile | 360(M+H) | 0.907, A | 1H NMR (600 MHz CHLOROFORM-d) d ppm 1.52 (3 H, d, J=6.6 Hz), 1.93 (1 H, br. s.), 3.95 (3 H, s), 4.96 (2 H, s), 5.28 (1 H, d, J=6.6 Hz), 6.96 (1 H, d, J=7.8 Hz), 7.31 - 7.39 (2 H, m), 7.45 - 7.50 (1 H, m), 7.52 (1 H, d, J=8.7 Hz) | 012 |
| 218 | 1 | 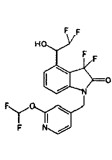 | (enantiomer 1) 4-(2,2-difluoro-1-hydroxyethyl)-1-{[2-(difluoromethoxy) pyridin-4-yl]methyl}-3,3-difluoro-1,3-dihydro-2H-indol-2-one | 407(M+H) | 1.037, A | 1H NMR (600 MHz, CHLOROFORM-d) d ppm 2.65 - 2.73 (1 H, m), 4.90 (2 H, s), 5.19 - 5.30 (1 H, m), 5.79 - 6.04 (1 H, m), 6.67 - 6.73 (1 H, m), 6.76 - 6.81 (1 H, m), 6.97 - 7.03 (1 H, m), 7.31 - 7.60 (3 H, m), 8.15 - 8.21 (1 H, m) | 0.010 |
| 219 | 1 | 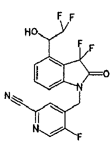 | (enantiomer 1) 4-{[4-(2,2-difluoro-1-hydroxyethyl)-3,3 difluoro-2-oxo-2,3-dihydro-1H-indol-1-yl]methyl}-5-fluoropyridine-2-carbonitrile | 384(M+H) | 0.924, A | 1H NMR (600 MHz, CHLOROFORM-d) d ppm 2.71 - 2.76 (1 H, m), 5.00 (2 H, s), 5.21 - 5.30 (1 H, m) 5.81 - 6.04 (1 H, m), 6.77 - 6.82 (1 H, m), 7.45 - 7.64 (3 H, m), 8.60 - 8.63 (1 H, m) | 018 |
| 220 | 1 | 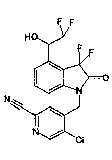 | (enantiomer 1) 5-chloro-4-{[4-(2,2-difluoro-1-hydroxyethyl)-3,3-difluoro-2-oxo-2,3-dihydro-1H-indol-1-yl)methyl}pyridine-2-carbonitrile | 400(M+H) | 0.987, A | 1H NMR (600 MHz, CHLOROFORM-d) d ppm 2.66 - 2.69 (1 H, m), 5.03 (2 H, s), 5.24 - 5.32 (1 H, m), 5.84 - 6.06 (1 H, m), 6.66 - 6.70 (1 H m), 7.38 - 7.40 (1 H, m), 7.47 - 7.57 (2 H, m), 8.73 - 8.75 (1 H, m) | 0.17 |
| 221 | 1 | 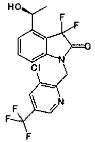 | 1-{[3-chloro-5-(trifluoromethyl) pyridin-2-yl]methyl}-3,3-difluoro-4-[(1S)-1-hydroxyethyl]-1,3-dihydro-2H-indol-2-one | 405(M-H) | 1.054, A | 1H NMR (600 MHz, CHLOROFORM-d) d ppm 1.56 (3 H d, J=6.6 Hz), 1.93 - 2.03 (1 H, m), 5.17 (2 H, s), 5.29 - 5.37 (1 H, m), 6.59 - 6.65 (1 H, m). 7.30 - 7.45 (2 H, m), 7.96 (1 H, d, J=1.7 Hz), 8.65 (1 H, s) | 0033 |
| 222 | 1 | 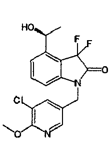 | 1-[(5-chloro-6-methoxypyridin-3-yl)methyl]-3,3-difluoro-4-[(1S)-1-hydroxyethyl]-1,3-dihydro-2H-indol-2-one | 369(M+H) | 1.005, A | 1H NMR (600 MHz, CHLOROFORM-d) d ppm 1.52 - 1.54 (3 H, m) 1.93 (1 H, d, J=3.30 Hz) 4.00 (3 H, s) 4.75 - 4.85 (2 H, m) 5.26 - 5.33 (1 H, m) 6.73 (1 H, d, J=7.84 Hz) 737 (1 H, d, J=826 Hz) 7.42 - 7.48 (1 H, m) 7.61 (1 H, d, J=2.48 Hz) 8.06 (1 H, d, J=2.06 Hz) | 0.0086 |
| 223 | 1 | 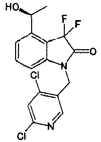 | 1-[(4,6-dichloropyridin-3-yl)methyl]-3,3-difluoro-4-[(1S)-1-hydroxyethyl]-1,3-dihydro-2H-indol-2-one | 373(M+H) | 1.042, A | 1H NMR (600 MHz, CHLOROFORM-d) d ppm 1.54 (3 H, br. s.), 1.96 (1 H, d, J=3.3 Hz), 4.94 - 5.06 (2 H, m), 5.31 (1 H, d, J=6.6 Hz), 6.64 (1 H, d, J=7.4 Hz), 7.37 - 7.47 (3 H, m), 8.23 (1 H, s) | 0.066 |
| 224 | 1 | 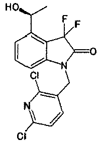 | 1-[(2,6-dichloropyridin-3-yl)methyl]-3,3-difluoro-4-[(1S)-1-hydroxyethyl]-1,3-dihydro-2H-indol-2-one | 373(M+H) | 1.071, A | 1H NMR (600 MHz, CHLOROFORM-d) d ppm 1 54 (3 H, s), 1.96 (1 H, br. s.). 498 (2 H, s), 5.31 (1 H, d, J=7.0 Hz), 6.67 (1 H, d, J=7.4 Hz), 7.25 (1 H, s). 7.39 - 7.42 (1 H, m), 7.44 - 7.48 (2 H, m) | 074 |

**[Table 1-29]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 225 | 1 | 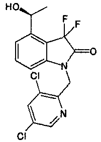 | 1-[(3,5-dichloropyridin-2-yl)methyl)-3,3-difluoro-4-[(1S)-1-hydroxyethyl]-1,3-dihydro-2H-indol-2-one | 373(M+H) | 1.093, A | 1H NMR (600 MHz, CHLOROFORM-d) d ppm 1.54 - 1.56 (3 H, m), 1.95 (1 H, br. s.), 5.09 (2 H, s), 5.32 (1 H, d, J=6.6 Hz) 6.65 (1 H, d, J=7.8), 7.32 - 7.36 (1 H, m), 7.36 - 7.42 (1 H, m), 7.74 (1 H, d, J=2.1 Hz), 8.36 (1 H, d, J=2.1 Hz) | 0.057 |
| 226 | 1 | 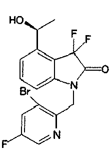 | 1-[(3-bromo-5-fluoropyridin-2-yl)methyl]-3,3-difluoro-4-[(1S)-1-hydroxyethyl]-1,3-dihydro-2H-indol-2-one | 401(M+H) | 0.992, A | 1H NMR (600 MHz, CHLOROFORM-d) d ppm 1.54 - 1.56 (3 H, m), 1.96 (1 H, br. s.), 5.08 (2 H, s), 5.28 - 5.37 (1 H, m), 6.61 (1 H, d, J=7.02 Hz), 7.33 - 7.40 (2 H, m), 7.67 (1 H, dd, J=7.43, 2.48 Hz), 8.31 (1 H, d, J=2.48 Hz) | 0040 |
| 227 | 1 | 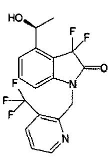 | 3,3-difluoro-4-[(1S)-1-hydroxyethyl)-1-{[3-(trifluoromethyl) pyridin-2-yl]methyl}-1,3-dihydro-2H-indol-2-one | 373(M+H) | 0.985, A | 1H NMR (600 MHz, CHLOROFORM-d) d ppm 1.56 (3 H d, J=6.61 Hz), 1.96 (1 H, d, J=3.30 Hz), 5.13 - 5.24 (2 H, m), 5.31 - 5.39 (1H, m), 6.51 (1 H, d, J=6.61 Hz), 7.29 - 7.40 (3 H, m), 7.99 (1 H, d, J=7.02 Hz), 8.61 (1 H, d, J=4.13 Hz) | 0.38 |
| 228 | 1 | 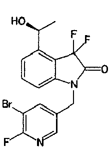 | 1-[(5-bromo-6-fluoropyridin-3-yl)methyl)-3,3-difluoro-4-[(1S)-1-hydroxyethyl]-1,3-dihydro-2H-indol-2-one | 401(M+H) | 0.982, A | 1H NMR (600 MHz, CHLOROFORM-d) d ppm 1.53 - 1.54 (3 H, m), 1.95 (1 H, d, J=3.30 Hz), 4.82 - 4.91 (2 H, m), 5.25 - 5.34 (1 H, m), 6,70 (1 H, d, J=7.43 Hz), 7.40 (1 H, d, J=7.84 Hz), 7.45 - 7.51 (1 H, m), 7.94 (1 H, dd, J=7.84, 2.06 Hz) 8.16 (1 H, d, J=0.83 Hz) | 0.0021 |
| 229 | 1 | 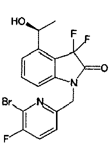 | 1-[(6-bromo-5-fluoropyridin-2-yl)methyl]-3,3-difluoro-4-[(1S)-1-hydroxyethyl]-1,3-dihydro-2H-indol-2-one | 401(M+H) | 0.991, A | 1H NMR (600 MHz, CHLOROFORM-d) d ppm 1.52 - 1.54 (3 H, m), 1.93 (1 H, d, J=3.30 Hz), 4.97 (2 H, s), 5.26 - 5.33 (1 H, m), 6.88 (1 H, d, J=7.84 Hz), 7.22 - 7.26 (1 H, m) 7.35 - 7.42 (2 H, m), 7.44 - 7.49 (1 H, m) | 00017 |
| 230 | 1 | 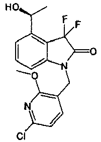 | 1-[(6-chloro-2-methoxypyridin-3-yl)methyl]-3,3-difluoro-4-[(1S)-1-hydroxyethyl]-1,3-dihydro-2H-indol-2-one | 369(M+H) | 1.126, A | 1H NMR (600 MHz CHLOROFORM-d) d ppm 1.53 (3H, d, J=6.2 Hz), 1.93 (1 H, br. s.), 4.03 (3 H, s), 4.82 (2 H, s), 5.30 (1 H, d, J=6.2 Hz), 6.74 (1 H, d, J=7.8 Hz), 6.88 (1 H, d, J=7 8 Hz), 7.36 (1 H, d, J=7.8 Hz), 7.39 - 7.47 (2 H, m) | 0063 |
| 231 | 1 | 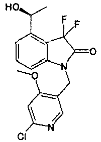 | 1-[(6-chloro-4-methoxypyridin-3-yl)methyl]-3,3-difluoro-4-[(1S)-1-hydroxyethyl]-1,3-dihydro-2H-indol-2-one | 369(M+H) | 0.932, A | 1H NMR (600 MHz, CHLOROFORM-d) d ppm 1.52 (3 H, d, J=6.6 Hz), 1.95 (1 H, br. s.). 3.90 (3 H, s), 4.84 (2 H, d, J=3.7 Hz), 5.28 (1 H, d, J=5.8 Hz), 6.70 (1 H, d, J=7.8 Hz), 6.82 (1 H, s), 7.34 (1 H, d, J=7.8 Hz), 7.37 - 7.44 (1 H, m), 8.14 (1 H, s) | 014 |
| 232 | 1 | 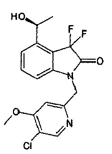 | 1-[(5-chloro-4-methoxypyridin-2-yl)methyl]-3,3-difluoro-4-[(1S)-1-hydroxyethyl]-1,3-dihydro-2H-indol-2-one | 369(M+H) | 0.994, A | 1H NMR (600 MHz, CHLOROFORM-d) d ppm 1.53 (3 H, d, J=6.2 Hz), 1.94 (1 H, d. J=3.3 Hz), 3.90 (3 H, s), 4.93 (2 H, s), 5.25 - 5.33 (1 H, m), 6.84 (1 H s), 6.97 (1 H, d, J=7.4 Hz), 7.35 (1 H, d, J=8.3 Hz), 7.41 - 7.48 (1 H, m), 8.37 (1 H, s) | 0 032 |

**[Table 1-30]**

| Compound No | Example | Structural formula | Compound Name | (ESI pos.) m/z (ESI neg.) m/z | LCMS RT (min) condition | 1H-NMR | 1050 (*µ*M) |
|---|---|---|---|---|---|---|---|
| 233 | 1 | 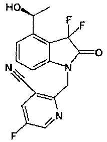 | 2-({3,3-difluoro-4-[(1S)-1-hydroxyethyl]-2-oxo-2,3-dihydro-1H-indol-1-yl}methyl)-5-fluoropyridine-3-carbonitrile | 348(M+H) | 0.892, A | 1H NMR (600 MHz, CHLOROFORM-d) d ppm 1.54 - 1.57 (3 H, m), 1.96 (1 H, d, J=3.30 Hz), 5.20 (2 H, s), 5.28 - 5.37 (1 H, m), 6.64 (1 H, d, J=7.43 Hz), 7.34 - 7.47 (2 H, m), 7.73 (1 H, dd, J=7.43, 2.89 Hz). 8 57 (1 H d, J=2.89 Hz) | 0.18 |
| 234 | 1 | 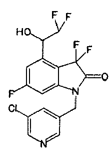 | 1-[(5-chloropyridin-3-yl)methyl]-4-(2,2-difluoro-1-hydroxyethyl)-3,3,6-trifluoro-1,3-dihydro-2H-indol-2-one | 393(M+H) | 0.932, A | 1H NMR (600 MHz, CHLOROFORM-d) d ppm 3.49 (1 H, br. s.), 4.88 (2 H, s), 5.16 - 5.27 (1 H, m), 5.77 - 6.01 (1 H, m), 6.48 - 6.55 (1 H, m), 7.08 - 7.16 (1 H, m), 7.57 - 7.67 (1 H, m), 8.42 - 8.59 (2 H, m) | 062 |
| 235 | 1 | 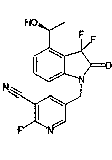 | 5-({3,3-difluoro-4-[(1S)-1-hydroxyethyl)-2-oxo-2,3-dihydro-1H-indol-1-yl}methyl)-2-fluoropyridine-3-carbonitrile | 370(M+Na) | 0.874, A | 1H NMR (600 MHz, CHLOROFORM-d) d ppm 1.54 - 1.55 (3 H, m), 1.95 (1 H, d, J=3.30 Hz), 4.92 (2 H, q, J=16.10 Hz) 5.25 - 5.35 (1 H, m), 6.68 (1 H, d, J=7.43 Hz), 7.41 - 7.45 (1 H, m), 7.47 - 7.54 (1 H, m), 8.03 (1 H, dd, J=7.64, 2.27 Hz), 8.47 (1 H, d, J=1.65 Hz) | 0023 |
| 236 | 1 | 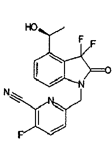 | 6-({3,3-difluoro-4-[(1S)-1-hydroxyethyl]-2-oxo-2,3-dihydro-1H-indol-1-yl}methyl)-3-fluoropyridine-2-carbonitrile | 348(M+H) | 0891, A | 1H NMR (600 MHz, CHLOROFORM-d) d ppm 1.53 (3 H, d, J=6.61 Hz), 1.93 (1 H, d, J=3.30 Hz), 5.01 (2 H, s), 5.25 - 5.34 (1 H, m), 6.90 (1 H, d, J=7.43 Hz), 7.39 (1 H, d, J=7.84 Hz), 7.45 - 7.52 (1 H, m), 7.56 - 7.66 (2 H, m) | 0 0040 |
| 237 | 1 | 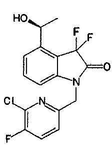 | 1-[(6-chloro-5-fluoropyridin-2-yl)methyl]-3,3-difluoro-4-[(1S)-1-hydroxyethyl]-1,3-dihydro-2H-indol-2-one | 357(M+H) | 1.016, A | 1 H NMR (600 MHz CHLOROFORM-d) d ppm 1.52 - 1.54 (3 H, m), 1.92 (1 H, d, J=3.30 Hz), 4.96 (1 H, s), 5.26 - 5.33 (2 H, m), 6.88 (1 H, d, J=7 84 Hz), 7.19 - 7.25 (1 H, m), 7.37 (1 H, d, J=8.26 Hz), 7.42 - 7.49 (2 H, m) | 0.0029 |
| 238 | 1 | 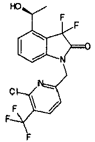 | 1-{[6-chloro-5-(trifluoromethyl) pyridin-2-yl]methyl}-3,3-difluoro-4-[(1S)-1-hydroxyethyl]-1,3-dihydro-2H-indol-2-one | 405(M-H) | 1.036, A | 1H NMR (600 MHz, CHLOROFORM-d) d ppm 1.49 - 1.59 (3 H, m), 1.91 - 2.00 (1 H, m), 5.03 (2 H, s), 5.27 - 5.33 (1 H, m), 6.76 - 6.88 (1 H, m), 7.28 - 7.33 (1 H, m), 7.35 - 7.42 (1 H, m), 7.43 - 7.52 (1 H, m), 7.93 - 8.03 (1 H, m) | 0.0062 |
| 239 | 1 | 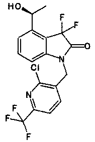 | 1-{[2-chloro-6-(trifluoromethyl) pyridin-3-yl]methyl}-3,3-difluoro-4-[(1S)-1-hydroxyethyl]-1,3-dihydro-2H-indol-2-one | 405(M-H) | 1.035, A | 1H NMR (600 MHz, CHLOROFORM-d) d ppm 1.56 (3 H, d, J=6.2 Hz), 1.92 - 2 00 (1 H, m), 5.06 (2 H, s), 5.27 - 5.38 (1 H, m), 6.61 - 6.68 (1 H, m), 7.38 - 7.50 (2 H, m), 7.55 - 7.67 (2H, m) | 0.22 |
| 240 | 1 | 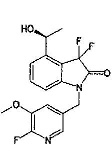 | 3,3-difluoro-1-[(6-fluoro-6-methoxypyridin-3-yl)methyl]-4-[(1S)-1-hydroxyethyl]-1,3-dihydro-2H-indol-2-one | 353(M+H) | 0.915, A | 1H NMR (600 MHz, CHLOROFORM-d) d ppm 1.53 (3 H, d, J=6.19 Hz), 1.93 (1 H, d, J=3.30 Hz), 3.87 (3 H, s), 4.81 - 4.90 (2 H, m), 5.26 - 5.33 (1 H, m), 6.75 (1 H, d, J=7.84 Hz), 7.22 (1 H, dd, J=9.29, 1.86 Hz), 7.38 (1 H d, J=7.84 Hz), 7.44 - 7.48 (1 H, m), 7.75 (1H, s) | 0.060 |

**[Table 1-31]**

| Compound No | Example | Structural formula | Compound Name | (ESI pos.) m/z (ESI neg.) m/z | LCMS RT (min) condition | 1H-NMR | IC50 (*µ*M) |
|---|---|---|---|---|---|---|---|
| 241 | 1 | 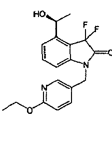 | 1-[(6-ethoxypyridin-3-yl)methyl]-3,3-difluoro-4-[(1S)-1-hydroxyethyl]-1,3-dihydro-2H-ndol-2-one | 349(M+H) | 1.008, A | 1H NMR (600 MHz CHLOROFORM-d) d ppm 1.37 (3 H, t, J=7.02 Hz) 15.2 (3 H, d, J=6 19 Hz) 1.92 (1 H, d. J=3.30 Hz), 433 (2 H, q, J=7.29 Hz), 476 - 485 (2 H, m) 5.23 - 532 (1 H, m), 6.70 (1H, d, J=8.67 Hz), 6 74 (1 H, d J=7 84 Hz), 7 34 (1 H, d, J=8.26 Hz) 7.40 - 7.45 (1 H, m), 7.52 (1 H. dd, J=8.46, 2 68 Hz), 8 14 (1 H. d J=2.48 Hz) | 0.066 |
| 242 | 3 | 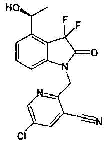 | 5-chloro-2-({3,3-difluoro-4-[(1S)-1-hydroxyethyl]-2-oxo-2,3-dihydro-1H-indol-1-yl}methyl)pyridine-3-carbonitrile | 364(M+H) | 0.966, A | 1H NMR (600 MHz CHLOROFORM-d) ppm 1.55 (3 H, d, J=6.6 Hz), 1.97 (1 H, d, J=2.5 Hz) 5.20 (2 H d, J=1.7 Hz), 5.33 (1 H, d, J=5.8Hz) 6.63 (1H, d, J=7.4 Hz), 7.36 - 7.46 (2 H m). 7 98 (1 H d, J=2.5 Hz) 8.64 (1 H, d J=2.1 Hz) | 0.083 |
| 243 | 1 | 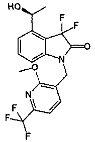 | 3,3-difluoro-4-[(1S)-1-hydroxyethyl]-1-{[2-methoxy-6-(trifluoromethyl) pyridin-3-yl]methyl}-1,3-dihydro-2H-indol-2-one | 403(M+H) | 1.085, A | 1H NMR (600 MHz, CHLOROFORM-d) ppm 1.54 (3 H, d, J=6.6 Hz), 1.87 - 2.01 (1 H, m), 407 (3 H, s), 4.90 (2H, s), 5.24 - 5.37 (1 H, m), 6.66 - 6.76 (1 H, m), 7.23 (1 H, s), 7.34 - 7.41 (1 H, m), 7.41 - 7.48 (1 H, m), 7.51 - 7.58 (1 H, m) | 0057 |
| 244 | 1 | 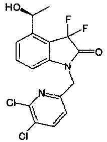 | 1-[(5,6-dichloropyndin-2-yl)methyl]-3,3-difluoro-4-[(1S)-1-hydroxyethyl]-1,3-dihydro-2H-indol-2-one | 373(M+H) | 1.077, A | 1H NMR (600 MHz, CHLOROFORM-d) ppm 1.53 (3 H, d, J=6.6 Hz) 1.94 (1 H, d, J=3.3 Hz), 4.96(2 H, s). 5.30 (1 H, d, J=6.2 Hz), 6.86 (1 H, d, J=7.8 Hz) 7.17 (1 H, d. J=7.8 Hz), 7.37 (1 H, d, J=8.3 Hz), 7.43 - 7.49 (1 H, m), 7.74 (1 H, d, J=8.3 Hz) | 0.0097 |
| 245 | 1 | 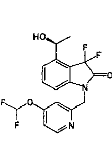 | 1-{[4-(difluoromethoxy) pyridin-2-yl]methyl}-3,3-difluoro-4-[(1S)-1-hydroxyethyl]-1,3-dihydro-2H-indol-2-one | 371(M+H) | 0.921, A | 1H NMR (600 MHz, CHLOROFORM-d) ppm 1.52 - 1.56 (3 H, m) 1.94 (1 H, br s.), 5.00 (2 H, s), 5.28 - 5 33 (1 H, m) 6.47 - 6.73 (7 H, m), 6.87 (1 H, d, J=7.8 Hz), 6.97 - 7.00 (2 H, m), 7.36 (1 H, d, J=7.8 Hz), 7.42 - 7.46 (1 H, m), 8.51-8.54 (1 H, m) d | 0.11 |
| 246 | 1 | 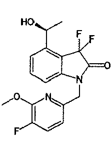 | 3,3-difluoro-1-[(5-fluoro-6-methoxypyridin-2-yl)methyl]-4-[(1S)-1-hydroxyethyl]-1,3-dihydro-2H-indol-2-one | 353(M+H) | 1.019, A | 1H NMR (600 MHz CHLOROFORM-d) d ppm 1.53 (3 H, d, J=6 19 Hz), 1.94 (1 H, d, J=3.30 Hz), 3.88 (3 H, s), 4.82 - 4.93 (2 H, m), 5.25 - 5.35 (1 H, m), 6.80 - 6.89 (2 H, m), 7.27 - 7.30 (1 H, m), 7.35 (1 H, d, J=8.26 Hz), 7.40 - 7.45 (1 H, m) | 0055 |
| 247 | 1 | 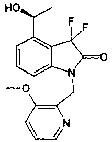 | 3,3-difluoro-4-[(1S)-1-hydroxyethyl]-1-[(3-methoxypyridin-2-yl)methyl]-1,3-dihydro-2H-indol-2-one | 335(M+H) | 0.850, A | 1H NMR (600 MHz, CHLOROFORM-d) d ppm 1.53 (3 H, d J=6 19 Hz). 1 92 (1 H, d, J=3.30 Hz), 3 84 (3 H s), 5.00 - 5.12 (2 H, m), 5.25 - 5 35 (1 H, m), 6.78 (1 (2 H, m), 5.25 - 5.35 (1 H, m), 6.78 (1 H, d, J=8.26 Hz), 7.12 - 7.16 (1 H, m), 7.18 - 7.22 (1 H, m), 7.27 - 7.30 (1 H, m), 7.32 - 7.37 (1 H, m), 8.12 (1 H, dd, J=4 95. 1 24 Hz) | 058 |
| 248 | 1 | 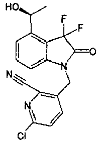 | 6-chloro-3-({3,3-difluoro-4-[(1S)-1-hydroxyethyl]-2-oxo-2,3-dihydro-1H-indol-1-yl}methyl)pyridine-2-carbonitrile | 386(M+Na) | 0.991, A | 1H NMR (600 MHz, CHLOROFORM-d) d ppm 1.53 (3 H, d, J=6.6 Hz), 1.85 (1 H, d, J=3.3 Hz), 5.11 (2 H, s), 5.30 (1 H, d, J=6.6 Hz), 6.82 (1 H, d, J=7.8 Hz), 7.42 (1H d, J=7.8 Hz), 7.48 - 7 56 (2 H, m), 7.76 (1 H, d J=8.3 Hz) | 1.2 |

**[Table 1-32]**

| Compound No | Example | Structural formula | Compound Name | (ESI pos.) m/z (ESI neg.) m/z | LCMS RT (min) condition | 1H-NMR | Chiral HPLC analysis conditions (Column used) (Solvent system) (Flow rate) | Chiral HPLC RT (min) | IC50 (µ M) |
|---|---|---|---|---|---|---|---|---|---|
| 249 | 8 | 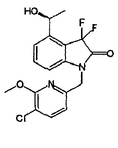 | 1-[(5-chloro-6-methoxypyridin-2-yl)methyl]-3,3-difluoro-4-[(1S)-1-hydroxyethyl]-1,3-dihydro-2H-indol-2-one | 369(M+H) | 1.088, A | 1H NMR (500 MHz, CHLOROFORM-d) d ppm 1.53 (3 H, d, J=6.5 Hz), 1.94 (1 H, d, J=3.1 Hz), 3.89 (3 H, s), 4.88 (2 H, s), 5.31 (1 H, br, s.), 6.80 - 6.86 (2 H, m), 7.32 - 7.37 (1 H, m), 7.39 - 7.46 (1 H, m), 7 59 (1 H, d, J=7 5 Hz), | | | 0058 |
| 250 | 1 | 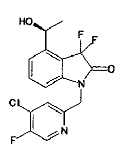 | 1-[(4-chloro-5-fluoropyridin-2-yl)methyl]-3,3-difluoro-4-[(1S)-1-hydroxyethyl]-1,3-dihydro-2H-indol-2-one | 357(M+H) | 1.015, A | 1H NMR (500 MHz CHLOROFORM-d) d ppm 1.53 (3 H, d, J=6.52 Hz), 1.95 (1 H, d, J=2.74 Hz), 4.96 (2 H, s), 5.22 - 5.39 (1 H, m), 6.82 - 6.91 (1 H, m), 7.35 - 7.41 (2 H, m), 7.42 - 7.47 (1 H, m), 8.42 (1 H, s) | | | 0.0098 oose |
| 251 | 1 | 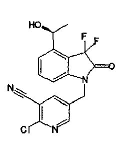 | 2-chloro-5-({3,3-difluoro4-[(1S)-1-hydroxyethyl]-2-oxo-2,3-dihydro-1H-indol-1-yl}methyl)pyridine-3-carbonitrile | 386(M+Na) | 0.952, A | 1H NMR (500 MHz, CHLOROFORM-d) d ppm 1.55 (3 H. s), 1.96 (1 H, d, J=3.1 Hz), 4.84 - 5.01 (2 H, m), 5.31 (1 H, dd, J=6.0, 3.6 Hz), 6.66 (1 H, d, J=7.5 Hz),7.41 - 7.46 (1 H, m), 7.46 - 7.53 (1 H, m), 7.93 (1 H, d, J=2.4 Hz), 8.61 (1 H, d, J=2.4 Hz) | | | 0.020 |
| 252 | 1 | 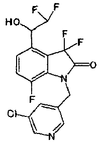 | 1-[(5-chloropyridin-3-yl)methyl]-4-(2,2-difluoro-1-hydroxyethyl)-3,3,7-trifluoro-1,3-dihydro-2H-indol-2-one | 393(M+H) | | | | | 0046 |
| 253 | 1 | 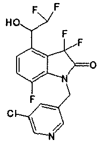 | (enantiomer 1) 1-[(5-chloropyridin-3-yl)methyl]-4-(2,2-difluoro-1-hydroxyethyl)-3,3,7-trifluoro-1,3-dihydro-2H-indol-2-one | 393(M+H) | 0.995, A | 1H NMR (600 MHz CHLOROFORM-d) d ppm 2.59 (1 H, d, J=4.1 Hz), 5.04 (2 H, s), 5.17 - 5.24 (1 H, m), 5.77 - 5.98 (1 H, m), 7.28 - 7.32 (1 H, m), 7.40 (1H, dd, J=8.7, 4.1 Hz), 7.66 - 7.68 (1 H, m), 8.52 - 8.53 (1 H, m), 8.55 (1 H, d. J=2.5 Hz) | AD3, 4.6*150mm Hex. / IPA=80 / 20 1 ml/min | 582 7.67 Faster | 0047 |
| 254 | 1 | 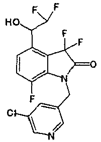 | (enantiomer 2) 1-[(5-chloropyridin-3-yl)methyl]-4-(2,2-difluoro-1-hydroxyethyl)-3,3,7-trifluoro-1,3-dihydro-2H-indol-2-one | 393(M+H) | | | AD3, 4.6*150mm Hex / IPA=80 / 20 1 ml/min. | 5.82, 7.67 Later | 68 |
| 255 | 1 | 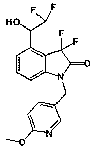 | (enantiomer 1) 4-(2,2-difluoro-1-hydroxyethyl)-3,3-difluoro-1-[(6-methoxypyridin-3-yl)methyl]-1,3-dihydro-2H-indol-2-one | 371(M+H) | 0.952, A | 1H NMR (600 MHz. CHLOROFORM-d) d ppm 2.58 (1 H, d, J=3.3 Hz), 3.92 (3 H, s), 4.78 - 4.88 (2 H, m), 5.22 (1 H, d, J=12.4 Hz), 5.78 - 6.01 (1 H, m), 6.71 - 6.76 (1 H, m), 6.86 (1 H, d, J=7.8 Hz), 7.36 (1 H. d, J=7.8 Hz), 7.48 (1 H, t, J=7.8 Hz), 7.53 (1 H, dd, J=8.3, 2.5 Hz), 8.17 (1 H, d. J=2.1 Hz) | | | 0.069 |
| 256 | 1 | 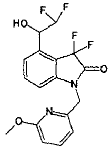 | (enantiomer 1) 4-(2,2-difluoro-1-hydroxyethyl)-3,3-difluoro-1-[(6-methoxypyridin-2-yl)methyl)-1,3-dihydro-2H-indol-2-one | 371(M+H) | 1.027, A | 1H NMR (600 MHz. CHLOROFORM-d) d ppm 2.52 - 2.60 (1 H, m), 3.74 - 3.81 (3 H, m), 4.86 - 4.95 (2 H, m), 5.19 - 5.28 (1 H, m), 5.78 - 6.03 (1 H, m), 6.64 (1 H, dd, J=8.5, 3.5 Hz), 6.85 (1 H, dd, J=7.4, 3.3 Hz), 6.99 (1 H, d, J=7.8 Hz) 7.34 (1 H, dd, J=8.1, 3.5 Hz), 747 (1 H, td. J=7.9 3.5 Hz), 7.51 - 7.56 (1H, m) | | | 0.0096 |

**[Table 1-33]**

| Compound No. | Example | Structural formula | Compound Name | (ESI pos.) m/z (ESI neg.) m/z | LCMS RT (min) condition | 1H-NMR | IC50 (µM) |
|---|---|---|---|---|---|---|---|
| 257 | 1 | 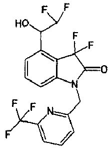 | (enantiomer 1) 4-(2,2-difluoro-1-hydroxyethyl)-3,3-difluoro-1-{[6-(trifluoromethyl) pyridin-2-yl)methyl}-1,3-dihydro-2H-indol-2-one | 409(M+H) | 1.053, A | 1H NMR (600 MHz. CHLOROFORM-d) d ppm 2.54 - 2.58 (1 H, m), 5.05 - 5.12 (2 H, m), 5.19 - 5.27 (1 H, m), 5.79 - 6.01 (1 H, m), 7.10 (1 H, d, J=7 8 Hz), 7.38 (1 H, d, J=7.8 Hz), 7.48 - 7.54 (2 H, m), 7.64 (1 H, d, J=7.8 Hz), 7.88 (1 H, t, J=7.8 Hz) | 0.11 |
| 258 | 1 | 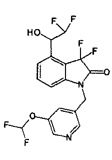 | (enantiomer 1) 4-(2,2-difluoro-1-hydroxyethyl)-1-{[5-(difluoromethoxy) pyridin-3-yl]methyl}-3,3-difluoro-1,3-dihydro-2H-indol-2-one | 407(M+H) | 0.922, A | 1H NMR (600 MHz CHLOROFORM-d) d ppm 2.59 - 2.62 (1 H, m), 4.94 (2 H, s), 5.20 - 5.28 (1 H, m), 5.80 - 6.01 (1 H, m), 6.42 - 6.68 (1 H, m), 6.81 (1 H, d, J=7.8 Hz), 7.39 - 7.44 (2 H, m), 7.51 (1 H, t, J=8.1 Hz), 8.46 - 8.48 (1 H, m), 8.49 - 8.52 (1 H m) | 0.12 |
| 259 | 3 | 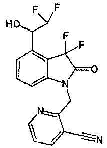 | (enantiomer 1) 2-([4-(2,2-difluoro-1-hydroxyethyl)-3,3-difluoro-2-oxo-2,3-dihydro-1H-indol-1-yl)methyl}pyridine-3-carbonitrile | 366(M+H) | 0.883, A | 1H NMR (600 MHz. CHLOROFORM-d) d ppm 2 59 (1 H, d. J=4.1 Hz), 5.20 - 5.32 (3 H, m), 5.81 - 6.07 (1 H, m), 6.76 (1 H, d, J=7.8 Hz), 7.35 - 7.41 (2 H, m), 7.44 - 7.51 (1 H, m), 8.01 (1 H, dd, J=7.8, 1.7 Hz), 8.69 (1 H, dd, J=5.0, 1.7 Hz) | 1.9 |
| 260 | 1 | 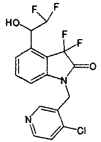 | (enantiomer 1) 1-[(4-chloropyridin-3-yl)methyl]-4-(2,2-difluoro-1-hydroxyethyl)-3,3-difluoro-1,3-dihydro-2H-indol-2-one | 375(M+H) | 0.878, A | 1H NMR(600 MHz, CHLOROFORM-d) d ppm 262 (1 H, d, J=4.1 Hz), 5.06 (2 H, d, J=3.7 Hz), 5.26 (1 H, br, s), 5.79 - 6.03 (1 H, m), 6.78 (1 H, d, J=7.4 Hz), 7.37 - 7.42 (2 H, m), 7.46 - 7.51 (1 H, m), 8.46 (1 H, s), 8.49 (1 H, d, J=5.0 Hz) | 1.5 |
| 261 | 1 | 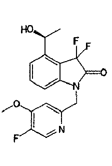 | 3,3-difluoro-1-[(5-fluoro-4-methoxypyridin-2-yl)methyl]-4-[(1S)-1-hydroxyethyl]-1,3-dihydro-2H-indol-2-one | 353(M+H) | 0.918, A | 1H NMR (600 MHz CHLOROFORM-d) d ppm 1.53 (3 H, d, J=6.61 Hz), 1 95 (1 H, d, J=2.89 Hz), 3.89 (3 H, s), 4.92 (2 H, s), 5.24 - 5.33 (1 H m), 6.91 (1 H, d, J=6.61 Hz), 6.98 (1 H d J=7.84 Hz), 7.35 (1 H, d, J=8.26 Hz), 7.44 (1 H, t. J=7.84 Hz), 8 24 (1 H, d, J=2.89 Hz) | 0.25 |
| 262 | 3 | 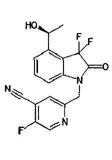 | 2-{(3,3-difluoro-4-[(1S)-1-hydroxyethyl]-2-oxo-2,3-dihydro-1H-indol-1-yl}methyl)-5-fluoropyridine-4-carbonitrile | 348(M+H) | 0.919, A | 1H NMR (600 MHz, CHLOROFORM-d) d ppm 1.54 - 5.55 (3 H, m), 1.94 (1 H, d J=3 30 Hz), 4.97 - 5 07 (2 H, m), 5.25 - 5.36 (1 H, m), 6.82 (1 H, d, J=7 84 Hz), 7.36 - 7 49 (2 H, m), 7 55 (1 H, d, J=4.54 Hz), 8.63 (1 H, s) | 0.062 |
| 263 | 1 | 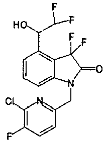 | (enantiomer 1) 1-[(6-chloro-5-fluoropyridin-2-yl)methyl]-4-(2,2-difluoro-1-hydroxyethyl)-3,3-difluoro-1,3-dihydro-2H-indol-2-one | 393(M+H) | 1.050, A | 1H NMR (600 MHz CHLOROFORM-d) d ppm 2.56 (1 H, d, J=3.7 Hz), 4.94 - 5.00 (2 H, m), 5.21 (1 H, br, s.), 5.77 - 6.03 (1 H, m), 7.04 (1 H, d, J=8.3 Hz), 7.25 (1 H, d, J=3.3 Hz), 7.39 (1 H, d, J=8.3 Hz), 7 47 (1 H, t, J=8.1 Hz), 7 53 (1 H, t, J=8.1 Hz) | 0.011 |
| 264 | 1 | 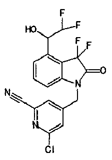 | (enantiomer 1)6-chloro-4-{[4-(2,2-difluoro-1-hydroxyethyl)-3,3-difluoro-2-oxo-2,3-dihydro-1 H-indol-1-yl]methyl}pyridine-2-carbonitrile | 422(M+Na) | 1.022, A | 1H NMR (600 MHz. CHLOROFORM-d) d ppm 2.63 (1 H, d, J=4.1 Hz), 4.93 (2 H, s), 5.27 (1 H, br, s.), 5.80 - 6.07 (1 H, m). 6.70 (1 H, d, J=8.3 Hz), 7.45 (1 H, s), 7.48 (1 H, d, J=8.3 Hz), 7 52 (1 H, s), 7.54 - 7.50 (1 H, m) | 0.17 |

**[Table 1-34]**

| Compound No | Example | Structural formula | Compound Name | (ESI pos.) m/z (ESI neg.) m/z | LCMS RT (min) condition | 1H-NMR | IC50 (µ M) |
|---|---|---|---|---|---|---|---|
| 265 | 1 | 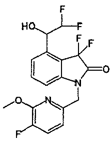 | (enantiomer 1) 4-(2,2-difluoro-1-hydroxyethyl)-3,3-difluoro-1-[(5-fluoro-6-methoxypyridin-2-yl)methyl]-1,3-dihydro-2H-indol-2-one | 389(M+H) | 1.075, A | 1H NMR (600 MHz, CHLOROFORM-d) d ppm 257 (1 H, d. J=4 .13 Hz), 388 (3 H, s), 4.84 - 4.93 (2 H, m), 5.20 - 5.28 (1 H, m), 5.80 - 6.01 (1 H, m), 6.85 (1 H, dd, J=7.84, 2.48 Hz), 6.99 (1 H, d, J=7.84 Hz), 7.29 (1 H, dd, J=9.91, 7.84 Hz), 7.38 (1 H, d J=7.84 Hz), 7.49 (1 H, t, J=8.05 Hz) | 093 |
| 266 | 1 | 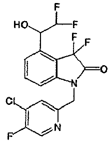 | (enantiomer 1) 1-[(4-chloro-5-fluoropyridin-2-yl)methyl]-4-(2,2-difluoro-1-hydroxyethyl)-3,3-difluoro-1,3-dihydro-2H-indol-2-one | 393(M+H) | 1.062, A | 1H NMR (600 MHz, CHLOROFORM-d) d ppm 259 (1 H, d J=4.13 Hz), 4.96 (2 H, s), 5.20 - 5.28 (1 H, m), 5.80 - 6.02 (1 H, m), 7.02 (1 H, d, J=7.84 Hz), 7.38 (1 H, d, J=8.26 Hz) 7.41 (1 H, d, J=5.37 Hz) 7.50 (1 H t, J=7 95 Hz), 8.42 (1 H, d, J=0.83 Hz), | 0.021 |
| 267 | 1 | 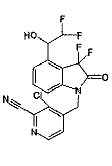 | (enantiomer 1) 3-chloro-4-{[4-(2,2-difluoro-1-hydroxyethyl)-3,3-difluoro-2-oxo-2,3-dihydro-1 H-indol-1-yl]methyl}pyridine-2-carbonitrile | 400(M+H) | 1.009, A | 1H NMR (600 MHz CHLOROFORM-d) d ppm 2.65 (1 H, d, J=4.1 Hz), 5.06 (2 H, d, J=4.5 Hz), 5.27 (1 H, br, s.), 5.79 - 6.06 (1 H, m), 6.69 (1 H, d, J=7.4 Hz), 7.24 (1 H, d, J=5.0 Hz), 7.46 (1H, d, J=8.3 Hz), 7.50 - 7.59 (1 H, m), 8.56 (1 H, d, J=4.5 Hz) | 0.26 |
| 268 | 1 | 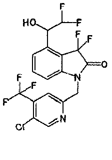 | (enantiomer 1) 1-{[5-chloro-4-(trifluoromethyl) pyridin-2-yl]methyl}-4-(2,2-difluoro-1-hydroxyethyl)-3,3-difluoro-1,3-dihydro-2H-indol-2-one | 443(M+H) | 1.160, A | 1H NMR (600 MHz, CHLOROFORM-d) d ppm 2.59 (1 H, d, J=4.1 Hz), 5.04 (2 H, s), 5.20 - 5.27 (1 H, m), 5.80 - 6.02 (1 H, m), 7.01 (1 H, d, J=7.8 Hz), 7.40 (1 H, d, J=7.8 Hz), 7.49 - 7.54 (1 H, m), 7.59 (1 H, s). 8.68 (1 H, s) | 0.016 |
| 269 | 1 | 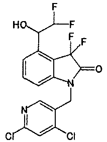 | (enantiomer 1) 1-[(4,6-dichloropyridin-3-yl)methyl]-4-(2,2-difluoro-1-hydroxyethyl)-3,3-difluoro-1,3-dihydro-2H-indol-2-one | 409(M+H) | 1.077, A | 1H NMR (600 MHz, CHLOROFORM-d) d ppm 2.61 (1 H, d, J=4.1 Hz), 4.97-5.05 (2 H, m), 5.21 - 5.28 (1 H, m), 5.80 - 6.02 (1 H, m), 6.78 (1 H, d, J=8.3 Hz), 7.40 - 7.44 (1 H, m), 7.45 (1 H, s) 7.48 - 7.53 (1 H, m), 8.25 (1 H, s) | 033 |
| 270 | 1 | 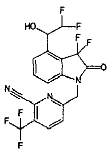 | (enantiomer 1) 6-{[4-(2,2-difluoro-1-hydroxyethyl)-3,3-difluoro-2-oxo-2,3-dihydro-1H-indol-1-yl)methyl}-3-(trifluoromethyl) pyridine-2-carbonitrile | 434(M+H) | 1.078, A | 1H NMR (600 MHz CHLOROFORM-d) d ppm 2.58 (1 H, d, J=4.1 Hz) 5.06 - 5.16 (2 H, m), 5.19 - 5.27 (1 H, m), 5.79 - 6.02 (1 H, m), 7.02 (1 H, d J=8.3 Hz), 7.40 - 7.45 (1 H, m), 7.56 (1 H, t. J=8.1 Hz), 7 68 - 7.73 (1H m), 8.13 (1 H, d, J=8.3 Hz) | 0.0084 |
| 271 | 1 | 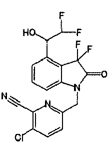 | (enantiomer 1) 3-chloro-6-[4-(2,2-difluoro-1-hydroxyethyl)-3,3-difluoro-2-oxo-2,3-dihydro-1H-indol-1-yl]methyl}pyridine-2-carbonitrile | 422(M+Na) | 1.034, A | 1H NMR (600 MHz. CHLOROFORM-d) d ppm 2.57 (1 H, d, J=4.1 Hz), 4.97 - 5.06 (2 H, m), 5.18 - 5.26 (1 H, m), 5.78 - 6.01 (1 H, m), 7.04 (1 H, d, J=7.8 Hz), 7.38 - 7.44 (1 H, m), 7.50 - 7 58 (2 H, m), 7.87 (1 H, d. J=8.7 Hz) | 0.0055 |
| 272 | 3 | 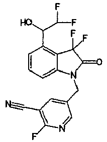 | (enantiomer 1) 5-{[4-(2,2-difluoro-1-hydroxyethyl)-3,3-difluoro-2-oxo-2,3-dihydro-1 H-indol-1-yl]methyl}-2-fluoropyridine-3-carbonitrile | 406(M+Na) | 0.984, A | 1H NMR (600 MHz, CHLOROFORM-d) d ppm 2.61 (1 H, d, J=4.1 Hz), 4.94 (2 H, d, J=5.8 Hz), 5.25 (1 H, br, s.), 5.77 - 6.03 (1 H, m), 6.82 (1 H, d, J=7.8 Hz) 7 45 (1 H, d, J=8.3 Hz), 7 51 - 7.60 (1 H, m), 8.05 (1 H, dd, J=7.8, 2.5 Hz), 8.48 (1 H, J=2.1 Hz) | 0.027 |

**[Table 1-35]**

| Compound No | Example | Structural formula | Compound Name | (ESI pos.) m/z (ESI neg.) m/z | LCMS RT (min) condition | 1H-NMR | Chiral HPLC analysis conditions (Column used) (Solvent system) (Flow rate) | Chiral HPLC RT (mm) | IC50 (µ M) |
|---|---|---|---|---|---|---|---|---|---|
| 273 | 1 | 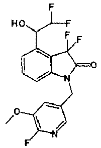 | (enantiomer 1) 4-(2,2-difluoro-1-hydroxyethyl)-3,3-difluoro-1-[(6-fluoro-5-methoxypyridin-3-yl)methyl]-1,3-dihydro-2H-indol-2-one | 389(M+H) | 0.979, A | 1H NMR (600 MHz. CHLOROFORM-d) d ppm 2.58 (1 H. d. J=4.1 Hz), 384 (3 H, s), 4.84 (2 H, d, J=2.9 Hz), 5.19 (1 H, br, s.), 5.74 - 5.99 (1 H, m), 6.85 (1 H, d, J=7.8 Hz), 7 19 (1 H dd. J=9.3, 1.9 Hz), 7.37 (1 H, d, J=7.8 Hz), 7.49 (1 H, t, J=8.1 Hz), 7.72 (1 H, s) | | | 0.14 |
| 274 | 1 | 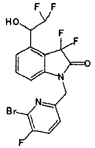 | (enantiomer 1) 1-[(6-bromo-5-fluoropyridin-2-yl)methyl]-4-(2,2-difluoro-1-hydroxyethyl)-3,3-difluoro-1,3-dihydro-2H-indol-2-one | 437(M+H) | 1.019, A | 1H NMR (600 MHz, CHLOROFORM-d) d ppm 2.55 - 2.62 (1 H, m), 4.98 (2 H, d. J=2.9 Hz), 5.18 - 5.27 (1 H, m), 5.80 - 6.03 (1 H, m), 7.00 - 7.07 (1 H, m), 7.22 - 7.29 (1 H, m), 7.34 - 7.45 (2 H, m), 7.47 - 7.58 (1 H, m) | | | 0.0046 |
| 275 | 3 | 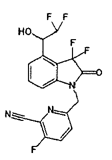 | (enantiomer 1) 6-{[4-(2,2-difluoro-1-hydroxyethyl)-3,3-difluoro-2-oxo-2,3-dihydro-1H-indol-1-yl]methyl}-3-fluoropyridine-2-carbonitrile | 406(M+Na) | 0.922, A | 1H NMR (600 MHz CHLOROFORM-d) d ppm 2.56 - 2.63 (1 H, m), 5.02 (2 H, d, J=5.4 Hz), 5.17- 5.27 (1 H, m), 5.77 - 6 03 (1 H, m), 7.02 - 7.09 (1 H, m), 7.37 - 7.43 (1 H, m), 7.52 - 7.58 (1 H, m), 7.60 - 7.67 (2 H, m) | | | 0.0091 |
| 276 | 3 | 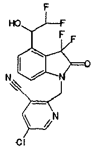 | (enantiomer 1) 5-chloro-2-{[4-(2,2-difluoro-1-hydroxyethyl)-3,3-difluoro-2-oxo-2,3-dihydro-1H-indol-1-yl]methyl}pyridine-3-carbomtrile | 400(M+H) | 1.030, A | 1H NMR (600 MHz, CHLOROFORM-d) d ppm 2.58 (1 H, d, J=4.13 Hz), 5.21 (2 H, s), 5.23 - 5.31 (1 H, m), 5.79 - 6.05 (1 H, m), 6.76 (1 H, d, J=7 43 Hz), 7.40 (1 H, d J=826 Hz). 749 (1 H, t, J=8.05 Hz), 7.99 (1 H, d. J=2.48 Hz), 8.64 (1 H, d, J=2.48 Hz), | | | 0.20 |
| 277 | 9 | 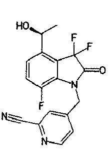 | 4-({3,3,7-trifluoro-4-[(1 S)-1-hydroxyethyl]-2-oxo-2,3-dihydro-1H-indol-1-yl}methyl) pyridine-2-carbonitrile | 348(M+H) | 0.920, A | 1H NMR (600 MHz, CHLOROFORM-d) d ppm 1.50 - 1.55 (3 H, m), 5.03 - 5.11 (2 H, m), 5.28 - 5.32 (1 H, m), 7.21 - 7.26 (1 H, m), 7.41 (1 H, dd, J=8.9, 4.3 Hz), 7.45 (1 H, dd J=5.0, 1.2 Hz), 7.62 (1 H, s), 8.71 (1 H, d, J=4.5 Hz) | AD3, 4.6*150mm Hex / IPA=80 / 20 1ml/min | 7.65, 10.11 Faster | 0014 |
| 278 | 1 | 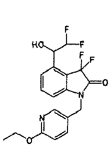 | (enantiomer 1) 4-(2,2-difluoro-1-hydroxyethyl)-1-[(6-ethoxypyridin-3-yl)methyl]-3,3-difluoro-1,3-dihydro-2H-indol-2-one | 385(M+H) | 1.099, A | 1 H NMR (600 MHz. CHLOROFORM-d) d ppm 1.38 (3 H, t, J=7.02 Hz), 2.59 (1 H, br, s.), 4.33 (2 H, q, J=7.02 Hz), 4.77 - 4.88 (2 H, m), 5.15 - 5.28 (1 H, m), 5.74 - 6.02 (1 H, m), 6.71 (1 H, d, J=8.67 Hz) 6.86 (1 H, d J=7.84 Hz), 7.36 (1 H, d, J=7.84 Hz), 7.48 (1 H, t, J=8.05 Hz), 7.52 (1 H, d, J=8.59 Hz), 815 (1 H, d, J=2.48 Hz) | | | 021 |

**[Table 1-36]**

| Compound No | Example | Structural formula | Compound Name | (ESI pos.) m/z (ESI neg.) m/z | LCMS RT (min) condition | 1H-NMR |
|---|---|---|---|---|---|---|
| 279 | 1 | 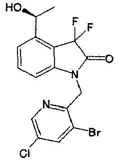 | 1-[(3-bromo-5-chloropyridin-2-yl)methyl]-3,3-difluoro-4-[(1S)-1-hydroxyethyl]-1,3-dihydro-2H-indol-2-one | 417(M+H) | 1.108, A | 1H NMR (600 MHz, CHLOROFORM-d) d ppm 1 55 (3 H, d, J=6.6 Hz), 1 95 (1 H, br. s.), 5.07 (2 H, s), 5.32 (1 H, br. s.), 6.60 (1 H, d, J=7.0 Hz), 7.32 - 7.42 (2 H, m), 7.90 (1 H, d, J=2.1 Hz), 8 36 - 8.40 (1 H. m) |
| 280 | 1 | 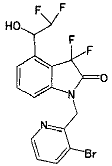 | (enantiomer 1) 1-[(3-bromopyridin-2-yl)methyl]-4-(2,2-difluoro-1-hydroxyethyl)-3,3-difluoro-1,3-dihydro-2H-indol-2-one | 419(M+H) | 1.019, A | 1H NMR (600 MHz, CHLOROFORM-d) d ppm 2 57 (1 H, d, J=4 1 Hz), 5 12 (2 H, s), 5.26 (1 H, br. s.), 5.82 - 6.07 (1 H, m), 6 75 (1 H. d. J=7. 8 Hz), 7 12 (1 H, dd, J=8.3, 4.5 Hz), 7.35 (1 H, d, J=8.3 Hz), 7.40 - 7 47 (1 H. m), 7 88 (1 H, dd, J=8.1, 1.4 Hz), 8 42 (1 H, dd, J=4.5, 1 2 Hz) |
| 281 | 1 | 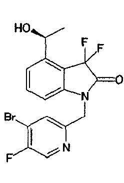 | 1-[(4-bromo-5-fluoropyridin-2-yl)methyl]-3,3-difluoro-4-[(1S)-1-hydroxyethyl]-1,3-dihydro-2H-indol-2-one | 401(M+H) | 1.032, A | 1H NMR (600 MHz, CHLOROFORM-d) d ppm 1.52 - 1.54 (3 H, m), 1.93 (1 H, d, J=2 89 Hz), 4.91 - 5 00 (2 H, m), 5.26 - 5.34 (1 H, m), 6.88 (1 H, d, J=7 84 Hz), 7 37 (1 H. d, J=8 26 Hz), 7.42 - 7.48 (1 H, m), 7.54 (1 H, d, J=5 37 Hz), 8 37 (1 H, s) |
| 282 | 1 | 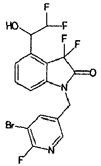 | (enantiomer 1) 1-[(5-bromo-6-fluoropyridin-3-yl)methyl]-4-(2,2-difluoro-1-hydroxyethyl)-3,3-difluoro-1,3-dihydro-2H-indol-2-one | 437(M+H) | 1.062, A | 1H NMR (600 MHz, CHLOROFORM-d) d ppm 2 61 (1 H, d, J=4.1 Hz), 4.88 (2 H, d, J=2.1 Hz), 524 (1 H, d, J=10.3 Hz), 5.78 - 6.02 (1 H, m), 6.83 (1 H, d, J=7 8 Hz), 7.42 (1 H, d, J=7.8 Hz), 7 54 (1 H, t, J=7 8 Hz), 7 94 (1 H. dd, J=8 1, 2.3 Hz), 8.17 (1 H, s) |
| 283 | 1 | 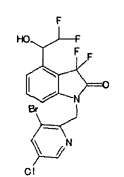 | (enantiomer 1) 1-[(3-bromo-5-chloropyridin-2-yl)methyl]-4-(2,2-difluoro-1-hydroxyethyl)-3,3-difluoro-1,3-dihydro-2H-indol-2-one | 453(M-H) | 1.140, A | 1H NMR (600 MHz. CHLOROFORM-d) d ppm 2.57 (1 H, d, J=4.13 Hz), 5.08 (2 H, s), 5.21 - 5.32 (1 H, m), 5.80 - 6.05 (1 H, m), 6 73 (1 H, d, J=7 84 Hz), 7.36 (1 H, d, J=7 84 Hz), 7 42 - 7 47 (1 H, m), 7 91 (1 H, d, J=2.06 Hz), 8 38 (1 H. d, J=2.06 Hz) |

### Test Example 1 [Glycine Uptake Inhibition Experiment]

A glycine uptake experiment was conducted in accordance with the method published in Neuron, 8, 927-935, 1992. In the experiment, T98G cells (glioma cells) expressing human type 1 glycine transporter (GlyT1) were used. The T98G cells were seeded in a 96-well plate at 2.0 × 10⁴ cells/well and cultured overnight in a CO₂ incubator. The test substance was dissolved in a 100% DMSO solution and then dissolved in a 10 mM HEPES buffer solution (pH 7.4) containing 150 mM sodium chloride, 1 mM calcium chloride, 5 mM potassium chloride, 1 mM magnesium chloride, 10 mM glucose and 0.2% bovine serum albumin. After removing the cell culture medium, the test substance was subjected to a 10-min pretreatment. Subsequently, the test substance and [³H] glycine (final concentration: 250 nM) were added to the cells and reaction was performed at room temperature for 15 minutes. After the end of the reaction, the extracellular fluid was aspirated with a manifold to remove excess labeled glycine present outside the cells, and then the cells were lysed with a 0.5 M aqueous sodium hydroxide solution. The glycine content in the cells was determined by measuring the radioactivity in the cell lysate with a liquid scintillation counter. Glycine uptake in the presence of 10 µM ALX5407 was defined as non-specific uptake, and the value calculated by subtracting the amount of the non-specific uptake from the total uptake in the absence of 10 µM ALX5407 was defined as specific uptake. In addition, glycine uptake inhibitory activity (IC₅₀ value) was calculated from an inhibition curve at the concentrations of each test substance ranging from 10⁻⁹ to 10⁻⁵ M. It should be noted that ALX5407 is an HCl salt of N-[(3R)-3-([1,1'-biphenyl]-4-yloxy)-3-(4-fluorophenyl)propyl]-N-methylglycine. IC₅₀ values of the compounds of the present invention are shown in Table 1.

### INDUSTRIAL APPLICABILITY

The inventive compounds have glycine transporter (GlyT1)-inhibiting activity, and thus, are effective in the prevention or treatment of diseases associated with the glycine transporter which are, specifically, schizophrenia, Alzheimer's disease, cognitive impairment, dementia, anxiety disorders (e.g., generalized anxiety disorder, panic disorder, obsessive-compulsive disorder, social anxiety disorder, post-traumatic stress disorder, specific phobias, acute stress disorder), depression, drug dependence, spasm, tremor, pain, Parkinson's disease, attention deficit hyperactivity disorder, bipolar disorder, eating disorder, sleep disorders or the like.

## Claims

1. A compound represented by formula [I] or a pharmaceutically acceptable salt thereof: 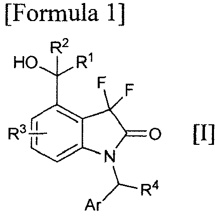 wherein
Ar represents a phenyl group optionally substituted with one to three substituents selected from substituent group 1, a bicyclic heterocyclyl group optionally substituted with one to three substituents selected from substituent group 1, or a monocyclic heteroaryl group optionally substituted with one to three substituents selected from substituent group 1,
substituent group 1 is the group consisting of a halogen atom, a C₁₋₆ alkyl group, a C₁₋₆ haloalkyl group, a C₁₋₆ alkoxy group, a cyano group, a triazolyl group, a C₁₋₆ haloalkoxy group, and a C₃₋₆ cycloalkyl group,
R¹ and R² are the same or different and are each a hydrogen atom, a C₁₋₆ alkyl group, or a C₁₋₆ haloalkyl group, or
together with the carbon atom to which they are attached, optionally form a cyclopropane ring, a cyclobutane ring, or an oxetane ring,
R³ represents a hydrogen atom or a halogen atom, and
R⁴ represents a hydrogen atom or a C₁₋₆ alkyl group.

2. A compound represented by formula [I] or a pharmaceutically acceptable salt thereof: 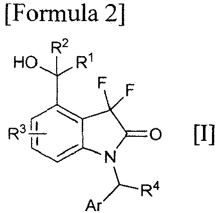 wherein
Ar represents a phenyl group optionally substituted with one to three substituents selected from substituent group 1, a bicyclic heterocyclyl group optionally substituted with one to three substituents selected from substituent group 1, or a monocyclic heteroaryl group optionally substituted with one to three substituents selected from substituent group 1,
substituent group 1 is the group consisting of a halogen atom, a C₁₋₆ alkyl group, a C₁₋₆ haloalkyl group, a C₁₋₆ alkoxy group, a cyano group, and a triazolyl group,
R¹ and R² are the same or different and are each a hydrogen atom, a C₁₋₆ alkyl group, or a C₁₋₆ haloalkyl group, or
together with the carbon atom to which they are attached, optionally form a cyclopropane ring, a cyclobutane ring, or an oxetane ring,
R³ represents a hydrogen atom or a halogen atom, and
R⁴ represents a hydrogen atom or a C₁₋₆ alkyl group.

3. The compound according to claim 1 or 2 or a pharmaceutically acceptable salt thereof, wherein Ar is a pyridyl group optionally substituted with one to three substituents selected from substituent group 1.

4. The compound according to claim 1 or 2 or a pharmaceutically acceptable salt thereof, wherein Ar is a pyridyl group substituted with one to three substituents selected from the group consisting of a halogen atom, a cyano group, a methyl group substituted with one to three halogen atoms, and a methoxy group substituted with one to three halogen atoms.

5. The compound according to any one of claims 1 to 4 or a pharmaceutically acceptable salt thereof, wherein R⁴ is a hydrogen atom.

6. The compound according to any one of claims 1 to 5 or a pharmaceutically acceptable salt thereof, wherein R¹ is a C₁₋₆ alkyl group, or a C₁₋₆ haloalkyl group, and R² is a hydrogen atom.

7. The compound according to claim 1 or a pharmaceutically acceptable salt thereof selected from the group consisting of
1-[(6-bromo-5-fluoropyridin-2-yl)methyl]-3,3-difluoro-4-[(1S)-1-hydroxyethyl]-1,3-dihydro-2H-indol-2-one,
1-[(5-bromo-6-fluoropyridin-3-yl)methyl]-3,3-difluoro-4-[(1S)-1-hydroxyethyl]-1,3-dihydro-2H-indol-2-one,
1-(3-chlorobenzyl)-3,3-difluoro-4-[(1S)-1-hydroxyethyl]-1,3-dihydro-2H-indol-2-one,
1-[(6-chloro-5-fluoropyridin-2-yl)methyl]-3,3-difluoro-4-[(1S)-1-hydroxyethyl]-1,3-dihydro-2H-indol-2-one,
6-({3,3-difluoro-4-[(1S)-1-hydroxyethyl]-2-oxo-2,3-dihydro-1H-indol-1-yl}methyl)-3-fluoropyridine-2-carbonitrile,
1-[(6-bromo-5-fluoropyridin-2-yl)methyl]-4-(2,2-difluoro-1-hydroxyethyl)-3,3-difluoro-1,3-dihydro-2H-indol-2-one,
3-chloro-6-{[4-(2,2-difluoro-1-hydroxyethyl)-3,3-difluoro-2-oxo-2,3-dihydro-1H-indol-1-yl]methyl}pyridine-2-carbonitrile,
1-[(6-chloropyridin-2-yl)(2H2)methyl]-3,3-difluoro-4-[(1S)-1-hydroxyethyl]-1,3-dihydro-2H-indol-2-one,
3,3-difluoro-4-[(1S)-1-hydroxyethyl]-1-{[5-(trifluoromethyl)furan-2-yl]methyl}-1,3-dihydro-2H-indol-2-one,
1-{[6-chloro-5-(trifluoromethyl)pyridin-2-yl]methyl}-3,3-difluoro-4-[(1S)-1-hydroxyethyl]-1,3-dihydro-2H-indol-2-one,
1-[(6-chloropyridin-2-yl)methyl]-3,3-difluoro-4-(1-hydroxyethyl)-1,3-dihydro-2H-indol-2-one,
3,3-difluoro-4-[(1S)-1-hydroxyethyl]-1-[3-(trifluoromethyl)benzyl]-1,3-dihydro-2H-indol-2-one,
1-[(5-chloropyridin-3-yl)methyl]-3,3,5-trifluoro-4-(1-hydroxyethyl)-1,3-dihydro-2H-indol-2-one,
3-chloro-6-({3,3-difluoro-4-[(1S)-1-hydroxyethyl]-2-oxo-2,3-dihydro-1H-indol-1-yl}methyl)pyridine-2-carbonitrile,
1-[(6-chloropyridin-2-yl)methyl]-4-(2,2-difluoro-1-hydroxyethyl)-3,3-difluoro-1,3-dihydro-2H-indol-2-one,
1-{[5-chloro-4-(trifluoromethyl)pyridin-2-yl]methyl}-3,3-difluoro-4-[(1S)-1-hydroxyethyl]-1,3-dihydro-2H-indol-2-one,
6-{[4-(2,2-difluoro-1-hydroxyethyl)-3,3-difluoro-2-oxo-2,3-dihydro-1H-indol-1-yl]methyl}-3-(trifluoromethyl)pyridine-2-carbonitrile,
1-[(5-chloro-6-methoxypyridin-3-yl)methyl]-3,3-difluoro-4-[(1S)-1-hydroxyethyl]-1,3-dihydro-2H-indol-2-one,
1-[(5,6-dichloropyridin-3-yl)methyl]-3,3-difluoro-4-[(1S)-1-hydroxyethyl]-1,3-dihydro-2H-indol-2-one,
1-(2,1,3-benzoxadiazol-5-ylmethyl)-3,3-difluoro-4-[(1S)-1-hydroxyethyl]-1,3-dihydro-2H-indol-2-one,
3,3-difluoro-1-(3-fluorobenzyl)-4-[(1S)-1-hydroxyethyl]-1,3-dihydro-2H-indol-2-one,
6-{[4-(2,2-difluoro-1-hydroxyethyl)-3,3-difluoro-2-oxo-2,3-dihydro-1H-indol-1-yl]methyl}-3-fluoropyridine-2-carbonitrile,
6-({3,3-difluoro-4-[(1S)-1-hydroxyethyl]-2-oxo-2,3-dihydro-1H-indol-1-yl}methyl)-3-(trifluoromethyl)pyridine-2-carbonitrile,
4-(2,2-difluoro-1-hydroxyethyl)-3,3-difluoro-1-[(6-methoxypyridin-2-yl)methyl]-1,3-dihydro-2H-indol-2-one,
1-[(5,6-dichloropyridin-2-yl)methyl]-3,3-difluoro-4-[(1S)-1-hydroxyethyl]-1,3-dihydro-2H-indol-2-one,
1-[(4-chloro-5-fluoropyridin-2-yl)methyl]-3,3-difluoro-4-[(1S)-1-hydroxyethyl]-1,3-dihydro-2H-indol-2-one,
4-(2,2-difluoro-1-hydroxyethyl)-1-{[2-(difluoromethoxy)pyridin-4-yl]methyl}-3,3-difluoro-1,3-dihydro-2H-indol-2-one,
1-[(6-chloro-5-fluoropyridin-2-yl)methyl]-4-(2,2-difluoro-1-hydroxyethyl)-3,3-difluoro-1,3-dihydro-2H-indol-2-one,
1-[(6-chloropyrazin-2-yl)methyl]-3,3-difluoro-4-[(1S)-1-hydroxyethyl]-1,3-dihydro-2H-indol-2-one,
3,3-difluoro-4-[(1S)-1-hydroxyethyl]-1-[(2-methoxypyridin-4-yl)methyl]-1,3-dihydro-2H-indol-2-one,
1-[(2-chloropyridin-4-yl)methyl]-4-(2,2-difluoro-1-hydroxyethyl)-3,3-difluoro-1,3-dihydro-2H-indol-2-one,
4-({3,3,7-trifluoro-4-[(1S)-1-hydroxyethyl]-2-oxo-2,3-dihydro-1H-indol-1-yl}methyl)pyridine-2-carbonitrile,
1-[(5-chloropyridin-3-yl)methyl]-3,3-difluoro-4-(1-hydroxyethyl)-1,3-dihydro-2H-indol-2-one,
1-[(6-chloropyridin-2-yl)methyl]-3,3-difluoro-4-(1-hydroxyethyl)-1,3-dihydro-2H-indol-2-one,
3-({3,3-difluoro-4-[(1S)-1-hydroxyethyl]-2-oxo-2,3-dihydro-1H-indol-1-yl}methyl)benzonitrile,
4-(2,2-difluoro-1-hydroxyethyl)-3,3-difluoro-1-(3-fluorobenzyl)-1,3 -dihydro-2H-indol-2-one,
1-{[5-chloro-4-(trifluoromethyl)pyridin-2-yl]methyl}-4-(2,2-difluoro-1-hydroxyethyl)-3,3-difluoro-1,3-dihydro-2H-indol-2-one,
1-[(6-chloropyridin-2-yl)methyl]-3,3-difluoro-4-(2,2,2-trifluoro-1-hydroxyethyl)-1,3-dihydro-2H-indol-2-one,
1-[(6-chloropyridin-2-yl)methyl]-3,3-difluoro-4-(2-fluoro-1-hydroxyethyl)-1,3-dihydro-2H-indol-2-one,
3,3-difluoro-4-[(1S)-1-hydroxyethyl]-1-{[6-(trifluoromethyl)pyridin-2-yl]methyl}-1,3-dihydro-2H-indol-2-one,
2-({3,3-difluoro-4-[(1S)-1-hydroxyethyl]-2-oxo-2,3-dihydro-1H-indol-1-yl}methyl)-7-fluoro-3-methylquinazolin-4(3H)-one,
1-[(2-chloropyridin-4-yl)methyl]-3,3-difluoro-4-(1-hydroxyethyl)-1,3-dihydro-2H-indol-2-one,
3,3-difluoro-4-[(1S)-1-hydroxyethyl]-1-(3-methoxybenzyl)-1,3-dihydro-2H-indol-2-one,
1-[(4-chloropyridin-2-yl)methyl]-3,3-difluoro-4-[(1S)-1-hydroxyethyl]-1,3-dihydro-2H-indol-2-one,
4-({3,3-difluoro-4-[(1S)-1-hydroxyethyl]-2-oxo-2,3-dihydro-1H-indol-1-yl}methyl)-3-fluoropyridine-2-carbonitrile,
1-benzyl-3,3-difluoro-4-[(1S)-1-hydroxyethyl]-1,3-dihydro-2H-indol-2-one,
2-chloro-5-({3,3-difluoro-4-[(1S)-1-hydroxyethyl]-2-oxo-2,3-dihydro-1H-indol-1-yl}methyl)pyridine-3-carbonitrile,
1-benzyl-4-(2,2-difluoro-1-hydroxyethyl)-3,3-difluoro-1,3-dihydro-2H-indol-2-one,
1-[(4-chloro-5-fluoropyridin-2-yl)methyl]-4-(2,2-difluoro-1-hydroxyethyl)-3,3-difluoro-1,3-dihydro-2H-indol-2-one,
1-[(4-bromopyridin-2-yl)methyl]-3,3-difluoro-4-[(1S)-1-hydroxyethyl]-1,3-dihydro-2H-indol-2-one,
5-({3,3-difluoro-4-[(1S)-1-hydroxyethyl]-2-oxo-2,3-dihydro-1H-indol-1-yl}methyl)-2-fluoropyridine-3-caxbonitrile,
1-[(2-chloropyridin-4-yl)methyl]-3,3-difluoro-4-(1-hydroxyethyl)-1,3-dihydro-2H-indol-2-one,
1-[(2-cyclopropylpyridin-4-yl)methyl]-3,3-difluoro-4-[(1S)-1-hydroxyethyl]-1,3-dihydro-2H-indol-2-one,
6-({3,3-difluoro-4-[(1S)-1-hydroxyethyl]-2-oxo-2,3-dihydro-1H-indol-1-yl}methyl)pyridine-2-carbonitrile,
1-[(6-chloropyrazin-2-yl)methyl]-4-(2,2-difluoro-1-hydroxyethyl)-3,3-difluoro-1,3-dihydro-2H-indol-2-one,
3,3-difluoro-4-[(1S)-1-hydroxyethyl]-1-{[2-(trifluoromethyl)pyridin-4-yl]methyl}-1,3-dihydro-2H-indol-2-one,
3,3-difluoro-1-[(2-methoxypyridin-4-yl)methyl]-4-[(1R)-2,2,2-trifluoro-1-hydroxyethyl]-1,3-dihydro-2H-indol-2-one,
1-[(6-chloropyridin-2-yl)methyl]-4-(2,2-difluoro-1-hydroxyethyl)-3,3-difluoro-1,3-dihydro-2H-indol-2-one,
3,3-difluoro-4-[(1S)-1-hydroxyethyl]-1-{[6-(trifluoromethyl)pyridin-3-yl]methyl}-1,3-dihydro-2H-indol-2-one,
5-{[4-(2,2-difluoro-1-hydroxyethyl)-3,3-difluoro-2-oxo-2,3-dihydro-1H-indol-1-yl]methyl}-2-fluoropyridine-3 -carbonitrile,
3,3-difluoro-4-[(1S)-1-hydroxyethyl]-1-(thiophen-3-ylmethyl)-1,3-dihydro-2H-indol-2-one,
2-({3,3-difluoro-4-[(1S)-1-hydroxyethyl]-2-oxo-2,3-dihydro-1H-indol-1-yl}methyl)-3-methylquinazolin-4(3H)-one,
3-({3,3-difluoro-4-[(11S)-1-hydroxyethyl]-2-oxo-2,3-dihydro-1H-indol-1-yl}methyl)-2-methylisoquinolin-1 (2H)-one,
3,3-difluoro-4-[(1S)-1-hydroxyethyl]-1-{[5-(trifluoromethyl)pyridin-2-yl]methyl}-1,3-dihydro-2H-indol-2-one,
4-[(2,2-difluoro-1-hydroxyethyl]-3,3-difluoro-1-{[2-(trifluoromethyl)pyridin-4-yl]methyl}-1,3-dihydro-2H-indol-2-one,
1-(1,3-benzoxazol-6-ylmethyl)-3,3-difluoro-4-[(1S)-1-hydroxyethyl]-1,3-dihydro-2H-indol-2-one,
1-(1,3-benzoxazol-2-ylmethyl)-3,3-difluoro-4-[(1S)-1-hydroxyethyl]-1,3-dihydro-2H-indol-2-one,
3,3-difluoro-4-[(1S)-1-hydroxyethyl]-1-(quinoxalin-2-ylmethyl)-1,3-dihydro-2H-indol-2-one,
3,3-difluoro-4-[(1S)-1-hydroxyethyl]-1-[(6-methoxypyridin-3-yl)methyl]-1,3-dihydro-2H-indol-2-one,
3,3-difluoro-4-[(1S)-1-hydroxyethyl]-1-[(3-methylquinoxalin-2-yl)methyl]-1,3-dihydro-2H-indol-2-one,
1-{[2-(difluoromethyl)pyridin-4-yl]methyl}-3,3-difluoro-4-[(1S)-1-hydroxyethyl]-1,3-dihydro-2H-indol-2-one,
1-[(5-chloropyridin-3-yl)methyl]-3,3-difluoro-4-(1-hydroxyethyl)-1,3-dihydro-2H-indol-2-one,
1-[(5-chloro-4-methoxypyridin-2-yl)methyl]-3,3-difluoro-4-[(1S)-1-hydroxyethyl]-1,3-dihydro-2H-indol-2-one,
1-{[3-chloro-5-(trifluoromethyl)pyridin-2-yl)methyl}-3,3-difluoro-4-[(1S)-1-hydroxyethyl]-1,3-dihydro-2H-indol-2-one,
3-({3,3-difluoro-2-oxo-4-[(1R)-2,2,2-trifluoro-1-hydroxyethyl]-2,3-dihydro-1H-indol-1-yl}methyl)quinoxalin-2(1H)-one,
3,3-difluoro-4-[(1S)-1-hydroxyethyl]-1-[(1-methyl-1H-benzimidazol-2-yl)methyl]-1,3-dihydro-2H-indol-2-one,
3-({3,3-difluoro-4-[(1S)-1-hydroxyethyl]-2-oxo-2,3-dihydro-1H-indol-1-yl}methyl)quinoxalin-2(1H)-one,
6-chloro-4-({3,3-difluoro-4-[(1S)-1-hydroxyethyl]-2-oxo-2,3-dihydro-1H-indol-1-yl}methyl)pyridine-2-carbonitrile,
3,3-difluoro-4-[(1S)-1-hydroxyethyl]-1-{[5-(2H-1,2,3-triazol-2-yl)pyridin-3-yl]methyl}-1,3-dihydro-2H-indol-2-one,
1-(1,3-benzothiazol-2-ylmethyl)-3,3-difluoro-4-[(1S)-1-hydroxyethyl]-1,3-dihydro-2H-indol-2-one,
3,3-difluoro-4-[(1S)-1-hydroxyethyl]-1-(quinolin-2-ylmethyl)-1,3-dihydro-2H-indol-2-one,
1-[(5-chloropyridin-3-yl)methyl]-3,3-difluoro-4-(2,2,2-trifluoro-1-hydroxyethyl)-1,3-dihydro-2H-indol-2-one,
1-[(2-chloropyridin-4-yl)methyl]-3,3-difluoro-4-[(1R)-2,2,2-trifluoro-1-hydroxyethyl]-1,3-dihydro-2H-indol-2-one,
4-({3,3-difluoro-4-[(1S)-1-hydroxyethyl]-2-oxo-2,3-dihydro-1H-indol-1-yl}methyl)pyridine-2-carbonitrile,
4-(2,2-difluoro-1-hydroxyethyl)-3,3-difluoro-1-{[5-(2H-1,2,3-triazol-2-yl)pyridin-3-yl]methyl}-1,3-dihydro-2H-indol-2-one,
3,3-difluoro-4-[(1S)-1-hydroxyethyl]-1-(quinolin-3-ylmethyl)-1,3-dihydro-2H-indol-2-one,
3,3-difluoro-4-[(1S)-1-hydroxyethyl]-1-{[5-(trifluoromethyl)pyridin-3-yl]methyl}-1,3-dihydro-2H-indol-2-one,
1-[(3-bromo-5-fluoropyridin-2-yl)methyl]-3,3-difluoro-4-[(1S)-1-hydroxyethyl]-1,3-dihydro-2H-indol-2-one,
1-[(6-chloropyrazin-2-yl)methyl]-3,3-difluoro-4-[(1R)-2,2,2-trifluoro-1-hydroxyethyl]-1,3-dihydro-2H-indol-2-one,
2-({3,3-difluoro-4-[(1S)-1-hydroxyethyl]-2-oxo-2,3-dihydro-1H-indol-1-yl}methyl)pyridine-3-carbonitrile,
3-({3,3-difluoro-4-[(1S)-1-hydroxyethyl]-2-oxo-2,3-dihydro-1H-indol-1-yl}methyl)-1-methylquinoxalin-2(1H)-one,
6-{[4-(2,2-difluoro-1-hydroxyethyl)-3,3-difluoro-2-oxo-2,3-dihydro-1H-indol-1-yl]methyl}pyridine-2-carbonitrile,
5-chloro-4-({3,3-difluoro-4-[(1S)-1-hydroxyethyl]-2-oxo-2,3-dihydro-1H-indol-1-yl}methyl)pyridine-2-carbonitrile,
1-{[2-(difluoromethoxy)pyridin-4-yl]methyl}-3,3-difluoro-4-[(1S)-1-hydroxyethyl]-1,3-dihydro-2H-indol-2-one,
1-[(4-chloropyridin-2-yl)methyl]-3,3-difluoro-4-[(1R)-2,2,2-trifluoro-1-hydroxyethyl]-1,3-dihydro-2H-indol-2-one,
2-({3,3-difluoro-2-oxo-4-[(1R)-2,2,2-trifluoro-1-hydroxyethyl]-2,3-dihydro-1H-indol-1-yl}methyl)-3-methylquinazolin-4(3H)-one,
1-{[6-(difluoromethyl)pyridin-2-yl]methyl}-3,3-difluoro-4-[(1S)-1-hydroxyethyl]-1,3-dihydro-2H-indol-2-one,
1-[(5-chloropyridin-2-yl)methyl]-3,3-difluoro-4-[(1S)-1-hydroxyethyl]-1,3-dihydro-2H-indol-2-one,
2-{[4-(2,2-difluoro-1-hydroxyethyl)-3,3-difluoro-2-oxo-2,3-dihydro-1H-indol-1-yl]methyl}-7-fluoro-3-methylquinazolin-4(3H)-one,
3,3-difluoro-4-[(1S)-1-hydroxyethyl]-1-{[2-(1H-1,2,4-triazol-1-yl)pyridin-4-yl]methyl}-1,3-dihydro-2H-indol-2-one,
1-{[2-(difluoromethyl)pyridin-4-yl]methyl}-3,3-difluoro-4-[(1R)-2,2,2-trifluoro-1-hydroxyethyl]-1,3-dihydro-2H-indol-2-one,
5-chloro-1-[(5-chloropyridin-3-yl)methyl]-3,3-difluoro-4-[(1S)-1-hydroxyethyl]-1,3-dihydro-2H-indol-2-one,
1-[(5-chloropyridin-3-yl)methyl]-4-(2,2-difluoro-1-hydroxyethyl)-3,3,7-trifluoro-1,3-dihydro-2H-indol-2-one,
1-[(5-chloropyridin-3-yl)methyl]-4-(2,2-difluoro-1-hydroxyethyl)-3,3,7-trifluoro-1,3-dihydro-2H-indol-2-one,
4-(2,2-difluoro-1-hydroxyethyl)-3,3-difluoro-1-(quinolin-3-ylmethyl)-1,3-dihydro-2H-indol-2-one,
4-(2,2-difluoro-1-hydroxyethyl)-3,3-difluoro-1-[(2-methoxypyridin-4-yl)methyl]-1,3-dihydro-2H-indol-2-one,
3-({3,3-difluoro-4-[(1S)-1-hydroxyethyl]-2-oxo-2,3-dihydro-1H-indol-1-yl}methyl)isoquinolin-1 (2H)-one,
3,3-difluoro-1-[(2-fluoropyridin-4-yl)methyl]-4-[(1S)-1-hydroxyethyl]-1,3-dihydro-2H-indol-2-one,
1-[(6-bromopyridin-3-yl)methyl)-3,3-difluoro-4-[(1S)-1-hydroxyethyl)-1,3-dihydro-2H-indol-2-one,
1-[(6-chloropyridin-2-yl)methyl]-3,3-difluoro-4-(2,2,2-trifluoro-1-hydroxyethyl)-1,3-dihydro-2H-indol-2-one,
3,3-difluoro-4-[(1S)-1-hydroxyethyl)-1-{[4-(trifluoromethyl)pyridin-2-yl]methyl}-1,3-dihydro-2H-indol-2-one,
3,3-difluoro-1-[(6-fluoropyridin-3-yl)methyl]-4-[(1S)-1-hydroxyethyl)-1,3-dihydro-2H-indol-2-one,
4-(2,2-difluoro-1-hydroxyethyl)-3,3-difluoro-1-{[4-(trifluoromethyl)pyridin-2-yl]methyl}-1,3-dihydro-2H-indol-2-one,
1-[(2-cyclopropylpyridin-4-yl)methyl]-4-(2,2-difluoro-1-hydroxyethyl)-3,3-difluoro-1,3-dihydro-2H-indol-2-one,
2-{[3,3-difluoro-4-(1-hydroxyethyl)-2-oxo-2,3-dihydro-1H-indol-1-yl]methyl}-3-methylquinazolin-4(3H)-one,
3,3-difluoro-4-[(1S)-1-hydroxyethyl]-1-[(2-methyl-2H-indazol-3-yl)methyl]-1,3-dihydro-2H-indol-2-one,
3,3-difluoro-1-[(5-fluoro-6-methoxypyridin-2-yl)methyl]-4-[(1S)-1-hydroxyethyl]-1,3-dihydro-2H-indol-2-one,
4-(2,2-difluoro-1-hydroxyethyl)-3,3-difluoro-1-{[5-(trifluoromethyl)pyridin-2-yl]methyl}-1,3-dihydro-2H-indol-2-one,
3,3-difluoro-4-[(1S)-1-hydroxyethyl]-1-{[2-methoxy-6-(trifluoromethyl)pyridin-3-yl]methyl}-1,3-dihydro-2H-indol-2-one,
1-[(5-chloropyridin-3-yl)methyl]-4-(2,2-difluoro-1-hydroxyethyl)-3,3-difluoro-1,3-dihydro-2H-indol-2-one,
1-[(5-chloropyridin-3-yl)methyl]-4-(2,2-difluoro-1-hydroxyethyl)-3,3,5-trifluoro-1,3-dihydro-2H-indol-2-one,
1-[(3,5-dichloropyridin-2-yl)methyl]-3,3-difluoro-4-[(1S)-1-hydroxyethyl]-1,3-dihydro-2H-indol-2-one,
3-({3,3-difluoro-2-oxo-4-[(1R)-2,2,2-trifluoro-1-hydroxyethyl]-2,3-dihydro-1H-indol-1-yl}methyl)isoquinolin-1(2H)-one,
1-[(5-chloro-6-methoxypyridin-2-yl)methyl]-3,3-difluoro-4-[(1S)-1-hydroxyethyl]-1,3-dihydro-2H-indol-2-one,
1-[(3-chloropyridin-2-yl)methyl]-3,3-difluoro-4-[(1S)-1-hydroxyethyl]-1,3-dihydro-2H-indol-2-one,
6-{[3,3-difluoro-2-oxo-4-(2,2,2-trifluoro-1-hydroxyethyl)-2,3-dihydro-1H-indol-1-yl]methyl}pyridine-2-carbonitrile,
3,3-difluoro-1-[(6-fluoro-5-methoxypyridin-3-yl)methyl]-4-[(1S)-1-hydroxyethyl]-1,3-dihydro-2H-indol-2-one.

8. A pharmaceutical composition comprising, as an active ingredient, the compound or pharmaceutically acceptable salt thereof according to any one of claims 1 to 7.

9. An agent for preventing or treating diseases of schizophrenia, Alzheimer's disease, cognitive impairment, dementia, anxiety disorders, depression, drug dependence, spasm, tremor, pain, Parkinson's disease, attention deficit hyperactivity disorder, bipolar disorder, eating disorder, or sleep disorders, which comprises, as an active ingredient, the compound or pharmaceutically acceptable salt thereof according to any one of claims 1 to 7.
